# EUROPEAN PATENT APPLICATION

(11) **EP 1 679 069 A1**
(43) Date of publication of application: **12.07.2006**
(21) Application number: 04792910.4
(22) Date of filing: 19.10.2004
(51) Int. Cl.: A61K 31/275

(54) **NOVEL PIPERIDINE DERIVATIVE**

(30) Priority: 21.10.2003 JP 2003361256
(71) Applicant: Dainippon Sumitomo Pharma Co., Ltd., Osaka 541-8524 (JP)
(72) Inventor: BAN, H., Dainippon Sumitomo Pharma. Co., Ltd, Suita-shi, Osaka 564-0053 (JP); OHNUMA, S., Sumitomo Pharma. Co., ltd., Osaka-shi, Osaka 553-0001 (JP); TSUBOYA, N., Dainippon Sumitomo Pharma.Co., Ltd, Suita-shi, Osaka 564-0053 (JP); ASANO, S., Dainippon Sumitomo Pharma.Co.,Ltd, Suita-shi, osaka 564-0053 (JP)
(74) Representative: Vossius & Partner
(86) International application number: PCT/JP2004/015773
(87) International publication number: WO 2005/037269

(57) **Abstract**

The invention provides a compound of the following formula (1): 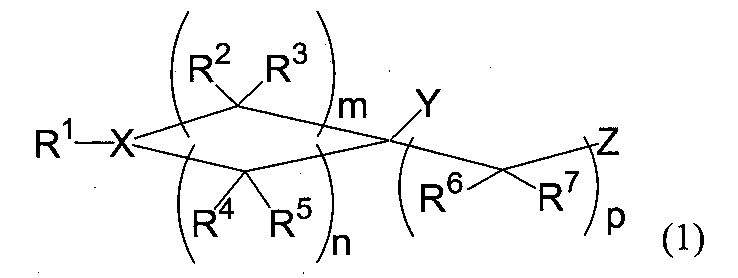 wherein m, n, and p are independently an integer of 0 - 4, provided 3 ≤ m + n ≤ 8; X is nitrogen atom or a group of the formula: C-R¹⁵; Y is a substituted or unsubstituted aromatic group, etc.; R¹⁵, R¹, R², R³, R⁴ , R⁵, R⁶ and R⁷ are hydrogen atom, a substituted or unsubstituted alkyl group, etc.; and Z is hydrogen atom, cyano group, etc.,
or a prodrug thereof, or a pharmaceutically acceptable salt thereof, which exhibits an action for enhancing LDL receptor expression, and is useful as a medicament for treating hyperlipidemia, atherosclerosis, etc.

## Description

### TECHNICAL FIELD

The invention relates to medicaments for enhancing low density lipoprotein (hereinafter, optionally abbreviated to "LDL") receptor expression.

### BACKGROUND ART

LDL receptors in hepatocyte play an important role that controls the plasma cholesterol concentration. That is, it has become clear that the inhibition of cholesterol synthesis in hepatocyte by a 3-hydroxy-3-methylglutaryl coenzyme A (HMG-CoA) reductase inhibitor indirectly enhances LDL receptor expression and then plasma cholesterol, especially LDL cholesterol, is reduced following the enhancement of an uptake of LDL from plasma.

A HMG-CoA reductase inhibitor is clinically admired as a medicament for lowering plasma cholesterol. For a patient with familial hypercholesterolemia with marked hypercholesterolemia or a patient with coronary artery disease, however, the inhibitor does not achieve the target low cholesterol level. Accordingly, a new hypolipidemic drug having the effect to sharply lower the plasma LDL concentration for such patients has been desirable.

While the HMG-CoA reductase inhibitor indirectly enhances LDL receptor synthesis via inhibition of the cholesterol synthesis, a medicament for enhancing LDL receptor expression can be expected to reduce the plasma LDL concentration more sharply by enhancing the expression of LDL receptors directly.

Recently, it has been cleared that the LDL receptor is expressed as a receptor protein having a function to uptake LDL after a transcription and a glycosylation and then uptake LDL from plasma into cells (Goldstein & Brown, Nature, 343, p.425, 1990; and Goldstein & Brown, Science, 232, p.34, 1986). Accordingly, a medicament which has cholesterol lowering effect with a new mechanism via enhancing the protein expression of LDL-R may be developed.

4-Cyano-4-(3-methoxyphenyl)-1-phenylpiperidine is known as a piperidine derivative (see e.g. the Journal of Heterocyclic Chemistry, 1983, 20, p.771), but any usage for lowering LDL cholesterol concentration, etc. is not disclosed therein.

### DISCLOSURE OF INVENTION

An object of the present invention is to provide a compound having an action for enhancing LDL receptor protein expression, and is useful for the treatment of hyperlipidemia, especially hypercholesterolemia. Another object of the present invention is to provide a compound which is useful for regulating LDL receptor synthesis, lowering plasma LDL cholesterol level, and preventing and/or treating arteriosclerosis.

The present inventors have intensively studied in order to solve the above-mentioned problems, and have found that a piperidine derivative of the following formula (1), a prodrug thereof, or a pharmaceutically acceptable salt thereof (hereinafter, optionally abbreviated to "the compound(s) of the invention") exhibits a potent action for enhancing LDL receptor expression, and they have accomplished the present invention.

That is, the present invention relates to the following embodiments:
[1] A medicament for enhancing low density lipoprotein receptor expression comprising as an active ingredient a compound of the formula (1): 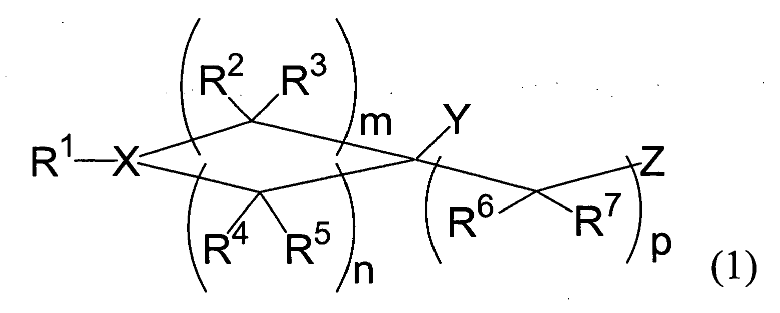 wherein
   m, n, and p are independently an integer of 0 - 4, provided 3 ≤ m + n ≤ 8;
   X is nitrogen atom or a group of the formula: C-R¹⁵;
   R¹⁵ is hydrogen atom, a substituted or unsubstituted alkyl group, a substituted or unsubstituted aromatic group, or a group of the formula: -NR¹⁹R²⁰ wherein
   R¹⁹ and R²⁰ are each independently hydrogen atom; a substituted or unsubstituted lower alkyl group; a substituted or unsubstituted cycloalkyl group; a saturated heterocyclic group comprising 3 - 8 carbon atoms as ring components which includes one -NR²¹- (R²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted lower alkoxycarbonyl group; a substituted or unsubstituted aromatic group; a substituted or unsubstituted aralkyl group; or a substituted or unsubstituted heteroarylalkyl group; or alternatively
   R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group;
   Y is a substituted or unsubstituted alkyl group; a substituted or unsubstituted alkenyl group; a substituted or unsubstituted alkynyl group; a substituted or unsubstituted cycloalkyl group; a substituted or unsubstituted aromatic group; or a group of the formula: - C(=O)R⁸ wherein R⁸ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
   R¹ is hydrogen atom; a substituted or unsubstituted alkyl group; a substituted or unsubstituted alkenyl group; a substituted or unsubstituted alkynyl group; a substituted or unsubstituted cycloalkyl group; a saturated heterocyclic group comprising 3 - 8 carbon atoms as ring components which includes one -NR²³- (R²³ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted aromatic group; or a group of the formula: - C(=O)R¹⁴ wherein R¹⁴ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
   R², R³, R⁴, R⁵, R⁶, and R⁷ are the same or different and are selected from the group consisted of hydrogen atom, hydroxyl group, a substituted or unsubstituted alkyl group, a substituted or unsubstituted alkoxy group, a substituted or unsubstituted alkoxycarbonyl group, a substituted or unsubstituted aralkyl group, a substituted or unsubstituted heteroarylalkyl group, a substituted or unsubstituted aralkyloxy group, and a substituted or unsubstituted heteroarylalkyloxy group; and when each of R², R³, R⁴, R⁵, R⁶, and/or R⁷ exists plurally, each thereof is independently selected from the aforementioned group; alternatively
      one or plural combinations of R² and R³, R⁴ and R⁵, and R⁶ and R⁷ may combine to form oxo group; alternatively
   R² and R⁴ may combine to form an alkylene group; alternatively
      any two of the carbon atoms substituted with R² and R³, or R⁴ and R⁵ may combine to form double bond when the two carbons are located adjacently; and
   Z is hydrogen atom, hydroxyl group, carboxy group, cyano group, phthalimide group, halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aromatic group, a substituted or unsubstituted lower alkoxycarbonyl group, a substituted or unsubstituted carbamoyl group, a substituted or unsubstituted benzyloxycarbonyl group, a substituted or unsubstituted aralkyloxy group, a substituted or unsubstituted heteroarylalkyloxy group, a substituted or unsubstituted aryloxy group, a substituted or unsubstituted heteroaryloxy group, a substituted or unsubstituted lower alkoxy group, a substituted or unsubstituted lower alkanoyloxy group, a substituted or unsubstituted lower alkylthio group, a substituted or unsubstituted lower alkylsulfinyl group, a substituted or unsubstituted lower alkylsulfonyl group, a substituted or unsubstituted benzenesulfonyloxy group, a substituted or unsubstituted lower alkoxycarbonylamino group, or a group of the formula: -NR⁹R¹⁰ wherein
   R⁹ and R¹⁰ are each independently hydrogen atom, a substituted or unsubstituted lower alkyl group, a substituted or unsubstituted cycloalkyl group, a substituted or unsubstituted lower alkoxycarbonyl group, a substituted or unsubstituted aromatic group, a substituted or unsubstituted acyl group, a substituted or unsubstituted aralkyl group, or a substituted or unsubstituted heteroarylalkyl group; or alternatively
   R⁹ and R¹⁰ may combine together with the nitrogen atom bound with R⁹ and R¹⁰ to form a saturated cyclic amino group comprising 3 - 8 carbon atoms as ring components, which may further include one -NR¹¹- (R¹¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group,
      or a prodrug thereof, or a pharmaceutically acceptable salt thereof.
[2] The medicament for enhancing low density lipoprotein receptor expression according to [1] for treating hyperlipidemia or arteriosclerosis.
[3] A compound of the formula (1'): 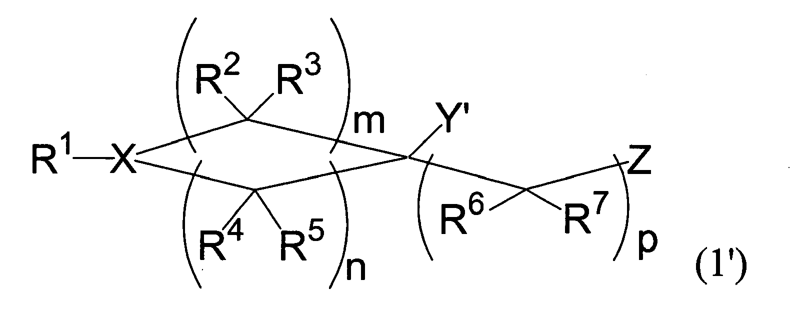 wherein
   m, n, and p are independently an integer of 0 - 4, provided 3 ≤ m + n ≤ 8;
   X is nitrogen atom or a group of the formula: C-R¹⁵;
   R¹⁵ is hydrogen atom, a substituted or unsubstituted alkyl group, a substituted or unsubstituted aromatic group, or a group of the formula: -NR¹⁹R²⁰ wherein
   R¹⁹ and R²⁰ are each independently hydrogen atom; a substituted or unsubstituted lower alkyl group; a substituted or unsubstituted cycloalkyl group; a saturated heterocyclic group comprising 3 - 8 carbon atoms as ring components which includes one -NR²¹- (R ²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted lower alkoxycarbonyl group; a substituted or unsubstituted aromatic group, a substituted or unsubstituted aralkyl group; or a substituted or unsubstituted heteroarylalkyl group; or alternatively
   R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group;
   Y' is a substituted or unsubstituted cycloalkyl group; a substituted or unsubstituted aromatic group; or a group of the formula: -C(=O)R^{8a} wherein R^{8a} is a substituted or unsubstituted cycloalkyl group, or a substituted or unsubstituted aromatic group;
   R¹ is hydrogen atom; a substituted or unsubstituted alkyl group; a substituted or unsubstituted alkenyl group; a substituted or unsubstituted alkynyl group, a substituted or unsubstituted cycloalkyl group; a saturated heterocyclic group comprising 3 - 8 carbon atoms as ring components which includes one -NR²³- (R²³ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted aromatic group; or a group of the formula: - C(=O)R¹⁴ wherein R¹⁴ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
   R², R³, R⁴, R⁵, R⁶, and R⁷ are the same or different and are selected from the group consisted of hydrogen atom, hydroxyl group, a substituted or unsubstituted alkyl group, a substituted or unsubstituted alkoxy group, a substituted or unsubstituted alkoxycarbonyl group, a substituted or unsubstituted aralkyl group, a substituted or unsubstituted heteroarylalkyl group, a substituted or unsubstituted aralkyloxy group, or a substituted or unsubstituted heteroarylalkyloxy group; and when each of R², R³, R⁴, R⁵, R⁶, and/or R⁷ exists plurally, each thereof is independently selected from the aforementioned group; alternatively
      one or plural combinations of R² and R³, R⁴ and R⁵, and R⁶ and R⁷ may combine to form oxo group; alternatively
   R² and R⁴ may combine to form an alkylene group; alternatively
      any two of the carbon atoms substituted with R² and R³, or R⁴ and R⁵ may combine to form double bond when the two carbons are located adjacently; and
   Z is hydrogen atom, hydroxyl group, carboxy group, cyano group, phthalimide group, halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aromatic group, a substituted or unsubstituted lower alkoxycarbonyl group, a substituted or unsubstituted carbamoyl group, a substituted or unsubstituted benzyloxycarbonyl group, a substituted or unsubstituted aralkyloxy group, a substituted or unsubstituted heteroarylalkyloxy group, a substituted or unsubstituted aryloxy group, a substituted or unsubstituted heteroaryloxy group, a substituted or unsubstituted lower alkoxy group, a substituted or unsubstituted lower alkanoyloxy group, a substituted or unsubstituted lower alkylthio group, a substituted or unsubstituted lower alkylsulfinyl group, a substituted or unsubstituted lower alkylsulfonyl group, a substituted or unsubstituted benzenesulfonyloxy group, a substituted or unsubstituted lower alkoxycarbonylamino group, or a group of the formula: -NR⁹R¹⁰ wherein
   R⁹ and R¹⁰ are each independently hydrogen atom, a substituted or unsubstituted lower alkyl group, a substituted or unsubstituted cycloalkyl group, a substituted or unsubstituted lower alkoxycarbonyl group, a substituted or unsubstituted aromatic group, a substituted or unsubstituted acyl group, a substituted or unsubstituted aralkyl group, or a substituted or unsubstituted heteroarylalkyl group; or alternatively
   R⁹ and R¹⁰ may combine together with the nitrogen atom bound with R⁹ and R¹⁰ to form a saturated cyclic amino group comprising 3 - 8 carbon atoms as ring components, which may further include one -NR¹¹- (R¹¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group; and
      provided that Z is not cyano group when both Y' and R¹ are unsubstituted phenyl group,
      or a prodrug thereof, or a pharmaceutically acceptable salt thereof.
[4] The compound according to [3] wherein
   X is nitrogen atom, and R² and R⁴ combine to form an alkylene; or alternatively
   X is a group of the formula: C-R¹⁵,
   or a prodrug thereof, or a pharmaceutically acceptable salt thereof.
[5] The compound according to any one of [3] and [4] wherein Y' is a substituted or unsubstituted aromatic group,
   or a prodrug thereof, or a pharmaceutically acceptable salt thereof.
[6] The compound according to [5] wherein R¹ is a substituted or unsubstituted aromatic group,
   or a prodrug thereof, or a pharmaceutically acceptable salt thereof.
[7] The compound according to [6] wherein Y' is a substituted or unsubstituted phenyl group, or a substituted or unsubstituted pyridyl group,
   or a prodrug thereof, or a pharmaceutically acceptable salt thereof.
[8] The compound according to [7] wherein
   R¹ is phenyl group, pyridyl group, pyrimidinyl group, benzoxazolyl group, or benzothiazolyl group, which may be optionally substituted with one or more substituents,
   or a prodrug thereof, or a pharmaceutically acceptable salt thereof.
[9] The compound according to [8] wherein
   R¹ is a substituted phenyl group or a substituted pyridyl group, wherein the substituents on the phenyl group or pyridyl group are the same or different and are selected from one or more of hydroxyl group or a lower alkoxy group, or a prodrug thereof, or a pharmaceutically acceptable salt thereof.
[10] The compound according to any one of [3] - [5] wherein
   X is the formula: C-R¹⁵, and
   R¹⁵ is a group of the formula: -NR¹⁹R²⁰,
   or a prodrug thereof, or a pharmaceutically acceptable salt thereof.
[11] The compound according to [10] wherein in the formula: -NR¹⁹R²⁰
   R¹⁹ is hydrogen atom, and
   R²⁰ is a substituted or unsubstituted aromatic group, a substituted or unsubstituted aralkyl group, or a substituted or unsubstituted heteroarylalkyl group, or alternatively
R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) as a ring component and may optionally have one or more substituents on the carbon atoms of the saturated cyclic amino group,
   or a prodrug thereof, or a pharmaceutically acceptable salt thereof.
[12] The compound according to [10] wherein
   R¹⁵ is a group of the formula: -NR¹⁹R²⁰,
   R¹⁹ is hydrogen atom,
   R²⁰ is a substituted or unsubstituted aromatic group, a substituted or unsubstituted aralkyl group, or a substituted or unsubstituted heteroarylalkyl group, and
   the configuration between R¹⁵ and Y' is trans, or a prodrug thereof, or a pharmaceutically acceptable salt thereof.
[13] The compound according to [12] wherein R²⁰ is a substituted or unsubstituted aralkyl group, or a substituted or unsubstituted heteroarylalkyl group,
   or a prodrug thereof, or a pharmaceutically acceptable salt thereof.
[14] The compound according to [12] wherein R²⁰ is a substituted benzyl group wherein the substituent is sulfamoyl group,
   or a prodrug thereof, or a pharmaceutically acceptable salt thereof.
[15] The compound according to [10] wherein
   R¹⁵ is a group of the formula: -NR¹⁹R²⁰;
   R¹⁹ is hydrogen atom;
   R²⁰ is a saturated heterocyclic group comprising 3 - 8 carbon atoms as ring components which includes one -NR²¹⁻(R²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; and
   the configuration between R¹⁵ and Y' is trans,
   or a prodrug thereof, or a pharmaceutically acceptable salt thereof.
[16] The compound according to [10] wherein
   R¹⁵ is a group of the formula: -NR¹⁹R²⁰ wherein R¹⁹ and R²⁰ combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) as a ring component and may optionally have one or more substituents on the carbon atoms of the saturated cyclic amino group; and
   the configuration between R¹⁵ and Y' is cis,
   or a prodrug thereof, or a pharmaceutically acceptable salt thereof.
[17] The compound according to any one of [9] - [16] wherein
   every R², R³, R⁴, R⁵, R⁶, and R⁷ is hydrogen atom, or alternatively
   one or plural combinations of R² and R³, R⁴ and R⁵, and R⁶ and R⁷ combine to form oxo group; and the others are all hydrogen atom,
   or a prodrug thereof, or a pharmaceutically acceptable salt thereof.
[18] The compound according to [17] wherein
   every R², R³, R⁴, and R⁵ is hydrogen atom, and
   R⁶ and R⁷ combine to form oxo group, or both R⁶ and R⁷ are hydrogen atom,
   or a prodrug thereof, or a pharmaceutically acceptable salt thereof.
[19] The compound according [18] wherein Z is hydroxyl group, cyano group, a lower alkoxy group or a group of the formula: -NR⁹R¹⁰,
   or a prodrug thereof, or a pharmaceutically acceptable salt thereof.
[20] The compound according to [19] wherein
   Y' is a substituted phenyl group wherein the substituents on the phenyl group are the same or different and are selected from one or more of hydroxyl group or a lower alkoxy group,
   or a prodrug thereof, or a pharmaceutically acceptable salt thereof.
[21] The compound according to any one of [3] - [20]
   wherein Z is cyano group, or a prodrug thereof, or a pharmaceutically acceptable salt thereof.
[22] The compound according to any one of [3] - [21] wherein
   m is 2 or 3,
   n is 2, and
   every R², R³, R⁴, R⁵, R⁶, and R⁷ is hydrogen atom,
   or a prodrug thereof, or a pharmaceutically acceptable salt thereof.
[23] The compound according to any one of [3] - [22] wherein p is 0, or a prodrug thereof, or a pharmaceutically acceptable salt thereof.
[24] A pharmaceutical composition comprising as an active ingredient the compounds set forth in any one of [3] -
[23], or a prodrug thereof, or a pharmaceutically acceptable salt thereof.
[25] A medicament for enhancing low density lipoprotein receptor expression comprising as an active ingredient the compounds set forth in any one of [3] - [23], or a prodrug thereof, or a pharmaceutically acceptable salt thereof.
[26] A hypolipidemic drug or antiarteriosclerotic drug comprising as an active ingredient the compound set forth in any one of [3] - [23], or a prodrug thereof, or a pharmaceutically acceptable salt thereof.
[27] A method for treating hyperlipidemia or arteriosclerosis comprising administering to a pacient in need of the treatment a therapeutically effective dose of the compound set forth in any one of [3] - [23], or a prodrug thereof, or a pharmaceutically acceptable salt thereof.
[28] Use of the compound set forth in any one of [3] - [23], or a prodrug thereof, or a pharmaceutically acceptable salt thereof, for the manufacture of a hypolipidemic drug or antiarteriosclerotic drug.
   The compounds of the invention exhibit an action for enhancing LDL receptor expression and an action for regulating LDL receptor synthesis, and useful for lowering plasma LDL cholesterol level and preventing and treating arteriosclerosis.

### BRIEF DESCRIPTION OF DRAWINGS

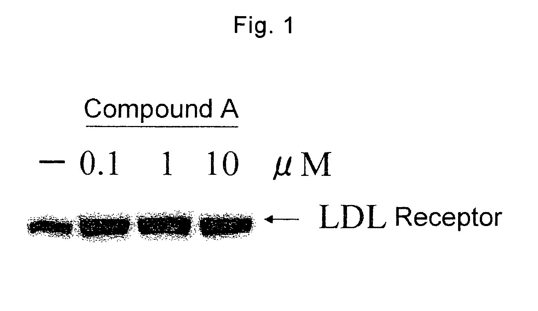

Figure 1 shows the result of immunoblot analysis for LDL receptor protein about the compound of Examples 1-4 of Experiment 1. Each lane shows the result of control, 0.1 µM, 1 µM, and 10 µM (the compound concentration) in the order from the left side. The arrow indicates the location of LDL receptor protein.

### BEST MODE FOR CARRYING OUT THE INVENTION

The enhancement of LDL receptor expression means an enhancement of expression amount of LDL receptor protein, and also refers to an increase of LDL receptor expression or an upregulation of LDL receptor expression.

Each group in the present invention is explained below. Unless defined otherwise, the definition for each group should be also applied to cases wherein said group is a part of another substituent.

The "alkyl group" may include, for example, a straight chain or branched chain C₁₋₁₅ alkyl group, and examples thereof are methyl, ethyl, propyl, 2-propyl, butyl, 2-butyl, 2-methylpropyl, 1,1-dimethylethyl, pentyl, 3-pentyl, 3-methylbutyl, hexyl, 3-hexyl, 4-methylpentyl, 4-heptyl, octyl, 4-octyl, decyl and the like. Preferable one is a straight chain or branched chain C₁₋₆ alkyl group.

The "alkenyl group" may include, for example, a straight chain or branched chain C₂₋₁₅ alkenyl group, and examples thereof are vinyl, allyl, 2-propenyl, 2-methyl-2-propenyl, 2-butenyl, 3-butenyl, 3-methyl-2-butenyl, 4-pentenyl, 1-hexenyl, 3-hexenyl, 3-ethyl-2-pentenyl, 4-ethyl-3-hexenyl and the like. Preferable one is a straight chain or branched chain C₂₋₆ alkenyl group.

The "alkynyl group" may include, for example, a straight chain or branched chain C₂₋₁₅ alkynyl group, and examples thereof are ethynyl, 2-propynyl, 3-butynyl, 4-pentynyl, 3-hexynyl, 5-methyl-2-hexynyl, 6-methyl-4-heptynyl and the like. Preferable one is a straight chain or branched chain C₂₋₆ alkynyl group.

The "alkoxy group" may include, for example, the above-mentioned alkyl group linked to an oxygen atom.

The "alkoxycarbonyl group" may include, for example, the above-mentioned alkyl group linked to the oxygen atom side of the formula: -C(=O)O-.

The substituted alkyl group, the substituted alkenyl group, the substituted alkynyl group, the substituted alkoxy group, the substituted alkoxycarbonyl group, the substituted aralkyl group, the substituted heteroarylalkyl group, the substituted alkanoyl group, the substituted aralkyloxy group, and the substituted heteroarylalkyloxy group may have one or more substituents which are same or different, and the substituents are, for example, a substituted or unsubstituted aromatic group, a substituted or unsubstituted cycloalkyl group, halogen atom, cyano group, hydroxyl group, a lower alkoxy group, a lower alkanoyloxy group, amino group, a mono-lower alkylamino group, a di-lower alkylamino group, carbamoyl group, a lower alkylaminocarbonyl group, a di-lower alkylaminocarbonyl group, a lower alkoxycarbonylamino group, carboxy group, a lower alkoxycarbonyl group, a lower alkylthio group, a lower alkylsulfinyl group, a lower alkylsulfonyl group, a lower alkanoylamino group, a lower alkylsulfonamide group and the like.

More specifically, the substituted alkyl group is, for example, trifluoromethyl group, 2,2,2-trifluoroethyl group, methoxymethyl group, etc.

The halogen atom is, for example, fluorine, chlorine, bromine and iodine. The preferable halogen atom is fluorine atom.

The cycloalkyl group may include, for example, a C₃ - C₈ cycloalkyl group, such as cyclopropyl, cyclobutyl, cyclopentyl, cyclohexyl, cycloheptyl, and cyclooctyl.

The substituted cycloalkyl group, the substituted cycloalkanecarbonyl group, the cycloalkenecarbonyl group, and the substituted saturated heterocycle-carbonyl group may have one or more substituents which are same or different, and the substituents are, for example, a lower alkyl group, halogen atom, cyano group, hydroxyl group, a lower alkoxy group, a lower alkanoyloxy group, amino group, a mono-lower alkylamino group, a di-lower alkylamino group, carbamoyl group, a lower alkylaminocarbonyl group, a di-lower alkylaminocarbonyl group, a lower alkoxycarbonylamino group, carboxyl group, a lower alkoxycarbonyl group, a lower alkylthio group, a lower alkylsulfinyl group, a lower alkylsulfonyl group, a lower alkanoylamino group, a lower alkylsulfonamide group and the like. The preferable halogen atom is fluorine atom.

The term "lower" used herein means that the alkyl moiety of the substituent having "lower" is a lower alkyl group, and the lower alkyl group includes a lower alkyl group having 1 to 6 carbon atoms such as methyl, ethyl, propyl, 2-propyl, butyl, pentyl, hexyl, etc. The lower alkanoyl group and the lower alkanoyl moiety of a substituent partially having the lower alkanoyl group may include a C₁ - C₆ lower alkyl group linked to a carbonyl group.

When a lower alkyl group and a lower alkanoyl group, or a group containing the lower alkyl or alkanoyl group as a partial structure have a substituent(s), the substituent(s) may be one or more substituents which are the same or different and include, for example, halogen atom, cyano group, a lower alkoxy group, a heteroaryl group, etc. The preferable halogen atom is fluorine atom.

The substituted carbamoyl group has one or two substituents which are the same or different, and the substituent includes, for example, a substituted or unsubstituted alkyl group.

The aromatic group includes an aryl group and a heteroaryl group.

The aryl group and the aryl moiety of the aryloxy group may include, for example, an aryl group having 13 or less carbon atoms such as phenyl group, naphthyl group and fluorenyl group. The preferable aryl moiety is an aryl group having 10 or less carbon atoms such as phenyl group and naphthyl group.

The heteroaryl group and the heteroaryl moiety of the heteroaryloxy group include, for example, a 5- or 6-membered mono-cyclic group having 1 to 3 nitrogen atoms; a 5- or 6-membered mono-cyclic group having 1 to 2 nitrogen atoms, and one oxygen or sulfur atom; a 5-membered mono-cyclic group having one oxygen or sulfur atom; a bicyclic group formed by condensing a 6-membered ring and a 5- or 6-membered ring and having 1 to 4 nitrogen atoms and optional one oxygen or sulfur atom; a bicyclic group formed by condensing a 5-membered ring and a 6-membered ring and having one oxygen or sulfur atom; a bicyclic group formed by condensing a 5-membered ring and another 5-membered ring and having 1 to 2 nitrogen atoms and optional one oxygen or sulfur atom; etc., in more detail, 2-pyridyl, 3-pyridyl, 4-pyridyl, 2-pyrimidinyl, 4-pyrimidinyl, 5-pyrimidinyl, 2-thienyl, 3-thienyl, 3-oxadiazolyl, 1-imidazolyl, 2-imidazolyl, 4-imidazolyl, 2-thiazolyl, 3-isothiazolyl, 2-oxazolyl, 3-isooxazolyl, 2-furyl, 3-furyl, 2-pyrrolyl, 3-pyrrolyl, 2-quinolyl, 4-quinolyl, 8-quinolyl, 2-quinazolinyl, 8-purinyl, 1,3-benzoxazol-2-yl, 2-benzothiophenyl, 5-imidazo[2,1-b]thiazolyl, 1-triazolyl, 1-tetrazolyl, 3-indolyl, 3-pyrazolyl, 4- pyrazolyl, 3-pyrazolon-4-yl, 2-benzofuranyl, 5-dihydrobenzofuranyl, etc.

The aralkyl group and the aralkyl moiety of the aralkyloxy group include an alkyl group substituted with the above-mentioned aryl group, and the preferable alkyl moiety is methyl or ethyl.

The heteroarylalkyl group and the heteroarylalkyl moiety of the heteroarylalkyloxy group include an alkyl group substituted with the above-mentioned heteroaryl group, and the preferable alkyl moiety is methyl.

The aryl moiety, heteroaryl moiety or hetero aromatic moiety of the substituted aromatic group, the substituted benzyloxycarbonyl group, the substituted aryloxy group, the substituted heteroaryloxy group, the substituted benzenesulfonyl group, the substituted benzenesulfonyloxy group, the substituted aralkyl group, the substituted heteroarylalkyl group, the substituted aroyl group, the substituted heteroaromatic acyl group, the substituted aralkyloxy group, the substituted heteroarylalkyloxy group, the substituted phenyl group, the substituted pyridyl group, the substituted pyrimidinyl group, the substituted benzoxazolyl group, and the substituted benzothiazolyl group may have one or more substituents which are same or different, and
the substituents are, for example, halogen atom, cyano group, nitro group, hydroxyl group, methylenedioxy group, a lower alkyl group, a lower alkyl group substituted with one or more halogen atoms (e.g. trifluoromethyl group), an alkenyl group (optionally substituted with carboxy group, phenyl group, etc.), an alkynyl group, an aryl group (optionally substituted with halogen atom, a lower alkyl group, a lower alkoxy group, etc.), a heteroaryl group, a lower alkoxy group, a lower alkoxy group substituted with hydroxyl group, a lower alkoxy group substituted with a lower alkoxy group, a lower alkoxy group substituted with one or more halogen atoms, phenoxy group, benzyloxy group, a lower alkanoyl group, a lower alkanoyloxy group, amino group, a mono-lower alkylamino group, a di-lower alkylamino group (optionally substituted with hydroxyl group), morpholino group, pyrrolidino group, piperazinyl group, arylamino group, diarylamino group, a lower alkoxycarbonylamino group, carbamoyl group, an unsubstituted aminosulfonyl group, a lower alkylaminocarbonyl group, a di-lower alkylaminocarbonyl group, carboxy group, sulfo group, a lower alkoxycarbonyl group, a lower alkylthio group, an arylthio group (optionally substituted with a lower alkyl group), a lower alkylsulfinyl group, a lower alkylsulfonyl group, an arylsulfonyl group, a lower alkanoylamino group, a lower alkylsulfonamide group, an arylsulfonamide group, a lower aminosulfonyl group, a group of the formula: -O-E-A wherein
O is oxygen atom, E is a divalent hydrocarbon comprising 1 - 8 carbon atoms which may optionally have a double bond, A is hydrogen atom, hydroxyl group, carboxyl group, a lower alkoxycarbonyl group, benzyloxycarbonyl group, halogen atom, cyano group, benzyloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamide group, phthalimide group, a cycloalkyl group, an aryl group (optionally substituted with halogen atom, an alkyl group, or a lower alkoxy group, etc.), heteroaryl group (optionally substituted with halogen atom, an alkyl group, or a lower alkoxy group, etc.), or a group of the formula: -NR¹⁶R¹⁷ wherein R¹⁶ and R¹⁷ are each independently hydrogen atom, a substituted or unsubstituted lower alkyl group, a substituted or unsubstituted cycloalkyl group, a substituted or unsubstituted lower alkoxycarbonyl group, an aromatic group (optionally substituted with halogen atom, an alkyl group, or a lower alkoxy group, etc.), an aralkyl group (optionally substituted with halogen atom, an alkyl group, or a lower alkoxy group, etc.) or a heteroarylalkyl group (optionally substituted with halogen atom, an alkyl group, or a lower alkoxy group, etc.), or alternatively R¹⁶ and R¹⁷ combine together with the nitrogen atom bound with R¹⁶ and R¹⁷ to form a saturated cyclic amino group comprising 3 - 8 carbon atoms as ring components which may further include one -NR¹⁸- (R¹⁸ is hydrogen atom, a substituted or unsubstituted lower alkyl group, an aromatic group (optionally substituted with halogen atom, an alkyl group, or a lower alkoxy group, etc.), a substituted or unsubstituted lower alkoxycarbonyl group, an aralkyl group (optionally substituted with halogen atom, an alkyl group, or a lower alkoxy group, etc.) or a heteroarylalkyl group (optionally substituted with halogen atom, an alkyl group, or a lower alkoxy group, etc.)) or one oxygen atom as a ring component and may optionally have one or more substituents on the carbon atoms of the saturated cyclic amino group or a group of the formula: -C(=O)NR¹⁶R¹⁷ wherein R¹⁶ and R¹⁷ are as defined above.

The divalent hydrocarbon comprising 1 - 8 carbon atoms which may optionally have a double bond includes, for example, an alkylene group (e.g., methylene, ethylene, trimethylene, tetramethylene, pentamethylene, hexamethylene, etc.), an alkenylene group (e.g., propenylene, butenylene, etc.), and an alkynylene (e.g., ethynylene, propynylene, butynylene, etc.). These groups may be either a straight chain one or a branched chain one.

The saturated cyclic amino group comprising 3 - 8 carbon atoms as ring components which is formed by combining R¹⁹ and R²⁰, R⁹ and R¹⁰, or R¹⁶ and R¹⁷ together with the nitrogen atom bound with R¹⁹ and R²⁰, R⁹ and R¹⁰, or R¹⁶ and R¹⁷ respectively includes, for example, 1-pyrrolidinyl, 1-piperidino, 1-piperazinyl, morpholino, 1-(4-methyl)piperazinyl, etc. The substituent on the carbon atoms of the saturated cyclic amino group includes halogen atom, a substituted or unsubstituted lower alkyl group, a substituted or unsubstituted lower alkoxy group, an aromatic group (optionally substituted with halogen atom, an alkyl group, a lower alkoxy group, etc.), an aralkyl group (optionally substituted with halogen atom, an alkyl group, or a lower alkoxy group, etc.) or a heteroarylalkyl group (optionally substituted with halogen atom, an alkyl group, or a lower alkoxy group, etc.).

The saturated heterocyclic group comprising 3 - 8 carbon atoms as ring components which includes one -NR²¹⁻or -NR²³-, or one oxygen atom includes, for example, piperidyl, pyrrolidinyl, tetrahydrofuranyl, tetrahydropyranyl, etc. The substituent on the carbon atoms of the saturated heterocyclic group includes halogen atom, a substituted or unsubstituted lower alkyl group, a substituted or unsubstituted lower alkoxy group, an aromatic group (optionally substituted with halogen atom, an alkyl group, a lower alkoxy group, etc.), an aralkyl group (optionally substituted with halogen atom, an alkyl group, or a lower alkoxy group, etc.), a heteroarylalkyl group (optionally substituted with halogen atom, an alkyl group, or a lower alkoxy group, etc.).

The acyl group includes formyl group; a C₂ - C₆ alkanoyl group such as acetyl and propanoyl; a C₄ - C₇ cycloalkanecarbonyl group such cyclopropanecarbonyl, cyclobutanecarbonyl, cyclopentanecarbonyl and cyclohexanecarbonyl; a C₃ - C₆ cycloalkenecarbonyl group such as cyclopentenecarbonyl, cyclohexenecarbonyl; a C₆ - C₁₀ aroyl group such as benzoyl, toluoyl and naphthoyl; a saturated heterocycle-carbonyl group comprising a 5- or 6-membered saturated heterocycle which has 1 to 2 hetero atoms selected from the group consisting of nitrogen atom, oxygen atom and sulfur atom, such as 2-piperidinecarbonyl and 3-morpholinecarbonyl; a hetero aromatic acyl group comprising a 5- or 6-membered hetero aromatic ring which has 1 to 2 hetero atoms selected from the group consisting of nitrogen atom, oxygen atom and sulfur atom, such as furoyl, thenoyl, nicotinoyl and isonicotinoyl.

The substituent of the substituted acyl group includes the substituents exemplified in each above-mentioned definition.

The preferable group for Y is, for example, a substituted phenyl group, and more preferable group for Y is a lower alkoxy-substituted phenyl group.

The preferable group for R¹ is, for example, hydrogen atom, a substituted or unsubstituted phenyl group, benzyl group, pyridyl group and 2,2,2-trifluoroethyl group. More preferable group for R¹ is a substituted phenyl group.

The preferable group for R¹⁵ is, for example, a group of the formula: -NR¹⁹R²⁰, especially the followings are preferable:
(1) R¹⁹ and/or R²⁰ are a substituted or unsubstituted aromatic group, and
(2) R¹⁹ and R²⁰ combine together with the nitrogen atom bound with R¹⁹ and R²⁰ to form a saturated cyclic amino group comprising 3 - 8 carbon atoms as ring components, which further includes one -NR²¹- as a ring component wherein R²¹ is substituted or unsubstituted aromatic group.

When R¹⁵ is the above group, the compound wherein R¹ is hydrogen atom is especially preferable.

The preferable group for Z is, for example, hydroxyl group, cyano group, a lower alkoxy group and a group of the formula: -NR⁹R¹⁰, further more preferably cyano group.

The double bond formed by combining any two of the carbon atoms substituted with R², R³, R⁴, R⁵ and Y when the two carbons are located adjacently can exist single or plurally in the ring of the compound (1). Preferably the double bond exists single therein.

The alkylene includes a C₁ - C₃ alkylene, for example, methylene, ethylene, trimethylene, etc.

The partial structure of the formula: 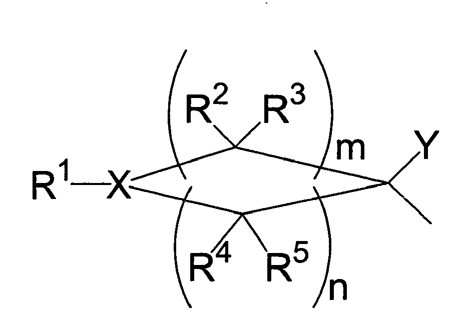 in the case of "R² and R⁴ combine to form an alkylene group" includes, for example, the following formulas:

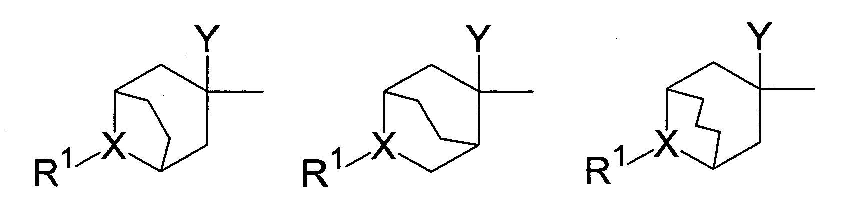

The "prodrug" includes a compound which can be easily hydrolyzed in a living body to reproduce the compound of the formula (1). The "prodrug" includes, for example, when such a compound of the formula (1) has a carboxyl group, then the compound wherein said carboxyl group is replaced by an alkoxycarbonyl group, an alkylthiocarbonyl group, or an alkylaminocarbonyl group; or when a compound of the formula (1) has an amino group, then the compound wherein said amino group is substituted with an alkanoyl group to form an alkanoylamino group, the compound wherein said amino group is substituted with an alkoxycarbonyl group to form an alkoxycarbonylamino group, or the compound wherein said amino group is converted into an acyloxymethylamino group or a hydroxyamine. When a compound of the formula (1) has a hydroxy group, the prodrug thereof includes, for example, a compound wherein said hydroxy group is converted into an acyloxy group by substitution with the above-mentioned acyl group, a compound wherein said hydroxy group is converted into a phosphate ester, or a compound wherein said hydroxy group is converted to an acyloxymethyloxy group. The alkyl moiety of the group used for making a prodrug may be the above-mentioned alkyl groups, and said alkyl group may be optionally substituted, for example, by an alkoxy group having 1 to 6 carbon atoms, etc. Preferable examples include, when taking the compounds wherein a carboxyl group is converted into an alkoxycarbonyl group as an example, a lower (e.g., having 1 to 6 carbon atoms) alkoxycarbonyl group substituted with an alkoxy group such as methoxycarbonyl, methoxymethoxy-carbonyl, ethoxymethoxycarbonyl, 2-methoxyethoxycarbonyl, 2-methoxyethoxymethoxycarbonyl and pivaloyloxymethoxycarbonyl.

The pharmaceutically acceptable salt includes, for example, a salt with a mineral acid such as hydrochloric acid, hydrobromic acid, sulfuric acid, phosphoric acid, etc.; a salt of an organic carboxylic acid such as formic acid, acetic acid, fumaric acid, maleic acid, oxalic acid, citric acid, malic acid, tartaric acid, aspartic acid, glutamic acid, etc.; a salt of a sulphonic acid such as methanesulfonic acid, benzenesufonic acid, p-toluenesulfonic acid, hydroxybenzenesulfonic acid, dihydroxy-benzenesulfonic acid, etc.; and an alkali metal salt such as a sodium salt and a potassium salt; an alkaline earth metal salt such as a calcium salt and a magnesium salt; a ammonium salt; a triethylamine salt, a pyridine salt, a picoline salt; an ethanolamine salt; a dichlorohexylamine salt; an N,N'-dibenzylethylenediamine salt; etc.

The present compounds may have a stereoisomer due to an asymmetric carbon atom thereof. In such cases, the present compounds also include each isomer or a mixture thereof.

The present compounds as mentioned above may be in the form of a free compound, a salt or a hydrate.

The compound of the above formula (1), or a prodrug thereof, or a pharmaceutically acceptable salt thereof may be administered either parenterally or orally. The present compounds can be formulated into liquid preparations such as solutions, emulsions, suspensions, etc., and can be administered in the form of an injection, and if necessary, buffering agents, solubilizers, isotonic agents, etc. may be added thereto. Further, the present compounds can also be administered rectally in the form of a suppository. The present compounds can also be administered orally in the form of a conventional administration form such as tablets (sugar-coated tablets, film-coated tablets), powders, granules, capsules (including soft capsules), syrups, and suspensions. These pharmaceutical preparations can be formulated in a conventional manner by mixing an active ingredient with conventional carriers or diluents, exipients, binding agents, lubricants, disintegration agents, or stabilizers in a conventional manner. If necessary, other additives such as preservatives, antioxidant substances, coloring agents, sweetening agents may be added thereto.

The dosage and the frequency of administration of the present compounds may vary according to the conditions, ages, weights of the patients and the administration form, etc., but when administered in the form of an injection, the present compounds can usually be administered in a dose of 0.1 to 100 mg per day in adult, once a day or divided into several dosage units (e.g., 2 to 4 times). When administered orally, the dosage is in the range of 0.1 to 1000 mg (preferably 1 to 400 mg), once a day or divided into several dosage units (e.g., 2 to 4 times).

The compounds of the present invention may be prepared by the following processes. In each process as mentioned below, even in cases where the use of the protecting group is not specified, when the starting compounds have a reactive functional group such as amino group as a substituent, then if necessary, the reaction may advantageously proceed by protecting these groups and then the protecting groups may be removed after the completion of the reaction or a series of reactions. The protection or deprotection can be carried out by the methods disclosed in literatures (e.g. PROTECTING GROUPS IN ORGANIC SYNTHESIS, 3rd ed., JOHN WILEY & SONS, INC.; New York (1999), or other literatures).
(A) Among the compounds of the present invention, the compound wherein p is 0, Z is cyano group, and X is the formula: C-R¹⁵, and the compound wherein p is 0, Z is cyano group, and X is nitrogen atom, and further neither m nor n is 0 may be prepared as follows. 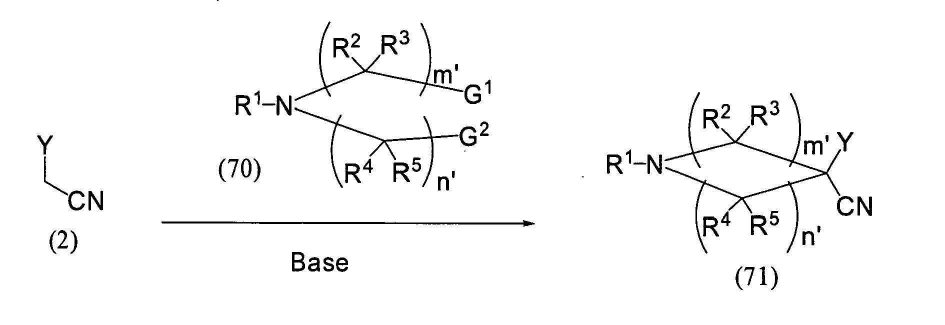 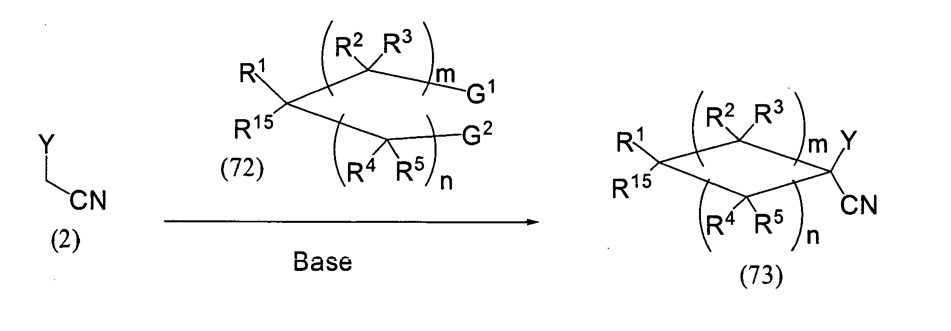 (wherein m' and n' are independently an integer of 1 - 4, provided 3 ≤ m' + n' ≤ 8; G¹ and G² are independently a leaving group such as iodine atom, bromine atom, chlorine atom, p-toluenesulfonyl group, etc.; and Y, m, n, R¹, R² R³, R⁴, R⁵ and R¹⁵ are as defined above)
   The compound of the formula (2) is reacted with the compound of the formula (70) or (72) in an amount of 1 to 3 mole equivalents in a solvent at -30°C to -10°C in the presence of a base such as sodium hydride in an amount of 2 to 5 mole equivalents to give the compound of the formula (71) or (73), respectively.
   The reaction is usually carried out in a solvent, and the solvent may be any solvent which does not disturb the reaction, including, for example, ethers such as diethyl ether, diisopropyl ether, tetrahydrofuran and 1,4-dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.
(B) Alternatively, among the compound of the formula (1), the compound of the formula (12) wherein p is 0, Z is cyano group, and X is nitrogen atom may be prepared, for exaple, as follows. 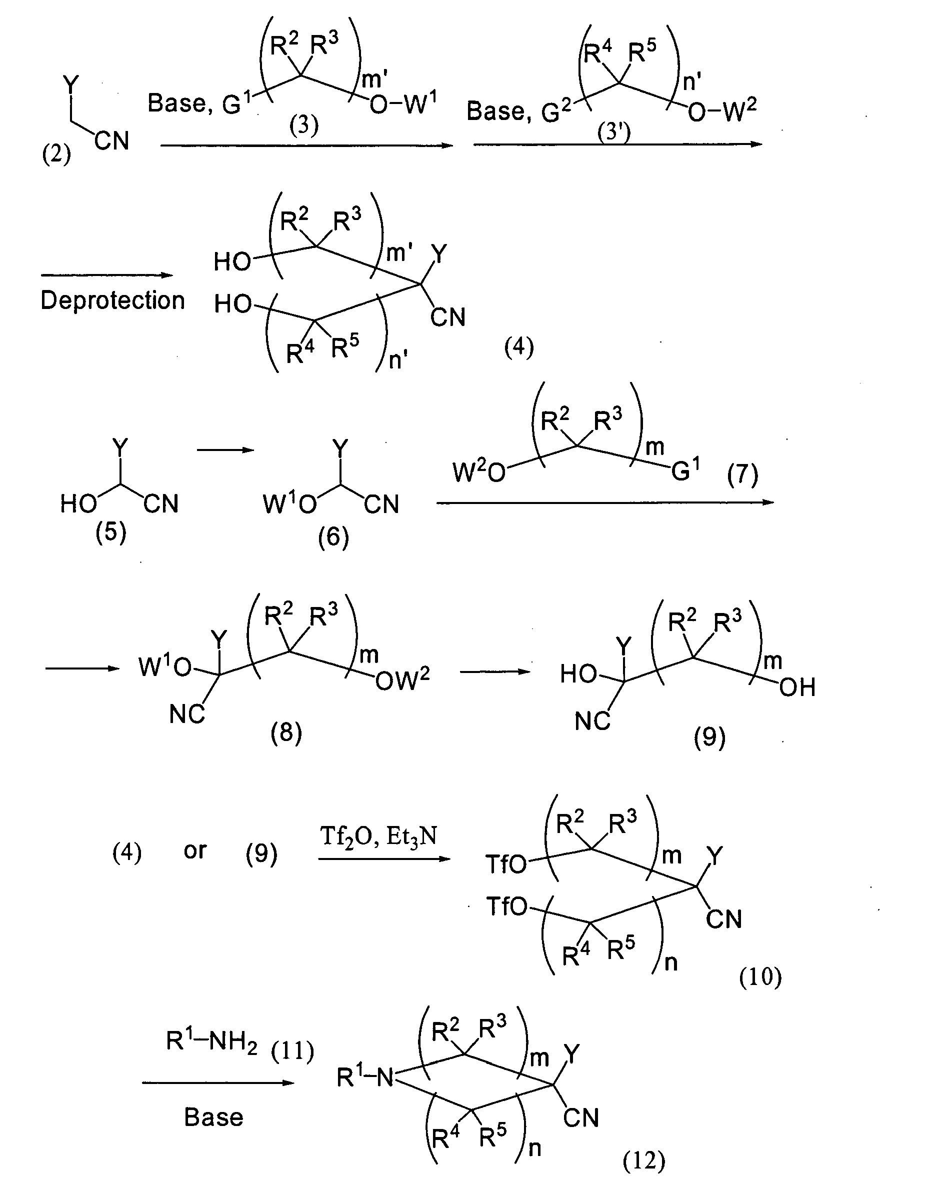 (wherein W¹ and W² are a protecting group of the hydroxyl group; Tf is trifluoromethanesulfonyl group; and G¹, G², m', n', Y, m, n, R¹, R², R³, R⁴ and R⁵ are as defined above)
   For the preparation of the compound of the formula (12), the route for preparing the compound of the formula (4) is employed. For example, the compound of the formula (2) is reacted with the compound of the formula (3) in an amount of 1 to 1.5 mole equivalent in the presence of a base such as sodium hydride in an amount of 1 to 1.5 mole equivalent or more at a temperature of from room temperature to a boiling point of the solvent to be used, and further reacted with the compound of the formula (3') in the presence of a base such as sodium hydride in an amount of 1 to 1.5 mole equivalent or more at a temperature of from room temperature to a boiling point of the solvent to be used, and then the protecting group for the hydroxy group of the resultant is removed to give the compound of the formula (4).
   The reaction is usually carried out in a solvent, and the solvent may be any solvent which does not disturb the reaction, including, for example, ethers such as diethyl ether, diisopropyl ether, tetrahydrofuran and 1,4-dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.
   On the other hand, for the preparation of the compound wherein n is 0, the route for preparing the compound of the formula (9) is employed. For example, the hydroxy group of the compound of the formula (5) is protected to give the compound of the formula (6), which is reacted with the compound of the formula (7) in an amount of 1 to 1.5 mole equivalent in an ether such as tetrahydrofuran (THF) under basic conditions: for example, in the presence of a lithium diisopropylamide in an amount of 1 to 1.5 mole equivalent at a temperature of from -78°C to room temperature, or in the presence of sodium hexamethyldisilazide in an amount of 1 to 1.5 mole equivalent at a temperature of from 0°C to under heating with reflux, followed by removing the protecting groups from the product.
   The protecting group for hydroxy group may be any one which does not disturb the reaction, and the preferable one is a silyl group such as tert-butyldimethylsilyl group.
   The deprotection may be carried out, for example, by the method disclosed in PROTECTIVE GROUPS IN ORGANIC SYNTHESIS, 3rd ed., JOHN WILEY & SONS, INC.: New York (1999), etc.
   The compound of the formula (4) or (9) thus obtained is reacted in a solvent with trifluoromethanesulfonic anhydride in an amount of 2 to 5 mole equivalents in the presence of a base in an amount of 2 to 5 mole equivalents at -30°C to -10°C to give the compound of the formula (10), and without isolating the compound of the formula (10), the compound of the formula (11) in an amount of 1 to 3 mole equivalents and a base such as triethylamine in an amount of 1 to 3 mole equivalents are added to the reaction mixture to give the compound of the formula (12).
   The reaction is usually carried out in a solvent, and the solvent may be any solvent which does not disturb the reaction, including, for example, ethers such as diethyl ether, diisopropyl ether, tetrahydrofuran and 1,4-dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.
(C) Alternatively, the compound wherein p is 0, Z is cyano group, and X is the formula: C-R²² may be also prepared, for example, as follows (wherein R²² is hydrogen atom, a substituted or unsubstituted alkyl group, or a substituted or unsubstituted aromatic group). 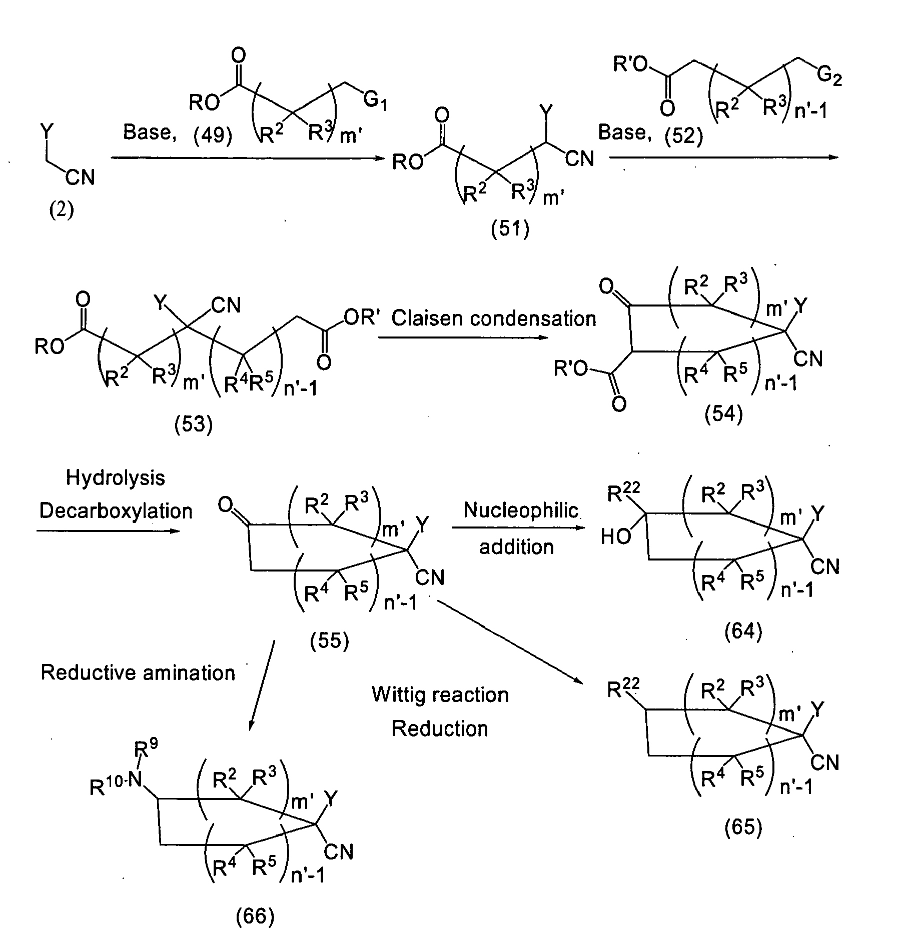 (wherein R and R' are each independently a lower alkyl group; and Y, m', n', R², R³, R⁴, R⁵, R⁹, R¹⁰, R²², G¹ and G² are as defined above)
   The compound of the formula (2) is reacted with the compound of the formula (49) in an amount of 1 to 5 mole equivalents in an ether such as tetrahydrofuran at a temperature of from ice-cooling to 100°C in the presence of a base such as sodium hydride in an amount of 1 to 5 mole equivalents or more to give the compound of the formula (51). Then, in a similar to the above-mentioned condition, the compound of the formula (51) can be reacted with the compound of the formula (52) to give the compound of the formula (53).
   Then, the compound of the formula (53) can be reacted usually in an alcoholic solvent such as ethanol in the presence of a base such as sodium ethoxide at a temperature of from room temperature to a boiling point of the solvent to be used to give the compound of the formula (54).
   And then, the compound of the formula (54) can be reacted usually in an alcoholic solvent such as ethanol in the presence of a base such as sodium hydroxide at a temperature of from room temperature to a boiling point of the solvent to be used give the compound of the formula (55).
   Then, the compound of the formula (55) can be reacted with a nucleophile in an amount of 1.0 - 5.0 mole equivalents usually in an ether such as tetrahydrofuran at 0°C to 120°C, preferably at a temperature of from room temperature to a boiling point of the solvent to be used to give the compound of the formula (64). The nucleophile includes, for example, a Grignard reagent such as R²²MgBr, etc.
   In addition, the compound of the formula (55) is reacted with a nucleophile such as a Wittig reagent in an amount of 1.0 - 5.0 mole equivalents usually in an ether such as tetrahydrofuran at 0°C to 120°C, preferably at a temperature of from room temperature to a boiling point of the solvent to be used, and then the reaction mixture can be hydrogenated at room temperature according to a conventional manner to give the compound of the formula (65).
   In addition, the compound of the formula (55) can be reacted with a reducing agent such as sodium triacetoxyborohydride in an amount of 1.0 - 5.0 mole equivalents and a compound of the formula: HNR⁹R¹⁰ (R⁹ and R¹⁰ are as defined above) in an amount of 1.0 to 5.0 mole equivalents usually in a halogenated hydrocarbon such as dichloroethane at 0°C to 120°C, preferably at a temperature of from room temperature to a boiling point of the solvent to be used to give the compound of the formula (66).
(D) Furthermore, the following compound may be prepared from the compound of the formula (64) obtained as the above process. 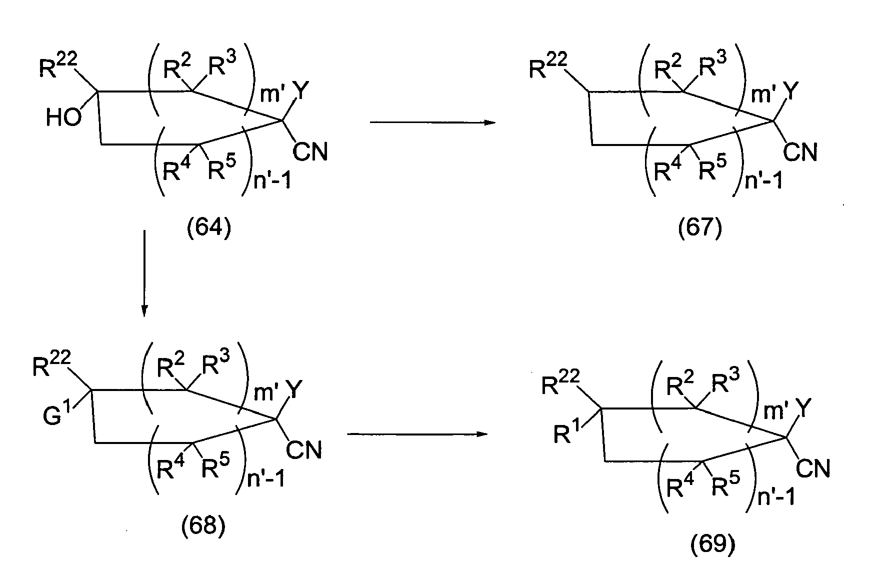 (wherein Y, m', n', R¹, R², R³, R⁴, R⁵, R²² and G¹ are as defined above)
   The compound of the formula (64) can be reduced with triethylsilane, etc. in an amount of 1.0 to 5.0 mole equivalents in a solvent such as trifluoroacetic acid at 0°C to 120°C, preferably at a temperature of from room temperature to a boiling point of the solvent to be used to give the compound of the formula (67).
   The compound of the formula (64) can be also led to the compound of the formula (68) by a conventional halogenation or methanesulfonation method, for example wherein the compound (64) is reacted with methanesulfonyl chloride in an amount of 2.0 to 3.0 mole equivalents or more in a halogenated hydrocarbon such as dichloromethane in the presence of a base such as triethylamine in an amount of 2.0 to 3.0 mole equivalents or more at a temperature of from room temperature to a boiling point of the solvent to be used to give the desired leaving group.
   The compound of the formula (68) thus obtained is reacted with a compound of the formula: R¹M (M means Li, MgBr, etc.) in a conventional manner to give the compound of the formula (69).
(E) In addition, the compound of the formula (1) may be also prepared as follows. 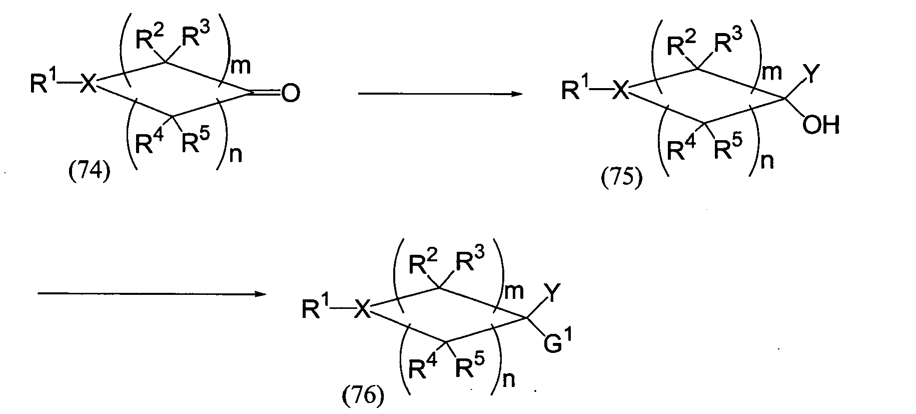 (wherein G¹, Y, m, n, R¹, R², R³, R⁴ and R⁵ are as defined above)
   The compound of the formula (74) is reacted with a Grignard reagent of the formula: e.g. YMgBr in an ether such as diethyl ether at a temperature of from 0°C to room temperature to give the compound of the formula (75).
   The compound of the formula (75) can be reacted to give the compound of the formula (76) in a similar manner in the above (C) wherein the compound of the formula (68) is prepared from the compound of the formula (64).
   The compound of the formula (76) thus obtained is reacted with various nucleophiles to construct various partial structures as shown in -(CR⁶R⁷)p-Z of the compound of the formula (1).
(F) The compound of the formula (74) is known compound, or can be prepared from a known compound in a conventional method. For example, the compound wherein X is nitrogen atom may be prepared as follows. 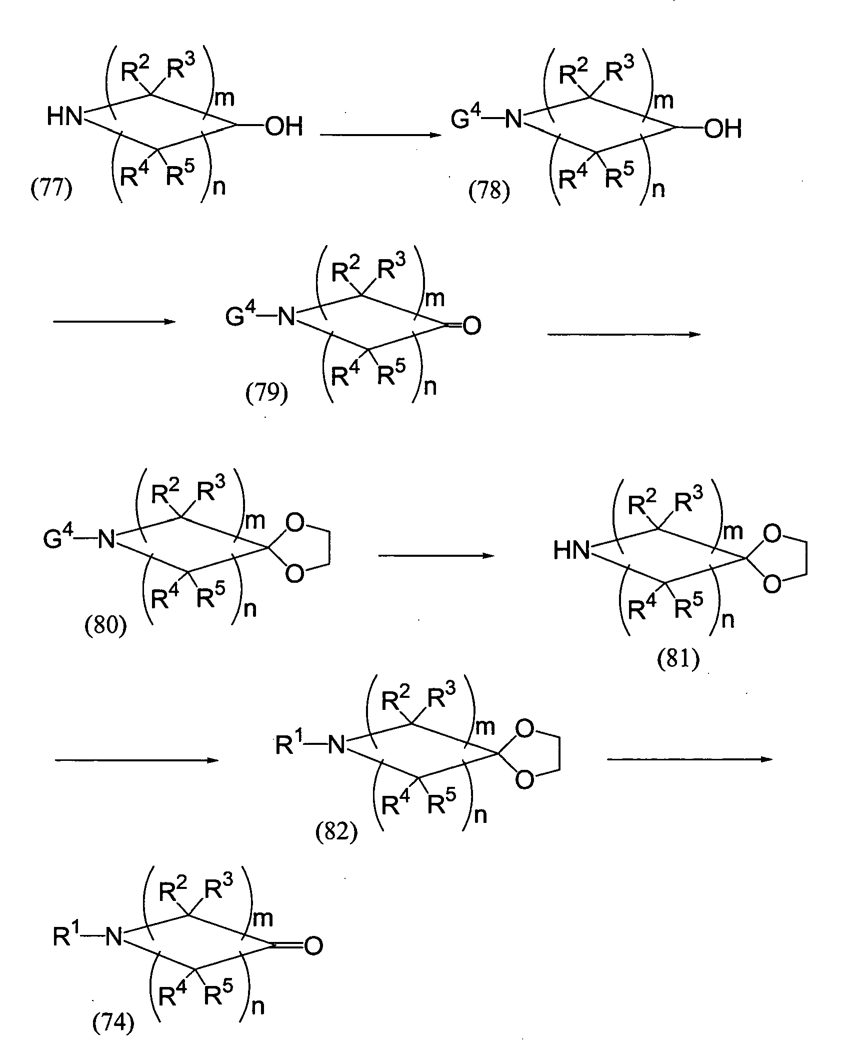 (wherein G⁴ is benzyloxycarbonyl group; and m, n, R¹, R², R³, R⁴ and R⁵ are as defined above)
   For example, the compound of the formula (77) is reacted with benzyloxycarbonylchloride or the like in an ether such as THF at a temperature of from 0°C to room temperature to give the compound of the formula (78).
   For example, the compound of the formula (78) is reacted with an oxidizing agent such as pyridine-sulfur trioxide and pyridinium chlorochromate in a polar solvent such as dimethylsulfoxide (DMSO) at a temperature of from 0°C to room temperature to give the compound of the formula (79).
   For example, the compound of the formula (79) is reacted with ethylene glycol or the like in a halogenated hydrocarbon such as dichloromethane for about one day at room temperature in the presence of an acid such as p-toluenesulfonic acid to give the compound of the formula (80).
   For example, the compound of the formula (80) is hydrogenated in an alcoholic solvent such as ethanol at room temperature in the presence of a catalyst such as palladium carbon to give the compound of the formula (81).
   The compound of the formula (81) is coupled with a compound of the formula: e.g. R¹-G¹ (R¹ and G¹ are as defined above) using a palladium catalyst under a base or using a base to give the compound of the formula (26).
   The coupling reaction using a palladium catalyst can be carried out with a palladium (0) catalyst (e.g. tris(dibenzylidene acetone)dipalladium, tetrakis(triphenylphosphine)palladium, etc.) and a phosphate ligand (e.g. 2,2'-bis(diphenylphosphino)-1,1'-binaphthyl, 1,1'-bis-(diphenylphosphino)ferrocene, etc.) in the presence of sodium tert-butoxide.
   The solvent may be any solvent which does not disturb the reaction, for example, a nonpolar solvent such as toluene is preferable.
   The coupling reaction using a base can be carried out with a base such as sodium hydride and triethylamine in a conventional manner.
   The solvent may be any solvent which does not disturb the reaction, for example, a polar solvent such as dimethylsulfoxide is preferable.
   For example, the compound of the formula (82) is reacted in a water-containing ether such as water-containing THF at room temperature in the presence of an acid such as p-toluenesulfonic acid to give the compound of the formula (74).
   The compound of the formula (70) or (72) used in the above process (A) is a known compound or can be prepared from a known compound in a known manner. For example, they can be prepared via the following process (G) or (H).
(G) The process for preparing the compound of the formula (70) is exemplified as follows. 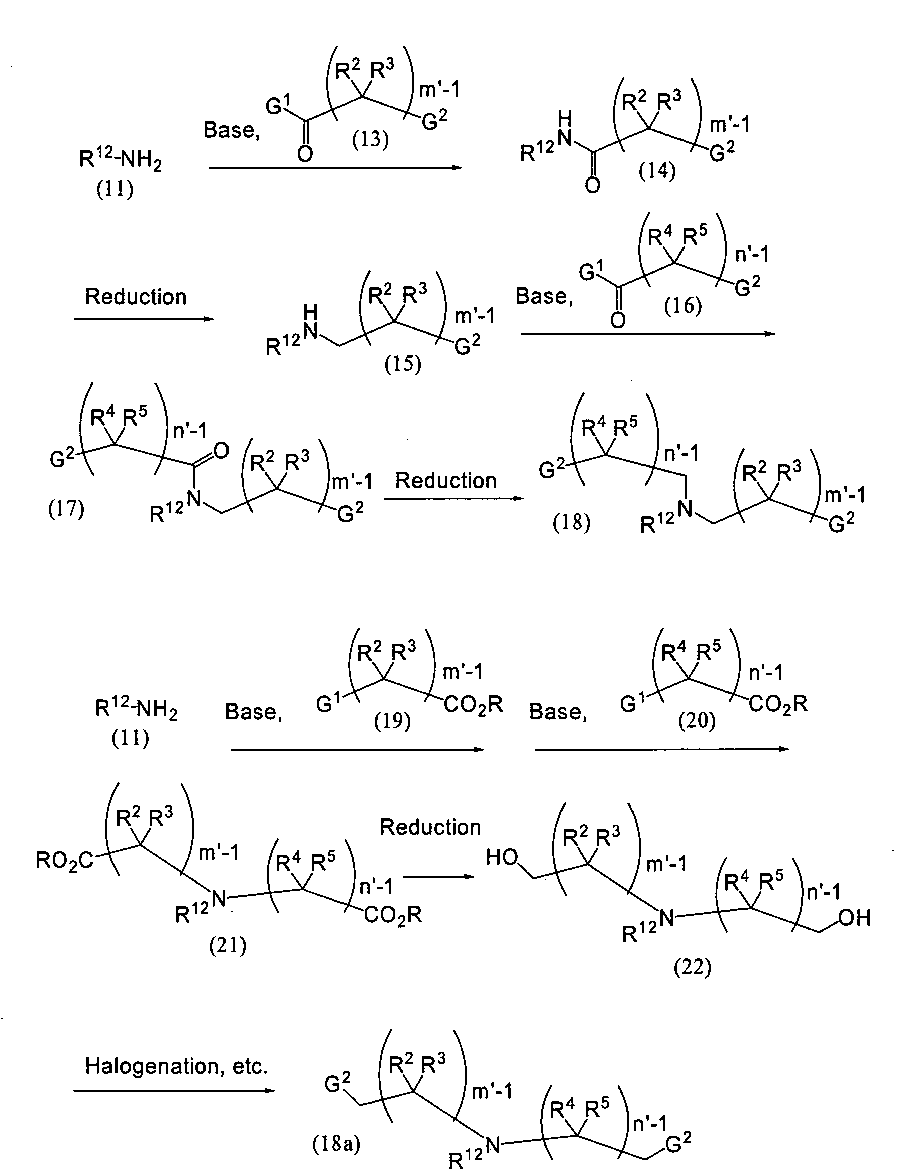 (wherein R is a lower alkyl group; R¹² is a substituted or unsubstituted alkyl group, a substituted or unsubstituted alkenyl group, a substituted or unsubstituted alkynyl group, a substituted or unsubstituted heteroarylalkyl group, a substituted or unsubstituted cycloalkyl group, or a substituted or unsubstituted aromatic group; and Y, G¹, G², m', n', R², R³, R⁴ and R⁵ are as defined above)
   The compound of the formula (11) is reacted with the compound of the formula (13) in an amount of 1 to 1.5 mole equivalent in the presence of a base such as triethylamine in an amount of 1 to 1.5 mole equivalent or more at a temperature of from room temperature to a boiling point of the solvent to be used to give the compound of the formula (14). Then, the compound of the formula (14) is reacted in the presence of a reducing agent such as BH₃·THF solution in an amount of 1 to 1.5 mole equivalent or more at a temperature of from room temperature to a boiling point of the solvent to be used to give the compound of the formula (15). And then, the compound of the formula (15) is reacted with the compound of the formula (16) in an amount of 1 to 1.5 mole equivalent at a temperature of from room temperature to a boiling point of the solvent to be used in the presence of a base such as triethylamine in an amount of 1 to 1.5 mole equivalent or more to give the compound of the formula (17). Then, the compound of the formula (17) is reacted at a temperature of from room temperature to a boiling point of the solvent to be used in the presence of a reducing agent such as BH₃·THF solution in an amount of 1 to 1.5 mole equivalent or more to give the compound of the formula (18).
   The reaction is usually carried out in a solvent, and the solvent may be any solvent which does not disturb the reaction, including, for example, ethers such as diethyl ether, diisopropyl ether, tetrahydrofuran and dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.
   In addition, the compound of the formula (11) is heated together with the compound of the formula (19) in an amount of 1 to 1.5 mole equivalent at 100 to 150°C in the presence of a base such as sodium carbonate in an amount of 1 to 1.5 mole equivalent or more without a solvent, and then the reaction mixture is reacted with the compound of the formula (20) at 100 to 150°C in the presence of a base such as sodium carbonate in an amount of 1 to 1.5 mole equivalent or more without a solvent to give the compound of the formula (21). Then, the compound of the formula (21) is reacted at a temperature of from room temperature to a boiling point of the solvent to be used in the presence of a reducing agent such as lithium aluminium hydride in an amount of 1 to 4.0 mole equivalents or more to give the compound of the formula (22). And then, the compound of the formula (22) can be also led to the compound of the formula (18a) by a conventional halogenation or methanesulfonation method, for example wherein the compound (22) is reacted with methanesulfonyl chloride in an amount of 2.0 to 3.0 mole equivalents or more in a halogenated hydrocarbon such as dichloromethane in the presence of a base such as triethylamine in an amount of 2.0 to 3.0 mole equivalents or more at a temperature of from room temperature to a boiling point of the solvent to be used to give the desired leaving group.
(H) The process for preparing the compound of the formula (72) is exemplified as follows. 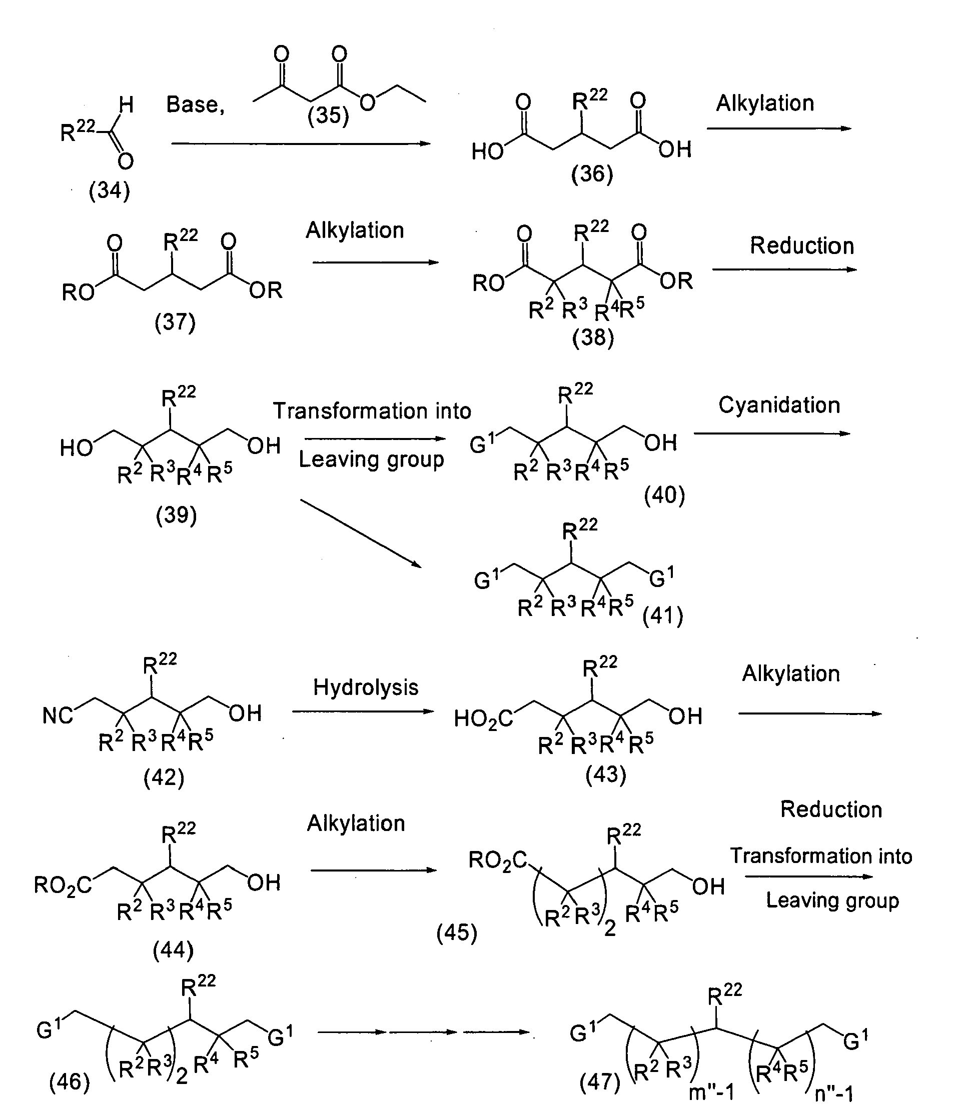 (wherein R is a lower alkyl group; m'' is 3 or 4; n'' is 2, 3 or 4; and Y, G¹, R²², R², R³, R⁴ and R⁵ are as defined above)
   The compound of the formula (34) is reacted with the compound of the formula (35) in amount of 2 to 5 mole equivalents at a temperature of from room temperature to 100°C in the presence of a base such as piperidine in a catalytic amount to an amount of 1.5 mole equivalent or more usually without a solvent to give the compound of the formula (36).
   Then, the compound of the formula (36) is reacted with an alkyl halide in an amount of 2.0 to 5.0 mole equivalents usually in a polar solvent such as N,N-dimethylformamide in the presence of a base such as potassium carbonate at 0°C to 120°C, preferably at a temperature of from room temperature to a boiling point of the solvent to be used to give the compound of the formula (37).
   And then, the compound of the formula (37) is reacted with a compound of the formula: R²-G¹ in an amount of 1.0 to 1.2 mole equivalent usually in a polar solvent such as N,N-dimethylformamide in the presence of a base such as sodium hydride at 0°C to 120°C, preferably at a temperature of from room temperature to a boiling point of the solvent to be used, and further reacted with optional compound(s) of R³-G¹, R⁴-G¹, and R⁵-G¹ in a similar manner to give the compound of the formula (38). The compound of the formula: R²-G¹, R³-G¹, R⁴-G¹, and R⁵-G¹ includes, for example, an alkyl halide, etc.
   Then, the compound of the formula (38) is reduced with lithium aluminium hydride or the like according to a conventional manner to give the compound of the formula (39).
   And then, the hydroxyl group of the compound of the formula (39) is transformed to a leaving group in a conventional manner, for example wherein the hydroxyl group is reacted with a sulfonyl chloride such as p-toluenesulfonyl chloride in an amount of 0.5 to 3.0 mole equivalents or more in pyridine in an amount of 0.5 to 3.0 mole equivalents or more at a temperature of from room temperature to a boiling point of the solvent to be used to give the compounds of the formulas (40) and (41).
   Next, the compound of the formula (40) is reacted with a cyanide salt such as potassium cyanide in an amount of 1.0 to 3.0 mole equivalents in a polar solvent such as dimethylsulfoxide at a temperature of from room temperature to 150°C to give the compound of the formula (42).
   And next, the compound of the formula (42) can be hydrolyzed in a conventional manner to give the compound of the formula (43).
   Then, the compound of the formula (43) is reacted with an alkyl halide in an amount of 1.0 to 5.0 mole equivalents usually in a polar solvent such as N,N-dimethylformamide in the presence of a base such as sodium hydride at 0°C to 120°C, preferably at a temperature of from room temperature to a boiling point of the solvent to be used to give the compound of the formula (44).
   And then, the compound of the formula (44) is reacted with a compound of the formula: R²-G¹ in an amount of 1.0 to 1.2 mole equivalent usually in a polar solvent such as N,N-dimethylformamide in the presence of a base such as potassium carbonate at 0°C to 120°C, preferably at a temperature of from room temperature to a boiling point of the solvent to be used, and then reacted with a compound of the formula: R³-G¹ in the amount of 1.0 to 1.2 mole equivalent in a similar manner to give the compound of the formula (45). The compound of the formula: R²-G¹ and R³-G¹ includes, for example an alkyl halide.
   Optionally, repeating the reactions from (38) to (45) can lead the compound of the formula (45) to the compound of the formula (46), and further to the compound of the formula (47).
   Transforming a partial structure of the compound obtained in the above method to the other variation makes each compound of the formula (1) having various partial structures.
(I) Transformation of R¹
   The compound of the formula (23) wherein X is nitrogen atom, and R¹ is the formula: -CHAr¹Ar², for example, can be transformed into a compound having any one of various R¹, for example, by the following process. The example of the compound of the formula (23) is the compound wherein p is 0 and Z is cyano group which can be prepared by the process (A) or (B). 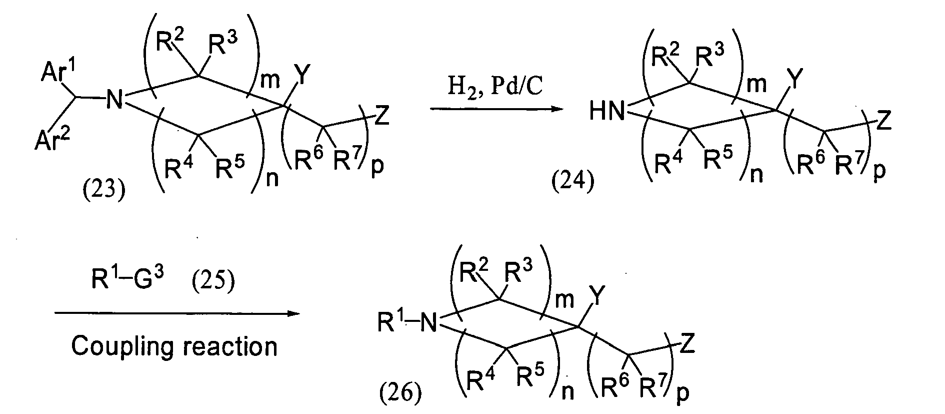 (wherein m, n, p, Z, R¹, R², R³, R⁴, R⁵, R⁶, R⁷ and Y are as defined above; G³ is a leaving group such as iodine atom, bromine atom and chlorine atom; Ar¹ is a substituted or unsubstituted phenyl group and Ar² is hydrogen atom, or a substituted or unsubstituted phenyl group wherein the substituent is a lower alkyl group, a lower alkoxy group, etc.)
   The compound of the formula (23) obtained according to the above processes (A), (B), and (D), and the compound of the formula (23) obtained according to the following processes (H) and (I) wherein X is nitrogen atom and R¹ is a group of the formula: -CHAr¹Ar² are hydrogenated in a conventional manner to give the compound of the formula (24).
   Then, the compound (24) is coupled with the compound of the formula (25) using a palladium catalyst in the presence of a base or using a base to give the compound of the formula (26).
   A coupling reaction using a palladium catalyst can be carried out in the presence of sodium tert-butoxide by using a palladium (0) catalyst such as tris(dibenzylidene acetone)dipalladium and tetrakis(triphenylphosphine)-palladium and a phosphine ligand such as 2,2'-bis(diphenyl-phosphino)-1,1'-binaphthyl and 1,1'-bis(diphenylphosphino)-ferrocene.
   The solvent may be any solvent which does not disturb the reaction, for example, a nonpolar solvent such as toluene is preferable.
   The coupling reaction using a base can be carried out with a base such as sodium hydride and triethylamine in a conventional manner.
   The solvent may be any solvent which does not disturb the reaction, for example, a polar solvent such as dimethylsulfoxide is preferable.
(J) Transformation of Z
   In the compound wherein Z is cyano group, which can be prepared by the above process, the Z can be transformed into various groups via the following process. 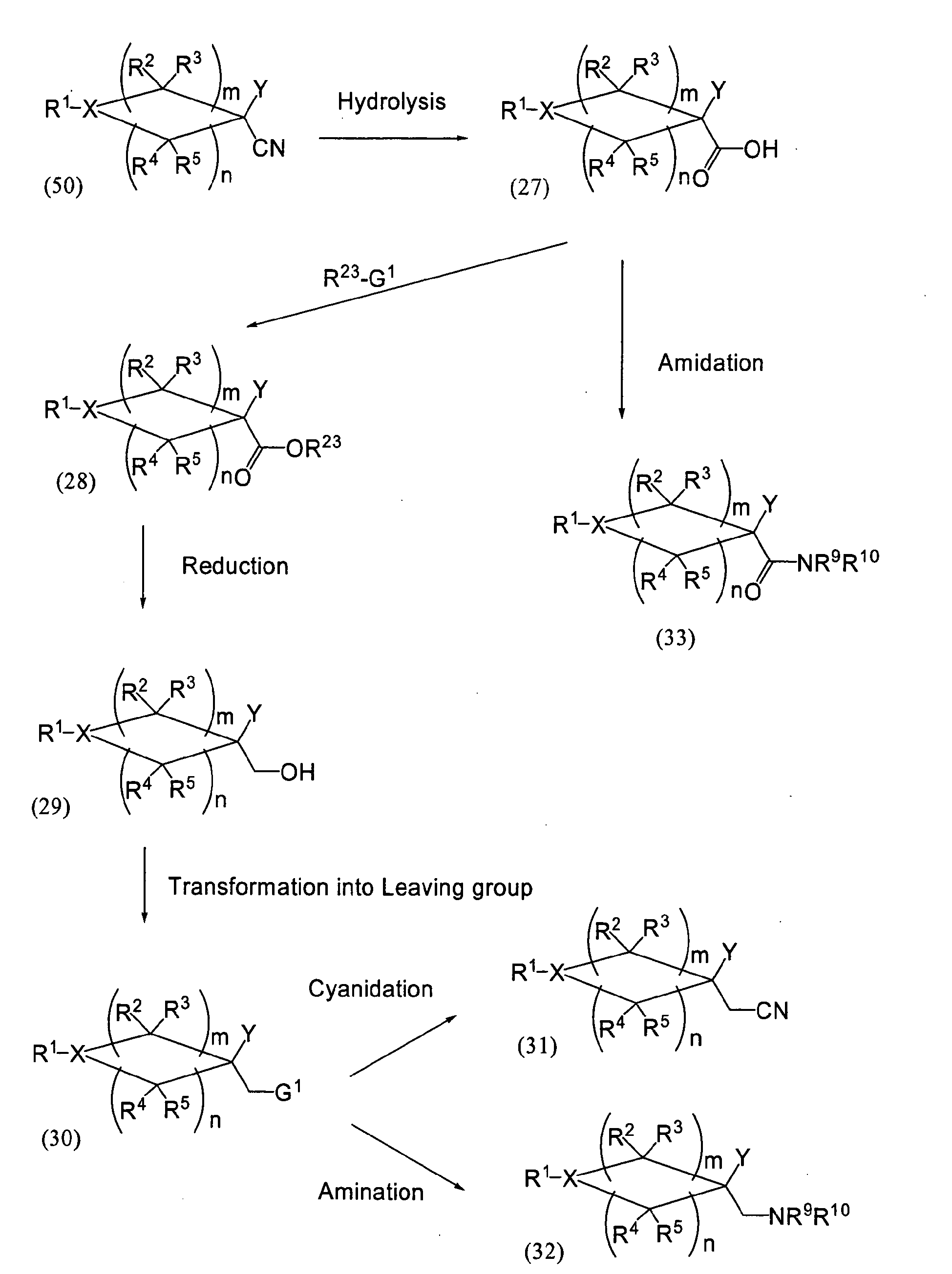 (wherein m, n, R¹, R², R³, R⁴, R⁵, R⁹, R¹⁰, Y and G¹ are as defined above; and R²³ is a substituted or unsubstituted alkyl group, or a substituted or unsubstituted aralkyl group)
   The compound of the formula (50) is hydrolyzed with 48% aqueous hydrobromic acid or the like to give the compound (27) of the invention.
   And the compound of the formula (27) is reacted with the compound of R²³-G¹ (R²³ and G¹ are as defined above) in an amount of 1 - 3.0 mole equivalents usually in a polar solvent such as N,N-dimethylformamide at 0°C to 120°C, preferably at a temperature of from room temperature to a boiling point of the solvent to be used to give the compound (28) of the invention.
   Next, the compound of the formula (28) is reduced with lithium aluminium hydride or the like in a conventional manner to give the compound (29) of the invention.
   When R¹ is a group of the formula: -C(=O)R¹⁴, for example, the compound of the formula (27) is reacted with acetyl chloride in an amount of 1 - 3 mole equivalents in a halogenated hydrocarbon such as dichloromethane at a temperature of from 0°C to room temperature to give a mixed anhydride thereof which is reduced with a reducing agent such as sodium borohydride in a conventional manner. In this way, the compound of the formula (29) can be prepared without reducing the carbonyl group of the formula: - C(=O)R¹⁴.
   And next, the compound of the formula (29) is reacted with an arylsulfonyl chloride such as p-toluenesulfonyl chloride in an amount of 2.0 to 3.0 mole equivalents or more in pyridine as a solvent in an amount of 2.0 - 3.0 mole equivalents at a temperature of from room temperature to a boiling point of the solvent to be used to give the compound of the formula (30).
   A variety of compounds can be prepared by nucleophilic displacement with the compound of the formula (30). As the nucleophilic displacement, cyanidation and amination are exemplified.
   For example, the compound of the formula (30) is reacted via cyanidation with a cyanide salt such as potassium cyanide in an amount of 1.0 to 3.0 mole equivalents or more in a polar solvent such as dimethylsulfoxide at a temperature of from room temperature to 150°C to give the compound (31) of the invention.
   For example, the compound of the formula (30) is reacted via amination with a compound of the formula: HNR⁹R¹⁰ in an amount of 1.0 to 3.0 mole equivalents or more at a temperature of from room temperature to 150°C in a polar solvent such as N,N-dimethylformamide or without a solvent to give the compound of the formula (32) wherein R⁹ and R¹⁰ are as defined above.
   In addition, the compound of the formula (27) is reacted with a compound of the formula: HNR⁹R¹⁰ in an amount of 1.0 to 3.0 mole equivalents or more and a condensing agent in an amount of 1.0 to 3.0 mole equivalents or more in a polar solvent such as N,N-dimethylformamide at a temperature of from room temperature to 150°C to give the compound of the formula (33). Alternatively, the compound of the formula (27) is led to an acid halide thereof by using a halogenating agent such as oxalyl chloride, and then the acid halide is reacted with the compound of the formula: NR⁹R¹⁰ in an amount of 1.0 to 3.0 mole equivalent or more in a halogenated hydrocarbon such as dichloromethane at a temperature of from room temperature to 150°C in the presence of a base such as triethylamine in an amount of 1.0 to 3.0 mole equivalent or more to give the compound of the formula (33).
   The condensing agent used herein is, for example, dicyclohexylcarbodiimide (DCC), 1,1'-carbonyldiimidazole, diethyl cyanophosphate (DEPC), 1-ethyl-3-(3'-dimethylaminopropyl)carbodiimide hydrochloride (WSC), etc.
   The reaction is usually carried out in a solvent, and the solvent may be any solvent which does not disturb the reaction, including, for example, ethers such as diethyl ether, diisopropyl ether, tetrahydrofuran and dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.
   Repeating the reactions from the compound of the formula (50) to the compound of the formula (31) can expand the number of methylene group corresponding to the partial structure: -CR⁶R⁷- of the compound of the formula (1) .
(K) Introduction of the group of the formula: - (CR⁶R⁷) - and transformation of Z
   From the compound of the formula (50) as a starting compound, thereto a group of the formula: -(CR⁶R⁷)-containing in the compound of the formula (1) can be introduced, the number of the group is expanded, and Z can be transformed. 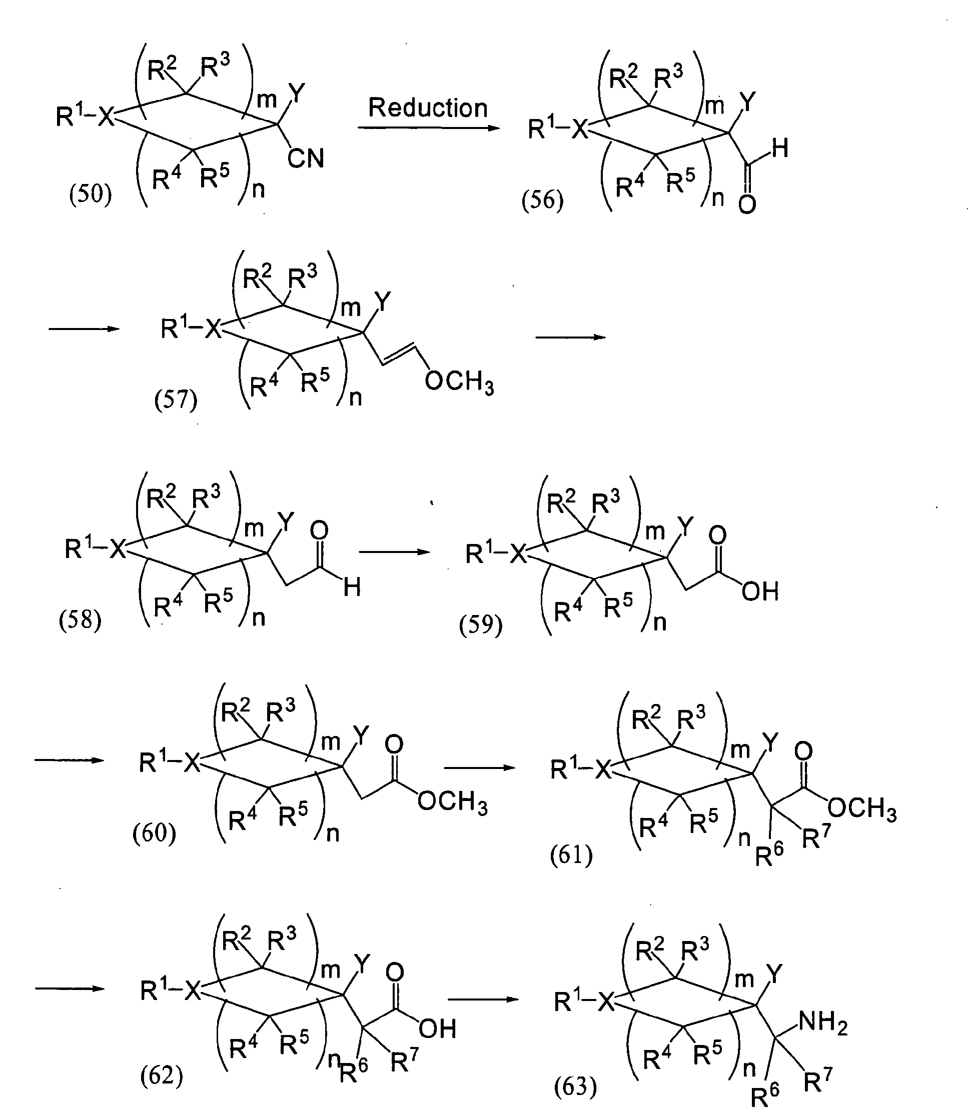 (wherein X, m, n, R¹, R², R³, R⁴, R⁵, R⁶, R⁷ and Y are as defined above)
   The compound of the formula (50) is reduced with diisobutyl aluminium hydride (DIBAL) in an amount of 1 to 1.5 mole equivalent in a halogenated hydrocarbon such as dichloromethane at a temperature of from ice-cooling to room temperature to give the compound of the formula (56).
   And then, for example, the compound (56) is reacted with a Wittig reagent such as (methoxymethyl)triphenylphosphonium chloride in an amount of 1 to 1.5 mole equivalent in an ether solvent such as tetrahydrofuran at - 78°C to room temperature in the presence of a strong base such as sodium amide (NaNH₂) in an amount of 1 to 1.5 mole equivalent to give the compound of the formula (57).
   And the compound (57) is reacted in a mixture of a halogenated hydrocarbon (e.g. dichloromethane) and trifluoroacetic acid (1:1) at a temperature of from ice-cooling to room temperature to give the compound of the formula (58).
   And then, for example, the compound (58) is oxidized with an oxidizing agent such as potassium permanganate (KMnO₄) in an amount of 1 to 3 mole equivalents in a harogenated hydrocarbon such as dichloromethane and chloroform at a temperature of from room temperature to 40°C to give the compound of the formula (59).
   Then, for example, the compound (59) is reacted with methyl iodide in an amount of 1 to 1.5 mole equivalent in N,N-dimethylformamide at a temperature of from room temperature to heating-temperature with reflux in the presence of a base such as potassium carbonate in an amount of about 3 mole equivalents to give the compound of the formula (60).
   Next, the compound (60) is reacted with a compound of the formula: R⁶-G¹ in an amount of 1 - 1.5 mole equivalent in an ether such as tetrahydrofuran at -78°C to room temperature in the presence of a strong base such as lithium diisopropylamide in an amount of 1 - 1.5 mole equivalents, and then reacted with a compound of the formula: R⁷-G² in an amount of 1 - 1.5 mole equivalent in the presence of a strong base such as lithium diisopropylamide in an amount of 1 - 1.5 mole equivalent to give the compound of the formula (61) wherein G¹, G², R⁶ and R⁷ are as defined above.
   And the compound (61) is hydrolyzed with a base such as sodium hydroxide or the like in an amount of 1 to 3 mole equivalents in a mixture of water-ethanol (1:4) at a temperature of from room temperature to heating-temperature with reflux to give the compound of the formula (62).
   Next, for example, the compound (62) is reacted with diphenylphosphoryl azide in an amount of 1 to 1.5 mole equivalent and triethylamine in an amount of 1 to 1.5 mole equivalent in N,N-dimethylformamide at a temperature of from ice-cooling to 60°C to give the compound of the formula (63) (Curtius rearrangement).
   In order that R⁶ and R⁷ combine to form the partial structure: oxo group, for example, the carboxy group of the above-mentioned compound of the formula (27), (59) or (62) is reacted with oxalyl chloride in a halogenated hydrocarbon such as dichloromethane at a temperature of from 0°C to room temperature to give an acid chloride which is then reacted with a Grignard reagent of the formula: ZMgBr (Z is as defined above), etc. in a ether solvent such as tetrahydrofuran (THF) to give the desired structure.
(L) As shown in the following scheme, the compound of the formula (61) can be transformed into the compound of the formula (31a) via a procedure from the carboxylic ester thereof to a cyanomethyl group, in a similar route from the compound of the formula (28) to the compound of the formula (31) in the above process (J). The compound (31a) is hydrolyzed to give the compound of the formula (27a) in a similar procedure transforming the compound of the formula (50) into the compound of the formula (27) in the above process (J), and then the compound (27a) can be transformed into the compound of the formula (61a) in a similar manner from the compound of the formula (59) to the compound of the formula (61) in the above process. In this way, the number of the group shown as the formula: -(CR⁶R⁷)- in the compound (1) can be expanded. 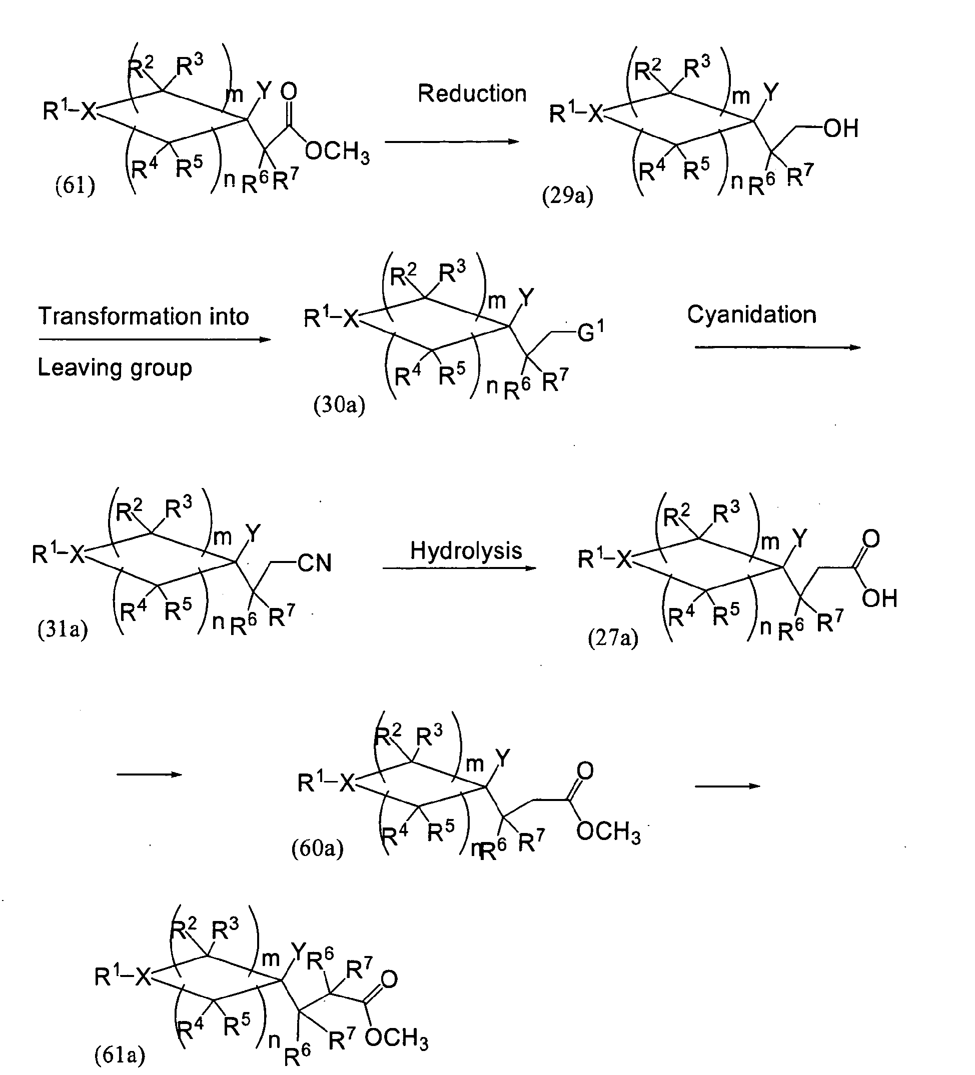 (wherein X, m, n, R¹, R², R³, R⁴, R⁵, R⁶, R⁷, Y and G¹ are as defined above)
(M) Among the compound of the formula (1), the compounds of the formulas (95), (96), (97) and (98) wherein R² and R⁴ combine together to an alkylene can be prepared, for example, as follows. 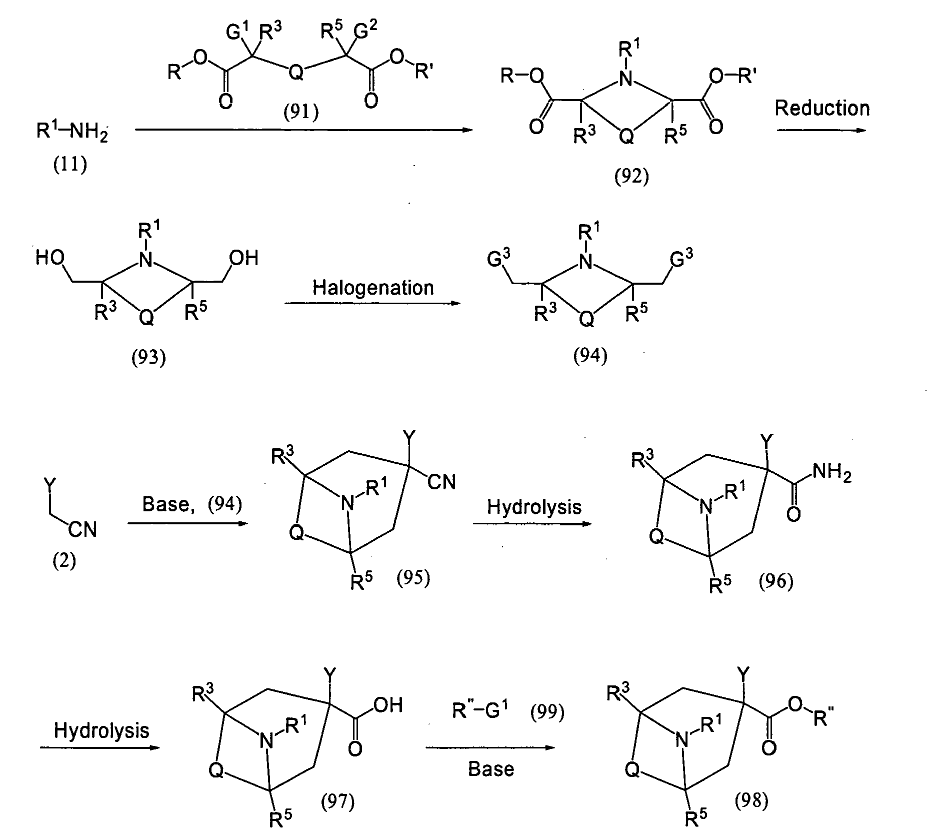 (wherein R¹, R³, R⁵, Y, G¹ and G² are as defined above; G³ is a leaving group such as chlorine atom and bromine atom; R'' is a lower alkyl group; and Q is an alkylene)
   Before showing preparation of the compound of the formula (95), the route preparing the compound of the formula (94) is shown. For example, the compound of the formula (91) is reacted with the compound of the formula (11) in an amount of 1.0 to 2.0 mole equivalents at a temperature of from room temperature to a boiling point of the solvent to be used to give the compound of the formula (92), then the compound (92) is reacted in the presence of a reducing agent such as lithium aluminium hydride in an amount of 1 to 1.5 mole equivalent or more at a temperature of from room temperature to a boiling point of the solvent to be used to give the compound of the formula (93), and then the compound of the formula (93) is reacted with thionyl chloride in an amount of 2.0 to 3.0 mole equivalents or more at a temperature of from 0°C to a boiling point of the solvent to be used to give the compound of the formula (94).

The reaction is usually carried out in a solvent, and the solvent may be any solvent which does not disturb the reaction, including, for example, ethers such as diethyl ether, diisopropyl ether, tetrahydrofuran and dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.

The compound of the formula (2) is reacted with the compound of the formula (9.4) obtained above in an amount of 1.0 to 2.0 mole equivalents in a solvent at -10°C to 50°C in the presence of a base such as sodium hydride in an amount of 2.0 to 4.0 mole equivalents to give the compound of the formula (95); then the compound of the formula (95) is reacted with basic aqueous solution such as aqueous potassium hydroxide in an amount of 2.0 to 3.0 mole equivalents or more at a temperature of from room temperature to a boiling point of the solvent to be used to give the compound of the formula (96); then the compound (96) is reacted with a solvent-amount of 48%-aqueous hydrobromic acid at a temperature of from room temperature to a boiling point of the solvent to be used to give the compound of the formula (97); and then the compound (97) is reacted with the compound of the formula (99) or the like in an amount of 2.0 to 10 mole equivalents or more in the presence of a base such as cesium carbonate in an amount of 2.0 to 10 mole equivalents or more at a temperature of from room temperature to a boiling point of the solvent to be used to give the compound of the formula (98).

The reaction is usually carried out in a solvent, and the solvent may be any solvent which does not disturb the reaction, including, for example, ethers such as diethyl ether, diisopropyl ether, tetrahydrofuran and dioxane; aromatic hydrocarbons such as benzene, toluene and xylene; esters such as methyl acetate, ethyl acetate and propyl acetate; halogenated hydrocarbons such as dichloromethane, chloroform, dichloroethane, chlorobenzene and dichlorobenzene; ketones such as acetone and methylethylketone; nitriles such as acetonitrile and isobutylonitrile; N,N-dimethylformamide; dimethylsulfoxide; etc.

The starting compounds used in the above-mentioned each process may be known compounds or may be prepared from a conventional known compound by a conventional method known by an ordinary skilled person in the art or a modified method thereof.

Each compound obtained in the above-mentioned processes may be isolated and purified by a conventional isolation method such as recrystallization, a conventional purification method using chromatography, solvent extraction method, or reprecipitation.

Any one of the products obtained in the processes may be in a salt or free form thereof, due to the reaction conditions. The product may be converted into a desired salt or free form by a conventional method.

The compounds of the present invention are exemplified below (wherein Me means methyl group).

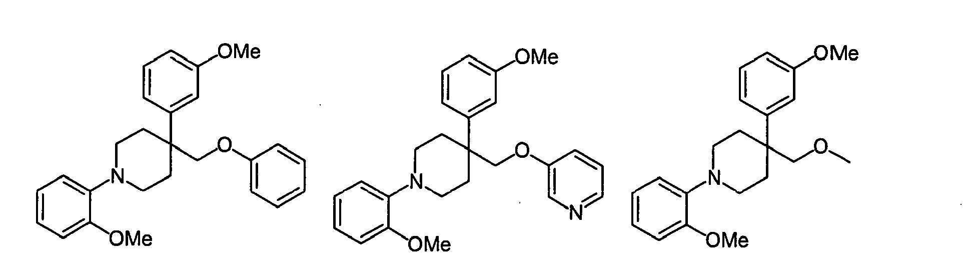

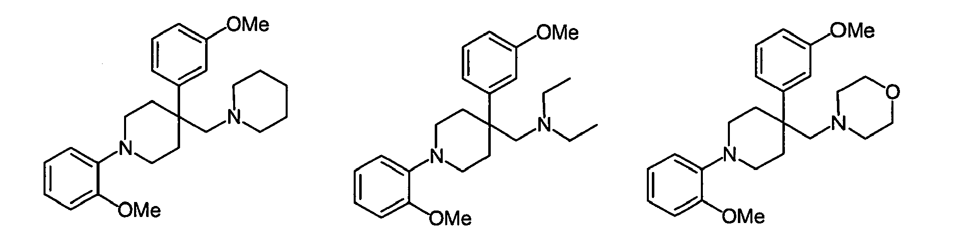

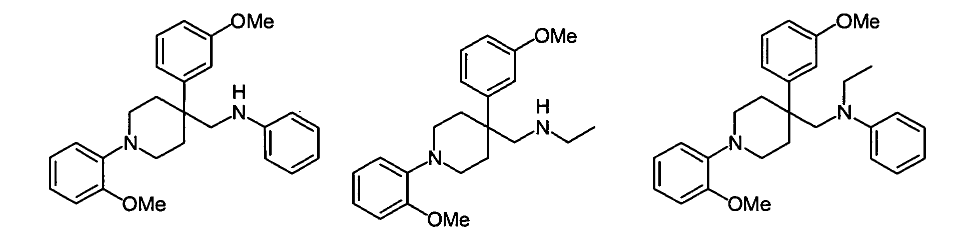

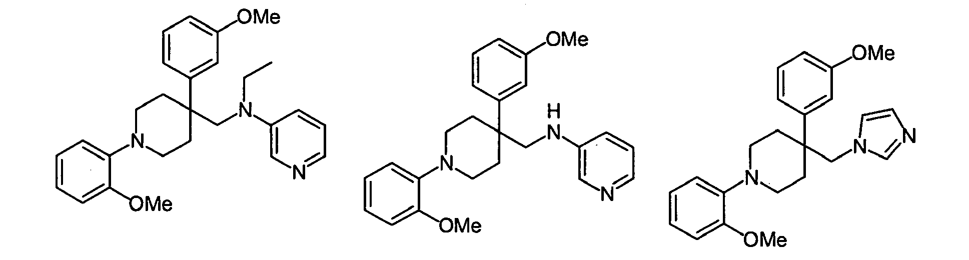

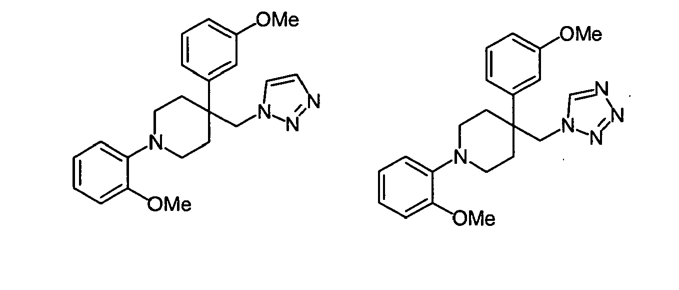

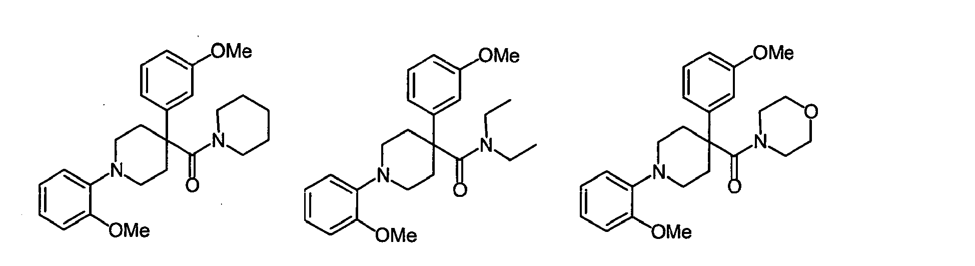

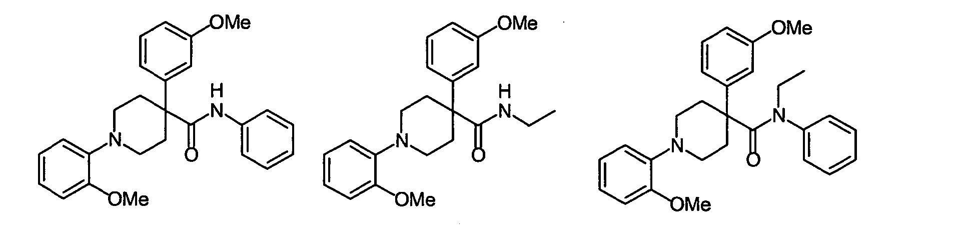

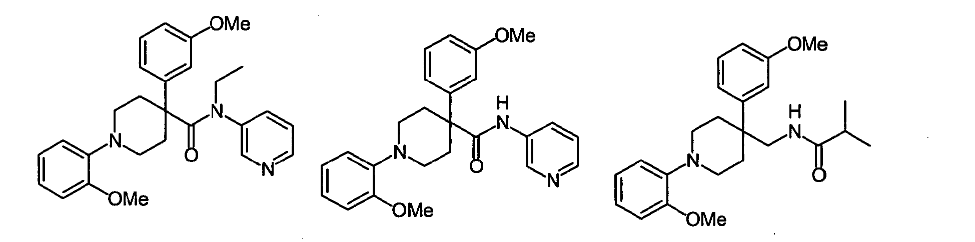

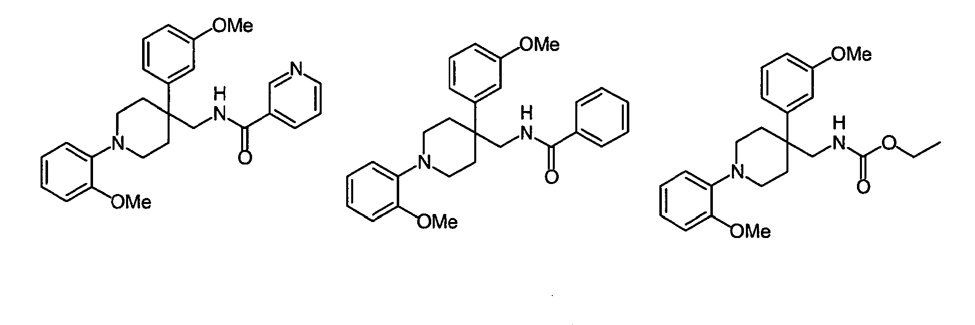

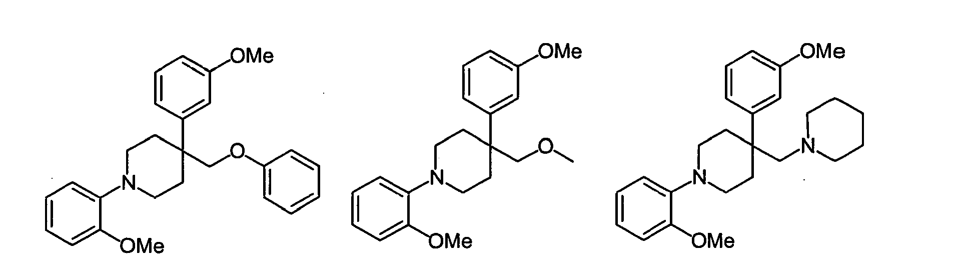

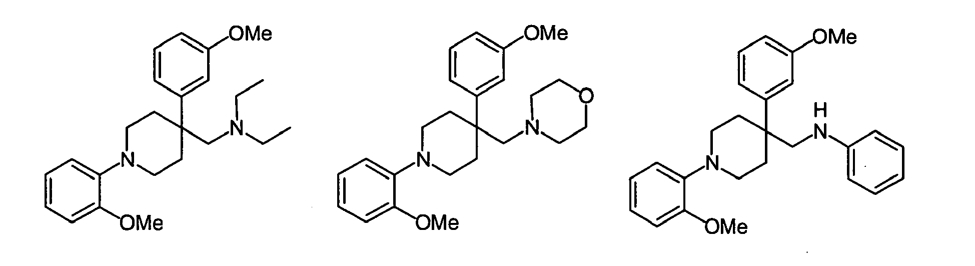

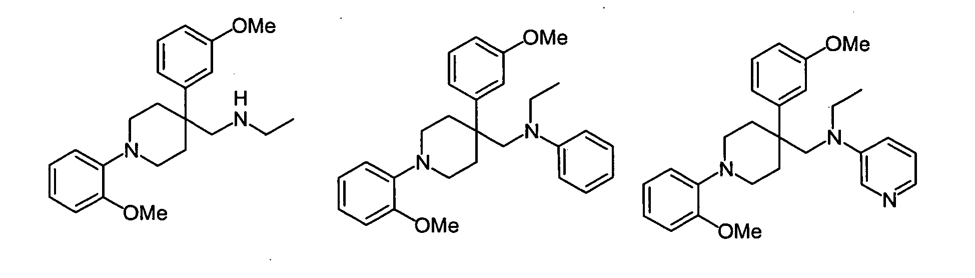

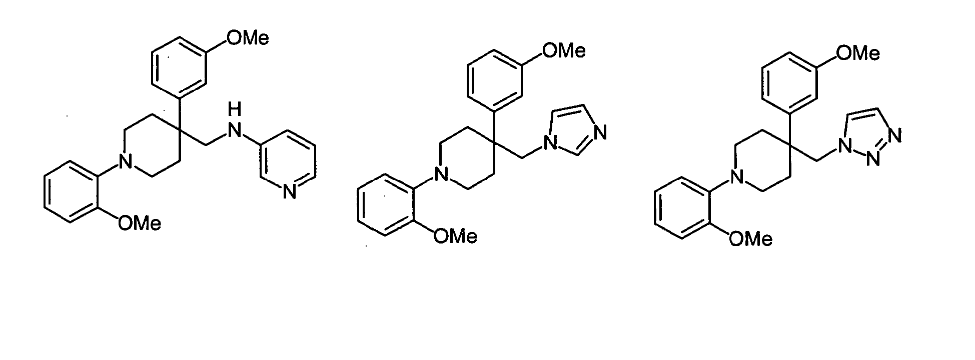

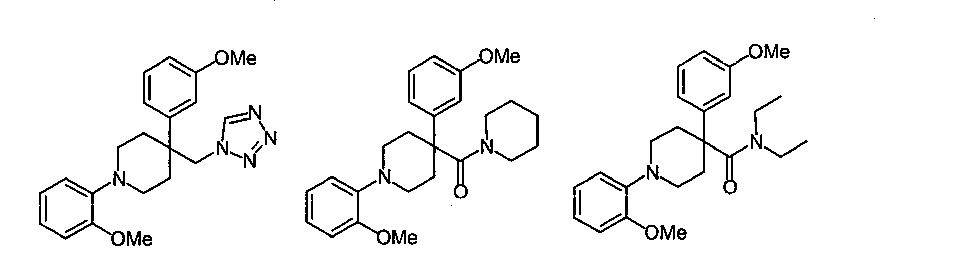

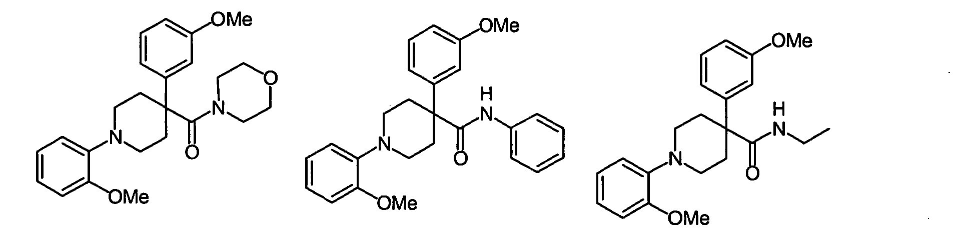

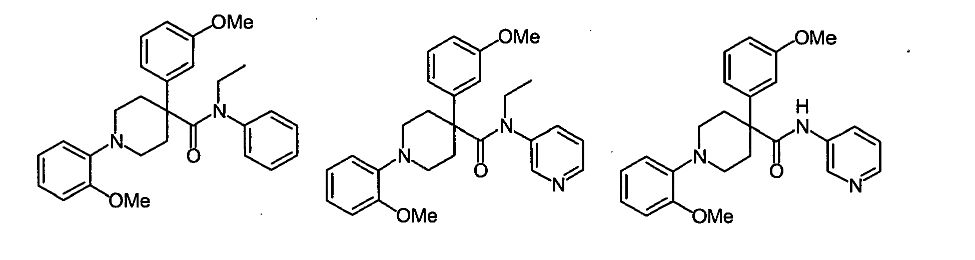

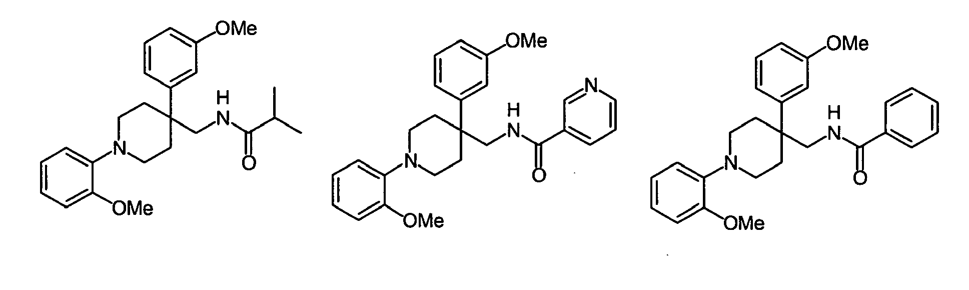

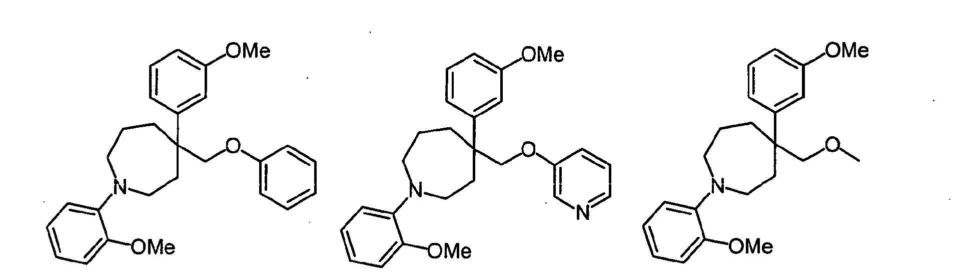

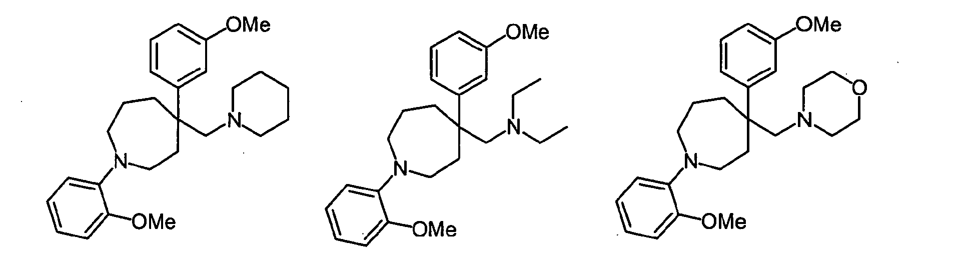

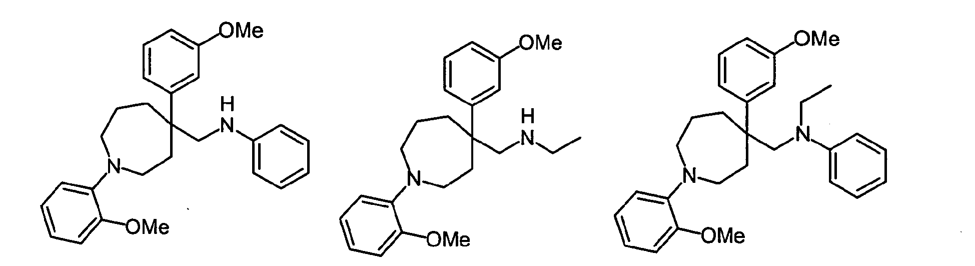

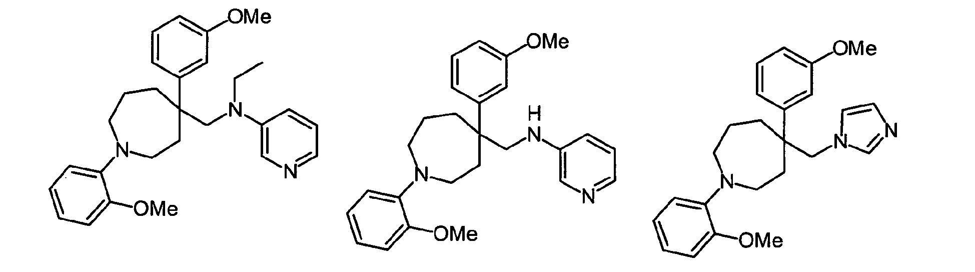

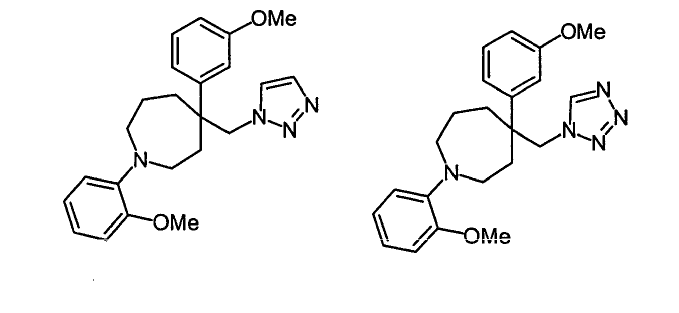

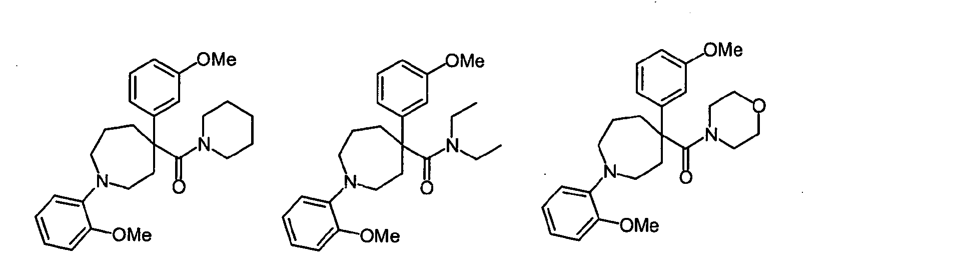

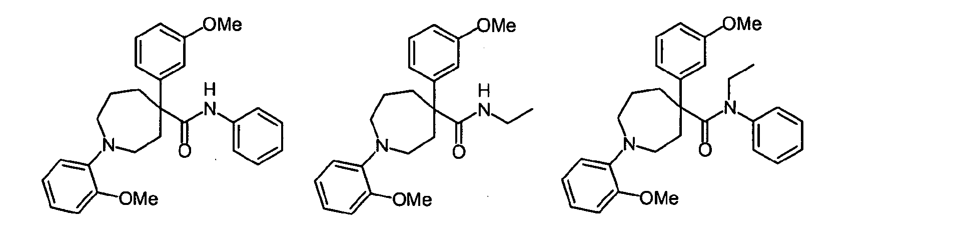

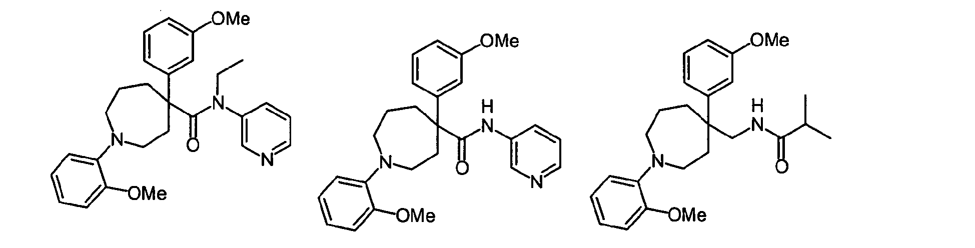

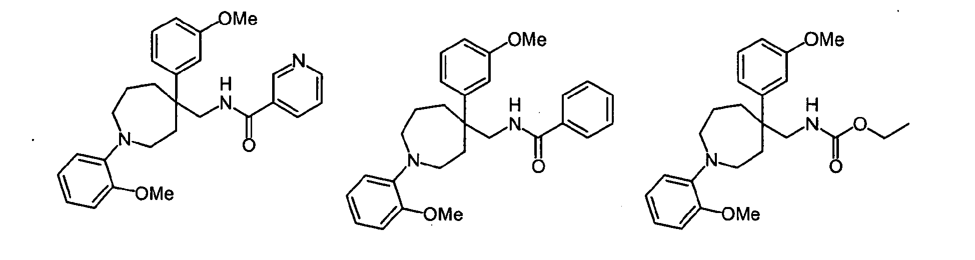

Hereinafter, the present invention is further illustrated by Reference Examples, Examples and Experiments, but should not be construed to be limited thereto. The analytical condition of high-performance liquid chromatography in respect to "retention time" in the following Examples and Reference Examples is defined as follows:
Column: octadecyl-bonded silica (ODS), Particle size: 5 µm, Pore size: 12 nm, Column length: 50 mm, Column I.D.: 4.6 mm
(Trade Name: YMC CombiScreen ODS-A (S-5 µm, 12 nm) 50 x 4.6 mm (YMC Co., Ltd.))
Flow rate: 3.5 ml/min
Wave length: 220 nm
Mobile phase:
solution A; 0.05% solution of trifluoroacetic acid in water solution B; 0.035% solution of trifluoroacetic acid in acetonitrile

Time program:

| Step | Time (min) | Solution A : Solution B |
|---|---|---|
| 1 | 0.0 - 0.5 | 90:10 |
| 2 | 0.5 - 4.2 | 90:10 → 1:99 |
| 3 | 4.2 - 4.4 | 1:99 → 99:1 |

### Reference example 1-1

### Preparation of 4-hydroxy-2-(2-hydroxyethyl)-2-(3-methoxyphenyl)butanenitrile

To a solution of sodium hydride (1.79 g, 44.8 mmol) in dimethylsulfoxide (DMSO, 60 mL) was added a solution of (3-methoxyphenyl)acetonitrile (2.50 ml, 17.9 mmol) and 2-bromoethyl tert-butyldimethylsilyl ether (9.22 ml, 43.0 mmol) in diethyl ether (20 mL) at room temperature, and the mixture was stirred overnight. After water was added thereto, the mixture was extracted with diethyl ether twice, and the organic layer was washed with water 3 times, dried over anhydrous magnesium sulfate, filtrated, and then the solvent was removed off in vacuo. Then, the residue was dissolved in tetrahydrofuran (THF, 100 mL), tetrabutylammonium fluoride (TBAF, 12.2 g, 46.5 mmol) was added thereto, and the mixture was stirred overnight at room temperature. After water was added thereto, the mixture was extracted with ethyl acetate twice, and the organic layer was washed with water twice, dried over anhydrous magnesium sulfate, filtrated, and then the solvent was removed off in vacuo. The residue was purified by silica gel chromatography to give 2.91 g of the title compound as a colorless oil.
¹H-NMR δ (DMSO-d₆); 1.97-2.02 (4H, m), 3.12-3.22 (2H, m), 3.32-3.45 (2H, m), 3.76 (3H, s), 4.59 (2H, t, J = 5.13), 6.87-7.03 (3H, m), 7.31-7.36 (1H, m).

### Reference example 1-2

### Preparation of 4-hydroxy-2-(2-hydroxyethyl)-2-(2-methoxyphenyl)butanenitrile

The title compound was prepared in a similar manner to Reference example 1-1.
¹H-NMR δ (DMSO-d₆); 1.98-2.18 (2H, m), 2.35-2.44 (2H, m), 3.15-3.24 (2H, m), 3.36-3.43 (2H, m), 3.82 (3H, s), 4.57 (2H, t, J = 5.10), 6.95-6.99 (1H, m), 7.07 (1H, d, J = 8.07), 7.30-7.38 (2H, m).

### Reference example 1-3

### Preparation of 4-hydroxy-2-(2-hydroxyethyl)-2-(4-methoxyphenyl)butanenitrile

The title compound was prepared in a similar manner to Reference example 1-1.
¹H-NMR δ (DMSO-d₆); 2.07-2.15 (4H, m), 3.12-3.19 (2H, m), 3.33-3.42 (2H, m), 3.74 (3H, s), 4.59 (2H, t, J = 5.13), 6.96 (2H, d, J = 8.79), 7.34 (2H, d, J = 8.79).

### Reference example 1-4

### Preparation of 4-hydroxy-2-(2-hydroxyethyl)-2-(3-fluorophenyl)butanenitrile

The title compound was prepared in a similar manner to Reference example 1-1.
¹H-NMR δ (DMSO-d₆); 2.15-2.31(4H, m), 3.19-3.29 (2H, m), 3.40-3.50 (2H, m), 4.69 (2H, t, J = 4.95), 7.20-7.55 (4H, m).

### Reference example 1-5

### Preparation of 4-hydroxy-2-(2-hydroxyethyl)-2-(3-trifluoromethylphenyl)butanenitrile

The title compound was prepared in a similar manner to Reference examplel-1.
¹H-NMR 5 (DMSO-d₆): 2.20-2.25 (4H, m), 3.19-3.20 (2H, m), 3.40-3.50 (2H, m), 4.64 (2H, t, J = 4.92), 7.64-7.80 (4H, m).

### Reference example 1-6

### Preparation of 4-hydroxy-2-(2-hydroxyethyl)-2-(3-benzyloxyphenyl)butanenitrile

The title compound was prepared in a similar manner to Reference example 1-1.
¹H-NMR δ (DMSO-d₆): 2.13-2.18 (4H, m), 3.12-3.22 (2H, m), 3.35-3.45 (2H, m), 4.60 (2H, t, J = 5.13), 5.11 (2H, s), 6.98-7.07 (3H, m), 7.32-7.49 (6H, m).

### Reference example 1-7

### Preparation of 4-hydroxy-2-(2-hydroxyethyl)-2-[3-(trifluoromethoxy)phenyl]butanenitrile

The title compound was prepared in a similar manner to Reference example 1-1.
High-performance liquid chromatography/mass spectrometry
m/z 289.9 (M+H)
retention time: 2.86 min.

### Reference example 2

### Ethyl N-(2-ethoxy-2-oxoethyl)-N-(2-methoxyphenyl)glycinate

To a mixture of o-anisidine (75.0 ml, 644 mmol) and ethyl bromoacetate (158 ml, 1.42 mol) was added sodium carbonate (162 g, 1.53 mol) at room temperature, and the mixture was stirred for 4 hours heating at 130°C. Further, ethyl bromoacetate (59.2 ml, 0.531 mol) and sodium carbonate (56.3 g, 0.531 mol) were added thereto, and the mixture was stirred for 3 hours heating at 130°C. After the reaction mixture was cooled to room temperature, ethyl acetate (500 mL) was added thereto, and then the sodium carbonate was filtrated off. After the solvent was removed off in vacuo, the residue was purified by silica gel chromatography to give 165 g of the title compound as a colorless oil.
¹H-NMR δ (DMSO-d₆): 1.17 (6H, t, J = 7.93), 3.67 (3H, s), 6.16 (4H, q, J = 7.93), 6.65-6.91 (4H, m).

### Reference example 3

### 2,2'-[(2-Methoxyphenyl)imino]diethanol

To a suspension of lithium aluminium hydride (29.6 g, 0.779 mol) in tetrahydrofuran (THF, 100 mL) was slowly added a solution of ethyl N-(2-ethoxy-2-oxoethoxy)-N-(2-methoxyphenyl)glycinate (115 g, 0.389 mol) in tetrahydrofuran (THF, 500 mL) at room temperature (15°C - 20°C) over 1 hour, and then the reaction mixture was stirred at the same temperature for 2 hours. Thereto 14.5 N aqueous ammonia (76 mL) was added over 30 minutes in an ice bath, tetrahydrofuran (500 mL) was added, and then furthermore 14.5 N aqueous ammonia (153 mL) was added to quench the reaction. Consequently the precipitated salt was filtrated through Celite®. The solvent was removed off in vacuo to give 71.3 g of the title compound as a colorless oil.
¹H-NMR δ (DMSO-d₆): 3.16 (4H, t, J = 6.60), 3.40 (4H, t, J = 4.77, 6.60), 3.74 (3H, s), 4.40 (2H, t, J = 4.77), 6.80-6.98 (4H, m).

### Reference example 4

### N,N-Bis(2-chloroethyl)-2-methoxyaniline

To a solution of 2,2'-[(2-methoxyphenyl)imino]-diethanol (70.0 g, 331 mmol) and triethylamine (102 ml, 729 mmol) in dichloromethane (CH₂Cl₂, 100 mL) was added methanesulfonyl chloride (56.4 ml, 729 mmol) in an ice bath, and then the reaction mixture was stirred at room temperature for 2 hours. Consequently, the mixture was heated under reflux for 5 hours, and then the solvent was removed off in vacuo. The reactant was extracted with ethyl acetate, and the organic layer was washed with water twice and saturated aqueous sodium hydrogencarbonate once, dried over anhydrous magnesium sulfate, filtrated, and the solvent was removed off in vacuo. The residue was purified by silica gel chromatography to give 75.8 g of the title compound as a colorless oil.
¹H-NMR δ (DMSO-d₆): 3.45 (4H, t, J = 6.78), 3.57 (4H, t, J = 6.78), 3.78 (3H, s), 6.84-7.01 (4H, m).

### Reference example 5

### Dimethyl 3-phenylpentanedioate

To a solution of 3-phenylglutaric acid (5.00 g, 24.0 mmol) and potassium carbonate (16.6 g, 120 mmol) in N,N-dimethylformamide (DMF, 200 mL) was added methyl iodide (3.29 ml, 52.8 mmol) at room temperature, and then the reaction mixture was stirred at 35°C for 4 hours. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was crystallized with hexane to give 4.18 g of the title compound as a white crystal.
¹H-NMR δ (DMSO-d₆) : 2.63 (2H, dd, J = 8.43, 15.7), 2.75 (2H, dd, J = 8.43, 15.7), 3.41-3.50 (1H, m), 3.50 (6H, s), 7.16-7.30 (5H, m).

### Reference example 6

### 3-Phenylpentane-1,5-diol

To a suspension of lithium aluminium hydride (1.26 g, 33.9 mol) in tetrahydrofuran (THF, 100 mL) was slowly added a solution of diethyl 3-phenylpentanedioate (2.00 g, 8.47 mol) in tetrahydrofuran (THF, 10 mL) at room temperature, and then the reaction mixture was stirred at the same temperature overnight. Under ice-cooling, 10% (W/W) aqueous solution of Rochelle salt (48 mL) was added thereto to quench the reaction, and then the precipitated salt was filtrated with Celite®. The solvent was removed off in vacuo to give 1.67 g of the title compound as a colorless oil.
¹H-NMR δ (DMSO-d₆): 1.58-1.81 (4H, m), 2.72-2.82 (4H, m), 3.11-3.26 (4H, m), 4.32 (2H, t, J = 4.95), 7.13-7.28 (5H, m).

### Reference example 7

### [3-Bromo-1-(2-chloroethyl)propyl]benzene

To a solution of 3-phenylpentane-1,5-diol (470 mg, 2.61 mmol) and triethylamine (0.836 ml, 6.00 mmol) in dichloromethane (CH₂Cl₂, 30 mL) was added methanesulfonyl chloride (0.444 ml, 5.74 mmol) at room temperature, and then the reaction mixture was stirred at the same temperature for 2 hours. Water was added thereto to quench the reaction, and then the mixture was extracted with ethyl acetate. The organic layer was washed with saturated aqueous ammonium chloride twice and saturated aqueous sodium hydrogencarbonate once, dried over anhydrous magnesium sulfate, filtrated, and then the solvent was removed off in vacuo. The resulting reaction mixture was dissolved in N,N-dimethylformamide (DMF, 30 mL). Lithium bromide (907 mg, 10.4 mmol) was added thereto at room temperature and then the reaction mixture was heated to 60°C and stirred for 3 hours under heating. Water was added thereto to quench the reaction, and then the mixture was extracted with ethyl acetate. The organic layer was washed with water, dried over anhydrous magnesium sulfate, filtrated, and then the solvent was removed off in vacuo. The residue was purified by silica gel chromatography to give 630 mg of the title compound as a colorless oil.
¹H-NMR δ (DMSO-d₆): 2.16 (4H, dt, J = 7.14, 7.14), 2.98 (1H, tt, J = 7.35, 7.35), 3.13 (2H, dd, J = 7.86), 3.37 (2H, dd, J = 7.86), 7.22-7.36 (5H, m).

### Reference example 8

### N-(3-Chloropropyl)-2-methoxyaniline

To a suspension of o-anisidine (3.00 ml, 26.6 mmol) and potassium carbonate (11.0 g, 79.8 mmol) in N,N-dimethylformamide (DMF, 50 mL) was added 1-bromo-3-chloropropane (2.37 ml, 23.9 mmol) at room temperature, and then the mixture was stirred at 55°C for 5 hours. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 2.00 g of the title compound as a colorless oil.
¹H-NMR δ (DMSO-d₆): 1.99 (2H, t, J = 6.57), 3.17 (2H, t, J = 6.57), 3.70 (2H, t, J = 6.57), 3.75 (3H, s), 4.91 (1H, t, J = 5.85) 6.50-6.56 (2H, m), 6.73-6.80 (2H, m).

### Reference example 9

### 2-Chloro-N-(3-chloropropyl)-N-(2-methoxyphenyl)acetamide

To a solution of N-(3-chloropropyl)-2-methoxyaniline (1.00 g, 5.00 mmol) and triethylamine (0.906 ml, 6.50 mmol) in dichloromethane (CH₂Cl₂, 20 mL) in an ice bath was added chloroacetyl chloride (0.478 ml, 6.00 mmol), and then the reaction mixture was stirred overnight at room temperature. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 1.27 g of the title compound as a colorless oil.
¹H-NMR δ (DMSO-d₆): 2.01 (2H, m), 3.56 (2H, m), 3.76-3.82 (4H, m), 3.86 (3H, s), 6.99-7.05 (2H, m) 7.18-7.21 (1H, m), 7.36-7.42 (1H, m).

### Reference example 10

### N-(2-Chloroethyl)-N-(3-chloropropyl)-2-methoxyaniline

To a solution of 2-chloro-N-(3-chloropropyl)-N-(2-methoxyphenyl)acetamide (847 mg, 3.07 mmol) in tetrahydrofuran (THF, 20 mL) in an ice bath was added a solution of borane-tetrahydrofuran in tetrahydrofuran (1.13 M, 4.60 mmol), and then the reaction mixture was stirred overnight at room temperature. Methanol was added thereto to quench the reaction, and then the solvent was removed off in vacuo. The mixture was extracted with ethyl acetate, the organic layer was washed with saturated aqueous ammonium chloride twice. The organic layer was dried over anhydrous magnesium sulfate, filtrated, and the solvent was removed off in vacuo. The residue was purified by silica gel chromatography to give 370 mg of the title compound as a colorless oil.
¹H-NMR δ (DMSO-d₆): 1.79 (2H, tt, J = 6.78, 6.78), 3.25 (2H, t, J = 6.78), 3.34 (2H, t, J = 6.78), 3.58 (2H, t, J = 6.78), 3.77 (3H, s) 6.82-6.88 (1H, m), 6.94-7.00 (3H, m).

### Reference example 11

### N,N-Bis(2-chloroethyl)aniline

To a solution of N-phenyldiethylamine (10.0 g, 5.52 mmol) in toluene (200 mL) was added thionyl chloride (9.70 ml, 132 mmol) at room temperature, and then the reaction mixture was heated to 100 °C and stirred for 1 hour. Saturated aqueous sodium hydrogencarbonate was added thereto to quench the reaction, and then the mixture was extracted with diethyl ether twice. The organic layer was washed with water twice, dried over anhydrous magnesium sulfate, filtrated, and then the solvent was removed off in vacuo. The residue was purified by silica gel chromatography to give 5.86 g of the title compound as a colorless oil.
¹H-NMR δ (DMSO-d₆): 3.60-3.66 (4H, m), 3.71-3.76 (4H, m), 6.68-6.71 (2H, m), 6.75-6.80 (1H, m), 7.24-7.29 (2H, m).

### Reference example 12

### Preparation of 1-(3-methoxyphenyl)-4-oxocyclohexane-carbonitrile

To a suspension of sodium hydride (1.37 g, 31.5 mmol) in N,N-dimethylformamide (DMF, 50 mL) in an ice bath was added a solution of methyl acrylate (3.22 ml, 35.8 mmol) and (3-methoxyphenyl)acetonitrile (2.00 ml, 14.3 mmol) in N,N-dimethylformamide (DMF, 10 mL). The reaction mixture was warmed to room temperature and stirred for 3 hours, and then saturated aqueous ammonium chloride was added thereto to quench the reaction. The mixture was extracted with ethyl acetate, and the organic layer was washed with saturated aqueous ammonium chloride once, dried over anhydrous magnesium sulfate, filtrated, and then the solvent was removed off in vacuo. The residue was purified by silica gel chromatography to give 3.29 g as a colorless oil. The colorless oil (2.29 g, 7.97 mmol) was dissolved in a 1,4-dioxane (100 mL) - water (20 mL) solution, potassium hydroxide (984 mg, 17.5 mmol) was added thereto, and the solution was heated under reflux for 4 hours. Saturated aqueous ammonium chloride was added thereto to quench the reaction, and then the mixture was extracted with ethyl acetate. The organic layer was washed with saturated aqueous ammonium chloride twice, dried over anhydrous magnesium sulfate, filtrated, and the solvent was removed off in vacuo. The residue was purified by silica gel chromatography to give 1.07 g of the title compound as a colorless oil.
¹H-NMR δ (DMSO-d6); 2.34-2.50 (8H, m), 2.64-2.77 (2H, m), 3.78 (3H, s), 6.94-6.98 (1H, m), 7.14-7.20 (2H, m), 7.35-7.40 (1H, m).

### Reference example 13

### Preparation of 1-(3-methoxyphenyl)-4-oxocyclohexane-carboxylic acid

### Preparation of dimethyl 4-hydroxy-1-(3-methoxyphenyl)-cyclohex-3-ene-1,3-dicarboxylate

To a solution of sodium hydride (13.2 g, 303.6 mmol) in dimethylformamide (350 mL) was added methyl 3-methoxyphenylacetate (25 g, 138 mmol) and methyl acrylate (31 ml, 345 mmol) in dimethylformamide (160 mL) at 0°C, then the reaction mixture was stirred at room temperature for 3 hours. The reaction mixture was poured into aqueous hydrochloric acid and extracted with ethyl acetate. The organic layer was washed with brine, dried over anhydrous magnesium sulfate, filtrated, and concentrated in vacuo. The residue was purified by silica gel chromatography to give 42.8 g of the title compound as a colorless oil.
High-performance liquid chromatography/mass spectrometry
m/z 321(M+H)
Retention time: 3.61 min.

### Preparation of 1-(3-methoxyphenyl)-4-oxocyclohexane-carboxylic acid

To a solution of dimethyl 4-hydroxy-1-(3-methoxyphenyl)cyclohex-3-ene-1,3-dicarboxylate (31 g, 96.7 mmol) in dioxane (260 mL) was added a solution of potassium hydroxide (21.7 g, 387 mmol) in water (260 mL), and then the reaction mixture was stirred under reflux for 4 hours. The reaction mixture was poured to aqueous hydrochloric acid and extracted with ethyl acetate. The organic layer was washed with brine, dried over anhydrous magnesium sulfate, filtrated, and concentrated in vacuo. The residue was purified by silica gel chromatography to give 8.57 g of the title compound as a white solid.
High-performance liquid chromatography/mass spectrometry
m/z 249 (M+H)
Retention time: 2.65 min.

### Reference example 14

### Preparation of methyl 1-(3-methoxyphenyl)-4-oxocyclohexane-carboxylate

To a solution of 1-(3-methoxyphenyl)-4-oxocyclohexane-carboxylic acid (4 g, 16.51 mmol) in dimethylformamide (40 mL) was added methyl iodide (1.5 ml, 48,3 mmol), and then the mixture was stirred at room temperature for 22 hours. The reaction mixture was poured to water, then saturated aqueous ammonium chloride was added thereto, and the mixture was extracted with ethyl acetate. The organic layer was washed with brine, dried over anhydrous magnesium sulfate, and filtrated. The filtrate was concentrated in vacuo, and the residue was purified by crystallization and with a silica gel chromatography to give 3.88 g of the title compound as a white solid.
High-performance liquid chromatography/mass spectrometry
m/z 263 (M+H)
Retention time: 3.37 min.

### Example 1-1

### Preparation of 1-(4-fluorophenyl)-4-(3-methoxyphenyl)-piperidine-4-carbonitrile

To a solution of 4-hydroxy-2-(2-hydroxyethyl)-2-(3-methoxyphenyl)butanenitrile (70 mg, 0.195 mmol) in acetonitrile (6 mL), trifluoromethanesulfonic anhydride (0.11 ml, 0.626 mmol) and triethylamine (0.080 ml, 0.626 mmol) were successively added at -30 to -20°C, and then the mixture was stirred for 15 minutes under the same condition. To the reaction mixture at -30 to -20°C, 4-fluoroaniline (0.037 ml, 0.387 mmol) and triethylaniline (0.066 ml, 0.387 mmol) were added, and the mixture was gradually warmed to room temperature and then stirred for 2 hours. Water was added thereto to quench the reaction, and then the mixture was extracted with ether. The organic layer was washed with water twice, dried over anhydrous magnesium sulfate, filtrated, and then concentrated in vacuo. The residue was purified by preparative thin-layer chromatography (preparative TLC) to give 53.5 mg of the title compound as a colorless oil.
¹H-NMR δ (DMSO-d₆) : 2.12-2.27 (4H, m), 2.88-2.97 (2H, m), 3.72-3.78 (5H, m), 6.93-6.97 (1H, m), 7.03-7.15 (6H, m), 7.35-7.40 (1H, m).

### Example 1-2

### Preparation of 1-benzhydryl-4-(3-methoxyphenyl)piperidine-4-carbonitrile

The title compound was prepared in a similar manner to Example 1-1.
¹H-NMR δ (DMSO-d₆): 2.08 (4H, m), 2.18-2.26 (2H, m), 2.88-2.93 (2H, m), 3.78 (3H, s), 4.45 (1H, s), 6.91-6.94 (1H, m), 7.03-7.05 (1H, m), 7.09-7.12 (1H, m), 7.16-7.21 (2H, m), 7.27-7.37 (5H, m), 7.44-7.47 (4H, m).

### Example 1-3

### Preparation of 1-(2-methoxyphenyl)-4-(2-methoxyphenyl)-piperidine-4-carbonitrile

The title compound was prepared in a similar manner to Example 1-1.
¹H-NMR δ (DMSO-d₆): 2.02-2.12 (2H, m), 2.42 (2H, d, J = 12.3), 2.93 (2H, t, J = 11.7), 3.49 (2H, t, J = 12.3), 3.77 (3H, s), 3.86 (3H, s), 6.86-7.04 (5H, m), 7.15 (1H, d, J = 7.89), 7.36-7.40 (3H, m).

### Example 1-4

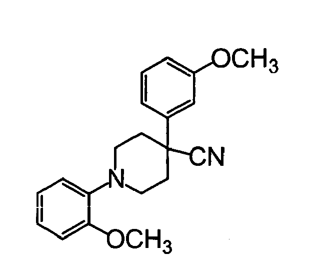

### Preparation of 1-(2-methoxyphenyl)-4-(3-methoxyphenyl)-piperidine-4-carbonitrile

The title compound was prepared in a similar manner to Example 1-1.
¹H-NMR δ (DMSO-d₆): 2.16-2.20 (4H, m), 2.85-2.93 (2H, m), 3.49-3.53 (2H, m), 3.79 (6H, m), 6.87-7.02 (5H, m), 7.08-7.17 (2H, m), 7.35-7.40 (1H, m).

### Example 1-5

### Preparation of 1-(2-methoxyphenyl)-4-(4-methoxyphenyl)-piperidine-4-carbonitrile

The title compound was prepared in a similar manner to Example 1-1.
¹H-NMR δ (DMSO-d₆): 2.06-2.22 (4H, m), 2.84-2.91 (2H, m), 3.50 (2H, d, J = 12.1), 3.78 (3H, s), 3.81 (3H, s), 6.87-7.01 (6H, m), 7.47-7.51 (2H, m).

### Example 1-6

### Preparation of 1-(2-methoxyphenyl)-4-(3-fluorophenyl)-piperidine-4-carbonitrile

The title compound was prepared in a similar manner to Example 1-1.
¹H-NMR δ (DMSO-d₆): 2.13-2.27 (4H, m), 2.85-2.92 (2H, m), 3.49-3.53 (2H, m), 3.78 (3H, s), 6.91-7.02 (3H, m), 7.20-7.26 (2H, m), 7.42-7.55 (4H, m).

### Example 1-7

### Preparation of 1-(2-methoxyphenyl)-4-(3-trifluoromethylphenyl)piperidine-4-carbonitrile

The title compound was prepared in a similar manner to Example 1-1.
¹H-NMR δ (DMSO-d₆): 2.07-2.19 (4H, m), 2.87-2.94 (2H, m), 3.51-3.56 (2H, m), 3.79 (3H, s), 6.92-7.03 (3H, m), 7.68-7.96 (5H, m).

### Example 1-8

### Preparation of 1-(2-methoxyphenyl)-4-(3-benzyloxyphenyl)-piperidine-4-carbonitrile

The title compound was prepared in a similar manner to Example 1-1.
¹H-NMR δ (DMSO-d₆): 1.98-2.08 (4H, m), 2.80-2.90 (2H, m), 3.48-3.52 (2H, m), 3.78 (3H, s), 5.13 (1H, s), 5.15 (1H, s), 6.96-7.18 (7H, m), 7.33-7.46 (6H, m).

### Example 1-9

### Preparation of 1-(diphenylmethyl)-4-[3-(trifluoromethoxy)-phenyl]piperidine-4-carbonitrile

The title compound was prepared in a similar manner to Example 1-1.
High-performance liquid chromatography/mass spectrometry
m/z 437 (M+H)
Retention time: 3.34 min.

### Example 1-10

### Preparation of 4-[3-(benzyloxy)phenyl]-1-(2-methoxyphenyl)-piperidine-4-carbonitrile

The title compound was prepared in a similar manner to Example 1-1.
High-performance liquid chromatography/mass spectrometry
m/z 399 (M+H)
Retention time: 3.74 min.

### Example 2

### Preparation of 1-(2-hydroxyphenyl)-4-(3-methoxyphenyl)-piperidine-4-carbonitrile

To a solution of 2-aminophenol (2.00 g, 18.3 mmol) and triethylamine (3.32 ml, 23.8 mmol) in N,N-dimethylformamide (DMF, 100 mL) in an ice bath, trimethylsilyl chloride (2.79 ml, 22.0 mmol) was added, and then the mixture was warmed to room temperature and stirred for 2 hours. Water was added thereto to quench the reaction, and then the mixture was extracted with diethyl ether. The organic layer was washed with water twice, dried over anhydrous magnesium sulfate, filtrated, and then concentrated in vacuo to give 3.31 g of a reaction mixture.

Besides, to a solution of 4-hydroxy-2-(2-hydroxyethyl)-2-(3-methoxyphenyl)butanenitrile (400 mg, 1.70 mmol) in acetonitrile (50 mL), trifluoromethanesulfonic anhydride (0.629 ml, 3.74 mmol) and triethylamine (0.521 ml, 3.74 mmol) were successively added at -30 to -20°C, and then the mixture was stirred for 15 minutes under the same condition. Thereto a part (400 mg) of the above-obtained reaction mixture (3.31 g) and triethylaniline (0.616 ml, 4.42 mmol) were added at -30 to -20°C, and the mixture was gradually warmed to room temperature and then stirred for 1 hour. Water was added thereto to quench the reaction, the mixture was extracted with diethyl ether. The organic layer was washed with water once, dried over anhydrous magnesium sulfate, filtrated, then and concentrated in vacuo to give 625 mg of a reaction mixture. Then, the reaction mixture was dissolved in tetrahydrofuran (20 mL). Thereto tetrabutyl ammonium fluoride (943 mg, 3.61 mmol) was added at room temperature, and the mixture was stirred for 3 hours under the same condition. Saturated aqueous ammonium chloride was added thereto to quench the reaction, and then the mixture was extracted with ethyl acetate. The organic layer was washed with saturated aqueous ammonium chloride once and saturated aqueous sodium hydrogencarbonate once, dried over anhydrous magnesium sulfate, and then concentrated in vacuo. The residue was purified by crystallization with diethyl ether to give 255 mg of the title compound as a light brown powder.
High-performance liquid chromatography/mass spectrometry
m/z 309.6 (M+H)
Retention time: 2.92 min.

### Example 3

### Preparation of 1-(2-benzyloxyphenyl)-4-(3-methoxyphenyl)-piperidine-4-carbonitrile

To a suspension of 1-(2-hydroxyphenyl)-4-(3-methoxyphenyl)piperidine-4-carbonitrile (155 mg, 0.503 mmol) and potassium carbonate (209 mg, 1.51 mmol) in N,N-dimethylformamide (DMF, 10 mL), benzyl bromide (0.078 ml, 0.654 mmol) was added at room temperature, and then the mixture was stirred for 4 hours under the same condition. Water was added thereto to quench the reaction, and then the mixture was extracted with ethyl acetate. The organic layer was washed with water twice, dried over anhydrous magnesium sulfate, filtrated, and then concentrated in vacuo. The residue was purified by silica gel chromatography to give 184 g of the title compound as an amorphous.
¹H-NMR δ (DMSO-d₆): 2.09-2.25 (4H, m), 2.90-2.97 (2H, m), 3.56-3.60 (2H, m), 3.79 (3H, s), 5.12 (2H, s), 6.92-7.13 (7H, m), 7.29-7.49 (6H, m).

### Example 4

### 1-[2-(2-methoxyethoxy)phenyl]-4-(3-methoxyphenyl)-piperidine-4-carbonitrile

To a suspension of sodium hydride (31.2 mg, 0.779 mmol) in N,N-dimethylformamide (DMF, 20 mL) was added 1-(2-hydroxyphenyl)-4-(3-methoxyphenyl)piperidine-4-carbonitrile (200 mg, 0.649 mmol) at room temperature followed by 2-bromoethylmethylether (0.073 ml, 0.779 mmol), and then the mixture was stirred for 4 hours under the same condition. Saturated aqueous ammonium chloride was added thereto to quench the reaction, and then the mixture was extracted with ethyl acetate. The organic layer was washed with saturated aqueous ammonium chloride twice and saturated aqueous sodium hydrogencarbonate once, dried over anhydrous magnesium sulfate, filtrated, and then concentrated in vacuo. The residue was purified by silica gel chromatography to give 221 mg of the title compound as a colorless oil.
High-performance liquid chromatography/mass spectrometry
m/z 367.3 (M+H)
Retention time: 3.01 min.

### Example 5

### Preparation of 4-(3-methoxyphenyl)piperidine-4-carbonitrile

A suspension of 1-benzhydryl-4-(3-methoxyphenyl)-piperidine-4-carbonitrile (34.3 mg, 0.090 mmol), acetic acid (0.005 ml, 0.090 mmol) and 10% palladium hydroxide (3.4 mg) in methanol (20 mL) was stirred under hydrogen atmosphere for 3 hours. The reaction mixture was filtrated through Celite® , and the solvent was removed from the filtrate. To the residue, saturated aqueous sodium hydrogencarbonate was added, and the mixture was extracted with ethyl acetate. The ethyl acetate layer was washed with saturated sodium hydrogencarbonate once, dried over anhydrous magnesium sulfate, filtrated, and then concentrated in vacuo. The residue was purified by preparative TLC to give 12.9 mg of the title compound as an oil.
¹H-NMR δ (DMSO-d₆):1.86-2.06 (4H, m), 2.79-2.87 (2H, m), 3.07-3.11 (2H, m), 3.77 (3H, s), 6.92-6.96 (1H, m), 7.00-7.08 (2H, m), 7.33-7.39 (1H, m).

### Example 6-1

### Preparation of 1-(3-pyridyl)-4-(3-methoxyphenyl)piperidine-4-carbonitrile

To a solution of tris(dibenzylidene acetone)-dipalladium (Pd₂(dba)₃, 14.0 mg, 10 mol%) and 2,2'-bis-(diphenylphosphino)-1,1'-binaphthyl (BINAP, 17.0 mg, 20 mol%) in tetrahydrofuran (THF, 100 mL) were added 3-iodopyridine (34 mg, 0.166 mmol), 4-(3-methoxyphenyl)-piperidine-4-carbonitrile (30 mg, 0.139 mmol) and sodium tert-butoxide (47 mg, 0.417 mmol) at room temperature, and then the mixture was heated under reflux for 5 hours. Brine was added thereto to quench the reaction, and the mixture was extracted with ethyl acetate. The organic layer was washed with brine twice, dried over anhydrous magnesium sulfate, filtrated, and then concentrated in vacuo. The residue was purified by preparative TLC to give 28.0 mg of the title compound as an oil.
High-performance liquid chromatography/mass spectrometry
m/z 294.1 (M+H)
Retention time: 2.96 min.

### Example 6-2

### Preparation of 1-(4-pyridyl)-4-(3-methoxyphenyl)piperidine-4-carbonitrile

The title compound was prepared in a similar manner to Example 6-1.
High-performance liquid chromatography/mass spectrometry
m/z 294.1 (M+H)
Retention time: 2.86 min.

### Example 6-3

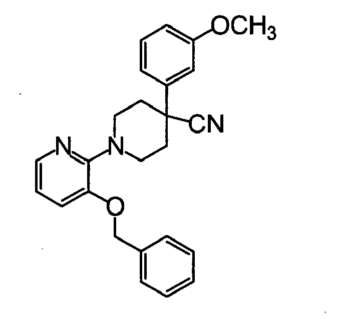

### 1-[3-(Benzyloxy)pyridin-2-yl]-4-(3-methoxyphenyl)-piperidine-4-carbonitrile

### Preparation of 3-(benzyloxy)-2-bromopyridine

To a solution of 2-bromo-3-pyridinol (1.74 g, 10.0 mmol) and potassium carbonate (4.15 g, 30.0 mmol) in N,N-dimethylformamide (DMF, 150 mL), benzyl bromide (1.49 ml, 12.5 mmol) was added at room temperature, and the mixture was warmed to 55 °C and then stirred for 3 hours. Water was added thereto to quench the reaction, and then the mixture was extracted with ethyl acetate. The organic layer was washed with water twice, dried over anhydrous magnesium sulfate, filtrated, and then concentrated in vacuo. The residue was purified by silica gel chromatography to give 2.61 g of the title compound as an oil.
¹H-NMR δ (DMSO-d₆):7.33-7.49 (6H, m), 7.58-7.61 (1H, m), 7.95-7.98 (1H, m).

The title compound was prepared using the obtained 3-(benzyloxy)-2-bromopyridine in a similar manner to Example 6-1.
High-performance liquid chromatography/mass spectrometry
m/z 400.3 (M+H)
Retention time: 3.17 min.

### Example 6-4

### 1-[3-(Methoxy)pyridin-2-yl]-4-(3-methoxyphenyl)piperidine-4-carbonitrile

The title compound was prepared in a similar manner to Example 6-1.
High-performance liquid chromatography/mass spectrometry
m/z 324.1 (M+H)
Retention time: 2.48 min.

### Example 6-5

### 1-[2-Methoxy-5-fluorophenyl]-4-(3-methoxyphenyl)piperidine-4-carbonitrile

The title compound was prepared in a similar manner to Example 6-1.
High-performance liquid chromatography/mass spectrometry
m/z 340.9 (M+H)
Retention time: 3.76 min.

### Example 6-6

### 1-[2-Benzyloxy-5-fluorophenyl]-4-(3-methoxyphenyl)-piperidine-4-carbonitrile

The title compound was prepared using 2-bromo-4-fluorophenol in a similar manner to Example 6-3.
¹H-NMR δ (DMSO-d₆): 2.10-2.24 (4H, m), 2.89-2.96 (2H, m), 3.06-3.65 (2H, m), 5.09 (2H, s), 6.74-7.13 (6H, m), 7.30-7.47 (6H, m).

### Example 7-1

### 4-(3-methoxyphenyl)-1-pyrimidin-2-ylpiperidine-4-carbonitrile

To a suspension of sodium hydride (18.5 mg, 0.555 mmol) in dimethylsulfoxide (DMSO, 15 mL) was added 4-(3-methoxyphenyl)piperidine-4-carbonitrile (200 mg, 0.462 mmol) at room temperature followed by 2-bromopyrimidine (88.2 mg, 0.555 mmol) at room temperature, and the mixture was stirred overnight under the same condition. Water was added thereto to quench the reaction, and then the mixture was extracted with ethyl acetate. The organic layer was washed with water twice, dried over anhydrous magnesium sulfate, filtrated, and then concentrated in vacuo. The residue was purified by preparative TLC to give 55.8 mg of the title compound as a colorless oil.
High-performance liquid chromatography/mass spectrometry
m/z 295.3 (M+H)
Retention time: 3.42 min.

### Example 7-2

### 1-(1,3-Benzoxazol-2-yl)-4-(3-methoxyphenyl)piperidine-4-carbonitrile

The title compound was prepared in a similar manner to Example 7-1.
High-performance liquid chromatography/mass spectrometry
m/z 334.5 (M+H)
Retention time: 3.51 min.

### Example 7-3

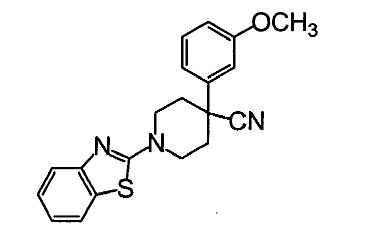

### 1-(1,3-Benzothiazol-2-yl)-4-(3-methoxyphenyl)piperidine-4-carbonitrile

The title compound was prepared in a similar manner to Example 7-1.
High-performance liquid chromatography/mass spectrometry
m/z 350.4 (M+H)
Retention time: 3.53 min.

### Example 8

### 4-(3-Methoxyphenyl)-1-(2,2,2-trifluoroethyl)piperidine-4-carbonitrile

To a solution of 4-(3-methoxyphenyl)piperidine-4-carbonitrile (250 mg, 1.162 mmol) and triethylamine (0.243 ml, 17.4 mmol) in dichloromethane (CH₂Cl₂, 15 mL) in an ice bath, 2,2,2-trifluoroethyl trifluoromethanesulfonate (322 mg, 1.39 mmol) was added, and the mixture was warmed to room temperature and stirred overnight. Furthermore, 2,2,2-trifluoroethyl trifluoromethanesulfonate (322 mg, 1.39 mmol) was added to the reaction mixture in an ice bath, and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 315 mg of the title compound as a colorless oil.
High-performance liquid chromatography/mass spectrometry
m/z 299.2 (M+H)
Retention time: 3.67 min.

### Example 9

### 1-Benzyl-4-(3-methoxyphenyl)piperidine-4-carbonitrile

To a solution of 4-(3-methoxyphenyl)piperidine-4-carbonitrile (1.00 g, 4.62 mmol) and potassium carbonate (1.92 g, 13.9 mmol) in N,N-dimethylformamide (DMF, 20 mL), benzyl bromide (0.665 ml, 5.09 mmol) was added at room temperature, and the mixture was stirred at the same temperature overnight. Water was added thereto to quench the reaction, and then the reaction mixture was extracted with ethyl acetate. The organic layer was washed with water twice, dried over anhydrous magnesium sulfate, filtrated, and then concentrated in vacuo. The residue was purified by silica gel chromatography to give 1.21 g of the title compound as a colorless oil.
¹H-NMR δ (DMSO-d₆):1.97-2.11 (4H, m), 2.27-2.35 (2H, m), 2.91-2.95 (2H, m), 3.55 (2H, s), 3.77 (3H, s), 6.90-6.94 (1H, m), 7.02-7.04 (1H, m), 7.08-7.11 (1H, m), 7.24-7.37 (6H, m).

### Example 10-1

### 1-(3-Methoxyphenyl)-4-phenylcyclohexanecarbonitrile

To a suspension of sodium hydride (100 mg, 2.62 mmol) in dimethylsulfoxide (DMSO, 50 mL), a solution of [3-chloro-1-(2-chloroethyl)propyl]benzene (400 mg, 1.31 mmol) and (3-methoxyphenyl)acetonitrile (0.182 ml, 1.28 mmol) in dimethylsulfoxide (DMSO, 5 mL) was added at room temperature, and the mixture was stirred under the same condition overnight. Saturated aqueous ammonium chloride was added thereto to quench the reaction, and then the mixture was extracted with ethyl acetate. The organic layer was washed with saturated aqueous ammonium chloride once and saturated aqueous sodium hydrogencarbonate once, dried over anhydrous magnesium sulfate, filtrated, and then concentrated in vacuo. The residue was purified by preparative TLC to give 305 mg of the title compound as a colorless oil.
¹H-NMR δ (DMSO-d₆):1.77-2.20 (8H, m), 2.67-2.76 (1H, m), 3.77 (3H, s), 6.92-6.97 (1H, m), 7.07-7.39(8H, m).

### Example 10-2

### Preparation of 1-phenyl-4-(3-methoxyphenyl)piperidine-4-carbonitrile

The title compound was prepared using the compound of Reference Example 11 in a similar manner to Example 10-1. ¹H-NMR δ (DMSO-d₆): 2.21-2.25 (4H, m), 3.19-3.28 (2H, m), 3.72-3.88 (2H, m), 3.84 (3H, s), 6.86-6.94 (2H, m), 6.98-7.02 (2H, m), 7.09-7.13 (2H, m), 7.26-7.37 (3H, m).

### Example 10-3

### Preparation of 1-(2-methoxyphenyl)-4-(3-methoxyphenyl)-piperidine-4-carbonitrile

The title compound was prepared using the compound of Reference Example 4 in a similar manner to Example 10-1. ¹H-NMR δ (DMSO-d₆):2.16-2.20 (4H, m), 2.85-2.93 (2H, m), 3.49-3.53 (2H, m), 3.79 (6H, m), 6.87-7.02 (5H, m) , 7.08-7.17 (2H, m), 7.35-7.40 (1H, m).

### Example 10-4

### 1-(2-Methoxyphenyl)-4-(3-methoxyphenyl)azepane-4-carbonitrile

The title compound was prepared using the compound of Reference Example 10 in a similar manner to Example 10-1. ¹H-NMR δ (DMSO-d₆): 2.15-2.60 (6H, m), 3.21-3.62 (4H, m), 3.83 (6H, s), 6.83-7.03 (5H, m), 7.09-7.16 (2H, m), 7.28-7.34.

### Example 10-5

### Preparation of 1-(2-methoxyphenyl)-4-(1-naphthyl)-piperidine-4-carbonitrile

The title compound was prepared using the compound of Reference Example 4 in a similar manner to Example 10-1. High-performance liquid chromatography/mass spectrometry
m/z 343.2 (M+H)
Retention time: 3.32 min.

### Example 10-6

### Preparation of 1-(2-methoxyphenyl)-4-(3-phenoxyphenyl)-piperidine-4-carbonitrile

The title compound was prepared using the compound of Reference Example 4 in a similar manner to Example 10-1. High-performance liquid chromatography/mass spectrometry
m/z 385.5 (M+H)
Retention time: 3.65 min.

### Example 10-7

### Preparation of 4-(3-bromophenyl)-1-(2-methoxyphenyl)-piperidine-4-carbonitrile

The title compound was prepared using the compound of Reference Example 4 in a similar manner to Example 10-1. High-performance liquid chromatography/mass spectrometry
m/z 371.2 (M+H)
Retention time: 3.42 min.

### Example 10-8

### Preparation of 1-(2-methoxyphenyl)-4-(3-methylphenyl)-piperidine-4-carbonitrile

The title compound was prepared using the compound of Reference Example 4 in a similar manner to Example 10-1. High-performance liquid chromatography/mass spectrometry
m/z 307.3 (M+H)
Retention time: 3.21 min.

### Example 10-9

### Preparation of 4-biphenyl-3-yl-1-(2-methoxyphenyl)-piperidine-4-carbonitrile

A suspension of 4-(3-bromophenyl)-1-(2-methoxyphenyl)-piperidine-4-carbonitrile (28 mg, 0.075 mmol), phenylboronic acid (11 mg, 0.09 mmol), tetrakistriphenylphosphine palladium (231 mg, 25mol%) and cesium carbonate (74.0 mg, 25 mol%) in 1,4-dioxane (DMF, 1.5 mL) was heated under reflux for 10 hours. Water was added thereto to quench the reaction, and then the mixture was extracted with ethyl acetate. The organic layer was washed with water twice, dried over anhydrous magnesium sulfate, filtrated, and then concentrated in vacuo. The residue was purified by silica gel chromatography to give 114 mg of the title compound as a yellow oil.
High-performance liquid chromatography/mass spectrometry
m/z 369.2 (M+H)
Retention time: 3.61 min.

### Example 11-1

### Methyl 1-(2-methoxyphenyl)-4-(3-methoxyphenyl)piperidine-4-carboxylate

A suspension of 1-(2-benzyloxyphenyl)-4-(3-methoxyphenyl)piperidine-4-carbonitrile (1.00g, 3.24 mmol) in 48 % aqueous hydrobromic acid (20 mL) was heated under reflux for 12 hours. The solvent was removed from the reaction mixture in vacuo and the precipitated solid was washed with ethyl acetate. The obtained reaction mixture was dissolved in N,N-dimethylformamide (DMF, 20 mL), and methyl iodide (0.666 ml, 10.7 mmol) and potassium carbonate (5.38 g, 38.9 mmol) were added thereto at room temperature. Then, the reaction mixture was warmed to 55°C and stirred for 4 hours. Water was added thereto to quench the reaction, and the mixture was extracted with ethyl acetate. The organic layer was washed with water twice, dried over anhydrous magnesium sulfate, filtrated, and then concentrated in vacuo. The residue was purified by silica gel chromatography to give 990 mg of the title compound as a colorless oil.
¹H-NMR δ (DMSO-d₆): 1.96-2.02 (2H, m), 2.62-2.69 (6H, m), 3.60 (3H, s), 3.75 (3H, s), 3.76 (3H, s), 6.84-6.98 (7H, m), 7.26-7.31 (1H, m).

### Example 11-2

### Methyl 4-(3-methoxyphenyl)-1-(3-methoxypyridin-2-yl)-piperidine-4-carboxylate

The title compound was prepared in a similar manner to Example 11-1.
High-performance liquid chromatography/mass spectrometry
m/z 357.2 (M+H)
Retention time: 2.76 min.

### Example 11-3

### Methyl 4-(3-methoxyphenyl)-1-pyrimidin-2-ylpiperidine-4-carboxylate

4-(3-Methoxyphenyl)-1-pyrimidin-2-ylpiperidine-4-carbonitrile (70 mg, 0.238 mmol) was added to 12N aqueous hydrochloric acid (5 mL). The suspension was heated under reflux for 12 hours, and then the solvent was removed from the suspension in vacuo. The precipitated solid was washed with ethyl acetate. The obtained reaction mixture was dissolved in N,N-dimethylformamide (DMF, 10 mL), and then methyl iodide (0.0593 ml, 0.952 mmol) and potassium carbonate (263 mg, 1.90 mmol) was added thereto at room temperature. The reaction mixture was warmed to 35°C and then stirred for 4 hours. Water was added into the reaction mixture to quench the reaction, and the mixture was extracted with ethyl acetate. The organic layer was washed with water twice, dried over anhydrous magnesium sulfate, filtrated, and then concentrated in vacuo. The residue was purified by preparative TLC to give 30.4 mg of the title compound as a colorless oil.
High-performance liquid chromatography/mass spectrometry
m/z 328.3 (M+H)
Retention time: 3.24 min.

### Example 12

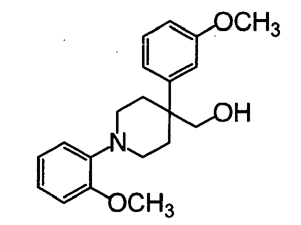

### [1-(2-Methoxyphenyl)-4-(3-methoxyphenyl)piperidin-4-yl]-methanol

To suspension of lithium aluminium hydride (51.4 mg, 1.35 mol) in tetrahydrofuran (THF, 30 mL) in an ice bath, a solution of methyl 1-(2-methoxyphenyl)-4-(3-methoxyphenyl)-piperidine-4-carboxylate (500 mg, 1.35 mol) in tetrahydrofuran (THF, 10 mL) was slowly added and then the mixture was warmed to room temperature and stirred overnight. 14.5N Aqueous ammonia (0.200 mL) was added to the reaction mixture in an ice bath to quench the reaction and then the precipitated salt was filtrated with Celite®. The solvent was removed from the filtrate in vacuo. The residue was purified by silica gel chromatography to give 388 mg of the title compound as a colorless oil. High-performance liquid chromatography/mass spectrometry
m/z 328.3 (M+H)
Retention time: 2.38 min.

### Example 13

### [1-(2-Methoxyphenyl)-4-(3-methoxyphenyl)piperidin-4-yl]methyl 4-methylbenzenesulfonate

To a solution of [1-(2-methoxyphenyl)-4-(3-methoxyphenyl)piperidin-4-yl]methanol (88.0 mg, 0.269 mmol) in pyridine (10 mL), para-toluenesulfonyl chloride (56.4 mg, 0.296 mmol) was added at room temperature, and then the mixture was warmed to 50°C and stirred for 4 hours. After evaporating the solvent in vacuo, the residue was extracted with ethyl acetate. The organic layer was washed with saturated aqueous ammonium chloride twice, dried over anhydrous magnesium sulfate, filtrated, and then concentrated in vacuo. The residue was purified by preparative TLC to give 33.3 mg of the title compound as a colorless oil.
High-performance liquid chromatography/mass spectrometry
m/z 482.4 (M+H)
Retention time: 3.11 min.

### Example 14

### [1-(2-Methoxyphenyl)-4-(3-methoxyphenyl)piperidin-4-yl]acetonitrile

A suspension of [1-(2-methoxyphenyl)-4-(3-methoxyphenyl)piperidin-4-yl]methyl 4-methylbenzenesulfonate (33.3 mg, 0.0690 mmol) and potassium cyanide (9.00 mg, 0.138 mmol) in dimethylsulfoxide (DMSO, 10 mL) was heated to 80°C and stirred for 4 hours. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by preparative TLC to give 20.1 mg of the title compound as a colorless oil.
High-performance liquid chromatography/mass spectrometry
m/z 337.6 (M+H)
Retention time: 3.07 min.

### Example 15

### 1-(2-Methoxyphenyl)-4-(3-methoxyphenyl)piperidine-4-carboxylic acid

To a solution of methyl 1-(2-methoxyphenyl)-4-(3-methoxyphenyl)piperidine-4-carboxylate (1.00 g, 4.62 mmol) in ethanol (5 mL), 2N aqueous lithium hydroxide (0.071 ml, 0.142 mmol) was added at room temperature, and the mixture was heated under reflux for 4 hours. Furthermore, thereto 2N aqueous lithium hydroxide (0.149 ml, 0.284 mmol) was added at room temperature and the mixture was heated under reflux for 4 hours. After removing off the solvent, the reaction mixture was suspended in 1,4-dioxane (3 mL) / water (2 mL) and further heated under reflux for 5 hours. Saturated aqueous ammonium chloride was added thereto to quench the reaction, and the mixture was extracted with ethyl acetate. The organic layer was washed with saturated aqueous ammonium chloride twice, dried over anhydrous magnesium sulfate, filtrated, and concentrated in vacuo. The residue was purified by crystallization with a mixture of ethyl acetate / hexane to give 35.2 mg of the title compound as a white crystal.
High-performance liquid chromatography/mass spectrometry
m/z 342.3 (M+H)
Retention time: 2.37 min.

### Example 16

### 1-(2-Methoxyphenyl)-4-(3-methoxyphenyl)piperidine-4-carboxamide

To a solution of 1-(2-methoxyphenyl)-4-(3-methoxyphenyl)piperidine-4-carboxylic acid (150 mg, 0.439 mmol) in dichloromethane (CH₂Cl₂, 10 mL) was added oxalyl chloride (0.0867 ml, 0.967 mmol) at room temperature followed by N,N-dimethylformamide (DMF, 1 drop), and the mixture was stirred at the same temperature for 4 hours. 14.5 N Aqueous ammonia (5.00 mL) was added to the reaction mixture in an ice bath to quench the reaction, and the mixture was warmed to room temperature and stirred for 1 hour. To the reaction mixture was added water, and then the mixture was extracted with ethyl acetate. The organic layer was washed with water twice, dried over anhydrous magnesium sulfate, filtrated, and then the solvent was removed off in vacuo. The residue was purified by crystallization with diethyl ether to give 96.8 mg of the title compound as a white crystal.
¹H-NMR δ (DMSO-d₆):1.88-1.95 (2H, m), 2.50-2.56 (2H, m), 2.71-2.78 (2H, m), 3.17-3.21 (2H, m), 3.74 (3H, s), 3.76 (3H, s), 6.78-7.00 (8H, m), 7.17 (1H, brd), 7.25 (1H, t, J = 8.04).

### Example 17

### 1-(2-Methoxyphenyl)-4-[3-(trifluoromethoxy)phenyl]-piperidine-4-carbonitrile

### Preparation of 4-[3-(trifluoromethoxy)phenyl]piperidine-4-carbonitrile

To a solution of 1-(diphenylmethyl)-4-[3-(trifluoro-methoxy)phenyl]piperidine-4-carbonitrile (24 2 mg, 0.554 mmol) in ethanol (7 mL), 10% palladium-carbon (48 mg) and ammonium formate (242 mg) were added, and then the mixture was stirred for two and half hours heating under reflux. The reaction mixture was filtrated and then the filtrate was concentrated in vacuo. The residue was purified by silica gel chromatography to give 94 mg of the title compound as a colorless oil.
High-performance liquid chromatography/mass spectrometry
m/z 271 (M+H)
Retention time: 2.80 min. (1 % - 99 %)

### 1-(2-Methoxyphenyl)-4-[3-(trifluoromethoxy)phenyl]-piperidine-4-carbonitrile

The title compound was prepared in a similar manner to Example 6-1.
¹H-NMR δ (CDCl₃) 2.19-2.24 (2H, m), 2.31-2.39 (2H, m), 3.09-3.15 (2H, m), 3.60-3.63 (2H, m), 3.89 (3H,s), 6.89-6.91 (1H, m), 6.94-6.98 (1H, m), 7.04-7.08 (2H, m), 7.21-7.24 (1H, m), 7.41-7.55 (3H, m).

### Example 18

### Preparation of 1-(2-methoxyphenyl)-4-(3-nitrophenyl)-piperidine-4-carbonitrile

### 4-Hydroxy-2-(2-hydroxyethyl)-2-(3-nitrophenyl)butanenitrile

To a suspension of sodium hydride (617 mg, 14.16 mmol) in dimethylformamide (16 mL) under 10°C, a solution of 3-nitrophenylacetonitrile (1.15 g, 7.09 mmol) and 2-(2-bromoethoxy)tetrahydro-2H-pyran (3.24 g, 15.5 mmol) in dimethylformamide (5 mL) was added, and the mixture was stirred for 3 hours at room temperature. The reaction mixture was poured into saturated aqueous ammonium chloride and extracted with ethyl acetate. The organic layer was washed with brine, dried over anhydrous magnesium sulfate, filtrated, and then the filtrate was concentrated in vacuo. The residue was purified by silica gel column chromatography. To the resulting oil was added methanol (15 mL) followed by p-toluenesulfonic acid mono-hydrate (90.7 mg), and then the mixture was stirred for three and half hours at room temperature. Water was added to the reaction mixture, the mixture was concentrated in vacuo. Then, saturated aqueous ammonium chloride was added thereto, and the mixture was extracted with ethyl acetate. The combined organic layer was washed with brine, dried over anhydrous magnesium sulfate, filtrated, and then the filtrate was concentrated in vacuo. The residue was purified by silica gel chromatography to give 740 mg of the title compound as a yellow solid.
High-performance liquid chromatography/mass spectrometry
m/z 251 (M+H)
Retention time: 2.09 min.

### Preparation of 1-(2-methoxyphenyl)-4-(3-nitrophenyl)-piperidine-4-carbonitrile

The title compound was prepared in a similar manner to Example 1-1.
High-performance liquid chromatography/mass spectrometry
m/z 338 (M+H)
Retention time: 3.26 min.

### Example 19

### Preparation of 4-(3-aminophenyl)-1-(2-methoxyphenyl)-piperidine-4-carbonitrile

To a mixture of 1-(2-methoxyphenyl)-4-(3-nitrophenyl)-piperidine-4-carbonitrile (400 mg, 1.185 mmol) in methanol (4 mL) / tetrahydrofuran (4 mL), 10% palladium-carbon (40 mg) was added, and the mixture was stirred under hydrogen atmosphere for 3 hours. The reaction mixture was filtrated, and the filtrate was concentrated in vacuo. The residue was purified by silica gel column chromatography followed by crystallization to give 277 mg of the title compound as a white solid.
High-performance liquid chromatography/mass spectrometry
m/z 308 (M+H)
Retention time: 2.19 min.

### Example 20

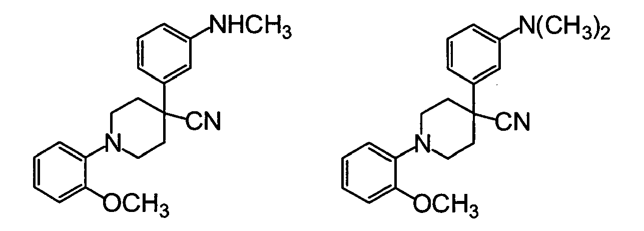

### Preparation of 1-(2-methoxyphenyl)-4-[3-(methylamino)-phenyl]piperidine-4-carbonitrile and 4-[3-(dimethylamino)-phenyl]-1-(2-methoxyphenyl)piperidine-4-carbonitrile

To a solution of (4-(3-aminophenyl)-1-(2-methoxyphenyl)piperidine-4-carbonitrile (50 mg,0.162 mmol) in 1,2-dichloroethane (1.5 mL) added formalin (30.5 mg, 0.356 mmol) and sodium triacetoxyborohydride (103 mg,0.486 mmol), and the mixture was stirred for 17 and half hours at room temperature. The reaction mixture was poured into saturated aqueous sodium hydrogencarbonate and extracted with ethyl acetate. The organic layer was washed with brine, dried over anhydrous magnesium sulfate, filtrated, and then the filtrate was concentrated in vacuo. The residue was purified by silica gel column chromatography to give 18 mg of 1-(2-methoxyphenyl)-4-[3-(methylamino)-phenyl]piperidine-4-carbonitrile and 23 mg of 4-[3-(dimethylamino)phenyl]-1-(2-methoxyphenyl)piperidine-4-carbonitrile each as a white crystal.
1-(2-Methoxyphenyl)-4-[3-(methylamino)phenyl]piperidine-4-carbonitrile
High-performance liquid chromatography/mass spectrometry
m/z 322 (M+H)
Retention time: 2.38 min.
4-[3-(Dimethylamino)phenyl]-1-(2-methoxyphenyl)piperidine-4-carbonitrile)
High-performance liquid chromatography/mass spectrometry
m/z 336 (M+H)
Retention time: 2.51 min.

### Example 21-1

### Preparation of 4-(3-chlorophenyl)-1-(2-methoxyphenyl)-piperidine-4-carbonitrile

To a suspension of sodium hydride (126 mg, 2.88 mmol) in dimethylformamide (8 mL) under 10°C was added a solution of 3-chlorobenzyl cyanide (200 mg,1.31 mmol) and N,N'-bis(2-chloroethyl)-2-methoxyaniline (326 mg, 1.32 mmol) in dimethylformamide (4 mL), and the mixture was stirred for 57 hours at room temperature. The reaction mixture was poured into ice-water and extracted with ethyl acetate. The organic layer was washed with brine, dried over anhydrous magnesium sulfate, filtrated, and the filtrate was concentrated in vacuo. The residue was purified by silica gel column chromatography to give 41 mg of the title compound as a yellow solid.
High-performance liquid chromatography/mass spectrometry
m/z 327 (M+H)
Retention time: 3.38 min.

### Example 21-2

### Preparation of 4-(3,5-dimethoxyphenyl)-1-(2-methoxyphenyl)-piperidine-4-carbonitrile

The title compound was prepared in a similar manner to Example 21-1.
High-performance liquid chromatography/mass spectrometry
m/z 353 (M+H)
Retention time: 3.24 min.

### Example 21-3

### Preparation of 1-(2-methoxyphenyl)-4-(3-thienyl)piperidine-4-carbonitrile

The title compound was prepared in a similar manner to Example 21-1.
High-performance liquid chromatography/mass spectrometry
m/z 299 (M+H)
Retention time: 2.96 min.

### Example 21-4

### Preparation of 1-(2-methoxyphenyl)-4-(2-thienyl)piperidine-4-carbonitrile

The title compound was prepared in a similar manner to Example 21-1.
High-performance liquid chromatography/mass spectrometry
m/z 299 (M+H)
Retention time: 3.24 min.

### Example 22

### Preparation of 4-(3-hydroxyphenyl)-1-(2-methoxyphenyl)-piperidine-4-carbonitrile

To a mixture of 4-[3-(benzyloxy)phenyl]-1-(2-methoxyphenyl)piperidine-4-carbonitrile (280 mg, 0.702 mmol) in methanol (4 mL) / tetrahydrofuran (0.8 mL), palladium-carbon (28 mg) was added, and the mixture was stirred under hydrogen atmosphere for eight and half hours at 40°C. The reaction mixture was filtrated, and the filtrate was concentrated in vacuo. The residue was purified by silica gel column chromatography to give 192 mg of the title compound as a white solid.
High-performance liquid chromatography/mass spectrometry
m/z 309 (M+H)
Retention time: 2.71 min.

### Example 23-1

### Preparation of 4-(3-ethoxyphenyl)-1-(2-methoxyphenyl)-piperidine-4-carbonitrile

To a solution of 4-(3-hydroxyphenyl)-1-(2-methoxyphenyl)piperidine-4-carbonitrile (35 mg, 0.113 mmol) in dimethylformamide (1 mL) were added ethyl iodide (0.009 ml, 0.113 mmol) and potassium carbonate (47 mg, 0.340 mmol), and then the mixture was stirred for four and half hours at 50°C. During the stirring process, ethyl iodide (0.003 ml, 0.037 mmol) and potassium carbonate (15 mg, 0.108 mmol) were added thereto. Water was added to the reaction solution, and then the mixture was extracted with ethyl acetate. The organic layer was washed with brine, dried over anhydrous magnesium sulfate, filtrated, and then the solvent was removed from the filtrate in vacuo. The residue was purified by thin-layer chromatography followed by crystallization to give 18 mg of the title compound as a white solid.
High-performance liquid chromatography/mass spectrometry
m/z 337 (M+H)
Retention time: 3.34 min.

### Example 23-2

### Preparation of 4-(3-isopropoxyphenyl)-1-(2-methoxyphenyl)-piperidine-4-carbonitrile

The title compound was prepared in a similar manner to Example 23-1.
high-performance liquid chromatography/mass spectrometry
m/z 351 (M+H)
retention time: 3.49 min.

### Example 24

### Preparation of 4-(3-fluorophenyl)-1-pyrimidin-2-yl-piperidine-4-carbonitrile

### 1-(Diphenylmethyl)-4-(3-fluorophenyl)piperidine-4-carbonitrile

The title compound was prepared in a similar manner to Example 1-1.
High-performance liquid chromatography/mass spectrometry
m/z 371 (M+H)
Retention time: 3.13 min.

### 4-(3-Fluorophenyl)piperidine-4-carbonitrile

The title compound was prepared in a similar manner to Example 17.
High-performance liquid chromatography/mass spectrometry
m/z 205 (M+H)
Retention time: 2.46 min. (1-99)

### 4-(3-Fluorophenyl)-1-pyrimidin-2-ylpiperidine-4-carbonitrile

The title compound was prepared in a similar manner to Example 6-1.
High-performance liquid chromatography/mass spectrometry
m/z 283 (M+H)
Retention time: 3.44 min.

### Example 25

### Preparation of 1-(5-bromopyrimidin-2-yl)-4-(3-methoxyphenyl)piperidine-4-carbonitrile

To a solution of 4-(3-methoxyphenyl)-1-pyrimidin-2-ylpiperidine-4-carbonitrile (80 mg, 0.271 mmol) in tetrahydrofuran (1.5 mL), N-bromosuccinimide (48 mg, 0.271 mmol) was added at 0°C, and then the mixture was stirred for 6 hours at room temperature. During the stirring process, more 5 mg of N-bromosuccinimide (0.028 mmol) was added thereto. The reaction mixture was poured into saturated aqueous sodium hydrogencarbonate, and extracted with ethyl acetate. The organic layer was washed with brine, dried over anhydrous sodium sulfate, filtrated, and then the filtrate was concentrated in vacuo. The residue was purified by silica gel column chromatography to give 91 mg of the title compound as a white solid.
High-performance liquid chromatography/mass spectrometry
m/z 372.9 (M+H)
Retention time: 4.24 min.

### Example 26-1

### Preparation of 1-benzoyl-4-(3-methoxyphenyl)piperidine-4-carbonitrile

To a solution of 4-(3-methoxyphenyl)piperidine-4-carbonitrile (100 mg, 0.46 mmol) in dichloromethane (2 mL) were added triethylamine (0.064 ml, 0.46 mmol) and benzoyl chloride (0.053 ml, 0.46 mmol) at 0°C, and then the mixture was stirred for two and half hours at room temperature. The reaction mixture was poured into saturated aqueous sodium hydrogencarbonate, and extracted with ethyl acetate. The ethyl acetate layer was washed with brine, dried over anhydrous sodium sulfate, filtrated, and then the filtrate was concentrated in vacuo. The residue was purified by silica gel column chromatography to give 133 mg of the title compound as a white solid.
High-performance liquid chromatography/mass spectrometry
m/z 321 (M+H)
Retention time: 3.36 min.

### Example 26-2

### Preparation of 1-acetyl-4-(3-methoxyphenyl)piperidine-4-carbonitrile

The title compound was prepared in a similar manner to Example 26-1.
High-performance liquid chromatography/mass spectrometry
m/z 259(M+H)
Retention time: 2.82 min.

### Example 27

### Preparation of 4-(3-methoxyphenyl)-1-[(4-methylphenyl)-sulfonyl]piperidine-4-carbonitrile

To a solution of 4-(3-methoxyphenyl)piperidine-4-carbonitrile (100 mg, 0.46 mmol) in dichloromethane (2 mL) were added triethylamine (0.064 ml, 0.46 mmol) and p-toluenesulfonyl chloride (88.0 mg, 0.46 mmol) at 0°C, and then the mixture was stirred for 16 and half hours at room temperature. The reaction mixture was poured into saturated aqueous sodium hydrogencarbonate and extracted with ethyl acetate. The ethyl acetate layer was washed with brine, dried over anhydrous sodium sulfate, filtrated, and then the filtrate was concentrated in vacuo. The residue was purified by silica gel column chromatography to give 151 mg of the title compound as a gray solid. High-performance liquid chromatography/mass spectrometry
m/z 371 (M+H)
Retention time: 2.82 min.

### Example 28

### Preparation of 1-cyclohexyl-4-(3-methoxyphenyl)piperidine-4-carbonitrile

To a solution of 4-(3-methoxyphenyl)piperidine-4-carbonitrile (80 mg, 0.37 mmol) in dichloroethane (2 mL) were added cyclohexanone (0.057 ml, 0.55 mmol) and sodium triacetoxyborohydride (156 mg, 0.738 mmol), and then the mixture was stirred for 15 hours at room temperature. The reaction mixture was poured into saturated aqueous sodium hydrogencarbonate, and extracted with ethyl acetate. The ethyl acetate layer was dried over anhydrous sodium sulfate, filtrated, and then the filtrate was concentrated in vacuo. The residue was purified by silica gel column chromatography to give 102 mg of the title compound as a white solid.
High-performance liquid chromatography/mass spectrometry
m/z 299 (M+H)
Retention time: 2.76 min.

### Example 29

### Preparation of ethyl {[1-(2-methoxyphenyl)-4-(3-methoxyphenyl)piperidin-4-yl]methyl}carbamate

To a solution of {[1-(2-methoxyphenyl)-4-(3-methoxyphenyl)piperidin-4-yl]methyl}amine (100 mg, 0.306 mmol) in dichloromethane (2 mL) were added triethylamine (0.042 ml, 0.306 mmol) and ethylchloroformate (0.03 ml, 0.306 mmol) at 0°C, and then the mixture was stirred for 16 and half hours at room temperature. The reaction mixture was poured into saturated aqueous sodium hydrogencarbonate, and extracted with ethyl acetate. The ethyl acetate layer was washed with brine, dried over anhydrous sodium sulfate, filtrated, and then the filtrate was concentrated in vacuo. The residue was purified by silica gel column chromatography to give 82 mg of the title compound as a colorless oil.
high-performance liquid chromatography/mass spectrometry
m/z 399 (M+H)
retention time: 2.42 min.

### Example 30-1

### Preparation of N-{[1-(2-methoxyphenyl)-4-(3-methoxyphenyl)-piperidin-4-yl]methyl}benzamide

To a solution of { [1- (2-methoxyphenyl) -4- (3-methoxyphenyl)piperidin-4-yl]methyl}amine (100 mg, 0.306 mmol) in dichloromethane (2 mL) were added triethylamine (0.042 ml, 0.306 mmol) and benzoyl chloride (0.355 ml, 0.306 mmol) at 0°C, and then the mixture was stirred for three and half hours at room temperature. The reaction mixture was poured into saturated aqueous sodium hydrogencarbonate and extracted with ethyl acetate. The ethyl acetate layer was washed with brine, dried over anhydrous sodium sulfate, filtrated, and then the filtrate was concentrated in vacuo. The residue was purified by silica gel column chromatography to give 119 mg of the title compound as a white solid.
High-performance liquid chromatography/mass spectrometry
m/z 431 (M+H)
Retention time: 2.57 min.

### Example 30-2

### Preparation of N-{[1-(2-methoxyphenyl)-4-(3-methoxyphenyl)-piperidin-4-yl]methyl}propanamide

The title compound was prepared in a similar manner to Example 30-1.
High-performance liquid chromatography/mass spectrometry
m/z 383 (M+H)
Retention time: 2.34 min.

### Example 31

### Preparation of N-benzyl-1-[1-(2-methoxyphenyl)-4-(3-methoxyphenyl)piperidin-4-yl]methylamine

To a solution of {[1-(2-methoxyphenyl)-4-(3-methoxyphenyl)piperidin-4-yl]methyl}amine (100 mg, 0.306 mmol) in dichloroethane (2 mL) were added benzaldehyde (0.031 ml, 0.306 mol), sodium triacetoxyborohydride (97 mg, 0.459 mmol) and acetic acid (0.017 ml, 0.306 mmol), and then the mixture was stirred for 16 hours at room temperature. The reaction mixture was poured into saturated aqueous sodium hydrogencarbonate and extracted with ethyl acetate. The ethyl acetate layer was washed with brine, dried over anhydrous sodium sulfate, filtrated, and then the filtrate was concentrated in vacuo. The residue was purified by silica gel column chromatography to give 84 mg of the title compound as a white solid.
High-performance liquid chromatography/mass spectrometry
m/z 417 (M+H)
Retention time: 2.30 min.

### Example 32-1

### Preparation of 1-(2-methoxyphenyl)-4-(3-methoxyphenyl)-4-[(methylthio)methyl]piperidine

### [1-(2-Methoxyphenyl)-4-(3-methoxyphenyl)piperidin-4-yl]methyl methanesulfonate

To a solution of [1-(2-methoxyphenyl)-4-(3-methoxyphenyl)piperidin-4-yl]methanol (300 mg, 0.916 mmol) in dichloromethane were added triethylamine (0.151 ml, 1.09 mol) and methanesulfonyl chloride (0.085 ml, 1.09 mmol) at 0°C, and then the mixture was stirred for two and half hours at room temperature. The reaction solution was poured into ice-water and extracted with ethyl acetate. The organic layer was washed with brine, dried over anhydrous magnesium sulfate, and then filtrated. The filtrate was concentrated in vacuo to give 326 mg of the title compound as a yellow oil.
High-performance liquid chromatography/mass spectrometry
m/z 406 (M+H)
Retention time: 2.44 min.

### 1-(2-Methoxyphenyl)-4-(3-methoxyphenyl)-4-[(methylthio)-methyl]piperidine

To a solution of [1-(2-methoxyphenyl)-4-(3-methoxyphenyl)piperidin-4-yl]methyl methanesulfonate (50 mg, 0.123 mmol) in dimethylformamide (1 mL), sodium thiomethoxide (9 mg, 0.123 mmol) was added, and then the mixture was stirred for seven and half hours at room temperature. During the stirring process, an appropriate amount of sodium thiomethoxide was added thereto. The reaction solution was poured into saturated aqueous ammonium chloride and extracted with ethyl acetate. The organic layer was washed with brine, dried over anhydrous magnesium sulfate, filtrated, and the filtrate was concentrated in vacuo. The residue was purified by silica gel column chromatography to give 10 mg of the title compound as a colorless oil.
high-performance liquid chromatography/mass spectrometry
m/z 358 (M+H)
retention time: 3.24 min.

### Example 32-2

### Preparation of 1-(2-methoxyphenyl)-4-(3-methoxyphenyl)-4-[(phenylthio)methyl]piperidine

The title compound was prepared in a similar manner to Example 32-1.
High-performance liquid chromatography/mass spectrometry
m/z 420 (M+H)
Retention time: 3.72 min.

### Example 33

### Preparation of 4-(methoxymethyl)-1-(2-methoxyphenyl)-4-(3-methoxyphenyl)piperidine

To a solution of [1-(2-methoxyphenyl)-4-(3-methoxyphenyl)piperidin-4-yl]methanol (50 mg, 0.152 mmol) in tetrahydrofuran (1 mL), sodium hydride (7 mg, 0.152 mmol) was added, and then stirred for 15 minutes at room temperature. And then methyl iodide (0.009 ml, 0.152 mmol) was added thereto and the mixture was further stirred for 21 hours at room temperature. During the stirring process, methyl iodide (0.009 ml, 0.152 mmol) was added thereto. The reaction solution was poured into saturated aqueous ammonium chloride and extracted with ethyl acetate. The organic layer was washed with brine, dried over anhydrous magnesium sulfate, filtrated, and then the filtrate was concentrated in vacuo. The residue was purified by silica gel column chromatography to give 10 mg of the title compound as a colorless oil.
High-performance liquid chromatography/mass spectrometry
m/z 342 (M+H)
Retention time: 2.53 min.

### Example 34

### Preparation of 1-(2-methoxyphenyl)-4-(3-methoxyphenyl)-4-(phenoxymethyl)piperidine

To a solution of phenol (13 mg, 0.135 mmol) in dimethylformamide (1 mL), sodium hydride (6 mg, 0.135 mmol) was added at 0°C, and then the mixture was stirred for 15 minutes at room temperature. And then a solution of [1-(2-methoxyphenyl)-4-(3-methoxyphenyl)piperidin-4-yl]methyl methanesulfonate (50 mg, 0.126 mmol) in dimethylformamide (0.5 mL) was added thereto at 0°C and the mixture was further stirred for 25 hours at room temperature. During the stirring process, a solution of phenol (13 mg, 0.135 mmol) and sodium hydride (6 mg, 0.135 mmol) in dimethylformamide (0.5 mL) was added thereto. The reaction solution was poured into saturated aqueous ammonium chloride and extracted with ethyl acetate. The organic layer was washed with brine, dried over anhydrous magnesium sulfate, filtrated, and the filtrate was concentrated in vacuo. The residue was purified by silica gel column chromatography to give 9.3 mg of title compound as a colorless oil.
High-performance liquid chromatography/mass spectrometry
m/z 404 (M+H)
Retention time: 3.51 min.

### Example 35-1

### Preparation of 1-(2-methoxyphenyl)-4-(3-methoxyphenyl)-4-vinylpiperidine

To a solution of methyl triphenylphosphonium bromide (219 mg, 0614 mmol) in tetrahydrofuran (3 mL) was added 0.614 ml of 1M sodium bis(trimethylsilyl)amide in tetrahydrofuran (0.614 mmol) at 0°C, and then the mixture was stirred for 1 hour at room temperature. Then, after cooling the reaction mixture in ice bath, a solution of 1-(2-methoxyphenyl)-4-(3-methoxyphenyl)piperidine-4-carboaldehyde (100 mg, 0.307 mmol) in tetrahydrofuran (1 mL) was added thereto, and the mixture was stirred for 17 hours at room temperature. The reaction mixture was poured into saturated aqueous ammonium chloride and extracted with ethyl acetate. The organic layer was washed with brine, dried over anhydrous magnesium sulfate, filtrated, and the filtrate was concentrated in vacuo. The residue was purified by silica gel column chromatography to give 65 mg of the title compound as a colorless oil.
High-performance liquid chromatography/mass spectrometry
m/z 324 (M+H)
Retention time: 2.67 min.

### Example 35-2

### Preparation of 1-(2-methoxyphenyl)-4-(3-methoxyphenyl)-4-(2-phenylvinyl)piperidine

The title compound was prepared in a similar manner to Example 35-1.
High-performance liquid chromatography/mass spectrometry
m/z 400 (M+H)
Retention time: 3.09 min.

### Example 35-3

### Preparation of 4-(2-chlorovinyl)-1-(2-methoxyphenyl)-4-(3-methoxyphenyl)piperidine

The title compound was prepared in a similar manner to Example 35-1.
High-performance liquid chromatography/mass spectrometry
m/z 358 (M+H)
Retention time: 2.82 min.

### Example 36

### Preparation of [1-(2-methoxyphenyl)-4-(3-methoxyphenyl)-piperidin-4-yl](phenyl)methanone

To a solution of 1-(2-methoxyphenyl)-4-(3-methoxyphenyl)piperidine-4-carboxylic acid in dichloromethane (20 mL) were added dimethylformamide (1 drop) and oxalyl chloride (0.523 ml, 5.84 mmol) at 0°C, and then the mixture was stirred for 4 hours at room temperature. During the stirring process, further dimethylformamide (0.02 mL) and oxalyl chloride (0.13 ml, 1.45 mmol) were added thereto. The reaction mixture was concentrated and the residual solvent was removed by azeotropic distillation with toluene. To a solution of the residue (100 mg, 0.252 mmol) in tetrahydrofuran (3 mL), 0.6 ml of 0.94 M phenyl lithium in cyclohexane/diethyl ether (0.564 mmol) was added at -78°C, and then the mixture was stirred for 1 hour at - 78°C. Then the mixture was gradually warm to room temperature and stirred for 26 hours. The reaction solution was poured into saturated aqueous ammonium chloride and extracted with ethyl acetate. The organic layer was washed with brine, dried over anhydrous magnesium sulfate, filtrated, and the filtrate was concentrated in vacuo. The residue was purified by silica gel column chromatography followed by crystallization to give 27 mg of the title compound as a colorless oil.
High-performance liquid chromatography/mass spectrometry
m/z 402 (M+H)
Retention time: 2.99 min.

### Example 37

### Preparation of 1-(2-methoxyphenyl)-4-(3-methoxyphenyl)-4-{[(4-methylphenyl)sulfonyl]methyl}piperidine

To a solution of [1-(2-methoxyphenyl)-4-(3-methoxyphenyl)piperidin-4-yl]methyl methanesulfonate (103 mg, 0.253 mmol) in dimethylformamide (2 mL), sodium 4-toluenesulfinate (58.6 mg, 0.328 mmol) was added, and then the mixture was stirred for 2 hours at room temperature and for seven and half hours at 60°C. During the stirring process, sodium 4-toluenesulfinate (16 mg, 0.089 mmol) was added thereto. The reaction mixture was poured into saturated aqueous ammonium chloride and extracted with ethyl acetate. The organic layer was washed with brine, dried over anhydrous magnesium sulfate, filtrated, and then the filtrate was concentrated in vacuo. The residue was purified by silica gel column chromatography to give 83 mg of the title compound as a light yellow oil. High-performance liquid chromatography/mass spectrometry
m/z 466 (M+H)
Retention time: 3.47 min.

### Example 38

### Preparation of [1-(2-methoxyphenyl)-4-(3-methoxyphenyl)-piperidin-4-yl]methyl benzylcarbamate.

To a solution of [1-(2-methoxyphenyl)-4-(3-methoxyphenyl)piperidin-4-yl]methanol (55 mg, 0.167 mmol) in tetrahydrofuran (1 mL), benzylisocyanate (0.044 ml, 0.334 mmol) was added, and then the mixture was stirred for 12 hours at 75°C. During the stirring process, benzylisocyanate (0.027 ml, 0.218 mmol) was twice added thereto. The reaction mixture was poured into brine and extracted with ethyl acetate. The organic layer was washed with brine, dried over anhydrous magnesium sulfate, filtrated, and then the filtrate was concentrated in vacuo. The residue was purified by thin-layer chromatography to give 63 mg of the title compound as a light yellow oil. High-performance liquid chromatography/mass spectrometry
m/z 461 (M+H)
Retention time: 2.84 min.

### Example 39

### Preparation of 2-{[1-(2-methoxyphenyl)-4-(3-methoxyphenyl)-piperidin-4-yl]methyl}-1H-isoindole-1,3(2H)-dione

To a mixture of {[1-(2-methoxyphenyl)-4-(3-methoxyphenyl)piperidin-4-yl]methyl}amine (50 mg, 0.153 mmol) in acetonitrile (1 mL) / water (1 mL), N-carboethoxyphthalimide (33 mg, 0.153 mmol) was added, and then the mixture was stirred for 26 hours at room temperature. Water was added to the reaction solution, and the mixture was extracted with ethyl acetate. The organic layer was washed with brine, dried over anhydrous magnesium sulfate, filtrated, and the filtrate was concentrated in vacuo. The residue was purified by silica gel column chromatography to give 26 mg of the title compound as a light yellow oil.
High-performance liquid chromatography/mass spectrometry
m/z 457 (M+H)
Retention time: 2.78 min.

### Example 40

### Preparation of 4-ethyl-1-(2-methoxyphenyl)-4-(3-methoxyphenyl)piperidine

To a mixture of 1-(2-methoxyphenyl)-4-(3-methoxyphenyl)-4-vinylpiperidine (38 mg, 0.117 mmol) in methanol (1 mL), 10% palladium-carbon (10 mg) was added, and then the mixture was stirred under hydrogen atmosphere for 3 hours at room temperature. The reaction solution was filtrated, and then the filtrate was concentrated in vacuo. The residue was purified by thin-layer chromatography to give 37 mg of the title compound as a colorless oil.
High-performance liquid chromatography/mass spectrometry
m/z 326 (M+H)
Retention time: 2.71 min.

### Example 41

### Preparation of 4-ethynyl-1-(2-methoxyphenyl)-4-(3-methoxyphenyl)piperidine

To a solution of 4-(2-chlorovinyl)-1-(2-methoxyphenyl)-4-(3-methoxyphenyl)piperidine (100 mg, 0.28 mmol) in toluene (2 mL), potassium t-butoxide (32 mg, 0.28 mmol) was added at 0°C, and then the mixture was stirred for 8 hours at 110°C. The reaction solution was poured into saturated aqueous ammonium chloride, and the mixture was extracted with ethyl acetate. The organic layer was washed with brine, dried over anhydrous magnesium sulfate, filtrated, and then the filtrate was concentrated in vacuo. The residue was purified by thin-layer chromatography to give 54 mg of the title compound as a white solid. High-performance liquid chromatography/mass spectrometry
m/z 322 (M+H)
Retention time: 2.73 min.

### Example 42-1

### Preparation of 4-(3-methoxyphenyl)-N,N-dimethyl-1-pyrimidin-2-ylpiperidine-4-carboxyamide hydrochloride

### a) 4-(3-Methoxyphenyl)-1-pyrimidin-2-ylpiperidine-4-carbonylchloride hydrochloride

To a solution of 4-(3-methoxyphenyl)-1-pyrimidin-2-ylpiperidine-4-carboxylic acid (1.0 g, 3.19 mmol) in dichloromethane (20 mL) at 0°C under nitrogen atmosphere were added N,N-dimethylformamide (2 drops) and oxalyl chloride (573 µl, 6.38 mmol), and then the mixture was warmed to room temperature. After 4 hours, the reaction solution was concentrated in vacuo, the residual solvent was removed by several azeotropic distillations with toluene. The residue was dried in vacuo to give 1.13 g of the title compound as a white solid.

### b) 4-(3-Methoxyphenyl)-N,N-dimethyl-1-pyrimidin-2-yl-piperidine-4-carboxyamide

At room temperature 4-(3-methoxyphenyl)-1-pyrimidin-2-ylpiperidine-4-carbonylchloride hydrochloride (50 mg, 0.151 mmol) was dissolved in dichloromethane (1 mL) under nitrogen atmosphere, and then dimethylamine·hydrochloride (14.7 mg, 0.181 mmol) and triethylamine (84 µl, 0.604 mmol) were added thereto. After stirring overnight, saturated aqueous sodium hydrogencarbonate was added thereto to quench the reaction, the reactant was extracted with ethyl acetate. The ethyl acetate layer was washed with saturated aqueous ammonium chloride, dried over anhydrous magnesium sulfate, filtrated, and then the solvent was removed off in vacuo. The residue was purified by thin-layer chromatography to give 44.8 mg of the title compound as a colorless oil.

### c) 4-(3-Methoxyphenyl)-N,N-dimethyl-1-pyrimidin-2-yl-piperidine-4-carboxyamide hydrochloride

To a solution of 4-(3-methoxyphenyl)-N,N-dimethyl-1-pyrimidin-2-ylpiperidine-4-carboxyamide (44.8 mg, 0.132 mmol) in ethyl acetate (1.0 mL), 1M hydrochloric acid/diethyl ether (158 ul, 0.158 mmol) was added at room temperature under nitrogen atmosphere. After 1 hour, diethyl ether (3.0 mL) was added to the reaction mixture, and the mixture was filtrated by aspiration to give 38.2 mg of the title compound as a white solid.
High-performance liquid chromatography/mass spectrometry
m/z 341.2 (M+H)
Retention time: 2.67 min.

### Example 42-2

### Preparation of N-(3-benzoylphenyl)-4-(3-methoxyphenyl)-1-pyrimidin-2-ylpiperidine-4-carboxyamide hydrochloride

The title compound was prepared in a similar manner to Example 42-1.
High-performance liquid chromatography/mass spectrometry
m/z 493.3 (M+H)
Retention time: 3.51 min.

### Example 42-3

### Preparation of N-ethyl-4-(3-methoxyphenyl)-1-pyrimidin-2-ylpiperidine-4-carboxyamide hydrochloride

The title compound was prepared in a similar manner to Example 42-1.
High-performance liquid chromatography/mass spectrometry
m/z 341.2 (M+H)
Retention time: 2.55 min.

### Example 43-1

### Preparation of 4-(3-methoxyphenyl)-N-methyl-1-pyrimidin-2-ylpiperidine-4-carboxyamide

The title compound was prepared in a similar manner to Example 42-1 a), b).
High-performance liquid chromatography/mass spectrometry
m/z 327.2 (M+H)
Retention time: 2.80 min.

### Example 43-2

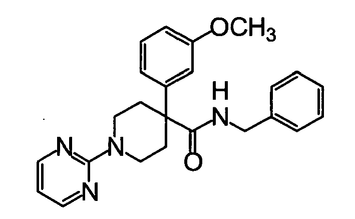

### Preparation of N-benzyl-4-(3-methoxyphenyl)-1-pyrimidin-2-ylpiperidine-4-carboxyamide

The title compound was prepared in a similar manner to Example 43-1.
High-performance liquid chromatography/mass spectrometry
m/z 403.4 (M+H)
Retention time: 3.36 min.

### Example 43-3

### Preparation of N-(diphenylmethyl)-4-(3-methoxyphenyl)-1-pyrimidin-2-ylpiperidine-4-carboxyamide

The title compound was prepared in a similar manner to Example 43-1.
High-performance liquid chromatography/mass spectrometry
m/z 479.3 (M+H)
Retention time: 3.84 min.

### Example 43-4

### Preparation of N-(3,5-di-tert-butylphenyl)-4-(3-methoxyphenyl)-1-pyrimidin-2-ylpiperidine-4-carboxyamide

The title compound was prepared in a similar manner to Example 43-1.
High-performance liquid chromatography/mass spectrometry
m/z 501.2 (M+H)
Retention time: 4.40 min.

### Example 43-5

### Preparation of 4-(3-methoxyphenyl)-N-(4-morpholin-4-yl-phenyl)-1-pyrimidin-2-ylpiperidine-4-carboxyamide

The title compound was prepared in a similar manner to Example 43-1.
¹H-NMR (DMSO-d₆) δ; 1.86 (2H, m), 2.61 (2H, m), 3.00 (4H, m), 3.23 (2H, m), 3.70 (4H, m), 3.73 (3H, s), 4.40 (2H, m), 6.59 (1H, dd, J=4.6, 4.6 Hz), 6.81 (1H, m), 6.83 (2H, d, J=9.0 Hz), 6.96 (1H, m), 7.01 (1H, d, J=8.0 Hz), 7.27 (1H, dd, J=8.0, 8.0 Hz), 7.37 (2H, d, J=9.0 Hz), 8.33 (2H, d, J=4.6 Hz), 9.14 (1H, s).

### Example 44

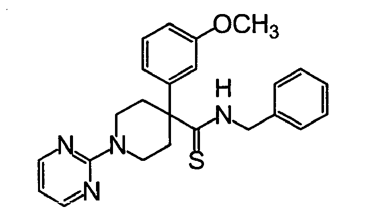

### Preparation of N-benzyl-4-(3-methoxyphenyl)-1-pyrimidin-2-ylpiperidine-4-carbothioamide

To a solution of N-benzyl-4-(3-methoxyphenyl)-1-pyrimidin-2-ylpiperidine-4-carboamide (12.0 mg, 0.0298 mmol) in toluene (0.200 mL), Lawesson's reagent (58.4 mg, 0.144 mmol) was added at room temperature, and then the mixture was stirred for 9 hours at 60°C. The reaction solution was filtrated through a Celite and then the filtrate was purified by thin-layer chromatography to give 6.88 mg of the title compound as a white crystal.
¹H-NMR δ (CDCl₃); 2.32-2.44 (2H, m), 2.67-2.78 (2H, m), 3.30-3.44 (2H, m), 3.79 (3H, s), 4.32-4.45 (2H, m), 4.74-4.77 (2H, m), 6.44 (1H, t, J = 4.7 Hz), 6.82-6.87 (1H, m), 6.97-7.05 (3H, m), 7.10-7.20 (1H, m), 7.23-7.28 (2H, m), 7.33 (1H, t, J = 7.9 Hz), 8.28 (2H, d, J = 4.7 Hz).

### Example 45-1

### Preparation of 4-(3-methoxyphenyl)-1-pyrimidin-2-yl-N-quinolin-5-ylpiperidine-4-carboxyamide hydrochloride

### a) 4-(3-Methoxyphenyl)-1-pyrimidin-2-yl-N-quinolin-5-yl-piperidine-4-carboxyamide

At room temperature 4-(3-methoxyphenyl)-1-pyrimidin-2-ylpiperidine-4-carbonylchloride hydrochloride (50 mg, 0.151 mmol) prepared in Example 42-1 a) was dissolved in dichloromethane (1 mL) under nitrogen atmosphere, and then 5-aminoquinoline (26.0 mg, 0.181 mmol) and triethylamine (84 µl, 0.604 mmol) were added thereto. After stirring the mixture overnight, saturated aqueous sodium hydrogencarbonate was added thereto to quench the reaction, and the mixture was extracted with ethyl acetate. The ethyl acetate layer was dried over anhydrous magnesium sulfate, filtrated, and then the solvent was removed off in vacuo. The residue was purified by thin-layer chromatography to give 46.0 mg of the title compound as a colorless oil.

### b) 4-(3-methoxyphenyl)-1-pyrimidin-2-yl-N-quinolin-5-yl-piperidine-4-carboxyamide hydrochloride

The title compound was prepared in a similar manner to Example 42-1 c).
High-performance liquid chromatography/mass spectrometry
m/z 440.4 (M+H)
Retention time: 2.40 min.

### Example 45-2

### Preparation of 4-(3-methoxyphenyl)-N-pyridin-3-yl-1-pyrimidin-2-ylpiperidine-4-carboxyamide hydrochloride

The title compound was prepared in a similar manner to Example 45-1.
High-performance liquid chromatography/mass spectrometry
m/z 390.2 (M+H)
Retention time: 2.32 min.

### Example 46

### Preparation of 2-{4-(3-methoxyphenyl)-4-[(4-phenyl-piperazin-1-yl)carbonyl]piperidin-1-yl}pyrimidine

At room temperature 4-(3-methoxyphenyl)-1-pyrimidin-2-ylpiperidine-4-carbonylchloride hydrochloride (50 mg, 0.151 mmol) prepared in Example 42-1 a) was dissolved in dichloromethane (1 mL) under nitrogen atmosphere, and then 4-phenylpiperazine (28.0 µl, 0.181 mmol) and triethylamine (84 µl, 0.604 mmol) were added thereto. After stirring the mixture overnight, saturated aqueous sodium hydrogencarbonate was added thereto to quench the reaction, and the mixture was extracted with ethyl acetate. The ethyl acetate layer was washed with saturated aqueous ammonium chloride, dried over anhydrous magnesium sulfate, filtrated, and then the solvent was removed from the filtrate in vacuo. The residue was washed with water to give 50.3 mg of the title compound as a white solid.
¹H-NMR (DMSO-d₆) δ; 1.85 (2H, m), 2.27 (2H, m), 2.83 (4H, m), 3.25 (2H, m), 3.40 (4H, m), 3.72 (3H, s), 4.47 (2H, m), 6.58 (1H, dd, J=4.8, 4.8 Hz), 6.81 (6H, m), 7.16 (2H, m), 7.29 (1H, dd, J=7.9, 7.9 Hz), 8.33 (2H, d, J=4.8 Hz).

### Example 47

### Preparation of N-bicyclo[2.2.1]hept-2-yl-4-(3-methoxyphenyl)-1-pyrimidin-2-ylpiperidine-4-carboxyamide

At room temperature 4-(3-methoxyphenyl)-1-pyrimidin-2-ylpiperidine-4-carbonylchloride hydrochloride (50 mg, 0.151 mmol) prepared in Example 42-1 a) was dissolved in dichloromethane (1 mL) under nitrogen atmosphere, and then (+/-)-endo-2-norbornylamine hydrochloride (27.0 mg, 0.181 mmol) and triethylamine (84 µl, 0.604 mmol) were added thereto. After stirring the mixture overnight, saturated aqueous sodium hydrogencarbonate was added thereto to quench the reaction, and the mixture was extracted with ethyl acetate. The ethyl acetate layer was washed with saturated aqueous ammonium chloride, dried over anhydrous magnesium sulfate, filtrated, and then the solvent was removed from the filtrate in vacuo. The residue was washed with diethyl ether/hexane to give 37.8 mg of the title compound as a white solid.
¹H-NMR (DMSO-d₆) δ; 0.99-1.33(7H, m), 1.76(3H, m), 2.07(1H, m), 2.28(1H, m), 2.58(2H, m), 3.13(2H, m), 3.72(3H, s), 3.86(1H, m), 4.36(2H, m), 6.57(1H, dd, J=4.5, 4.5 Hz), 6.81(1H, m), 6.90(1H, m), 6.95(1H, d, J=7.8 Hz), 7.23(1H, dd, J=7.8, 7.8 Hz), 7.27(1H, d, J=6.6 Hz), 8.32(2H, d, J=4.5 Hz).

### Example 48

### Preparation of 3-(3-methoxyphenyl)-8-pyrimidin-2-yl-8-azabicyclo[3.2.1]octane-3-carboxyamide

### a) Diethyl cis-1-benzylpyrrolidine-2,5-dicarboxylate

A solution of diethyl 2,5-dibromoadipate (10.0 g, 0.0278 mol) in benzene (30 mL) was heated under reflux. After stopping the heating, benzylamine (10 mL) was added dropwise to the reaction mixture over 50 minutes, and then the mixture was heated under reflux again. After 14 hours, the reaction mixture was filtrated in vacuo, and the filtrate was concentrated in vacuo. The residue was purified by silica gel column chromatography to give 1.42 g of the title compound as a white solid.
¹H-NMR (CDCl₃) δ; 1.19 (6H, t, J=7.1Hz), 2.06 (4H, m), 3.42 (2H, m), 3.94 (2H, s), 4.01 (2H, q, J=7.1 Hz), 4.02 (2H, q, J=7.1 Hz), 7.20-7.35 (5H, m).

### b) (Cis-1-benzylpyrrolidine-2,5-diyl)dimethanol

To a suspension of lithium aluminium hydride (671 mg, 0.0177 mol) in tetrahydrofuran (50 mL) at 0°C, a solution of diethyl cis-1-benzylpyrrolidine-2,5-dicarboxylate (3.6 g, 0.0118 mol) in tetrahydrofuran (14 mL) was added dropwise over 20 minutes, and then the mixture was stirred for 2 hours at room temperature. Water, 2N aqueous sodium hydroxide and water were added thereto in order, and the mixture was stirred overnight. The reaction mixture was filtrated through Celite®, and then the solvent was removed off in vacuo. The residue was purified by silica gel column chromatography to give 2.55 g of the title compound as a colorless oil.
¹H-NMR (CDCl₃) δ; 1.82 (4H, m), 2.07 (2H, brs.), 3.03 (2H, m), 3.37 (2H, ddd, J=11.0, 3.5Hz), 3.41 (2H, dd, J=11.0, 4.4Hz), 3.80 (2H, s), 7.30 (5H, m).

### c) Cis-1-benzyl-2,5-bis(chloromethyl)pyrrolidine hydrochloride

To a solution of (cis-1-benzylpyrrolidin-2,5-diyl)dimethanol (2.3 mg, 0.0104 mol) in chloroform (23 mL) at 0°C, thionyl chloride (2.72 g, 0.0229 mol) was slowly added dropwise, and then the mixture was heated under reflux for 30 minutes. The reaction mixture was concentrated in vacuo and the residue was washed with 2-propanol (25 mL) to give 1.83 g of the title compound as a white solid.
High-performance liquid chromatography/mass spectrometry
m/z 258.0 (M+H)
Retention time: 2.82 min.

### d) 8-Benzyl-3-(3-methoxyphenyl)-8-azabicyclo[3.2.1]octane-3-carbonitrile

To a suspension of 55% sodium hydride (42 mg, 0.956 mmol) in N,N-dimethylformamide (1.0 mL) at 0°C, a solution of cis-1-benzyl-2,5-bis(chloromethyl)pyrrolidine hydrochloride (100 mg, 0.33 mmol) and 3-methoxyphenylacetonitrile (45.4 mg, 0.308 mmol) in N,N-dimethylformamide (1.0 mL) was slowly added dropwise and furthermore the residual solution was added with N,N-dimethylformamide (1.0 mL), and then the mixture was stirred for 1 hour at room temperature. Additionally, the reaction mixture was stirred for more one hour at 45°C. Water was added thereto to quench the reaction, and the mixture was extracted with ethyl acetate. The ethyl acetate layer was dried over anhydrous magnesium sulfate, filtrated, and then the solvent was removed from the filtrate in vacuo. The residue was purified by silica gel column chromatography to give a crude product. It was purified by preparative thin-layer chromatography to give 10.8 mg of the title compound as a colorless oil.
¹H-NMR (CDCl₃) δ; 1.86-2.36 (8H, m), 3.28 (2H, m), 3.51 (2H, s), 2.82 (3H, s), 6.78 (1H, m), 7.02 (2H, m), 7.16-7.49 (6H, m).

### e) 8-Benzyl-3-(3-methoxyphenyl)-8-azabicyclo[3.2.1]octane-3-carboxyamide

To a solution of 8-benzyl-3-(3-methoxyphenyl)-8-azabicyclo[3.2.1]octane-3-carbonitrile (50.0 mg, 0.150 mmol) in dimethylsulfoxide (1 mL) at room temperature, 6N aqueous potassium hydroxide (1 mL) was added dropwise, and then the mixture was stirred for 6 hours at 100°C. The reaction solution was cooled to room temperature, diluted with water, and adjusted to pH=10 with concentrated hydrochloric acid. The mixture was extracted with ethyl acetate. The organic layer was dried over anhydrous magnesium sulfate, filtrated, and then the solvent was removed from the filtrate in vacuo. The residue was purified by silica gel column chromatography and then washed with hexane to give 29.9 mg of the title compound as a light yellow solid.
¹H-NMR (DMSO-d₆) δ; 1.84 (6H, m), 2.86 (2H, m), 3.11 (2H, m), 3.44 (2H, s), 3.71 (3H, s), 6.76 (1H, dd, J=7.9, 2.0Hz), 6.86 (3H, m), 7.17 (1H, dd, J=7.9, 7.9Hz), 7.21 (1H, m), 7.29 (5H, m).

### f) 3-(3-Methoxyphenyl)-8-pyrimidin-2-yl-8-azabicyclo-[3.2.1]octane-3-carboxyamide

To a solution of 8-benzyl-3-(3-methoxyphenyl)-8-azabicyclo[3.2.1]octane-3-carboxyamide (10.0 mg, 0.0285 mmol) in ethanol (0.5 mL) were added 10% palladium-carbon (2 mg) and ammonium formate (40 mg) at room temperature, and then the mixture was stirred for a hour under reflux. After cooling the reaction mixture to room temperature, it was filtrated through Celite®, and then the solvent was removed from the filtrate in vacuo. To a solution of the concentrated residue in N,N-dimethylformamide (0.5 mL) were added potassium carbonate (7.9 mg, 0.0571 mmol) and 2-chloropyrimidine (4.1 mg, 0.0285 mmol) at room temperature, and then the mixture was stirred for 2 hours at 70°C. Water was added to the reaction solution to quench the reaction, and the mixture was extracted with ethyl acetate. The ethyl acetate layer was dried over anhydrous magnesium sulfate, filtrated, and then the solvent was removed off in vacuo. The residue was purified by silica gel column chromatography to give 3.4 mg of the title compound as a white solid.
¹H-NMR (CDCl₃) δ; 2.00 (2H, m), 2.14 (2H, m), 2.31 (2H, m), 2.80 (2H, m), 3.75 (3H, s), 4.81 (2H, m), 6.44 (1H, dd, J=4.8, 4.8 Hz), 6.74 (1H, m), 6.83 (1H, m), 6.90 (1H, m), 7.18 (1H, dd, J=8.0, 8.0 Hz), 8.26 (2H, d, J=4.8 Hz).

### Example 49

### Preparation of methyl 8-(2-methoxyphenyl)-3-(3-methoxyphenyl)-8-azabicyclo[3.2.1]octane-3-carboxylate

### a) Diethyl cis-1-(2-methoxyphenyl)pyrrolidine-2,5-dicarboxylate

A solution of diethyl 2,5-dibromoadipate (8.0 g, 0.0222 mol) in toluene (25 mL) was heated at 80°C. After stopping the heating, 2-methoxyaniline (4.1 g, 0.333 mol) was added dropwise thereto over 40 minutes, and then the mixture was stirred for 11 hours at 100°C again. The reaction mixture was filtrated by aspiration, and the filtrate was concentrated in vacuo. The residue was purified by silica gel column chromatography to give 2.08 g of the title compound as a white solid.
¹H-NMR (CDCl₃) δ; 1.25 (6H, t, J=7.2Hz), 2.21 (4H, m), 3.71 (3H, s), 4.21 (4H, m), 4.36 (2H, m), 6.70 (1H, m), 6.82 (3H, m).

### b) [Cis-1-(2-methoxyphenyl)pyrrolidine-2,5-diyl]dimethanol

The title compound was prepared in a similar manner to Example 48 b).
¹H-NMR (CDCl₃) δ; 2.01 (4H, m), 3.08 (2H, m), 3.38 (4H, m), 3.41 (2H, m), 6.96 (1H, dd, J=8.3, 1.3 Hz), 7.01 (1H, ddd, J=7.7, 7.7, 1.3 Hz), 7.18 (1H, m), 7.28 (1H, dd, J=7.7, 1.7 Hz).

### c) Cis-2,5-bis(chloromethyl)-1-(2-methoxyphenyl)pyrrolidine hydrochloride

The title compound was prepared in a similar manner to Example 48 c).
¹H-NMR (DMSO-d₆) δ; 1.86 (2H, m), 2.07 (2H, m), 3.39 (2H, dd, J=10.5, 8.5 Hz), 3.54 (2H, dd, J=10.5, 3.3 Hz), 3.68 (2H, m), 3.80 (3H, s), 6.86 (1H, m), 6.96-7.11 (3H, m).

### d) Cis-2,5-bis(chloromethyl)-1-(2-methoxyphenyl)pyrrolidine

Cis-2,5-bis(chloromethyl)-1-(2-methoxyphenyl)-pyrrolidine hydrochloride (720 mg, 2.32 mmol) was suspended in diethyl ether and washed with saturated aqueous sodium hydrogencarbonate. The aqueous layer was extracted with diethyl ether again. The diethyl ether layer was washed with brine, dried over anhydrous magnesium sulfate, filtrated, and then the solvent was removed from the filtrate in vacuo to give 614 mg of the title compound as a light yellow solid.
¹H-NMR (CDCl₃) δ; 1.92 (2H, m), 2.16 (2H, m), 3.27 (2H, dd, J=10.5, 8.8 Hz), 3.51 (2H, dd, J=10.5, 3.3 Hz), 3.72 (2H, m), 3.85 (3H, s), 6.87 (1H, ddd, J=7.5, 7.5, 1.5 Hz) , 6.90 (1H, m), 7.00 (1H, dd, J=7.5, 1.5 Hz), 7.13 (1H, ddd, J=8.2, 7.5, 1.5 Hz).

### e) 8-(2-Methoxyphenyl)-3-(3-methoxyphenyl)-8-azabicyclo-[3.2.1]octane-3-carbonitrile

To a suspension of 55% sodium hydride (541 mg, 0.0124 mol) in N,N-dimethylformamide (16 mL) at 0°C, a solution of cis-2,5-bis(chloromethyl)-1-(2-methoxyphenyl)pyrrolidine (810 mg, 2.95 mmol) and 3-methoxyphenylacetonitrile (870 mg, 5.91 mmol) in N,N-dimethylformamide (14 mL) was added dropwise over 15 minutes and furthermore the residual solution was added with N,N-dimethylformamide (2.0 mL), and then the mixture was stirred for 2 hours at room temperature. After further stirring the mixture for 3 hours at 40°C, water was added thereto to quench the reaction, and then the mixture was extracted with ethyl acetate. The ethyl acetate layer was dried over anhydrous magnesium sulfate, filtrated, and then the solvent was removed off in vacuo. The residue was purified by silica gel column chromatography to give a crude product. It was washed with hexane/diethyl ether to give 472 mg of the title compound as a white solid.
¹H-NMR (CDCl₃) δ; 2.17 (2H, m), 2.32 (2H, m), 2.46-2.57 (4H, m), 3.76 (3H, s), 4.39 (2H, m), 6.80 (1H, m), 6.88 (4H, m), 6.99 (1H, dd, J=2.2, 2.2 Hz), 7.08 (1H, m), 7.26 (1H, m).

### f) 8-(2-Methoxyphenyl)-3-(3-methoxyphenyl)-8-azabicyclo-[3.2.1]octane-3-carboxyamide

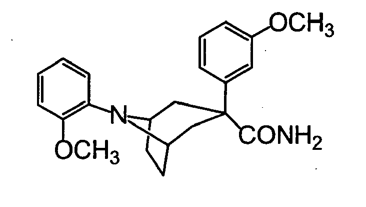

To a solution of 8-(2-methoxyphenyl)-3-(3-methoxyphenyl)-8-azabicyclo[3.2.1]octane-3-carbonitrile (285 mg, 0.818 mmol) in dimethylsulfoxide (4 mL) at room temperature, 6N aqueous potassium hydroxide (4 mL) was added dropwise, and then the mixture was stirred for 10 hours at 100°C. After cooling the reaction mixture at 0°C, water (12 mL) was added thereto, and the mixture was stirred for 30 minutes. The mixture was filtrated by aspiration to give 295 mg of the title compound as a white solid.
¹H-NMR (DMSO-d₆) δ; 1.73 (2H, m), 1.94 (4H, m), 2.89 (2H, m), 3.69 (3H, s), 3.72 (3H, s), 4.24 (2H, m), 6.67-6.86 (7H, m), 6.92 (1H, brs.), 7.15 (1H, dd, J=8.0,8.0 Hz), 7.34 (1H, brs.).

### g) Methyl 8-(2-methoxyphenyl)-3-(3-methoxyphenyl)-8-azabicyclo[3.2.1]octane-3-carboxylate

To 8-(2-methoxyphenyl)-3-(3-methoxyphenyl)-8-azabicyclo[3.2.1]octane-3-carboxyamide (345 mg, 0.941 mmol), concentrated hydrobromic acid (5.0 mL) was added at room temperature, and then the mixture was stirred for 14 hours under reflux. The reaction mixture was concentrated in vacuo, and the residue was washed with ethyl acetate to give a hydrobromide.

To a solution of the hydrobromide (470 mg) in N,N-dimethylformamide (5.0 mL) were added cesium carbonate (2.19 g, 5.65 mmol) and methyl iodide (279 µl, 3.77 mmol) at room temperature, and then the mixture was stirred for 4.5 hours. Another more cesium carbonate (1.88 mmol) and methyl iodide (1.88 mmol) were added thereto, and then the mixture was stirred for additional 14 hours. Water was added thereto to quench the reaction, and then the mixture was extracted with ethyl acetate. The ethyl acetate was washed with brine, dried over anhydrous magnesium sulfate, filtrated, and then the solvent was removed off in vacuo. The residue was purified by silica gel chromatography to give 136 mg of the title compound as a white solid.
¹H-NMR (CDCl₃) δ; 1.81 (2H, m), 1.92 (2H, m), 2.34 (2H, m), 2.97 (2H, m), 3.66 (3H, s), 3.77 (3H, s), 3.83 (3H, s), 4.29 (2H, m), 6.74 (1H, m), 6.82 (4H, m), 6.88 (1H, m), 6.92 (1H, d, J=8.0 Hz), 7.17 (1H, dd, J=8.0,8.0 Hz).

### Example 50

### Preparation of endo-9-(2-methoxyphenyl)-3-(3-methoxyphenyl)-9-azabicyclo[3.3.1]nonane-3-carboxylic acid

### a) Dimethyl cis-1-(2-methoxyphenyl)piperidine-2,6-dicarboxylate

To a solution of dimethyl 2,6-dibromoheptanoate (10.0 g, 0.0289 mol) in toluene (30 mL) at 80°C, 2-methoxyaniline (5.34 g, 0.0434 mol) was added dropwise, and then the mixture was stirred for 19 hours at 100°C. The reaction mixture was cooled to room temperature, and then filtrated through Celite®. The solvent was removed from the filtrate in vacuo and the residue was purified by silica gel column chromatography. The obtained crude product was purified by recrystallization with diisopropyl ether to give 1.24 g of the title compound as a white solid.
¹H-NMR (CDCl₃) δ; 1.56 (1H, m), 1.92 (5H, m), 3.48 (6H, s), 3.86 (3H, s), 4.21 (2H, m), 6.82 (2H, m), 7.09 (1H, m), 7.15 (1H, dd, J=7.7, 1.8 Hz).

### b) [Cis-1-(2-methoxyphenyl)piperidin-2,6-diyl]dimethanol

The title compound was prepared in a similar manner to Example 48 b).
¹H-NMR (CDCl₃) δ; 1.57 (1H, m), 1.68 (2H, m), 1.95 (3H, m), 2.90 (2H, m), 3.01 (2H, dd, J=10.6, 1.4 Hz), 3.11 (2H, m), 3.18 (2H, m), 3.90 (3H, s), 6.98 (1H, dd, J=8.2, 1.3 Hz), 7.03 (1H, ddd, J=7.7, 7.7, 1.3 Hz), 7.23 (1H, m), 7.33 (1H, dd, J=7.9, 1.6 Hz).

### c) Cis-2,6-bis(chloromethyl)-1-(2-methoxyphenyl)piperidine

To a solution of [cis-1-(2-methoxyphenyl)piperidin-2,6-diyl]dimethanol (980 mg, 3.90 mmol) in chloroform (10 mL) at 0°C, thionyl chloride (1.02 g, 8.58 mmol) was added dropwise, and then the mixture was stirred for 30 minutes at room temperature. After further stirring the mixture for 10 minutes under reflux, the solvent was removed from the mixture in vacuo. The residue was diluted with saturated aqueous sodium hydrogencarbonate, and extracted with diethyl ether. The diethyl ether layer was dried over anhydrous magnesium sulfate, filtrated, and then the solvent was removed off in vacuo. The residue was purified by silica gel column chromatography to give 805 mg of the title compound as a light yellow solid.
¹H-NMR (CDCl₃) δ; 1.51 (3H, m), 1.91 (1H, m), 2.00 (2H, m), 3.14 (2H, dd, J=11.0, 7.0 Hz), 3.19 (2H, d, J=11.0, 2.9 Hz), 3.42 (2H, m), 3.82 (3H, s), 6.89 (2H, m), 7.22 (2H, m).

### d) Exo-9-(2-methoxyphenyl)-3-(3-methoxyphenyl)-9-azabicyclo[3.3.1]nonane-3-carbonitrile and endo-9-(2-methoxyphenyl)-3-(3-methoxyphenyl)-9-azabicyclo[3.3.1]-nonane-3-carbonitrile

To a suspension of 55% sodium hydride (254 mg, 5.83 mmol) in N,N-dimethylformamide (4.0 mL) at 0°C, a solution of cis-2,6-bis(chloromethyl)-1-(2-methoxyphenyl)piperidine (400 mg, 1.39 mmol) and 3-methoxyphenylacetonitrile (409 mg, 2.78 mmol) in N,N-dimethylformamide (3.0 mL) was added dropwise. The residual solution was added with N,N-dimethylformamide (1.0 mL), and the mixture was stirred overnight at room temperature. Saturated aqueous ammonium chloride was added thereto to quench the reaction, and then the mixture was extracted with ethyl acetate. The ethyl acetate layer was washed with brine, dried over anhydrous magnesium sulfate, filtrated, and then the solvent was removed from the filtrate in vacuo. The residue was purified by silica gel column chromatography to give 218 mg of the exo form of the title compound as a light red and 150 mg of the endo form thereof as a light yellow solid. Exo-9-(2-methoxyphenyl)-3-(3-methoxyphenyl)-9-azabicyclo-[3.3.1]nonane-3-carbonitrile;
¹H-NMR (CDCl₃) δ; 1.69 (1H, m), 1.86 (2H, m), 2.06 (2H, m), 2.20 (1H, m), 2.34 (2H, dd, J=14.3, 2.6 Hz), 2.67 (2H, dd, J=14.3, 7.5 Hz), 3.62 (3H, s), 3.69 (3H, s), 4.14 (2H, m), 6.71-6.89 (5H, m), 7.02 (1H, dd, J=2.1, 2.1 Hz), 7.09(1H, m), 7.22 (1H, dd, J=8.0, 8.0 Hz).
Endo-9-(2-methoxyphenyl)-3-(3-methoxyphenyl)-9-azabicyclo-[3.3.1]nonane-3-carbonitrile;
¹H-NMR (CDCl₃) δ; 1.36 (2H, m), 1.68 (1H, m), 1.90-2.13 (5H, m), 2.74 (2H, m), 3.85 (3H, s), 3.88 (3H, s), 4.34 (2H, m), 6.87 (4H, m), 6.96 (1H, m), 7.20 (2H, m), 7.33 (1H, dd, J=7.9, 7.9 Hz).

### e) Endo-9-(2-methoxyphenyl)-3-(3-methoxyphenyl)-9-azabicyclo[3.3.1]nonane-3-carboxyamide

The title compound was prepared in a similar manner to Example 49 f).
¹H-NMR (DMSO-d₆) δ; 1.46 (3H, m), 1.82 (2H, m), 1.95 (1H, m), 2.45 (4H, m), 3.53 (3H, s), 3.63 (3H, s), 3.99 (2H, m), 6.59-6.74 (5H, m), 6.82 (1H, s), 6.89 (1H, m), 6.94 (1H, d, J=7.9 Hz), 7.05 (1H, s), 7.10 (1H, dd, J=7.9, 7.9 Hz).

### f) Methyl endo-9-(2-methoxyphenyl)-3-(3-methoxyphenyl)-9-azabicyclo[3.3.1]nonane-3-carboxylate

To endo-9-(2-methoxyphenyl)-3-(3-methoxyphenyl)-9-azabicyclo[3.3.1]nonane-3-carboxyamide (150 mg, 0.394 mmol), concentrated aqueous hydrobromic acid (3.0 mL) was added, and then the mixture was heated under reflux for 3 hours. The reaction mixture was concentrated in vacuo, and the residue was washed with methanol/ethyl acetate/diethyl ether, dissolved in N,N-dimethylformamide (3.0 mL), and at room temperature methyl iodide (98 µl, 1.58 mmol) and cesium carbonate (1.03 g, 3.15 mmol) was added thereto, and the mixture was stirred for 3 days. Saturated aqueous ammonium chloride was added thereto to quench the reaction, and the reactant was extracted with ethyl acetate. The ethyl acetate layer was washed with brine, dried over anhydrous magnesium sulfate, filtrated, and then the solvent was removed from the filtrate in vacuo. The residue was purified by silica gel column chromatography to give 166 mg of the title compound as a colorless oil.
¹H-NMR (CDCl₃) δ; 1.63 (4H, m), 2.01 (2H, m), 2.61 (2H, dd, J=14.3, 7.3 Hz), 2.71 (2H, dd, J=14.3, 2.7 Hz), 3.63 (3H, s), 3.64 (3H, s), 3.70 (3H, s), 4.11 (2H, m), 6.65-6.83 (5H, m), 6.94 (1H, m), 7.00 (1H, m), 7.13 (1H, dd, J=8.0, 8.0 Hz).

### g) Endo-9-(2-methoxyphenyl)-3-(3-methoxyphenyl)-9-azabicyclo[3.3.1]nonane-3-carboxylic acid

To a solution of methyl endo-9-(2-methoxyphenyl)-3-(3-methoxyphenyl)-9-azabicyclo[3.3.1]nonane-3-carboxylate (150 mg, 0.379 mmol) in dimethylsulfoxide (2.0 mL) at room temperature, 6N aqueous potassium hydroxide (1.0 mL) was added dropwise, and then the mixture was stirred for 1 hour at 100°C. The reaction mixture was cooled, diluted with water, and then adjusted to pH=5 using concentrated hydrocholic acid. The solid was collected by filtration of the mixture using aspiration, washed with water to give 114 mg of the title compound as a light green solid.
¹H-NMR (DMSO-d₆) δ; 1.51 (3H, m), 1.85 (3H, m), 2.41 (2H, dd, J=14.3, 7.3 Hz), 2.59 (2H, m), 3.30 (3H, s), 3.65 (3H, s), 3.99 (2H, m), 6.62-6.79 (5H, m), 6.88 (1H, m), 6.97 (1H, m), 7.14 (1H, dd, J=8.0, 8.0 Hz), 12.41 (1H, brs.).

### Example 51

### Preparation of methyl 4-(3-methoxyphenyl)piperidine-4-carboxylate

To a solution of methyl 1-(diphenylmethyl)-4-(3-methoxyphenyl)piperidine-4-carboxylate (600 mg, 1.44 mmol) in methanol (12 mL) and tetrahydrofuran (6.0 mL), 10%-palladium carbon (120 mg) was added at room temperature, and then the mixture was stirred under hydrogen atmosphere for 5.5 hours. The reaction mixture was filtrated through Celite®, and the solvent was removed from the filtrate in vacuo. The residue was purified by silica gel column chromatography to give 351 mg of the title compound as a colorless oil.
¹H-NMR (CDCl₃) δ; 1.83 (2H, m), 2.50 (2H, m), 2.78 (2H, m), 3.03 (2H, m), 3.66 (3H, s), 3.80 (3H, s), 6.81 (1H, m), 6.93 (1H, m), 6.98 (1H, m), 7.26 (1H, dd, J=8.1 Hz).

### Example 52

### Preparation of methyl {[1-(2-methoxyphenyl)-4-(3-methoxyphenyl)piperidin-4-yl]methyl}carbamate

To a solution of {[1-(2-methoxyphenyl)-4-(3-methoxyphenyl)piperidin-4-yl]methyl}amine (210 mg, 0.643 mmol) in dichloromethane (8.0 mL) at 0°C were added methyl chloroformate (55 µl, 0.708 mmol) and triethylamine (108 µl, 0.772 mmol), and then the mixture was stirred for 2 hours at room temperature. Water was added to the reaction mixture to quench the reaction, the mixture was extracted with chloroform. The chloroform layer was dried over anhydrous magnesium sulfate, filtrated, and then the solvent was removed from the filtrate in vacuo. The residue was purified by silica gel column chromatography to give 222 mg of the title compound as a colorless oil.
¹H-NMR (CDCl₃) δ; 2.07 (2H, m), 2.26 (2H, m), 2.92 (2H, m), 3.22 (2H, m), 3.41 (2H, d, J=6.4 Hz), 3.62 (3H, s), 3.82 (3H, s), 3.85 (3H, s), 4.33 (1H, m), 6.77-6.98 (7H, m), 7.30 (1H, dd, J=8.0, 8.0 Hz).

### Example 53

### Preparation of cis-1-(3-methoxyphenyl)-4-piperidin-1-yl-cyclohexanecarboxyamide

The title compound was prepared in a similar manner to Example 49 f).
¹H-NMR (DMSO-d₆) δ; 1.41 (10H, m), 1.68 (2H, m), 2.22 (1H, m), 2.40 (4H, m), 2.54 (2H, m), 3.71 (3H, s), 6.78 (1H, dd, J=8.0, 2.4 Hz), 6.85 (1H, s), 6.89 (1H, d, J=2.4 Hz), 6.94 (1H, d, J=8.0 Hz), 7.20 (1H, dd, J=8.0, 8.0 Hz).

### Example 54

### 4-(3-Methoxyphenyl)-1-pyrimidin-2-ylpiperidine-4-amine

To a solution of 4-(3-methoxyphenyl)-1-pyrimidin-2-ylpiperidine-4-carboxylic acid (200 mg, 0.638 mmol) in toluene (4.0 mL) were added triethylamine (98 µl, 0.702 mmol) and diphenylphosphoryl azide (142 µl, 0.657 mmol) dropwise at room temperature, and the mixture was stirred for 1.5 hours at 80°C. Additional triethylamine (44 µl, 0.319 mmol) and diphenylphosphoryl azide (28 ul, 0.128 mmol) were added thereto, and the mixture was stirred under heating for additional 1 hour. To the reaction mixture, 20% aqueous hydrochloric acid (4.0 mL) was added, and the mixture was stirred for 4 hours at 100°C. The reaction mixture was cooled to room temperature, the toluene was removed from the mixture in vacuo to give a aqueous layer. The aqueous layer was adjusted to pH=8 with aqueous sodium hydroxide. The precipitated solid was collected by aspirated filtration, and dried to give a crude product. The residue was purified by silica gel column chromatography to give 153 mg of the title compound as a colorless oil.
¹H-NMR (DMSO-d₆) δ; 1.62 (2H, m), 1.84 (2H, m), 1.92 (2H, s), 3.44 (2H, m), 3.73 (3H, s), 4.37 (2H, m), 6.55 (1H, dd, J=4.7, 4.7 Hz), 6.76 (1H, dd, J=8.0, 2.2 Hz), 7.06 (1H, d, J=8.0 Hz), 7.09 (1H, d, J=2.2 Hz), 7.20 (1H, dd, J=8.0, 8.0 Hz), 8.32 (2H, d, J=4.7 Hz).

### Example 55-1

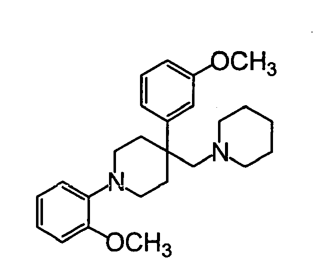

### Preparation of 1-(2-methoxyphenyl)-4-(3-methoxyphenyl)-4-(piperidin-1-ylmethyl)piperidine

To a solution of [1-(2-methoxyphenyl)-4-(3-methoxyphenyl)piperidin-4-yl]methanol (100 mg, 0.305 mmol) in pyridine (10 mL), p-toluenesulfonyl chloride (146 mg, 0.764 mmol) was added at room temperature. The reaction mixture was warmed at 50°C and stirred for 3 hours. Saturated aqueous ammonium chloride was added thereto to quench the reaction, and then the mixture was extracted with ethyl acetate. The organic layer was washed with saturated aqueous ammonium chloride 3 times, dried over anhydrous magnesium sulfate, filtrated, and then the solvent was removed from the filtrate in vacuo. The residual reaction mixture was dissolved in piperidine (5 mL) and heated under reflux for 2 hours. The piperidine was removed off in vacuo and the mixture was extracted with ethyl acetate. The organic layer was washed with water 3 times, dried over anhydrous magnesium sulfate, filtrated, and then the solvent was removed from the filtrate in vacuo. The residue was purified by silica gel chromatography to give 772.3 mg of the title compound as a colorless oil. High-performance liquid chromatography/mass spectrometry
m/z 395.3 (M+H)
Retention time: 2.44 min.

### Example 55-2

### 4-{[1-(2-Methoxyphenyl)-4-(3-methoxyphenyl)piperidin-4-yl]methyl}morpholine

The title compound was prepared using morpholine instead of piperidine in a similar manner to Example 55-1.
High-performance liquid chromatography/mass spectrometry
m/z 397.5 (M+H)
Retention time: 2.36 min.

### Example 55-3

### N-Ethyl-N-{[1-(2-methoxyphenyl)-4-(3-methoxyphenyl)-piperidin-4-yl]methyl}ethylamine

The title compound was prepared using diethylamine instead of piperidine in a similar manner to Example 55-1.
High-performance liquid chromatography/mass spectrometry
m/z 383.2 (M+H)
Retention time: 2.96 min.

### Example 56

### (6E)-3,5-Dihydroxy-7-[1-(2-methoxyphenyl)-4-(3-methoxyphenyl)piperidin-4-yl]hept-6-enoic acid

### 1-(2-Methoxyphenyl)-4-(3-methoxyphenyl)piperidine-4-carbaldehyde

To a solution of 1-(2-methoxyphenyl)-4-(3-methoxyphenyl)piperidine-4-carbonitrile (2.89 g, 8.96 mmol) in toluene (20 mL) in an ice bath, 10.7 ml of 1.01 M of diisobutyl aluminium hydride (DIBAL) in hexane (10.8 mmol) was added, and then the mixture was warmed to room temperature and stirred for 4 hours. 10% Aqueous hydrochloric acid was added thereto to quench the reaction, and then the reaction mixture was basified with 2N aqueous sodium hydroxide and extracted with toluene. The organic layer was washed with 2N aqueous sodium hydroxide once and water once, dried over anhydrous magnesium sulfate, filtrated, and then the solvent was removed from the filtrate in vacuo. The residue was purified by silica gel chromatography to give 3.34 g of the title compound as a light yellow oil.
¹H-NMR δ (DMSO-d₆); 2.23-2.34 (2H, m), 2.53-2.58 (2H, m), 2.81-2.90 (2H, m), 3.33-3.40 (2H, m), 3.81 (3H, s), 3.87 (3H, s), 6.82-7.11 (7H, m), 7.36 (1H, t, J = 7.14 Hz), 9.44 (1H, s).

### Methyl (6E)-3-{[tert-butyl(dimethyl)silyl]oxy}-7-[1-(2-methoxyphenyl)-4-(3-methoxyphenyl)piperidin-4-yl]-5-oxohept-6-enoate

To a solution of 1-(2-methoxyphenyl)-4-(3-methoxyphenyl)piperidine-4-carbaldehyde (213 mg, 0.654 mmol) and methyl 3-{[tert-butyl(dimethyl)silyl]oxy}-6-(dimethoxy-phosphoryl)-5-oxohexanoate (250 mg, 0.654 mmol) that was prepared according to the reference (J. Org. Chem., (1991), 56, 3744.), in acetonitrile (10 mL) in an ice bath; 0.654 ml of 1M sodium methoxide in methanol (0.654 mmol) was added dropwise, and then the mixture was warmed to room temperature and stirred overnight. Saturated aqueous ammonium chloride was added thereto to quench the reaction, and then the mixture was extracted with ethyl acetate. The organic layer was washed with saturated aqueous ammonium chloride twice, dried over anhydrous magnesium sulfate, filtrated, and then the solvent was removed from the filtrate in vacuo. The residue was purified by silica gel chromatography to give 99.8 mg of the title compound as a colorless oil.
¹H-NMR δ (DMSO-d₆); 2.24-2.30 (2H, m), 2.39-2.58 (4H, m), 2.69-2.86 (2H, m), 3.09-3.13 (4H, m), 3.66 (3H, s), 3.82 (3H, s), 3.88 (3H, s), 4.59-4.62 (1H, m), 6.00 (1H, d, J = 16.3), 6.77-7.00 (7H, m), 7.28 (1H, t, J = 8.04).

### Methyl (6E)-3-hydroxy-7-[1-(2-methoxyphenyl)-4-(3-methoxyphenyl)piperidin-4-yl]-5-oxohept-6-enoate

To a solution of methyl (6E)-3-{[tert-butyl(dimethyl)-silyl]oxy}-7-[1-(2-methoxyphenyl)-4-(3-methoxyphenyl)-piperidin-4-yl]-5-oxohept-6-enoate (99.0 mg, 0.170 mmol) in acetonitrile (10 mL) in an ice bath, 47% aqueous hydrogen fluoride (0.50 mmol) was added dropwise, and the mixture was warmed to room temperature and stirred for 2 hours. Saturated aqueous sodium hydrogencarbonate was added thereto to quench the reaction, and then the mixture was extracted with ethyl acetate. The ethyl acetate layer was washed with saturated aqueous sodium hydrogencarbonate twice, dried over anhydrous magnesium sulfate, filtrated, and then the solvent was removed off in vacuo. The residue was purified by silica gel chromatography to give 82.3 mg of the title compound as a colorless oil.
High-performance liquid chromatography/mass spectrometry
m/z 468.1 (M+H)
Retention time: 2.55 min.

### Methyl (6E)-3,5-dihydroxy-7-[1-(2-methoxyphenyl)-4-(3-methoxyphenyl)piperidin-4-yl]hept-6-enoate

To a solution of methyl (6E)-3-hydroxy-7-[1-(2-methoxyphenyl)-4-(3-methoxyphenyl)piperidin-4-yl]-5-oxohept-6-enoate (82.3 mg, 0.176 mmol) in tetrahydrofuran (THF, 10 mL) at -78°C, 0.178 ml of 1M diethylmethoxyborane in tetrahydrofuran (0.178 mmol) was added dropwise. Then, sodium borohydride (8.00 mg, 0.211 mmol) was added thereto at the same temperature, and the mixture was stirred for 2.5 hours. Saturated aqueous ammonium chloride was added thereto to quench the reaction, and then the mixture was extracted with ethyl acetate. The organic layer was washed with saturated aqueous ammonium chloride twice, dried over anhydrous magnesium sulfate, filtrated, and then the solvent was removed from the filtrate in vacuo. The residue was purified by silica gel chromatography to give 42.3 mg of the title compound as a colorless oil.
High-performance liquid chromatography/mass spectrometry
m/z 470.1 (M+H)
Retention time: 2.42 min.

### (6E)-3,5-Dihydroxy-7-[1-(2-methoxyphenyl)-4-(3-methoxyphenyl)piperidin-4-yl]hept-6-enoic acid

To a solution of methyl (6E)-3,5-dihydroxy-7-[1-(2-methoxyphenyl)-4-(3-methoxyphenyl)piperidin-4-yl]hept-6-enoate (21.2 mg, 0.0451 mmol) in ethanol (THF, 10 mL) at room temperature, 2.00 mL of 0.1 M sodium hydroxide in ethanol was added dropwise, and then the mixture was stirred overnight. Saturated aqueous ammonium chloride was added thereto to quench the reaction, and then the mixture was extracted with ethyl acetate. The organic layer was washed with saturated aqueous ammonium chloride twice, dried over anhydrous magnesium sulfate, filtrated, and then the solvent was removed from the filtrate in vacuo to give 20.4 mg of the title compound as a colorless oil.
High-performance liquid chromatography/mass spectrometry
m/z 456.6 (M+H)
Retention time: 2.36 min.

### Example 57

### Cis-1-(3-methoxyphenyl)-4-[4-(2-methoxyphenyl)piperazin-1-yl]cyclohexanecarbonitrile

A suspension of cis-4-amino-1-(3-methoxyphenyl)-cyclohexanecarbonitrile (100 mg, 0.434 mmol), N,N-bis(2-chloroethyl-2-methoxyaniline (108 mg, 0.654 mmol) and calcium carbonate (180 mg, 1.30 mmol) in N,N-dimethylformamide (DMF, 10 mL) was stirred for 2 days at 70°C. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 29.5 mg of the title compound as a colorless oil.
High-performance liquid chromatography/mass spectrometry
m/z 406.5 (M+H)
Retention time: 2.78 min.

### Example 58-1

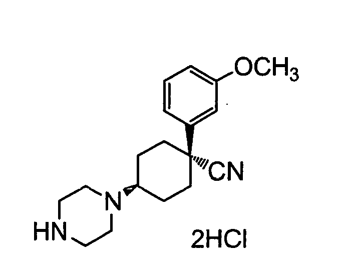

### Preparation of trans-1-(3-methoxyphenyl)-4-piperazin-1-yl-cyclohexanecarbonitrile dihydrochloride

### Preparation of tert-butyl 4-[trans-4-cyano-4-(3-methoxyphenyl)cyclohexyl]piperazine-1-carboxylate and tert-butyl 4-[cis-4-cyano-4-(3-methoxyphenyl)cyclohexyl]piperazine-1-carboxylate

A solution of 1-(3-methoxyphenyl)-4-oxocyclohexane-carbonitrile (2.00 g, 8.72 mmol) and t-butyl 1-piperazinecarboxylate (1.79 g, 9.59 mmol) in 1,2-dichloroethane (50 mL), sodium triacetoxyborohydride (2.77 g, 13.1 mmol) was added at room temperature, and then the mixture was stirred overnight. Saturated aqueous sodium hydrogencarbonate was added thereto to quench the reaction, and then the mixture was extracted with chloroform. The organic layer was washed with saturated aqueous sodium hydrogencarbonate twice, dried over anhydrous magnesium sulfate, filtrated, and then the solvent was removed from the filtrate in vacuo. The residue was purified by silica gel chromatography to give the title compound: tert-butyl 4-[trans-4-cyano-4-(3-methoxyphenyl)cyclohexyl]piperazine-1-carboxylate as a white crystal (740 mg) and tert-butyl 4-[cis-4-cyano-4-(3-methoxyphenyl)cyclohexyl]piperazine-1-carboxylate as a white crystal (3.26 g).
tert-Butyl 4-[trans-4-cyano-4-(3-methoxyphenyl)cyclohexyl]-piperazine-1-carboxylate
High-performance liquid chromatography/mass spectrometry
m/z 400.3 (M+H)
Retention time: 2.61 min.
tert-Butyl 4-[cis-4-cyano-4-(3-methoxyphenyl)cyclohexyl]-piperazin-1-carboxylate
High-performance liquid chromatography/mass spectrometry
m/z 400.3 (M+H)
Retention time: 2.65 min.

### Preparation of trans-1-(3-methoxyphenyl)-4-piperazin-1-yl-cyclohexanecarbonitrile dihydrochloride

tert-Butyl 4-[trans-4-cyano-4-(3-methoxyphenyl)-cyclohexyl]piperazine-1-carboxylate (500 mg, 1.25 mmol) was dissolved in 4M hydrochloric acid/1,4-dioxane solution (20 mL) and then the mixture was stirred for 5 hours at room temperature. The solvent was removed from the reaction mixture in vacuo and then ethyl acetate was added thereto. The precipitated crystal was collected by filtration to give 440 mg of the title compound as a white crystal.
High-performance liquid chromatography/mass spectrometry
m/z 300.3 (M+H)
Retention time: 1.67 min.
Melting point 159-160°C

### Example 58-2

### Preparation of cis-1-(3-methoxyphenyl)-4-piperazin-1-yl-cyclohexanecarbonitrile dihydrochloride

The title compound was prepared using tert-butyl 4-[cis-4-cyano-4-(3-methoxyphenyl)cyclohexyl]piperazine-1-carboxylate in a similar manner to Example 58-1. High-performance liquid chromatography/mass spectrometry
m/z 300.3 (M+H)
Retention time: 1.94 min.
Melting point >300°C

### Example 59

### Preparation of 4-[4-(diphenylmethyl)piperazin-1-yl]-1-(3-methoxyphenyl)cyclohexanecarbonitrile

To a solution of 1-(3-methoxyphenyl)-4-oxocyclohexane-carbonitrile (500 mg, 2.18 mmol), 1-(diphenylmethyl)-piperazine (660 mg, 2.62 mmol) and acetic acid (0.250 ml, 4.36 mmol) in methanol (50 mL) in an ice bath, sodium cyanoborohydride (165 mg, 2.62 mmol) was added, and then the mixture was warmed to room temperature and stirred overnight. Saturated aqueous sodium hydrogencarbonate was added thereto to quench the reaction, and then the mixture was extracted with ethyl acetate. The organic layer was washed with saturated aqueous sodium hydrogencarbonate twice, dried over anhydrous magnesium sulfate, and then the solvent was removed off in vacuo. The residue was purified by silica gel chromatography to give the title compound: 187 mg (Rf=0.7, hexane:ethyl acetate=1:1) as a colorless oil and 576 mg (Rf=0.2, hexane:ethyl acetate=1:1) as a colorless oil.
High-performance liquid chromatography/mass spectrometry
m/z 466.4 (M+H)
Retention time: 3.67 min.
High-performance liquid chromatography/mass spectrometry
m/z 466.4 (M+H)
Retention time: 3.69 min.

### Example 60

### Preparation of 4-[(4-benzoylphenyl)amino]-1-(3-methoxyphenyl)cyclohexanecarbonitrile

### Preparation of (4-aminophenyl)(phenyl)methanol

To a solution of 4-aminobenzophenone (1.00 g, 5.05 mmol) in ethanol (30 mL) in an ice bath, sodium borohydride (764 mg, 20.2 mmol) was added, and then the mixture was warmed to room temperature and stirred overnight. Saturated aqueous ammonium chloride was added thereto to quench the reaction, and then the mixture was extracted with ethyl acetate. The organic layer was washed with saturated aqueous ammonium chloride twice and brine twice dried over anhydrous magnesium sulfate, filtrated, and then the solvent was removed from the filtrate in vacuo. The residue was purified by silica gel chromatography to give 959 mg of the title compound as a light yellow oil.
¹H-NMR δ (DMSO-d₆); 2.24 (1H, br.), 3.64 (2H, br.), 5.74 (1H, s), 5.74 (1H, m), 6.69-6.77 (2H, m), 7.11-7.22 (1H, m), 7.23-7.40 (5H, m).

### Preparation of 4-[(4-benzoylphenyl)amino]-1-(3-methoxyphenyl)cyclohexanecarbonitrile

To a solution of 1-(3-methoxyphenyl)-4-oxocyclohexane-carbonitrile (400 mg, 2.01 mmol) and (4-aminophenyl)(phenyl)methanol (598 mg, 2.61 mmol) in 1,2-dichloroethane (30 mL), sodium triacetoxyborohydride (639 mg, 3.02 mmol) was added at room temperature, and then the mixture was stirred overnight. Saturated aqueous sodium hydrogencarbonate was added thereto to quench the reaction, and then the mixture was extracted with chloroform. The organic layer was washed with saturated aqueous sodium hydrogencarbonate twice, dried over anhydrous magnesium sulfate, filtrated, and then the solvent was removed from the filtrate in vacuo. The residue was purified by silica gel chromatography to give 1.00 g of the title compound as a light yellow oil.
High-performance liquid chromatography/mass spectrometry
m/z 413.2 (M+H)
Retention time: 3.11 min.

### Example 61

### Preparation of 4-({4-[hydroxy(phenyl)methyl]phenyl}amino)-1-(3-methoxyphenyl)cyclohexanecarbonitrile

A suspension of 4-[(4-benzoylphenyl)amino]-1-(3-methoxyphenyl)cyclohexanecarbonitrile (500 mg, 1.19 mmol) and manganese dioxide (1.30 g, 11.9 mmol) in chloroform (30 mL) was stirred for 4 hours at 40°C. The manganese dioxide was filtrated off using Celite®, and then the solvent was removed from the filtrate in vacuo. The residue was purified by silica gel chromatography to give 226 mg of the title compound as a light yellow crystal.
High-performance liquid chromatography/mass spectrometry
m/z 411.2 (M+H)
Retention time: 4.03 min.

### Example 62

### Preparation of cis-4-(4-acetylpiperazin-1-yl)-1-(3-methoxyphenyl)cyclohexanecarbonitrile

To a solution of cis-1-(3-methoxyphenyl)-4-piperazin-1-ylcyclohexanecarbonitrile dihydrochloride (50 mg, 0.134 mmol) and triethylamine (0.0654 ml, 0.469 mmol) in dichloromethane (5 mL) in an ice bath, acetyl chloride (0.0114 ml, 0.161 mmol) was added, and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 35.6 mg of the title compound as a white crystal.
High-performance liquid chromatography/mass spectrometry
m/z 342.3 (M+H)
Retention time: 2.76 min.

### Example 63

### Preparation of cis-4-(4-benzoylpiperazin-1-yl)-1-(3-methoxyphenyl)cyclohexanecarbonitrile

To a solution of cis-1-(3-methoxyphenyl)-4-piperazin-1-ylcyclohexanecarbonitrile dihydrochloride (50 mg, 0.134 mmol) and triethylamine (0.0654 ml, 0.469 mmol) in dichloromethane (5 mL) in an ice bath, benzoyl chloride (0.0187 ml, 0.161 mmol) was added, and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 51.4 mg of the title compound as a white crystal.
High-performance liquid chromatography/mass spectrometry
m/z 404.5 (M+H)
Retention time: 3.07 min.

### Example 64

### Preparation of cis-4-(4-benzylpiperazin-1-yl)-1-(3-methoxyphenyl)cyclohexanecarbonitrile

A solution of cis-1-(3-methoxyphenyl)-4-piperazin-1-ylcyclohexanecarbonitrile dihydrochloride (50 mg, 0.134 mmol) and triethylamine (0.0654 ml, 0.469 mmol) in dichloromethane (5 mL) in an ice bath, benzyl bromide (0.0191 ml, 0.161 mmol) was added, and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 36.2 mg of the title compound as a white crystal.
High-performance liquid chromatography/mass spectrometry
m/z 390.5 (M+H)
Retention time: 2.88 min.

### Example 65

### Preparation of cis-1-(3-methoxyphenyl)-4-[4-(methanesulfonyl)piperazin-1-yl]cyclohexanecarbonitrile

To a solution of cis-1-(3-methoxyphenyl)-4-piperazin-1-ylcyclohexanecarbonitrile dihydrochloride (50 mg, 0.134 mmol) and triethylamine (0.0654 ml, 0.469 mmol) in dichloromethane (5 mL) in an ice bath, methanesulfonyl chloride (0.0125 ml, 0.161 mmol) was added , and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 38.6 mg of the title compound as a white crystal. High-performance liquid chromatography/mass spectrometry
m/z 378.5 (M+H)
Retention time: 2.36 min.

### Example 66

### Preparation of cis-1-(3-methoxyphenyl)-4-{4-[(4-methylphenyl)sulfonyl]piperazin-1-yl}cyclohexanecarbonitrile

To a solution of cis-1-(3-methoxyphenyl)-4-piperazin-1-ylcyclohexanecarbonitrile dihydrochloride (50 mg, 0.134 mmol) and triethylamine (0.0654 ml, 0.469 mmol) in dichloromethane (5 mL) in an ice bath, p-toluenesulfonyl chloride (0.0125 ml, 0.161 mmol) was added, and then the mixture was warmed to room temperature and stirred overnight. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 57.9 mg of the title compound as a white crystal.
High-performance liquid chromatography/mass spectrometry
m/z 454.4 (M+H)
Retention time: 2.78 min.

### Example 67

### Preparation of cis-1-(3-methoxyphenyl)-4-(4-methyl-piperazin-1-yl)cyclohexanecarbonitrile

To a solution of cis-1-(3-methoxyphenyl)-4-piperazin-1-ylcyclohexanecarbonitrile dihydrochloride (50 mg, 0.134 mmol), triethylamine (0.0654 ml, 0.469 mmol) and potassium carbonate (92.6 mg, 0.670 mmol) in N,N-dimethylformamide (DMF, 1.5 mL), methyl iodide (0.010 ml, 0.161 mmol) was added at room temperature, and then the mixture was warmed to 40°C and stirred for 5 hours. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 9.30 mg of the title compound as a white crystal. High-performance liquid chromatography/mass spectrometry
m/z 314.3 (M+H)
Retention time: 1.96 min.

### Example 68

### Preparation of cis-1-(3-methoxyphenyl)-4-pyrimidin-2-yl-piperazin-1-yl)cyclohexanecarbonitrile

To a solution of cis-1-(3-methoxyphenyl)-4-piperazin-1-ylcyclohexanecarbonitrile dihydrochloride (50 mg, 0.134 mmol), triethylamine (0.0654 ml, 0.469 mmol) and potassium carbonate (92.6 mg, 0.670 mmol) in N,N-dimethylformamide (DMF, 1.5 mL) and 2-chloropyrimidine (23.0 mg, 0.201 mmol) were added at room temperature, and then the mixture was heated to 70°C and stirred for 5 hours. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 38.2 mg of the title compound as a white crystal.
High-performance liquid chromatography/mass spectrometry
m/z 378.5 (M+H)
Retention time: 2.44 min.

### Example 69

### Preparation of cis-1-(3-methoxyphenyl)-4-[4-(4-methoxy-pyrimidin-2-yl)piperazin-1-yl]cyclohexanecarbonitrile

To a solution of cis-1-(3-methoxyphenyl)-4-piperazin-1-ylcyclohexanecarbonitrile dihydrochloride (50 mg, 0.134 mmol), triethylamine (0.0654 ml, 0.469 mmol) and potassium carbonate (92.6 mg, 0.670 mmol) in N,N-dimethylformamide (DMF, 1.5 mL), 2-chloro-4-methoxypyrimidine (29.1 mg, 0.201 mmol) was added at room temperature, and then the mixture was heated to 70°C and stirred for 5 hours. Water was added thereto to quench the reaction, and then the mixture was extracted with ethyl acetate. The organic layer was washed with water twice, dried over anhydrous magnesium sulfate, filtrated, and the solvent was removed from the filtrate in vacuo. The residue was purified by silica gel chromatography to give 39.3 mg of the title compound as a white crystal.
High-performance liquid chromatography/mass spectrometry
m/z 408.4 (M+H)
Retention time: 2.36 min.

### Example 70

### Preparation of cis-4-[4-(1,3-benzoxazol-2-yl)piperazin-1-yl]-1-(3-methoxyphenyl)cyclohexanecarbonitrile

To a solution of cis-1-(3-methoxyphenyl)-4-piperazin-1-ylcyclohexanecarbonitrile dihydrochloride (50 mg, 0.134 mmol), triethylamine (0.0654 ml, 0.469 mmol) and potassium carbonate (92.6 mg, 0.670 mmol) in N,N-dimethylformamide (DMF, 1.5 mL), 2-chlorobenzoxazole (27.4 mg, 0.161 mmol) was added at room temperature, and then the mixture was heated at 70°C and stirred for 5 hours. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 47.2 mg of the title compound as a white crystal.
High-performance liquid chromatography/mass spectrometry
m/z 417.4 (M+H)
Retention time: 2.62 min.

### Example 71

### Preparation of cis-4-[4-(1,3-benzothiazol-2-yl)piperazin-1-yl]-1-(3-methoxyphenyl)cyclohexanecarbonitrile

To a solution of cis-1-(3-methoxyphenyl)-4-piperazin-1-ylcyclohexanecarbonitrile dihydrochloride (50 mg, 0.134 mmol), triethylamine (0.0654 ml, 0.469 mmol) and potassium carbonate (92.6 mg, 0.670 mmol) in N,N-dimethylformamide (DMF, 1.5 mL), 2-chlorobenzothiazole (27.3 mg, 0.161 mmol) was added at room temperature, and the mixture was warmed to 70°C and stirred for 5 hours. Water was added thereto to quench the reaction, and then the mixture was extracted with ethyl acetate. The organic layer was washed with water twice, dried over anhydrous magnesium sulfate, filtrated, and then the solvent was removed from the filtrate in vacuo. The residue was purified by silica gel chromatography to give 42.5 mg of the title compound as a white crystal.
High-performance liquid chromatography/mass spectrometry
m/z 433.4 (M+H)
Retention time: 2.76 min.

### Example 72

### Preparation of cis-4-anilino-1-(3-methoxyphenyl)-cyclohexanecarbonitrile

To a solution of 1-(3-methoxyphenyl)-4-oxocyclohexane-carbonitrile (50 mg, 0.218 mmol) and aniline (0.030 ml, 0.327 mmol) in 1,2-dichloroethane (5 mL), sodium triacetoxyborohydride (69.3 mg, 0.327 mmol) was added at room temperature, and then the mixture was stirred overnight. Saturated aqueous sodium hydrogencarbonate was added thereto to quench the reaction, and then the mixture was extracted with chloroform. The organic layer was washed with saturated aqueous sodium hydrogencarbonate twice, dried over anhydrous magnesium sulfate, filtrated, and then the solvent was removed from the filtrate in vacuo. The residue was purified by silica gel chromatography to give 39.1 mg of the title compound as a white crystal.
High-performance liquid chromatography/mass spectrometry
m/z 307.3 (M+H)
Retention time: 2.90 min.

### Example 73-1

### Preparation of cis-1-(3-methoxyphenyl)-4-[(2-phenylethyl)-amino]cyclohexanecarbonitrile

To a solution of cis-4-amino-1-(3-methoxyphenyl)-cyclohexanecarbonitrile (50.0 mg, 0.217 mmol), 50% phenylacetaldehyde/isopropyl alcohol (78.3 mg, 0.326 mmol) and acetic acid (0.0150 ml, 0.260 mmol) in methanol (5 mL) in an ice bath, sodium cyanoborohydride (20.5 mg, 0.326 mmol) was added, and the mixture was warmed to room temperature and stirred overnight. Saturated aqueous sodium hydrogencarbonate was added thereto to quench the reaction, and then the mixture was extracted with ethyl acetate. The organic layer was washed with saturated aqueous sodium hydrogencarbonate twice, dried over anhydrous magnesium sulfate, filtrated, the solvent was removed from the filtrate in vacuo. The residue was purified by preparative TLC to give 12.9 mg of the title compound as a colorless oil.
High-performance liquid chromatography/mass spectrometry
m/z 335.3 (M+H)
Retention time: 2.73 min.

### Example 73-2

### Preparation of cis-1-(3-methoxyphenyl)-4-[(3-phenylpropyl)-amino]cyclohexanecarbonitrile

The title compound was prepared using 3-phenylpropionaldehyde in a similar manner to Example 73-1. High-performance liquid chromatography/mass spectrometry
m/z 483.5 (M+H)
Retention time: 2.82 min.

### Example 74

### Trans-4-(benzylamino)-1-(3-methoxyphenyl)cyclohexane-carbonitrile

To a solution of trans-4-amino-1-(3-methoxyphenyl)-cyclohexanecarbonitrile (50 mg, 0.217 mmol) and benzaldehyde (0.0265 ml, 0.260 mmol) in 1,2-dichloroethane (5 mL), sodium triacetoxyborohydride (55.1 mg, 0.260 mmol) was added at room temperature, and then the mixture was stirred overnight. Water was added thereto to quench the reaction, and then the mixture was extracted with ethyl acetate. The organic layer was washed with water twice, dried over anhydrous magnesium sulfate, filtrated, and then the solvent was removed off in vacuo. The residue was purified by silica gel chromatography to give 26.7 mg of the title compound as a light yellow oil.
High-performance liquid chromatography/mass spectrometry
m/z 321.3 (M+H)
Retention time: 2.46 min.

### Example 75

### Preparation of cis-4-[benzyl(methyl)amino]-1-(3-methoxyphenyl)cyclohexanecarbonitrile

To a solution of cis-4-(benzylamino)-1-(3-methoxyphenyl)cyclohexanecarbonitrile (50 mg, 0.156 mmol) and potassium carbonate (64.7 mg, 0.468 mmol) in N,N-dimethylformamide (DMF, 1.0 mL), methyl iodide (0.011 ml, 0.172 mmol) was added at room temperature, and then the mixture was warmed to 70°C and stirred for 5 hours. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 23.7 mg of the title compound as a yellow oil.
High-performance liquid chromatography/mass spectrometry
m/z 335.3 (M+H)
Retention time: 2.61 min.

### Example 76

### Preparation of N-benzyl-N-[cis-4-cyano-4-(3-methoxyphenyl)-cyclohexyl]methanesulfonamide

To a solution of cis-4-(benzylamino)-1-(3-methoxyphenyl)cyclohexanecarbonitrile (50 mg, 0.156 mmol) and triethylamine (0.026 ml, 0.167 mmol) in dichloromethane (1.0 mL) in an ice bath, methanesulfonyl chloride (0.013 ml, 0.172 mmol) was added, and then the mixture was warmed to room temperature and stirred overnight. Water was added thereto to quench the reaction, and then the mixture was extracted with chloroform. The organic layer was washed with water once, dried over anhydrous magnesium sulfate, filtrated, and then the solvent was removed from the filtrate in vacuo. The residue was purified by silica gel chromatography to give 53.7 mg of the title compound as a colorless amorphous.
High-performance liquid chromatography/mass spectrometry
m/z 399.2 (M+H)
Retention time: 3.78 min.

### Example 77

### Preparation of N-benzyl-N-[cis-4-cyano-4-(3-methoxyphenyl)-cyclohexyl]acetamide

To a solution of cis-4-(benzylamino)-1-(3-methoxyphenyl)cyclohexanecarbonitrile (50 mg, 0.156 mmol) and triethylamine (0.028 ml, 0.203 mmol) in dichloromethane (1.0 mL) in an ice bath, acetyl chloride (0.013 ml, 0.187 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 56.1 mg of the title compound as a white crystal.
High-performance liquid chromatography/mass spectrometry
m/z 363.3 (M+H)
Retention time: 3.59 min.

### Example 78

### Preparation of N-benzyl-N-[cis-4-cyano-4-(3-methoxyphenyl)-cyclohexyl]-4-methylbenzenesulfonamide

To a solution of cis-4-(benzylamino)-1-(3-methoxyphenyl)cyclohexanecarbonitrile (50 mg, 0.156 mmol) in pyridine (1.0 mL), p-toluenesulfonyl chloride (35.7 mg, 0.187 mmol) was added at room temperature, and then the mixture was warmed to 50°C and stirred for 10 hours. Saturated aqueous ammonium chloride was added thereto to quench the reaction, and then the mixture was extracted with ethyl acetate. The organic layer was washed with saturated aqueous ammonium chloride once, dried over anhydrous magnesium sulfate, filtrated, and then the solvent was removed from the filtrate in vacuo. The residue was purified by silica gel chromatography to give 22.2 mg of the title compound as a white crystal. High-performance liquid chromatography/mass spectrometry
m/z 475.1 (M+H)
Retention time: 4.21 min.

### Example 79

### Preparation of N-benzyl-N-[cis-4-cyano-4-(3-methoxyphenyl)-cyclohexyl]benzamide

To a solution of cis-4-(benzylamino)-1-(3-methoxyphenyl)cyclohexanecarbonitrile (50 mg, 0.156 mmol) and triethylamine (0.0283 ml, 0.203 mmol) in dichloromethane (5 mL) in an ice bath, benzoyl chloride (0.0220 ml, 0.187 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 55.5 mg of the title compound as a white powder.
High-performance liquid chromatography/mass spectrometry
m/z 425.2 (M+H)
Retention time: 3.92 min.

### Example 80

### Cis-4-(diethylamino)-1-(3-methoxyphenyl)cyclohexane-carbonitrile

To a solution of cis-4-amino-1-(3-methoxyphenyl)-cyclohexanecarbonitrile (50 mg, 0.217 mmol) and potassium carbonate (120 mg, 0.868 mmol) in N,N-dimethylformamide (DMF, 1.0 mL), ethyl iodide (0.035 ml, 0.434 mmol) was added at room temperature, and then the mixture was warmed to 50°C and stirred for 5 hours. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 21.9 mg of the title compound as a colorless oil.
High-performance liquid chromatography/mass spectrometry
m/z 287.1 (M+H)
Retention time: 2.34 min.

### Example 81

### Preparation of trans-4-(4-benzylpiperazin-1-yl)-1-(3-methoxyphenyl)cyclohexanecarbonitrile

To a suspension of trans-1-(3-methoxyphenyl)-4-piperazin-1-ylcyclohexanecarbonitrile dihydrochloride (50 mg, 0.134 mmol) and potassium carbonate (92.6 mg, 0.670 mmol) in N,N-dimethylformamide (DMF, 1.0 mL) in an ice bath, benzyl bromide (0.019 ml, 0.161 mmol) was added, and then the mixture was warmed to room temperature and stirred overnight. Water was added thereto to quench the reaction, and then the mixture was extracted with ethyl acetate. The organic layer was washed with brine once, dried over anhydrous magnesium sulfate, filtrated, and then the solvent was removed from the filtrate in vacuo. The residue was purified by silica gel chromatography to give 23.1 mg of the title compound as a colorless amorphous.
High-performance liquid chromatography/mass spectrometry
m/z 390.2 (M+H)
Retention time: 2.38 min.

### Example 82

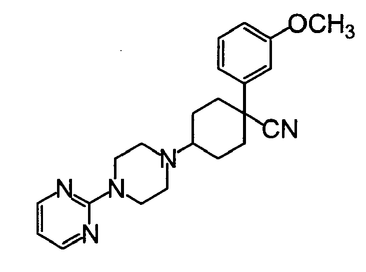

### Preparation of 1-(3-methoxyphenyl)-4-(4-pyrimidin-2-yl-piperazin-1-yl)cyclohexanecarbonitrile

To a suspension of trans-1-(3-methoxyphenyl)-4-piperazin-1-ylcyclohexanecarbonitrile dihydrochloride (50 mg, 0.134 mmol) and potassium carbonate (92.6 mg, 0.670 mmol) in N,N-dimethylformamide (DMF, 1.0 mL), 2-chloropyrimidine (23.0 mg, 0.201 mmol) was added at room temperature, and then the mixture was warmed to 70°C and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 31.7 mg of the title compound as a white powder. High-performance liquid chromatography/mass spectrometry
m/z 378.5 (M+H)
Retention time: 2.34 min.

### Example 83

### Preparation of 4-(benzyloxy)-1-(3-methoxyphenyl)-cyclohexanecarbonitrile

### 4-Hydroxy-1-(3-methoxyphenyl)cyclohexanecarbonitrile

To a solution of 1-(3-methoxyphenyl)-4-oxocyclohexane-carbonitrile (1.50 g, 6.54 mmol) in methanol (20 mL), sodium borohydride (297 mg, 7.85 mmol) was added at room temperature, and then the mixture was stirred overnight. Saturated aqueous ammonium chloride was added thereto to quench the reaction, and then the mixture was extracted with ethyl acetate. The organic layer was washed with saturated aqueous ammonium chloride once and brine once, dried over anhydrous magnesium sulfate, filtrated, and then the solvent was removed from the filtrate in vacuo. The residue was purified by silica gel chromatography to give 1.21 g of the title compound as a colorless oil.
¹H-NMR δ (DMSO-d₆); 1.47-1.60 (2H, m), 1.87-2.07 (6H, m), 3.45-3.56 (1H, m), 3.76 (3H, s), 4.79 (1H, d, J = 4.77), 6.89-6.92 (1H, m), 7.02-7.10 (2H, m), 7.33 (1H, t, J = 7.86).

### 4-(Benzyloxy)-1-(3-methoxyphenyl)cyclohexanecarbonitrile

To a solution of 55% sodium hydride (20.7 mg, 0.475 mmol) in N,N-dimethylformamide (DMF, 5 mL) in an ice bath, 4-hydroxy-1-(3-methoxyphenyl)cyclohexanecarbonitrile (100 mg, 0.432 mmol) and benzyl bromide (0.0167 ml, 0.519 mmol) were added, and then the mixture was warmed to room temperature and stirred overnight. Saturated aqueous ammonium chloride was added thereto to quench the reaction, and then the mixture was extracted with ethyl acetate. The organic layer was washed with saturated aqueous ammonium chloride once, dried over anhydrous magnesium sulfate, filtrated, and then the solvent was removed from the filtrate in vacuo. The residue was purified by silica gel chromatography to give 93.5 mg of the title compound as a white crystal.
¹H-NMR δ (DMSO-d₆); 1.53-1.65 (2H, m), 1.90-2.02 (2H, m), 2.09-2.20 (4H, m), 3.44-3.54 (1H, m), 3.76 (3H, s), 4.55 (1H, d, J = 4.77), 6.90-6.93 (1H, m), 7.03-7.11 (2H, m), 7.24-7.38 (6H, m).

### Example 84

### Preparation of cis-4-[benzyl(butyl)amino]-1-(3-methoxyphenyl)cyclohexanecarbonitrile

### N-Benzyl-N-[cis-4-cyano-4-(3-methoxyphenyl)cyclohexyl]-butanamide

To a solution of cis-4-(benzylamino)-1-(3-methoxyphenyl)cyclohexanecarbonitrile (100 mg, 0.312 mmol) and triethylamine (0.057 ml, 0.406 mmol) in dichloromethane (2.0 mL) in an ice bath, butyryl chloride (0.039 ml, 0.374 mmol) was added, and then the mixture was warmed to room temperature and stirred overnight. Saturated aqueous ammonium chloride was added thereto to quench the reaction, and then the mixture was extracted with ethyl acetate. The organic layer was washed over saturated aqueous ammonium chloride once, dried over anhydrous magnesium sulfate, filtrated, and then the solvent was removed from the filtrate in vacuo. The residue was purified by silica gel chromatography to give 120 mg of the title compound as a white crystal.
High-performance liquid chromatography/mass spectrometry
m/z 391.3 (M+H)
Retention time: 3.88 min.

### Cis-4-[benzyl(butyl)amino]-1-(3-methoxyphenyl)cyclohexane-carbonitrile

To a solution of N-benzyl-N-[cis-4-cyano-4-(3-methoxyphenyl)cyclohexyl]butanamide (85.9 mg, 0.220 mmol) in tetrahydrofuran (THF, 5.0 mL) in an ice bath, 1.1 M borane·tetrahydrofuran complex (0.30 ml, 0.330 mmol) was added, and then the mixture was warmed to room temperature and stirred overnight. 10% Hydrochloric acid/methanol solution was added thereto at room temperature, and then the mixture was warmed to 50°C and stirred for 1 hour. The solvent was removed off, and then saturated aqueous sodium hydrogencarbonate was added thereto to neutralize the residue. The mixture was extracted with ethyl acetate. The organic layer was washed with saturated aqueous sodium hydrogencarbonate once, dried over anhydrous magnesium sulfate, filtrated, the solvent was removed from the filtrate in vacuo to give 2.4 mg of the title compound as a yellow oil.
High-performance liquid chromatography/mass spectrometry
m/z 377.3 (M+H)
Retention time: 2.91 min.

### Example 85

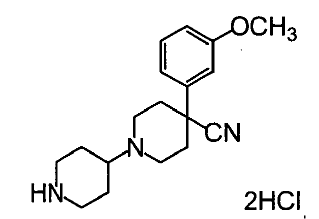

### Preparation of 4-(3-methoxyphenyl)-1,4'-bipiperidine-4-carbonitrile dihydrochloride

### tert-Butyl 4-cyano-4-(3-methoxyphenyl)-1,4'-bipiperidine-1'-carboxylate

To a solution of 4-(3-methoxyphenyl)piperidine-4-carbonitrile (2.47 g, 11.4 mmol) and 1-tert-butoxycarbonyl-4-piperidone (2.50 g, 12.5 mmol) in 1,2-dichloroethane (50 mL), sodium triacetoxyborohydride (3.62 g, 17.1 mmol) was added at room temperature, and the mixture was stirred overnight. Saturated aqueous sodium hydrogencarbonate was added thereto to quench the reaction, and then the mixture was extracted with chloroform. The organic layer was washed with saturated aqueous sodium hydrogencarbonate twice, dried over anhydrous magnesium sulfate, filtrated, and then the solvent was removed from the filtrate in vacuo. The residue was purified by silica gel chromatography to give 1.51 g of the title compound as a colorless oil.
High-performance liquid chromatography/mass spectrometry
m/z 400.3 (M+H)
Retention time: 2.61 min.

### 4-(3-Methoxyphenyl)1,4'-bipiperidine-4-carbonitrile dihydrochloride

tert-Butyl 4-cyano-4-(3-methoxyphenyl)-1,4'-bipiperidine-1'-carboxylate (1.51 g, 3.78 mmol) was dissolved in 4N hydrochloric acid/1,4-dioxane solution (15 mL), and the mixture was stirred overnight at room temperature. The solvent was removed off in vacuo, and then the residue was purified by crystallization with ethyl acetate to give 1.18 g of the title compound as a white solid.
High-performance liquid chromatography/mass spectrometry
m/z 300.3 (M+H)
Retention time: 0.28 min.
Melting point >300°C

### Example 86

### Preparation of 4-(3-methoxyphenyl)-1'-methyl-1,4'-bipiperidine-4-carbonitrile

To a solution of 4-(3-methoxyphenyl)-1,4'-bipiperidine-4-carbonitrile dihydrochloride (50 mg, 0.134 mmol) and potassium carbonate (92.6 mg, 0.670 mmol) in N,N-dimethylformamide (DMF, 1.0 mL), methyl iodide (0.013 ml, 0.201 mmol) was added at room temperature, and then the mixture was warmed to 35°C and stirred for 5 hours. Brine was added thereto to quench the reaction, and then the mixture was extracted with ethyl acetate. The organic layer was washed with brine twice, dried over anhydrous magnesium sulfate, filtrated, and then the solvent was removed from the filtrate in vacuo. The residue was purified by silica gel chromatography to give 22.0 mg of the title compound as a colorless oil.
High-performance liquid chromatography/mass spectrometry
m/z 314.3 (M+H)
Retention time: 0.69 min.

### Example 87

### Preparation of 4-(3-methoxyphenyl)-1'-(methylsulfonyl)-1,4'-bipiperidine-4-carbonitrile

To a solution of 4-(3-methoxyphenyl)-1,4'-bipiperidine-4-carbonitrile dihydrochloride (50 mg, 0.134 mmol) and triethylamine (0.094 ml, 0.670 mmol) in dichloromethane (1.0 mL) in an ice bath, methanesulfonyl chloride (0.013 ml, 0.172 mmol) was added, and then the mixture was warmed to room temperature and stirred overnight. Water was added thereto to quench the reaction, and then the mixture was extracted with chloroform. The organic layer was washed with water once, dried over anhydrous magnesium sulfate, filtrated, and then the solvent was removed from the filtrate in vacuo to give 35.1 mg of the title compound as a white solid.
High-performance liquid chromatography/mass spectrometry
m/z 378.5 (M+H)
Retention time: 2.73 min.

### Example 88

### Preparation of 4-(3-methoxyphenyl)-1'-pyrimidin-1,4'-bipiperidine-4-carbonitrile

To a suspension of 4-(3-methoxyphenyl)-1,4'-bipiperidine-4-carbonitrile dihydrochloride (50 mg, 0.134 mmol) and potassium carbonate (92.6 mg, 0.670 mmol) in N,N-dimethylformamide (DMF, 1.5 mL), 2-chloropyrimidine (23.0 mg, 0.201 mmol) was added at room temperature and then the mixture was heated to 70°C and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 41.2 mg of the title compound as a light brown powder. High-performance liquid chromatography/mass spectrometry
m/z 378.5 (M+H)
Retention time: 2.78 min.

### Example 89

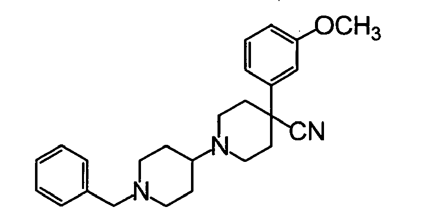

### Preparation of 1'-benzyl-4-(3-methoxyphenyl)-1,4'-bipiperidine-4-carbonitrile

To a solution of 4-(3-methoxyphenyl)-1,4'-bipiperidine-4-carbonitrile dihydrochloride (50 mg, 0.134 mmol) and potassium carbonate (92.6 mg, 0.670 mmol) in N,N-dimethylformamide (DMF, 1.0 mL) in an ice bath, benzyl bromide (0.019 ml, 0.161 mmol) was added, and then the mixture was warmed to room temperature and stirred overnight. Water was added thereto to quench the reaction, and then the mixture was extracted with ethyl acetate. The organic layer was washed with brine once, dried over anhydrous magnesium sulfate, filtrated, and then the solvent was removed from the filtrate in vacuo. The residue was purified by silica gel chromatography to give 15.6 mg of the title compound as a light yellow solid. High-performance liquid chromatography/mass spectrometry
m/z 390.2 (M+H)
Retention time: 2.02 min.

### Example 90

### Preparation of 4-(3-methoxyphenyl)-1'-[(4-methylphenyl)-sulfonyl]-1,4'-bipiperidine-4-carbonitrile

To a solution of 4-(3-methoxyphenyl)-1,4'-bipiperidine-4-carbonitrile dihydrochloride (50 mg, 0.134 mmol) and triethylamine (0.094 ml, 0.670 mmol) in dichloromethane (1.0 mL) in an ice bath, p-toluenesulfonyl chloride (38.3 mg, 0.201 mmol) was added, and then the mixture was warmed to room temperature and stirred overnight. Water was added thereto to quench the reaction, and then the mixture was extracted with chloroform. The organic layer was washed with water once, dried over anhydrous magnesium sulfate, filtrated, and the solvent was removed from the filtrate in vacuo to give 65.8 mg of the title compound as a light blue solid.
High-performance liquid chromatography/mass spectrometry
m/z 454.4 (M+H)
Retention time: 2.71 min.

### Example 91

### 1'-Acetyl-4-(3-methoxyphenyl)-1,4'-bipiperidine-4-carbonitrile

To a solution of 4-(3-methoxyphenyl)-1,4'-bipiperidine-4-carbonitrile dihydrochloride (50 mg, 0.156 mmol) and triethylamine (0.028 ml, 0.203 mmol) in dichloromethane (1.0 mL) in an ice bath, acetyl chloride (0.014 ml, 0.201 mmol) was added, and then the mixture was warmed to room temperature and stirred overnight. Water was added thereto to quench the reaction, and then the mixture was extracted with ethyl acetate, the organic layer was washed with water once, dried over anhydrous magnesium sulfate, filtrated, and then the solvent was removed from the filtrate in vacuo to give 44.6 mg of the title compound as a white crystal.
High-performance liquid chromatography/mass spectrometry
m/z 342.3 (M+H)
Retention time: 2.02 min.

### Example 92-1

### Preparation of trans-1-(3-ethoxyphenyl)-4-piperazin-1-yl-cyclohexanecarbonitrile dihydrochloride

### Methyl 5-cyano-2-hydroxy-5-(3-benzyloxyphenyl)cyclohex-1-ene-1-carboxylate

To a suspension of sodium hydride (14.2 g, 326 mmol) in N,N-dimethylformamide (DMF, 1.0 mL) were added 3-benzyloxyphenylacetonitrile (33.0 g, 148 mmol) and methyl acrylate (33.3 ml, 370 mmol) at -10°C, and then the mixture was warmed to room temperature and stirred overnight. Saturated aqueous ammonia was added thereto to quench the reaction, and then the mixture was extracted with ethyl acetate, and the organic layer washed with saturated aqueous ammonia twice, dried over anhydrous magnesium sulfate, filtrated, and then the solvent was removed from the filtrate in vacuo. The residue was purified by silica gel chromatography to give 39.3 g of the title compound as a yellow oil.
¹H-NMR δ (DMSO-d₆); 2.18-2.87 (6H, m), 3.77 (3H, s), 5.12 (2H, s), 7.02-7.48 (9H, m), 12.1 (1H, s).

### 1-[3-(Benzyloxy)phenyl]-4-oxocyclohexanecarbonitrile

To a solution of methyl 5-cyano-2-hydroxy-5-(3-benzyloxyphenyl)cyclohex-1-ene-1-carboxylate (35.0 g, 96.3 mmol) in 1,4-dioxane (400 mL), a solution of potassium hydroxide (11.9 g, 212 mmol) in water (80 mL) at room temperature, and then the mixture was heated to 130°C and stirred for 10 hours. Saturated aqueous ammonium chloride was added thereto to quench the reaction, and then the mixture was extracted with ethyl acetate. The organic layer was washed with saturated aqueous ammonium chloride once, dried over anhydrous magnesium sulfate, filtrated, and then the solvent was removed from the filtrate in vacuo. The residue was purified by silica gel chromatography followed by crystallization with hexanediethyl ether (4-1) to give 14.2 g of the title compound as a white crystal.
¹H-NMR δ (DMSO-d₆); 1.98-2.53 (4H, m), 2.62-2.75 (2H, m), 5.12 (2H, s), 7.01-7.03 (1H, m), 7.04-7.24 (2H, m), 7.30-7.48 (6H, m).

### tert-Butyl 4-{trans-4-[3-(benzyloxy)phenyl]-4-cyano-cyclohexyl}piperazine-1-carboxylate and tert-butyl 4-{cis-4-[3-(benzyloxy)phenyl]-4-cyanocyclohexyl}piperazine-1-carboxylate

To a solution of 1-[3-(benzyloxy)phenyl]-4-oxocyclohexanecarbonitrile (4.96 g, 16.3 mmol) and 1-tert-butoxycarbonylpiperazine (3.33 g, 17.9 mmol) in 1,2-dichloroethane (100 mL), sodium triacetoxyborohydride (5.18 g, 24.5 mmol) was added at room temperature, and then the mixture was stirred overnight. Saturated aqueous sodium hydrogencarbonate was added thereto to quench the reaction, and then the mixture was extracted with chloroform. The organic layer was washed with saturated aqueous sodium hydrogencarbonate twice, dried over anhydrous magnesium sulfate, filtrated, and then the solvent was removed from the filtrate in vacuo. The residue was purified by silica gel chromatography to give the title compound: 1.94 g of tert-butyl 4-{trans-4-[3-(benzyloxy)phenyl]-4-cyano-cyclohexyl}piperazine-1-carboxylate as a white crystal and 4.66 g of tert-butyl 4-{cis-4-[3-(benzyloxy)phenyl]-4-cyanocyclohexyl}piperazine-1-carboxylate as a white crystal.
tert-Butyl 4-{trans-4-[3-(benzyloxy)phenyl]-4-cyano-cyclohexyl}piperazine-l-carboxylate
High-performance liquid chromatography/mass spectrometry
m/z 476.5 (M+H)
Retention time: 3.01 min.
tert-Butyl 4-{cis-4-[3-(benzyloxy)phenyl]-4-cyano-cyclohexyl}piperazine-1-carboxylate
High-performance liquid chromatography/mass spectrometry
m/z 476.5 (M+H)
Retention time: 3.07 min.

### tert-Butyl 4-[trans-4-cyano-4-(3-hydroxyphenyl)cyclohexyl]-piperazine-1-carboxylate

A suspension of tert-butyl 4-{trans-4-[3-(benzyloxy)-phenyl]-4-cyanocyclohexyl}piperazine-1-carboxylate (1.60 g, 3.36 mmol) and 10% Pd/C (320 mg) in methanol (30 mL) was stirred under hydrogen atmosphere for 4 hours. The reaction mixture was filtrated through Celite®, and then the solvent was removed from the filtrate in vacuo. The residue was purified by crystallization with diethyl ether to give 572 mg of the title compound as a yellow solid. High-performance liquid chromatography/mass spectrometry
m/z 386.3 (M+H)
Retention time: 2.36 min.

### tert-Butyl 4-[trans-4-cyano-4-(3-ethoxyphenyl)cyclohexyl]-piperazine-1-carboxylate

To a solution of tert-butyl 4-[trans-4-cyano-4-(3-hydroxyphenyl)cyclohexyl]piperazine-1-carboxylate (50 mg, 0.130 mmol) and potassium carbonate (53.9 mg, 0.390 mmol) in N,N-dimethylformamide (DMF, 1.0 mL), ethyl iodide (0.014 ml, 0.169 mmol) was added at room temperature, and then the mixture was warmed to 50°C and stirred for 5 hours. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 33.9 mg of the title compound as a white solid.
High-performance liquid chromatography/mass spectrometry
m/z 414.3 (M+H)
Retention time: 2.71 min.

### Trans-1-(3-ethoxyphenyl)-4-piperazin-1-ylcyclohexane-carbonitrile dihydrochloride

tert-Butyl 4-[trans-4-cyano-4-(3-ethoxyphenyl)-cyclohexyl]piperazine-1-carboxylate (33.0 mg, 0.080 mmol) was dissolved in 4N hydrochloric acid/1,4-dioxane solution (15 mL), and then the mixture was stirred overnight at room temperature. The solvent was removed off in vacuo, and then the residue was purified by crystallization with ethyl acetate to give 21.4 mg of the title compound as a white solid.
High-performance liquid chromatography/mass spectrometry
m/z 314.3 (M+H)
Retention time: 2.02 min.
Melting point 189-191°C

### Example 92-2

### Preparation of trans-4-piperazin-1-yl-1-(3-propoxyphenyl)-cyclohexanecarbonitrile dihydrochloride

The title compound was prepared using 3-iodopropane as an alkylating agent in a similar manner to Example 92-1. High-performance liquid chromatography/mass spectrometry
m/z 328.3 (M+H)
Retention time: 2.28 min.
Melting point 159-161 °C

### Example 92-3

### Preparation of trans-1-[3-(cyclopentyloxy)phenyl]-4-piperazin-1-ylcyclohexanecarbonitrile dihydrochloride

The title compound was prepared using cyclopentyl iodide as an alkylating agent in a similar manner to Example 92-1.
High-performance liquid chromatography/mass spectrometry
m/z 354.4 (M+H)
Retention time: 2.44 min.
Melting point 250 °C

### Example 92-4

### Preparation of trans-1-(3-isopropoxyphenyl)-4-piperazin-1-ylcyclohexanecarbonitrile dihydrochloride

The title compound was prepared using 2-bromopropane as an alkylating agent in a similar manner to Example 92-1. High-performance liquid chromatography/mass spectrometry
m/z 328.3 (M+H)
Retention time: 2.11 min.
Melting point 240-250 °C

### Example 93

### Preparation of trans-1-(3-benzyloxyphenyl)-4-piperazin-1-ylcyclohexanecarbonitrile dihydrochloride

tert-Butyl 4-{trans-4-[3-(benzyloxy)phenyl]-4-cyano-cyclohexyl}piperazin-1-carboxylate (50.0 mg, 0.105 mmol) was dissolved in 4N hydrochloric acid/1,4-dioxane solution (2.0 mL), and then the mixture was stirred overnight at room temperature. The solvent was removed off in vacuo, and then the residue was purified by crystallization with ethyl acetate to give 40.4 mg of the title compound as a white solid.
High-performance liquid chromatography/mass spectrometry
m/z 376.5 (M+H)
Retention time: 2.44 min.
Melting point 250 °C

### Example 94

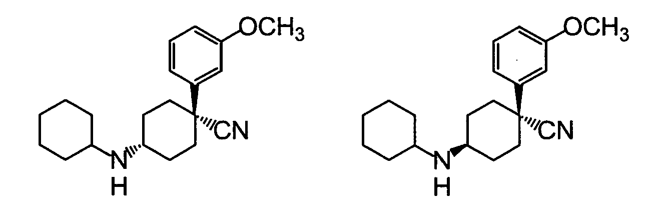

### Preparation of cis-4-(cyclohexylamino)-1-(3-methoxyphenyl)cyclohexanecarbonitrile and trans-4-(cyclohexyl-amino)-1-(3-methoxyphenyl)cyclohexanecarbonitrile

To a solution of 1-(3-methoxyphenyl)-4-oxocyclohexane-carbonitrile (100 mg, 0.436 mmol) in methanol (3 mL) were added cyclohexylamine (0.049 ml, 0.436 mmol), sodium cyanoborohydride (32.8 mg, 0.523 mmol) and acetic acid (0.074 ml, 1.56 mmol), and then the mixture was stirred for 18 and half hours at room temperature. The reaction mixture was poured into saturated aqueous sodium hydrogencarbonate, and extracted with ethyl acetate. The ethyl acetate layer was washed with brine, dried over anhydrous sodium sulfate, and then filtrated. The filtrate was concentrated in vacuo and the residue was purified by silica gel column chromatography to give 39 mg and 18 mg of the title compounds as a white solid.
Rf 0.19 (chloroform:methanol=10:1); ¹H-NMR δ (CDCl₃) 1.01-1.33 (5H, m), 1.60-1.89 (9H, m) 2.11-2.23 (4H, m), 2.62-2.77 (2H, m), 3.83 (3H, s), 6.86 (1H, ddd, J = 8.2, 2.4, 0.6 Hz), 7.02-7.08 (2H, m), 7.31 (1H, t, J = 8.0 Hz).
Rf 0.33 (chloroform:methanol=10:1);¹H-NMR δ (CDCl₃) 0.96-1.05 (2H, m), 1.12-1.31 (3H, m) 1.59-1.64 (1H, m), 1.70-1.74 (4H, m), 1.87-1.90 (4H, m), 1.95-2.03 (2H, m), 2.26-2.33(2H, m), 2.38-2.45 (1H, m), 3.13 (1H, t, J = 3.4 Hz), 3.83 (3H, s), 6.84 (1H, ddd, J = 8.2, 2.5, 0.7 Hz), 7.05-7.11 (2H, m), 7.31 (1H, t, J = 8.0 Hz).

### Example 95

### Preparation of cis-4-[(diphenylmethyl)amino]-1-(3-methoxyphenyl)cyclohexanecarbonitrile and trans-4-[(diphenylmethyl)amino]-1-(3-methoxyphenyl)cyclohexanecarbonitrile

To a solution of 1-(3-methoxyphenyl)-4-oxocyclohexane-carbonitrile (100 mg, 0.436 mmol) in dichloroethane (2 mL) were added benzhydrylamine (0.112 ml, 0.654 mmol) and sodium triacetoxyborohydride (138 mg, 0.654 mmol), and then the mixture was stirred for 16 and half hours at room temperature. The reaction mixture was poured into saturated aqueous sodium hydrogencarbonate, and extracted with ethyl acetate. The ethyl acetate layer was washed with brine, dried over anhydrous sodium sulfate, and then filtrated. The filtrate was concentrated in vacuo and the residue was purified by silica gel column chromatography to give 100 mg of cis-4-[(diphenylmethyl)amino]-1-(3-methoxyphenyl)cyclohexanecarbonitrile and 55 mg of trans-4-[(diphenylmethyl)amino]-1-(3-methoxyphenyl)cyclohexane-carbonitrile as a white solid.
Cis-4-[(diphenylmethyl)amino]-1-(3-methoxyphenyl)-cyclohexanecarbonitrile
High-performance liquid chromatography/mass spectrometry
m/z 397 (M+H)
Retention time: 3.07 min.
Trans-4-[(diphenylmethyl)amino]-1-(3-methoxyphenyl)-cyclohexanecarbonitrile
High-performance liquid chromatography/mass spectrometry
m/z 397 (M+H)
Retention time: 2.76 min.

### Example 96

### Preparation of cis-4-amino-1-(3-methoxyphenyl)cyclohexane-carbonitrile

To a solution of cis-4-[(diphenylmethyl)amino]-1-(3-methoxyphenyl)cyclohexanecarbonitrile (2g, 5.04 mmol) in ethanol (80 mL) were added palladium-carbon (500 mg) and ammonium formate (2 g), and then the mixture was stirred for 2 hours heating under reflux. The reaction mixture was filtrated off and the filtrate was concentrated in vacuo. The residue was purified by silica gel column chromatography to give 1.04 g of the title compound as a white solid.
High-performance liquid chromatography/mass spectrometry
m/z 231 (M+H)
Retention time: 2.13 min.

### Example 97-1

### Preparation of cis-1-(3-methoxyphenyl)-4-piperidin-1-yl-cyclohexanecarbonitrile

To a solution of cis-4-amino-1-(3-methoxyphenyl)-cyclohexanecarbonitrile (50 mg, 0.22 mmol) in dimethylformamide (5 mL) were added potassium carbonate (150.0 mg, 1.09 mmol) and 1,5-dibromopentane (54.9 mg,0.24 mmol), and then the mixture was stirred for 7 hours at 50°C. To the reaction mixture was added water and ethyl acetate, and the mixture was extracted with ethyl acetate, washed with brine, and then dried over anhydrous magnesium sulfate. After filtration, the solvent was removed from the filtrate in vacuo, and the residue was purified by silica gel chromatography to give 22.4 mg of the title compound. High-performance liquid chromatography/mass spectrometry
m/z 299 (M+H)
Retention time: 2.46 min.

### Example 97-2

### Preparation of trans-4-amino-1-(3-methoxyphenyl)-cyclohexanecarbonitrile

The title compound was prepared in a similar manner to Example 97-1.
High-performance liquid chromatography/mass spectrometry
m/z 231 (M+H)
Retention time: 2.11 min.

### Example 97-3

### Preparation of cis-1-(3-methoxyphenyl)-4-morpholin-4-yl-cyclohexanecarbonitrile

The title compound was prepared in a similar manner to Example 97-1.
High-performance liquid chromatography/mass spectrometry
m/z 301 (M+H)
Retention time: 2.32 min.

### Example 98-1

### Preparation of cis-4-[(4-chlorophenyl)amino]-1-(3-methoxyphenyl)cyclohexanecarbonitrile

A solution of cis-4-amino-1-(3-methoxyphenyl)-cyclohexanecarbonitrile (50 mg, 0.22 mmol), p-bromochlorobenzene (41.6 mg,0.22 mmol), palladium acetate (1.0 mg, 0.004 mmol), cesium carbonate (99.0 mg, 0.3 mmol) and 2,2'-bis(diphenylphosphino)-1,1'-binaphthyl (4.8 mg, 0.007 mmol) in dioxane (5 mL) was stirred for 7.5 hours at 90°C. Additional palladium acetate (4 mg) and 2,2'-bis(diphenyl-phosphino)-1,1'-binaphthyl (20 mg) were added thereto, and the mixture was stirred for 4 hours. And then additional p-bromochlorobenzene (500 mg) was added thereto, and the mixture was stirred for 10 hours. To the reaction mixture was added water, ethyl acetate and saturated aqueous ammonium chloride, and the mixture was extracted with ethyl acetate, and then the ethyl acetate layer was dried over anhydrous magnesium sulfate. After filtration, the solvent was removed from the filtrate in vacuo, and the residue was purified by silica gel chromatography to give 11.7 mg of the title compound.
High-performance liquid chromatography/mass spectrometry
m/z 341 (M+H)
Retention time: 3.84 min.

### Example 98-2

### Preparation of cis-1-(3-methoxyphenyl)-4-[(3-methylphenyl)-amino]cyclohexanecarbonitrile

The title compound was prepared in a similar manner to Example 98-1.
High-performance liquid chromatography/mass spectrometry
m/z 321 (M+H)
Retention time: 3.05 min.

### Example 99

### Preparation of cis-1-(3-methoxyphenyl)-4-[(4-methylphenyl)-amino]cyclohexanecarbonitrile

A mixture of cis-4-amino-1-(3-methoxyphenyl)-cyclohexanecarbonitrile (50 mg, 0.22 mmol), p-bromotoluene (40.9 mg, 0.24 mmol), dipalladium trisdibenzylidene-acetone (11.0 mg, 0.01 mmol) and sodium t-butoxide (29.2 mg, 0.30 mmol) in dioxane (5 mL) was stirred for 6 hours at 90°C, p-bromotoluene (80 mg) was thereto, and then the mixture was stirred for 8 hours at the same temperature. To the reaction mixture was added water, ethyl acetate and saturated aqueous ammonium chloride, and the mixture was extracted with ethyl acetate, washed with brine, and then dried over anhydrous magnesium sulfate. After filtration, the solvent was removed off in vacuo, and the residue was purified by silica gel chromatography to give 16.6 mg of the title compound.
High-performance liquid chromatography/mass spectrometry
m/z 321 (M+H) .
Retention time: 2.80 min.

### Example 100-1

### Preparation of cis-1-(3-methoxyphenyl)-4-[(2-methylphenyl)-amino]cyclohexanecarbonitrile

A mixture of cis-4-amino-1-(3-methoxyphenyl)-cyclohexanecarbonitrile (50 mg,0.26 mmol), o-bromotoluene (37.1 mg, 0.22 mmol), palladium acetate (10.0 mg, 0.004 mmol), sodium t-butoxide (31.3 mg, 0.33 mmol) and 2,8,9-triisobutyl-2,5,8,9-tetraaza-1-phosphabicycloundecane (29.7 mg, 0.087 mmol) in toluene (5 mL) was stirred for 12 hours at 90°C, and then o-bromotoluene (37 mg) was added thereto and the mixture was stirred for 10 hours at the same temperature. To the reaction mixture was added water, ethyl acetate and saturated aqueous ammonium chloride, the mixture was extracted with ethyl acetate, and then dried over anhydrous magnesium sulfate. After filtration, the solvent was removed off in vacuo and the residue was purified by preparative thin-layer chromatography to give 7.9 mg of the title compound.
High-performance liquid chromatography/mass spectrometry
m/z 321 (M+H)
Retention time: 3.32 min.

### Example 100-2

### Preparation of cis-4-[(3,5-dimethylphenyl)amino]-1-(3-methoxyphenyl)cyclohexanecarbonitrile

The title compound was prepared in a similar manner to Example 100-1.
High-performance liquid chromatography/mass spectrometry
m/z 335 (M+H)
Retention time: 3.05 min.

### Example 101-1

### Preparation of 4-{[cis-4-cyano-4-(3-methoxyphenyl)-cyclohexyl]amino}benzenesulfonamide

To a solution of 1-(3-methoxyphenyl)-4-oxocyclohexane-carbonitrile (50 mg, 0.22 mmol) in methanol (2 mL) were added p-aminobenzenesulfonamide (37.6 mg, 0.22 mmol) and acetic acid (62.4 mg, 1.09 mmol) followed by sodium cyanoborohydride (13.7 mg, 0.22 mmol), and then the mixture was stirred for 2.5 hours. To the reaction mixture was added aqueous sodium hydroxide, ethyl acetate and water, and then the mixture was extracted with ethyl acetate, washed with brine, dried over anhydrous magnesium sulfate, filtrated, and then the solvent was removed from the filtrate in vacuo. The residue was purified by silica gel chromatography to give 26.4 mg of the title compound. High-performance liquid chromatography/mass spectrometry
m/z 386 (M+H)
Retention time: 3.26 min.

### Example 101-2

### Preparation of 1-(3-methoxyphenyl)-4-{[4-(piperidin-1-yl-sulfonyl)phenyl]amino}cyclohexanecarbonitrile

The title compound was prepared in a similar manner to Example 101-1.
High-performance liquid chromatography/mass spectrometry
m/z 454 (M+H)
Retention time: 4.11 min.

### Example 101-3

### 2-{[Cis-4-cyano-4-(3-methoxyphenyl)cyclohexyl]amino}-benzenesulfonamide

The title compound was prepared in a similar manner to Example 101-1.
High-performance liquid chromatography/mass spectrometry
m/z 386 (M+H)
Retention time: 3.55 min.

### Example 101-4

### Preparation of 4-({(cis-4-cyano-4-(3-methoxyphenyl)-cyclohexyl]amino}methyl)benzenesulfonamide

The title compound was prepared in a similar manner to Example 101-1.
High-performance liquid chromatography/mass spectrometry
m/z 400 (M+H)
Retention time: 2.48 min.

### Example 101-5

### Preparation of methyl 4-({[cis-4-cyano-4-(3-methoxyphenyl)-cyclohexyl]amino}methyl)benzoate

The title compound was prepared in a similar manner to Example 101-1.
High-performance liquid chromatography/mass spectrometry
m/z 379 (M+H)
Retention time: 2.69 min.

### Example 101-6

### Preparation of methyl 4-({[trans-4-cyano-4-(3-methoxyphenyl)cyclohexyl]amino}methyl)benzoate

The title compound was prepared in a similar manner to Example 101-1.
High-performance liquid chromatography/mass spectrometry
m/z 379 (M+H)
Retention time: 2.67 min.

### Example 101-7

### Preparation of 4-({[trans-4-cyano-4-(3-methoxyphenyl)-cyclohexyl]amino}methyl)benzenesulfonamide

The title compound was prepared in a similar manner to Example 101-1.
High-performance liquid chromatography/mass spectrometry
m/z 400 (M+H)
Retention time: 2.34 min.

### Example 101-8

### Preparation of cis-1-(3-methoxyphenyl)-4-[(4-methylbenzyl)-amino]cyclohexanecarbonitrile

The title compound was prepared in a similar manner to Example 101-1.
High-performance liquid chromatography/mass spectrometry
m/z 335 (M+H)
Retention time: 2.78 min.

### Example 101-9

### Preparation of trans-1-(3-methoxyphenyl)-4-[(4-methylbenzyl)amino]cyclohexanecarbonitrile

The title compound was prepared in a similar manner to Example 101-1.
High-performance liquid chromatography/mass spectrometry
m/z 335 (M+H)
Retention time: 2.76 min.

### Example 101-10

### Preparation of cis-4-[(4-methoxybenzyl)amino]-1-(3-methoxyphenyl)cyclohexanecarbonitrile

The title compound was prepared in a similar manner to Example 101-1.
High-performance liquid chromatography/mass spectrometry
m/z 351 (M+H)
Retention time: 2.71 min.

### Example 101-11

### Preparation of trans-4-[(4-methoxybenzyl)amino]-1-(3-methoxyphenyl)cyclohexanecarbonitrile

The title compound was prepared in a similar manner to Example 101-1.
High-performance liquid chromatography/mass spectrometry
m/z 351 (M+H)
Retention time: 2.67 min.

### Example 101-12

### Preparation of cis-1-(3-methoxyphenyl)-4-[(pyridin-4-yl-methyl)amino]cyclohexanecarbonitrile

The title compound was prepared in a similar manner to Example 101-1.
¹H-NMR δ (DMSO-d₆) 1.39-1.50 (2H, m), 1.83-1.91 (2H, m), 2.03-2.07 (4H, m), 3.75-3.78 (5H, m), 6.90 (1H, m), 7.02-7.10 (2H, m), 7.29-7.38 (3H, m), 8.47 (2H, m).

### Example 102

### Preparation of ethyl 4-{[cis-4-cyano-4-(3-methoxyphenyl)-cyclohexyl]amino}benzoate

A mixture of cis-4-amino-1-(3-methoxyphenyl)-cyclohexanecarbonitrile (100 mg, 0.43 mmol), potassium carbonate (240 mg, 1.74 mmol) and ethyl p-fluorobenzoate (146.0 mg, 0.87 mmol) in dimethylsulfoxide (4 mL) was stirred for 5.5 hours at 120°C, and then potassium carbonate (360 mg) and ethyl p-fluorobenzoate (146 mg) were added thereto and the mixture was stirred for 11 hours. Cooling the reaction mixture, water was added thereto, and the mixture was extracted with ethyl acetate, washed with saturated aqueous ammonium chloride and brine successively, dried over anhydrous magnesium sulfate, filtrated, and then the solvent was removed from the filtrate in vacuo. The residue was purified by silica gel chromatography to give 48.2 mg of the title compound.
High-performance liquid chromatography/mass spectrometry
m/z 379 (M+H)
Retention time: 4.15 min.

### Example 103

### Preparation of 4-{[cis-4-cyano-4-(3-methoxyphenyl)-cyclohexyl]amino}benzoic acid

To a solution of ethyl 4-{[cis-4-cyano-4-(3-methoxyphenyl)cyclohexyl]amino}benzoate (25 mg) in methanol (4 mL), 2N aqueous lithium hydroxide was added, and then the mixture was stirred for 12 hours at 50°C. After removing the solvent in vacuo, the residue was dissolved in water, and then aqueous hydrochloric acid was added thereto. The precipitated crystal was collected by filtration to give 22.6 mg of the title compound.
High-performance liquid chromatography/mass spectrometry
m/z 351 (M+H)
Retention time: 3.53 min.

### Example 104

### Preparation of cis-1-(3-methoxyphenyl)-4-(pyrimidin-2-yl-amino)cyclohexanecarbonitrile

A solution of cis-4-amino-1-(3-methoxyphenyl)-cyclohexanecarbonitrile (80 mg, 0.347 mmol), 2-bromopyrimidine (55 mg, 0.347 mmol), dipalladium tris(dibenzylidene acetone) (9.5 mg, 0.01 mmol), 9,9-dimethyl-4,5-bis(diphenylphosphino)xanthene (18 mg, 0.031 mmol) and sodium t-butoxide (47 mg, 0.48 mmol) in toluene (1 mL) was stirred for 2 hours at 100°C. The reaction mixture was added to water, extracted with ethyl acetate, washed with brine, and concentrated in vacuo. The residue was purified by silica gel column chromatography to give 22 mg of the title compound as a white solid.
High-performance liquid chromatography/mass spectrometry
m/z 309 (M+H)
Retention time: 2.96 min.

### Example 105

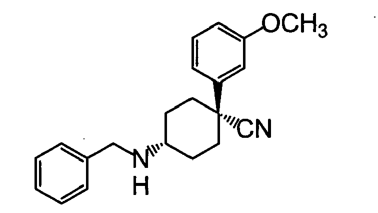

### Preparation of cis-4-(benzylamino)-1-(3-methoxyphenyl)-cyclohexanecarbonitrile

To a solution of cis-4-amino-1-(3-methoxyphenyl)-cyclohexanecarbonitrile (80 mg, 0.34 mmol) in methanol (2 mL) were added benzaldehyde (0.035 ml, 0.34 mmol) and sodium cyanoborohydride (24 mg, 0.38 mmol), and then the mixture was stirred for 43 hours at room temperature. The reaction mixture was poured into saturated aqueous sodium hydrogencarbonate, and extracted with ethyl acetate. The organic layer was washed with brine, dried over anhydrous magnesium sulfate, filtrated, and concentrated in vacuo. The residue was purified by silica gel column chromatography to give 61 mg of the title compound as a white solid.
High-performance liquid chromatography/mass spectrometry
m/z 321 (M+H)
Retention time: 2.55 min.

### Example 106

### Preparation of 4-({[cis-4-cyano-4-(3-methoxyphenyl)-cyclohexyl]amino}methyl)benzoic acid

Methyl 4-({[cis-4-cyano-4-(3-methoxyphenyl)-cyclohexyl]amino}methyl)benzoate (120 mg, 0.32 mmol) was dissolved in methanol (5 mL) by heating at 50°C. To the solution, aqueous lithium hydroxide (0.48 mL, 0.95 mmol) was added, and then the mixture was stirred overnight at room temperature. To the reaction mixture was added water and 2N aqueous hydrochloric acid, and the mixture was stirred at 0°C and then filtrated and dried to give 81.6 mg of the title compound.
¹H-NMR δ (DMSO-d₆) 1.49-1.56 (2H, m), 1.85-1.96 (2H, m), 1.95-2.10 (4H, m), 3.76 (3H, m), 3.90 (2H, m), 6.90 (1H, d, J = 8.15 Hz), 7.02 (1H, s), 7.08 (1H, d, J = 7.70 Hz), 7.32 (1H, dd, J = 7.89, 8.07 Hz), 7.50 (2H, d, J = 8.25 Hz), 7.90 (1H, d, J = 8.25 Hz).

### Example 107-1

### Preparation of 4-({[cis-4-cyano-4-(3-methoxyphenyl)-cyclohexyl]amino}methyl)benzamide

To a solution of 4-({[cis-4-cyano-4-(3-methoxyphenyl)cyclohexyl]amino}methyl)benzoic acid hydrochloride (50 mg, 0.124 mmol) in dimethylformamide (1.5 mL) were added 1-hydroxybenzotriazole (21.7 mg, 0.161 mmol), ammonium chloride (20 mg, 0.374 mmol), triethylamine (0.13 ml, 0.966 mmol) and 1-ethyl-3-(3-dimethylaminopropyl)-carbodiimide hydrochloride (31 mg, 0.161 mmol), and then the mixture was stirred for 15 and half hours at room temperature. The reaction mixture was poured to brine and extracted with ethyl acetate. The organic layer was washed with brine, dried over anhydrous magnesium sulfate, filtrated, and then concentrated in vacuo. The residue was purified by silica gel column chromatography to give 15 mg of the title compound as a light yellow solid.
High-performance liquid chromatography/mass spectrometry
m/z 364 (M+H)
Retention time: 2.32 min.

### Example 107-2

### Preparation of 4-({[cis-4-cyano-4-(3-methoxyphenyl)-cyclohexyl]amino}methyl)-N,N-dimethylbenzamide

The title compound was prepared in a similar manner to Example 107-1.
High-performance liquid chromatography/mass spectrometry
m/z 392 (M+H)
Retention time: 2.42 min.

### Example 108

### Preparation of 3-({[cis-4-cyano-4-(3-methoxyphenyl)-cyclohexyl]amino}methyl)benzenesulfonamide

### Methyl 3-(aminosulfonyl)-4-chlorobenzoate

To a suspension of 4-chloro-3-sulfamoylbenzoic acid (1 g, 4.24 mmol) in dichloromethane (20 mL) were added oxalyl chloride (0.4 mL, 4.46 mmol) and dimethylformamide (1 drop), and then the mixture was stirred overnight at room temperature. Methanol was added to the reaction mixture, the mixture was stirred for 1 hour, and then the solvent was removed from the mixture in vacuo. The residue was purified by silica gel chromatography to give 922.3 mg of the title compound.
¹H-NMR δ (DMSO-d₆) 3.89 (3H, s), 7.79-7.82 (3H, m), 8.11 (1H, d, J=6.06 Hz), 8.49 (1H, s).

### Methyl 3-(aminosulfonyl)benzoate

To a solution of methyl 3-(aminosulfonyl)-4-chlorobenzoate (200 mg, 0.80 mmol) in methanol (10 mL) were added palladium carbon (100 mg) and ammonium formate (505.1 mg, 8.01 mmol), and then the mixture was stirred for 2 hours at 90°C. The reaction mixture was left to room temperature, and then filtrated through Celite®. The filtrate was concentrated in vacuo and the residue was distributed with ethyl acetate and water. And then the mixture was extracted with ethyl acetate and the ethyl acetate layer was dried over anhydrous magnesium sulfate. After filtration, the solvent was removed off to give the title compound. The title compound was applied to the next step without further purification.
¹H-NMR δ (DMSO-d₆): 3.89 (3H, s), 7.52 (2H, br), 7.73 (1H, dd, J=7.88, 7.71 Hz), 8.07 (1H, d, J=7.88 Hz), 8.15 (1H, d, J=9.17 Hz), 8.39(1H, s).

### 3-(Aminosulfonyl)benzoic acid

To a solution of methyl 3-(aminosulfonyl)benzoate (149.6 mg) in methanol (7 mL), 2N aqueous lithium hydroxide (1.04 mL, 2.08 mmol) was added, and then the mixture was stirred for 3 hours at 50°C. After the reaction mixture was concentrated in vacuo, water and 2N aqueous hydrochloric acid were added thereto. The mixture was stirred at 0°C for 2 hours. And then the precipitate was collected by filtration and dried to give the title compound. The title compound was applied to the next step without further purification.
¹H-NMR δ (DMSO-d₆): 7.48 (2H, br), 7.70 (1H, dd, J = 7.88, 7.89 Hz), 8.03 (1H, d, J= 7.88 Hz), 8.12 (1H, d, J = 7.89 Hz), 8.38 (1H, s).

### 3-(Hydroxymethyl)benzenesulfonamide

To a suspension of 3-(aminosulfonyl)benzoic acid (120 mg, 0.60 mmol) in dichloromethane (5 mL) were added oxalyl chloride (55.6 µL, 0.63 mmol) and dimethylformamide (1 drop), and the mixture was stirred overnight at room temperature, and then the solvent was removed from the mixture in vacuo.

To a suspension of lithium aluminium hydride in tetrahydrofuran, a solution of the above chloride in tetrahydrofuran was added at 0°C, and the mixture was stirred for 3 hours at room temperature. The reaction mixture was cooled at 0°C and then water, 2N aqueous sodium hydroxide and water were added thereto in order and the mixture was stirred overnight. The reaction mixture was filtrated through Celite®, and then the filtrate was concentrated to give the title compound. The title compound was applied to the next step without further purification.
¹H-NMR δ (DMSO-d₆) 4.56 (2H, d, J = 5.68 Hz), 5.40 (1H, s), 7.31 (2H, br), 7.49 (2H, d, J = 5.13 Hz), 7.67 (1H, dd, J = 4.58, 4.77 Hz), 7.80 (1H, br).

### 3-(Bromomethyl)benzenesulfonamide

To a solution of 3-(hydroxymethyl)benzenesulfonamide (25 mg, 0.13 mmol) in dichloromethane (4 mL), a solution of phosphorus tribromide (76.0 mg, 0.28 mmol) in dichloromethane (1 mL) was added, and then the mixture was stirred overnight at room temperature. The reaction mixture was cooled at 0°C, and water, saturated aqueous sodium hydrogencarbonate was added thereto, and then the mixture was extracted with ethyl acetate twice, and dried over anhydrous magnesium sulfate. After filtration, the filtrate was concentrated in vacuo to give the title compound. The title compound was applied to the next step without further purification.
¹H-NMR δ (DMSO-d₆) 4.79 (2H, s), 7.41 (2H, br), 7.56 (1H, dd, J=7.71, 7.70 Hz), 7.66 (1H, d, J=7.88 Hz), 7.75 (1H, d, J=7.70 Hz), 7.89 (1H, s).

### 3-({[Cis-4-cyano-4-(3-methoxyphenyl)cyclohexyl]amino}-methyl)benzenesulfonamide

To a solution of cis-4-amino-1-(3-methoxyphenyl)-cyclohexanecarbonitrile (30.8 mg, 0.13 mmol) and N-methylmorpholine (73.5 µL, 0.67 mmol) in dimethylformamide, a solution of 3-(bromomethyl)benzenesulfonamide in DMF was added, and then the mixture was stirred for 4 hours at 50°C. Then, N-methylmorpholine (74 µL) was added thereto, and the mixture was stirred overnight. To the reaction mixture was added water and ethyl acetate. After distributing the mixture, the mixture was extracted with ethyl acetate, washed with water, and then dried over anhydrous sodium sulfate. The concentrated residue was purified by preparative thin-layer chromatography to give 153 mg of the title compound (5.6 mg, Y. 1.8%).
High-performance liquid chromatography/mass spectrometry
m/z 400(M+H)
Retention time: 2.49 min.

### Example 109

### Preparation of 3-(aminosulfonyl)-4-chloro-N-[cis-4-cyano-4-(3-methoxyphenyl)cyclohexyl]benzamide

To a suspension of 4-chloro-3-sulfamoylbenzoic acid (102.3 mg, 0.43 mmol) in dichloromethane (3 mL) were added oxalyl chloride (42.4 µL, 0.48 mmol) and dimethylformamide (1 drop), and the mixture was stirred overnight at room temperature, then the solvent was removed from the mixture in vacuo. Dichloromethane was added to the reaction mixture. To the mixture were added a solution of cis-4-amino-1-(3-methoxyphenyl)cyclohexanecarbonitrile (100 mg, 0.43 mol) in dichloromethane, and diisopropylethylamine (140.3 mg, 1.09 mmol), and then the mixture was stirred for 3 hours. To the reaction mixture was added ethyl acetate, water and saturated aqueous ammonium chloride. After separating the layers, the organic layer was washed with 0.5N aqueous sodium thiosulfate, dried over anhydrous magnesium sulfate, filtrated, and then the solvent was removed from the filtrate. The residue was purified by repulping with methanol to give 156.3 mg of the title compound.
High-performance liquid chromatography/mass spectrometry
m/z 448 (M+H)
Retention time: 3.36 min.

### Example 110

### Preparation of 3-(aminosulfonyl)-N-[cis-4-cyano-4-(3-methoxyphenyl)cyclohexyl]benzamide

To a suspension of 3-(aminosulfonyl)-4-chloro-N-[cis-4-cyano-4-(3-methoxyphenyl)cyclohexyl]benzamide (156.3 mg) in methanol (5 mL) were added ammonium formate (18.3 mg, 0.29 mmol) and palladium carbon (65 mg), and then the mixture was stirred for 5 hours at 80°C. After adding ammonium formate (30 mg) thereto, the mixture was stirred for another 1 hour, furthermore after adding ammonium formate (20 mg) thereto again, the mixture was stirred for another 6 hours. The reaction mixture was filtrated through Celite®, and the filtrate was concentrated. The residue was purified by repulping with methanol to give 41.9 mg of the title compound.
High-performance liquid chromatography/mass spectrometry
m/z 414 (M+H)
Retention time: 3.21 min.

### Example 111-1

### Preparation of N-[cis-4-cyano-4-(3-methoxyphenyl)-cyclohexyl]acetamide

The title compound was prepared using acetyl chloride as an acylating agent in a similar manner to Example 26-1.
High-performance liquid chromatography/mass spectrometry
m/z 273 (M+H)
Retention time: 2.99 min.

### Example 111-2

### Preparation of N-[cis-4-cyano-4-(3-methoxyphenyl)-cyclohexyl]benzamide

The title compound was prepared in a similar manner to Example 111-1.
High-performance liquid chromatography/mass spectrometry
m/z 335 (M+H)
Retention time: 3.63 min.

### Example 112

### Preparation of tert-butyl [cis-4-cyano-4-(3-methoxyphenyl)-cyclohexyl]carbamate

To a solution of cis-4-amino-1-(3-methoxyphenyl)-cyclohexanecarbonitrile (200 mg, 1.3 mmol) in tetrahydrofuran (10 mL), a solution of di-t-butyl carbonate (204.2 mg, 1.4 mmol) in tetrahydrofuran (2 mL) , and then the mixture was stirred overnight at room temperature. The solvent was removed from the mixture in vacuo and then the residue was purified by silica gel chromatography to give 255.9 mg of the title compound.
¹H-NMR δ (DMSO-d₆) 1.38 (9H, s), 1.51-1.64 (2H, m), 1.89-2.08 (6H, m), 3.76 (3H, s), 6.91 (1H, dd, J =2.57, 8.26 Hz), 6.96 (1H, d, J = 7.71 Hz), 7.05 (1H, dd, J = 1.84, 2.20 Hz), 7.09 (1H, d, J =7.70 Hz), 7.34 (1H, dd, J = 7.88, 8.07 Hz).

### Example 113

### Preparation of N-[cis-4-cyano-4-(3-methoxyphenyl)-cyclohexyl]-4-methylbenzenesulfonamide

To a solution of cis-4-amino-1-(3-methoxyphenyl)-cyclohexanecarbonitrile (30 mg, 0.13 mmol) in dichloromethane (3 mL) were added p-toluenesulfonyl chloride (27.3 mg, 0.14 mmol) and triethylamine (20 µL, 0.14 mmol), and then the mixture was stirred overnight at room temperature. To the reaction mixture was added water, ethyl acetate and saturated aqueous ammonium chloride. And then the organic layer was washed with saturated aqueous sodium hydrogencarbonate, water and brine successively, dried over anhydrous magnesium sulfate, filtrated, and the solvent was removed from the filtrate in vacuo. The residue was purified by preparative thin-layer chromatography to give 45.0 mg of the title compound.
¹H-NMR δ (DMSO-d₆) 1.56 (2H, m), 1.71-1.75 (2H, m), 1.90-1.93 (4H, m), 2.37 (3H, s), 3.16 (1H, br), 3.74 (3H, s), 6.87 (1H, d, J = 8.07 Hz), 7.00-7.06 (2H, m), 7.29 (1H, dd, 8.07, J = 7.88 Hz), 7.39 (2H, d, J = 8.25 Hz), 7.71 (2H, d, J = 8.25 Hz), 7.80 (1H, d, J = 7.34 Hz).

### Example 114

### Preparation of methyl cis-4-(benzylamino)-1-(3-methoxyphenyl)cyclohexanecarboxylate and methyl trans-4-(benzylamino)-1-(3-methoxyphenyl)cyclohexanecarboxylate

To a solution of methyl 1-(3-methoxyphenyl)-4-oxocyclohexanecarboxylate (500 mg, 1.90 mmol) in dichloroethane (10 mL) were added benzylamine (0.207 ml, 1.90 mmol), sodium triacetoxyborohydride (1.00 g, 4.75 mmol) and acetic acid (0.16 ml, 2.85 mmol), and then the mixture was stirred for 5 and half hours at room temperature. The reaction mixture was poured into saturated aqueous sodium hydrogencarbonate, and extracted with chloroform. The organic layer was washed with brine, dried over anhydrous sodium sulfate, filtrated, and then the solvent was removed from the filtrate in vacuo. The residue was purified by silica gel column chromatography to give 651 mg of the title compound as a colorless oil.
Rf 0.39 (chloroform:methanol=10:1)
High-performance liquid chromatography/mass spectrometry
m/z 354 (M+H)
Retention time: 2.53 min.
Rf 0.32 (chloroform:methanol=10:1)
High-performance liquid chromatography/mass spectrometry
m/z 354 (M+H)
Retention time: 2.65 min.

### Example 115

### Preparation of 4-(benzylamino)-1-(3-methoxyphenyl)-cycloheptanecarbonitrile

### 1-(3-Methoxyphenyl)-4-oxocycloheptanecarbonitrile

To a solution of 1-(3-methoxyphenyl)-4-oxocyclohexane-carbonitrile (200 mg, 0.872 mmol) in methanol (4 mL), sodium carbonate (8 mg, 0.0754 mmol) was added, and then N-nitroso-N-methylurethane (0.24 ml, 1.8 mmol) was added at 20°C or lower temperature, and the mixture was stirred for 4 hours at room temperature. During the stirring process, another N-nitroso-N-methylurethane (0.06 ml, 0.46 mmol) and sodium carbonate (3 mg, 0.0471 mmol) were added thereto. To the reaction mixture was added acetic acid (0.4 mL), and then the mixture was concentrated. Water was added to the residue, and the mixture was extracted with diethyl ether. The organic layer was washed with brine, dried over anhydrous magnesium sulfate, filtrated, and concentrated in vacuo. The residue was purified by silica gel column chromatography to give 96 mg of the title compound as a white solid.
¹H-NMR δ (CDCl₃) 1.91-2.29 (5H, m), 2.34-2.39 (1H, m), 2.51-2.74 (3H, m), 3.10-3.18 (1H, m), 3.83 (3H, s), 6.86-6.88 (1H, m), 7.02-7.04 (1H, m), 7.05-7.08 (1H, m), 7.33 (1H, t, J = 8.0 Hz).

### 4-(Benzylamino)-1-(3-methoxyphenyl)cycloheptanecarbonitrile

To a solution of 1-(3-methoxyphenyl)-4-oxocycloheptanecarbonitrile (90 mg, 0.369 mmol) in dichloroethane (2 mL) were added benzylamine (0.044 ml, 0.405 mol), acetic acid (0.032 ml, 0.553 mmol) and sodium triacetoxyborohydride (195 mg, 0.922 mmol), and then the mixture was stirred for 23 hours at room temperature. During the stirring process, another sodium triacetoxyborohydride (70 mg, 0.330 mmol) and benzylamine (0.025 ml, 0.228 mol) were added thereto. The reaction mixture was poured into saturated aqueous sodium hydrogencarbonate, and extracted with ethyl acetate. The organic layer was washed with brine, dried over anhydrous magnesium sulfate, filtratred, and concentrated in vacuo. The residue was purified by silica gel column chromatography to give 95 mg of the title compound as a colorless oil.
High-performance liquid chromatography/mass spectrometry
m/z 335 (M+H)
Retention time: 2.67 min.

### Example 116

### Preparation of 4-(benzylamino)-1-(3-methoxyphenyl)-cyclohexanol

### 8-(3-Methoxyphenyl)-1,4-dioxaspiro[4,5]decan-8-ol

To a solution of 3-bromoanisole (2 g, 10.69 mmol) in tetrahydrofuran (40 mL), 6.72 ml of 1.59 M n-butyl lithium in hexane (10.69 mmol) was added at -70°C, and the mixture was stirred for 30 minutes. Then, a solution of 1,4-dioxaspiro[4,5]decan-8-one (1.66 g, 10.69 mmol) in tetrahydrofuran (4 mL) was added thereto at -70°C, and then the mixture was gradually warmed to room temperature and stirred for 4 and half hours. The reaction mixture was poured into ice water and extracted with ethyl acetate. The organic layer was washed with brine, dried over anhydrous magnesium sulfate, filtrated, and concentrated in vacuo. The residue was purified by silica gel column chromatography to give 2.3 g of the title compound as a colorless oil.
¹H-NMR δ (CDCl₃) 1.51-1.63 (4H, m), 1.86-1.93 (4H, m), 3.72 (3H, s), 3.87 (4H, m), 4.88 (1H, s), 6.74-6.77 (1H, m), 6.96-7.01 (2H, m), 7.20 (1H, t, J= 7.9 Hz).

### 4-Hydroxy-4-(3-methoxyphenyl)cyclohexanone

To 8-(3-methoxyphenyl)-1,4-dioxaspiro[4,5]decan-8-ol (600 mg, 2.26 mmol), water (2.5 mL) and acetic acid (10 mL) were added, and then the mixture was stirred for 30 hours at room temperature. The reaction mixture was poured into aqueous sodium hydrogencarbonate to be neutralized and then extracted with ethyl acetate. The organic layer was washed with brine, dried over anhydrous sodium sulfate, and then filtrated. The filtrate was concentrated in vacuo and the residue was purified by silica gel column chromatography to give 440 mg of the title compound as a white solid.
¹H-NMR δ (CDCl₃) 1.77 (1H, s), 2.15-2.21 (2H, m), 2.26-2.39 (4H, m), 2.88-2.96 (2H, m), 3.83 (3H, s), 6.83-6.86 (1H, m), 7.06-7.10 (2H, m), 7.31 (1H, t, J = 7.9 Hz).

### 4-(Benzylamino)-1-(3-methoxyphenyl)cyclohexanol

The title compound was prepared in a similar manner to Example 115.
High-performance liquid chromatography/mass spectrometry
m/z 312 (M+H)
Retention time: 2.07 min.

### Example 117

### Preparation of tert-butyl 4-{[cis-4-cyano-4-(3-methoxyphenyl)cyclohexyl]amino}piperidine-1-carboxylate

To a solution of cis-4-amino-1-(3-methoxyphenyl)-cyclohexanecarbonitrile (1.00 g, 4.34 mmol) and 1-tert-butoxycarbonyl-4-piperidone (952 mg, 4.78 mmol) in 1,2-dichloroethane (20 mL), sodium triacetoxyborohydride (1.38 g, 6.51 mmol) was added at room temperature, and then the mixture was stirred overnight. Saturated aqueous sodium hydrogencarbonate was added thereto to quench the reaction and the mixture was extracted with ethyl acetate. The organic layer was washed with saturated aqueous sodium hydrogencarbonate twice, dried over anhydrous magnesium sulfate, filtrated, and then the solvent was removed off in vacuo. The residue was purified by silica gel chromatography followed by crystallization with hexane-ethyl acetate to give 1.34 g of the title compound as a white crystal.
High-performance liquid chromatography/mass spectrometry
m/z 414.3 (M+H)
Retention time: 2.69 min.

### Example 118

### Preparation of cis-1-(3-methoxyphenyl)-4-(piperidin-4-yl-amino)cyclohexanecarbonitrile dihydrochloride

tert-Butyl 4-{[cis-4-cyano-4-(3-methoxyphenyl)-cyclohexyl]amino}piperidine-1-carboxylate (1.00 g, 2.42 mmol) was dissolved in 4N hydrochloric acid/1,4-dioxane (10 mL), and then the mixture was stirred overnight at room temperature. The solvent was removed off in vacuo, and the residue was purified by crystallization with ethyl acetate to give 1.45 g of the title compound as a white solid.
High-performance liquid chromatography/mass spectrometry
m/z 314.3 (M+H)
Retention time: 1.80 min.

### Example 119

### Preparation of cis-1-(3-methoxyphenyl)-4-[(1-methyl-piperidin-4-yl)amino]cyclohexanecarbonitrile

To a solution of cis-1-(3-methoxyphenyl)-4-(piperidin-4-ylamino)cyclohexanecarbonitrile dihydrochloride (50 mg, 0.129 mmol) and potassium carbonate (89.1 mg, 0.645 mmol) in N,N-dimethylformamide (DMF, 1.0 mL), methyl iodide (0.010 ml, 0.168 mmol) was added at room temperature, and then the mixture was warmed to 50°C and stirred for 5 hours. Brine was added thereto to quench the reaction, and then the mixture was extracted with ethyl acetate, the organic layer was washed with brine twice, dried over anhydrous magnesium sulfate, filtrated, and then the solvent was removed from the filtrate in vacuo. The residue was purified by silica gel chromatography to give 17.8 mg of the title compound as a colorless oil.
High-performance liquid chromatography/mass spectrometry
m/z 328.6 (M+H)
Retention time: 1.38 min.

### Example 120

### Preparation of cis-4-[(1-benzylpiperidin-4-yl)amino]-1-(3-methoxyphenyl)cyclohexanecarbonitrile

To a suspension of cis-1-(3-methoxyphenyl)-4-(piperidin-4-ylamino)cyclohexanecarbonitrile dihydrochloride (50 mg, 0.129 mmol) and potassium carbonate (89.1 mg, 0.645 mmol) in N,N-dimethylformamide (DMF, 1.0 mL) in an ice bath, benzyl bromide (0.020 ml, 0.168 mmol) was added, and then the mixture was warmed to room temperature and stirred overnight. Water was added thereto to quench the reaction, and then the mixture was extracted with ethyl acetate. The organic layer was washed with brine once, dried with anhydrous magnesium sulfate, filtrated, and then the solvent was removed from the filtrate in vacuo. The residue was purified by silica gel chromatography to give 30.6 mg of the title compound as a light yellow solid.
High-performance liquid chromatography/mass spectrometry
m/z 404.5 (M+H)
Retention time: 2.71 min.

### Example 121

### Preparation of cis-1-(3-methoxyphenyl)-4-{[1-(methanesulfonyl)piperidin-4-yl]amino}cyclohexanecarbonitrile

To a solution of cis-1-(3-methoxyphenyl)-4-(piperidin-4-ylamino)cyclohexanecarbonitrile dihydrochloride (50 mg, 0.129 mmol) and triethylamine (0.090 ml, 0.645 mmol) in dichloromethane (1.0 mL) in an ice bath, methanesulfonyl chloride (0.013 ml, 0.172 mmol) was added, and then the mixture was warmed to room temperature and stirred overnight. Water was added thereto to quench the reaction, and then the mixture was extracted with chloroform. The organic layer was washed with water once, dried over anhydrous magnesium sulfate, filtrated, and then the solvent was removed from the filtrate in vacuo to give 8.6 mg of the title compound as a white solid.
High-performance liquid chromatography/mass spectrometry
m/z 392.2 (M+H)
Retention time: 2.34 min.

### Example 122

### Preparation of cis-1-(3-methoxyphenyl)-4-({1-[(4-methylphenyl)sulfonyl]piperidin-4-yl}amino)cyclohexane-carbonitrile

To a solution of cis-1-(3-methoxyphenyl)-4-(piperidin-4-ylamino)cyclohexanecarbonitrile dihydrochloride (50 mg, 0.129 mmol) and triethylamine (0.090 ml, 0.645 mmol) in dichloromethane (1.0 mL) in an ice bath, p-toluenesulfonyl chloride (32.0 mg, 0.168 mmol) was added, and then the mixture was warmed to room temperature and stirred overnight. Water was added thereto to quench the reaction, and then ethyl acetate was added thereto. The precipitated solid was collected by filtration to give 68.0 mg of the title compound as a white solid.
High-performance liquid chromatography/mass spectrometry
m/z 454.4 (M+H)
Retention time: 2.67 min.

### Example 123

### Preparation of cis-4-[(1-acetylpiperidin-4-yl)amino]-1-(3-methoxyphenyl)cyclohexanecarbonitrile

To a solution of cis-1-(3-methoxyphenyl)-4-(piperidin-4-ylamino)cyclohexanecarbonitrile dihydrochloride (50 mg, 0.129 mmol) and triethylamine (0.090 ml, 0.645 mmol) in dichloromethane (1.0 mL) in an ice bath, acetyl chloride (0.012 ml, 0.168 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed off in vacuo to give 11.0 mg of the title compound as a white crystal.
High-performance liquid chromatography/mass spectrometry
m/z 356.2 (M+H)
Retention time: 2.69 min.

### Example 124

### Preparation of cis-1-(3-methoxyphenyl)-4-[(1-pyrimidin-2-yl)amino]cyclohexanecarbonitrile

To a suspension of cis-1-(3-methoxyphenyl)-4-(piperidin-4-ylamino)cyclohexanecarbonitrile dihydrochloride (50 mg, 0.129 mmol) and potassium carbonate (89.1 mg, 0.645 mmol) in N,N-dimethylformamide (DMF, 1.0 mL), 2-chloropyrimidine (19.2 mg, 0.168 mmol) was added at room temperature, and then the mixture was warmed to 50°C and stirred overnight. Water was added thereto to quench the reaction, and then the mixture was extracted with ethyl acetate. The organic layer was washed once, dried over anhydrous magnesium sulfate, filtrated, and then the solvent was removed off in vacuo to give 43.8 mg of the title compound as a colorless oil.
High-performance liquid chromatography/mass spectrometry
m/z 392.2 (M+H)
Retention time: 2.38 min.

### Example 125-1

### Preparation of cis-4-(aminomethyl)-N-benzyl-4-(3-methoxyphenyl)cyclohexylamine

To a solution of lithium aluminium hydride (456 mg, 12.15 mmol) in tetrahydrofuran (50 mL), a solution of cis-4-(benzylamino)-1-(3-methoxyphenyl)cyclohexanecarbonitrile (1.3 g, 4.05 mmol) in tetrahydrofuran (10 mL) was added, and then the mixture was stirred for 3 hours heating under reflux. To the reaction mixture, water (0.46 m), 1N aqueous sodium hydroxide (0.46 mL), and water (1.4 mL) were added successively, and then the mixture was filtrated and concentrated to give 1.36 g of the title compound as a colorless oil.
High-performance liquid chromatography/mass spectrometry
m/z 325 (M+H)
Retention time: 2.00 min.

### Example 125-2

### Preparation of cis-4-(aminomethyl)-N-(biphenyl-4-ylmethyl)-4-(3-methoxyphenyl)cyclohexaneamine

The title compound was prepared in a similar manner to Example 125-1.
High-performance liquid chromatography/mass spectrometry
m/z 401 (M+H)
Retention time: 2.76 min.

### Example 126

### Preparation of cis-N-benzyl-4-(3-methoxyphenyl)-4-(piperidin-1-ylmethyl)cyclohexaneamine

To a solution of cis-4-(aminomethyl)-N-benzyl-4-(3-methoxyphenyl)cyclohexylamine (50 mg, 0.156 mmol) in dimethylformamide (1 mL) were added 1,5-dibromopentane (0.021 ml, 0.156 mmol) and potassium carbonate (64 mg, 0.468 mmol), and then the mixture was stirred for 7 hours at 50°C. During the stirring process, another potassium carbonate (64 mg, 0.468 mmol) was added thereto. The reaction mixture was poured into water and extracted with ethyl acetate. The organic layer was washed with brine, dried over anhydrous sodium sulfate, and then filtrated. The filtrate was concentrated in vacuo and the residue was purified by silica gel column chromatography and thin-layer chromatography to give 17 mg of the title compound as a colorless oil.
High-performance liquid chromatography/mass spectrometry
m/z 393 (M+H)
Retention time: 2.15 min.

### Example 127

### Preparation of cis-N-(biphenyl-4-ylmethyl)-4-[(ethylamino)-methyl]-4-(3-methoxyphenyl)cyclohexylamine

To a solution of cis-4-(aminomethyl)-N-(biphenyl-4-ylmethyl)-4-(3-methoxyphenyl)cyclohexylamine (50 mg, 0.125 mmol) and potassium carbonate (51.8 mg, 0.375 mmol) in N,N-dimethylformamide (DMF, 1.0 mL), ethyl iodide (0.025 ml, 0.275 mmol) was added at room temperature, and then the mixture was warmed to 50°C and stirred for 5 hours. Brine was added thereto to quench the reaction, and then the mixture was extracted with ethyl acetate. The organic layer was washed with brine twice, dried over anhydrous magnesium sulfate, filtrated, and then the solvent was removed from the filtrate in vacuo. The residue was purified by silica gel chromatography and preparative thin-layer chromatography to give 5.60 mg of the title compound as a colorless solid.
High-performance liquid chromatography/mass spectrometry
m/z 429.4 (M+H)
Retention time: 2.55 min.

### Example 128

### Preparation of benzyl {[cis-4-[(biphenyl-4-yl)amino]-1-(3-methoxyphenyl)cyclohexyl]methyl}amine

To a suspension of cis-4-(aminomethyl)-N-(biphenyl-4-ylmethyl)-4-(3-methoxyphenyl)cyclohexylamine (50 mg, 0.125 mmol) and potassium carbonate (86.4 mg, 0.625 mmol) in N,N-dimethylformamide (DMF, 1.0 mL) in an ice bath, benzyl bromide (0.015 ml, 0.125 mmol) was added, and then the mixture was warmed to room temperature and stirred overnight. Water was added thereto to quench the reaction, and then the mixture was extracted with ethyl acetate. The organic layer was washed with brine once, dried over anhydrous magnesium sulfate, filtrated, and then the solvent was removed from the filtrate in vacuo. The residue was purified by silica gel chromatography to give 19.7 mg of the title compound as a colorless oil.
High-performance liquid chromatography/mass spectrometry
m/z 491.3 (M+H)
Retention time: 2.67 min.

### Example 129

### Preparation of N-{[cis-4-[(biphenyl-4-ylmethyl)amino]-1-(3-methoxyphenyl)cyclohexyl]methyl}methanesulfonamide

To a solution of cis-4-(aminomethyl)-N-(biphenyl-4-ylmethyl)-4-(3-methoxyphenyl)cyclohexylamine (50 mg, 0.125 mmol) and triethylamine (0.088 ml, 0.625 mmol) in dichloromethane (1.0 mL) in an ice bath, methanesulfonyl chloride (0.012 ml, 0.138 mmol), and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 56.6 mg of the title compound as a colorless solid.
High-performance liquid chromatography/mass spectrometry
m/z 479.3 (M+H)
Retention time: 2.88 min.

### Example 130

### Preparation of N-{[cis-4-[(biphenyl-4-ylmethyl)amino]-1-(3-methoxyphenyl)cyclohexyl]methyl}-4-methylbenzenesulfonamide

To a solution of cis-4-(aminomethyl)-N-(biphenyl-4-ylmethyl)-4-(3-methoxyphenyl)cyclohexylamine (50 mg, 0.125 mmol) and triethylamine (0.088 ml, 0.625 mmol) in dichloromethane (1.0 mL) in an ice bath, p-toluenesulfonyl chloride (26.3 mg, 0.138 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 69.9 mg of the title compound as a colorless solid.
High-performance liquid chromatography/mass spectrometry
m/z 555.2 (M+H)
Retention time: 3.19 min.

### Example 131

### Preparation of N-{[cis-4-[(biphenyl-4-ylmethyl)amino]-1-(3-methoxyphenyl)cyclohexyl]methyl}acetamide

To a solution of cis-4-(aminomethyl)-N-(biphenyl-4-ylmethyl)-4-(3-methoxyphenyl)cyclohexylamine (50 mg, 0.125 mmol) and triethylamine (0.088 ml, 0.625 mmol) in dichloromethane (1.0 mL) in an ice bath, acetyl chloride (0.010 ml, 0.138 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 50.5 mg of the title compound as a colorless solid.
High-performance liquid chromatography/mass spectrometry
m/z 443.4 (M+H)
Retention time: 2.86 min.

### Example 132

### N-{[cis-4-[(biphenyl-4-ylmethyl)amino]-1-(3-methoxyphenyl)-cyclohexyl]methyl}benzamide

To a solution of cis-4-(aminomethyl)-N-(biphenyl-4-ylmethyl)-4-(3-methoxyphenyl)cyclohexylamine (50 mg, 0.125 mmol) and triethylamine (0.088 ml, 0.625 mmol) in dichloromethane (1.0 mL) in an ice bath, benzoyl chloride (0.016 ml, 0.138 mmol) was added, and then the mixture was warmed to room temperature and stirred overnight. Water was added thereto to quench the reaction, and then the mixture was extracted with ethyl acetate. The organic layer was washed with water once, dried over anhydrous magnesium sulfate, filtrated, and then the solvent was removed from the filtrate in vacuo. The residue was purified by silica gel chromatography to give 15.3 mg of the title compound as a colorless amorphous.
High-performance liquid chromatography/mass spectrometry
m/z 505.3 (M+H)
Retention time: 3.11 min.

### Example 133

### Preparation of cis-N-benzyl-4-[(biphenyl-4-ylmethyl)amino]-1-(3-methoxyphenyl)cyclohexanecarboxyamide

### Methyl cis-4-[(diphenylmethyl)amino]-1-(3-methoxyphenyl)-cyclohexanecarboxylate

To a solution of methyl 1-(3-methoxyphenyl)-4-oxocyclohexanecarboxylate (100 mg, 0.381 mmol) in methanol (2.0 mL) were added benzhydrylamine (72.0 µl, 0.419 mmol), acetic acid (44.0 µl, 0.762 mmol) and sodium cyanoborohydride (29.0 mg, 0.457 mmol) at 0°C, and then the mixture was stirred for 3 days at room temperature. Saturated aqueous sodium hydrogencarbonate was added thereto to quench the reaction. The mixture was extracted with ethyl acetate, dried over anhydrous magnesium sulfate, filtrated, and then the solvent was removed from the filtrate in vacuo. The residue was purified by silica gel column chromatography to give 74.0 mg of the title compound as a white solid.
¹H-NMR δ (CDCl₃) 1.28 (2H, m), 1.52 (2H, m), 2.04 (2H, m), 2.44 (1H, dddd, J = 11.0, 11.0, 4.0, 4.0 Hz), 2.57 (2H, m), 3.64 (3H, s), 3.76 (3H, s), 6.74 (1H, dd, J = 8.3, 2.6 Hz), 6.87 (1H, m), 6.91 (1H, m), 7.16-7.39 (11H, m).

### Methyl cis-4-[(tert-butoxycarbonyl)amino]-1-(3-methoxyphenyl)cyclohexanecarboxylate

To a solution methyl cis-4-[(diphenylmethyl)amino]-1-(3-methoxyphenyl)cyclohexanecarboxylate (1.20 g, 2.79 mmol) in methanol (24 mL) and tetrahydrofuran (12 mL) were added 10% palladium-carbon (240 mg) and di-tert-butyl carbonate (963 µl, 4.19 mmol) at room temperature, and then the mixture was stirred for 3 hours under hydrogen atmosphere (0.3 MPa). The reaction mixture was filtrated through Celite®, and the solvent was removed from the filtrate in vacuo. The residue was purified by silica gel chromatography to give 0.987 g of the title compound as a white solid.

### Cis-4-[(tert-butoxycarbonyl)amino]-1-(3-methoxyphenyl)-cyclohexanecarboxylic acid

To a solution of methyl cis-4-[(tert-butoxycarbonyl)-amino]-1-(3-methoxyphenyl)cyclohexanecarboxylate (800 mg, 2.20 mmol) in dimethylsulfoxide (8.0 mL), 6N aqueous potassium hydroxide (550 µl, 3.30 mmol) was added dropwise at room temperature, and then the mixture was stirred for 1.5 hours at 50°C. The reaction mixture was cooled to room temperature, diluted with water, and then adjusted to pH=4 with aqueous hydrochloric acid. The mixture was filtrated by aspiration and washed with water to give a crude product. The crude product was purified by silica gel chromatography to give 439 mg of the title compound as a white solid.
¹H-NMR δ (DMSO-d₆) 1.31 (2H, m), 1.3 (9H, s), 1.53 (2H, m), 1.75 (2H, m), 2.39(2H, m), 3.25 (1H, m), 3.72 (3H, s), 6.76 (1H, s), 6.79(1H, dd, J = 8.1, 2.4 Hz), 6.8 (1H, m), 6.95 (1H, d, J = 8.1Hz), 7.23 (1H, dd, J = 8.1, 8.1 Hz), 12.44 (1H, brs.).

### tert-Butyl [cis-4-[(benzylamino)carbonyl]-4-(3-methoxyphenyl)cyclohexyl]carbamate

To a solution of cis-4-[(tert-butoxycarbonyl)amino]-1-(3-methoxyphenyl)cyclohexanecarboxylic acid (80 mg, 0.228 mmol) in toluene were added oxalyl chloride (0.049 ml, 0.457 mmol) and dimethylformamide (0.001 mL), and then the mixture was stirred for 3 hours at room temperature. The reaction mixture was concentrated and the residual solvent was removed by azeotropic distillation with toluene. Dichloromethane (2 mL), triethylamine (0.095 ml, 0.684 mmol) and benzylamine (0.025 ml, 0.228 mmol) were added to the residue, and the mixture was stirred for 19 and half hours at room temperature. The reaction mixture was poured into aqueous ammonia and extracted with ethyl acetate. The organic layer was washed with brine, dried over anhydrous sodium sulfate, and then filtrated. The filtrate was concentrated in vacuo and the residue was purified by silica gel column chromatography to give 75 mg of the title compound as a white solid.
High-performance liquid chromatography/mass spectrometry
m/z 439 (M+H)
Retention time: 3.72 min.

### Cis-4-amino-N-benzyl-1-(3-methoxyphenyl)cyclohexanecarboxy-amide)

To a solution of tert-butyl [cis-4-[(benzylamino)-carbonyl]-4-(3-methoxyphenyl)cyclohexyl]carbamate (72 mg, 0.164 mmol) in 1,4-dioxane (1 mL), 4N hydrochloric acid/dioxane was added, and the mixture was stirred for three and half hours at room temperature. The reaction mixture was poured into saturated aqueous sodium hydrogencarbonate and extracted with ethyl acetate. The organic layer was washed with brine, dried over anhydrous sodium sulfate, and then filtrated. The filtrate was concentrated in vacuo to give 59 mg of the title compound as a colorless oil.
High-performance liquid chromatography/mass spectrometry
m/z 339 (M+H)
Retention time: 2.46 min.
Cis-N-benzyl-4-[(biphenyl-4-ylmethyl)amino]-1-(3-methoxyphenyl)cyclohexanecarboxyamide

The title compound was prepared in a similar manner to cis-4-(benzylamino)-1-(3-methoxyphenyl)cyclohexane-carbonitrile (Example 105).
High-performance liquid chromatography/mass spectrometry
m/z 505 (M+H)
Retention time: 3.11 min.

### Example 134

### Cis-4-(benzylamino)-1-(3-methoxyphenyl)cyclohexanecarboxy-amide

To a solution of cis-4-(benzylamino)-1-(3-methoxyphenyl)cyclohexanecarbonitrile (50 mg, 0.156 mmol) in dimethylsulfoxide (DMSO, 1.0 mL), 6N aqueous sodium hydroxide (0.5 mL) at room temperature, and the mixture was stirred for 20 hours at 100°C. Saturated aqueous ammonium chloride was added thereto to quench the reaction, and then the mixture was extracted with ethyl acetate twice. The organic layer was washed with saturated aqueous ammonium chloride once, dried over anhydrous magnesium sulfate, filtrated, and then the solvent was removed from the filtrate in vacuo. The residue was purified by silica gel chromatography to give 36.9 mg of the title compound as a white solid.
High-performance liquid chromatography/mass spectrometry
m/z 339.2 (M+H)
Retention time: 2.34 min.

### Example 135

### Trans-1-(3-methoxyphenyl)-4-piperazin-1-ylcyclohexane-carboxyamide

To a solution of trans-1-(3-methoxyphenyl)-4-piperazin-1-ylcyclohexanecarbonitrile (70 mg, 0.188 mmol) in dimethylsulfoxide (DMSO, 1.4 mL), 6N aqueous sodium hydroxide (0.7 mL) was added at room temperature, and then the mixture was stirred for 9 hours at 100°C. Then, di-tert-butyl dicarboxylate (98.3 mg, 0.564 mmol) was added thereto at room temperature, and then the mixture was stirred overnight. Saturated aqueous ammonium chloride was added thereto to quench the reaction, and then the mixture was extracted with ethyl acetate. The organic layer was washed with saturated aqueous ammonium chloride once, dried over anhydrous magnesium sulfate, filtrated, and then the solvent was removed off in vacuo. The obtained mixture was dissolved in 4N hydrochloric acid/1,4-dioxane (10 mL) and stirred overnight at room temperature. The solvent was removed off in vacuo and then the residue was purified by crystallization with ethyl acetate to give 15.9 mg of the title compound as a colorless amorphous.
High-performance liquid chromatography/mass spectrometry
m/z 318.5 (M+H)
Retention time: 0.29 min.

### Example 136

### Preparation of cis-4-[(biphenyl-4-ylmethyl)amino]-1-(3-methoxyphenyl)cyclohexanecarboxyamide

To a solution of cis-4-[(biphenyl-4-ylmethyl)amino]-1-(3-methoxyphenyl)cyclohexanecarbonitrile (100 mg, 0.252 mmol) in dimethylsulfoxide (1.3 mL), 6N aqueous potassium hydroxide (0.6 mL), and then the mixture was stirred for 20 hours at 100°C. The reaction mixture was poured into water and extracted with ethyl acetate. The organic layer was washed with brine, dried over anhydrous sodium sulfate, filtrated, and then concentrated in vacuo. The residue was purified by silica gel column chromatography to give 88 mg of the title compound as a white solid.
High-performance liquid chromatography/mass spectrometry
m/z 415 (M+H)
Retention time: 2.78 min.

### Example 137

To each reaction tube with filter (Libra Tube: HiPep Laboratories) was added tetrahydrofuran (2 mL) followed by cis-4-amino-1-(3-methoxyphenyl)cyclohexanecarbonitrile (55.3 mg, 0.24 mmol) and each of various aldehyde compounds (0.2 mmol in each), and each mixture was dissolved. Then, Macro porous (MP)-triacetoxyborohydride resin (each 2.02 mmol/gr: 0.5 mmol, 250 mg to each reaction mixture) was added thereto, and each mixture was orbital-stirred for 86 hours at room temperature.

Next, to each reaction tube, another polystyrene-aldehyde (PS-CHO) resin (0.2 mmol in each) and tetrahydrofuran (1.5 mL) was added, and each mixture was orbital-stirred at room temperature. After 6 hours, the reaction mixture was filtrated and the resin was washed with tetrahydrofuran several times. The filtrate and the wash were combined, and concentrated to dryness via a Speed Vac to give a crude product of the desired compound (as an acetate form).

### Purification A

The above acetate compound was dissolved in methanol, and purified by preparative HPLC. The collected fraction was concentrated to dryness via a Speed Vac to give a pure product thereof (as a trifluoroacetate compound). The trifluoroacetate compound was dissolved in methanol, Macro porous-carbonate (MP-carbonate) resin that was four times as heavy as the trifluoroacetate compound was added thereto and then the mixture was shaken. After 2 hours, the mixture was filtrated through a filter, and the purity of the filtrate was analyzed by HPLC before the filtrate was concentrated to dryness via a Speed Vac to give the compounds shown in Tables 1 to 9.

### Purification B

The above acetate compound was recrystallized with methanol or ethanol to give a crystal of the acetate compound. The crystal was dissolved in dichloromethane, and Macro porous-carbonate (MP-carbonate) resin that was four times as heavy as the acetate compound was added thereto, and the mixture was shaken. After 2 hours, the mixture was filtrated through a filter, and the purity of the filtrate was analyzed by HPLC before the filtrate was concentrated to dryness via a Speed Vac to give the compounds shown in Tables 1 to 9.

### Analytical method

Analytical condition:
MS detector Perkin-Elmer Sciex API 150EX Mass spectrometer (40eV),
HPLC Shimadzu LC 8A,
Column Shiseido CAPCELL PAK C18 (4.6 mm X 50 mm),
Gradient condition; A : 0.35%TFA/CH₃CN, B : 0.05%TFA/H₂O, 0.0-0.5 min A 10% B 90%
0.5-4.2 min Linear gradient from A 10% B 90% to A 99% B 1% 4.2-6.3 min A 99% B 1%.
UV 220 nm and 254 nm.

| Table 1 | | | | |
|---|---|---|---|---|
| 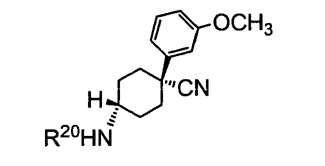 | | | | |

| No. | R²⁰ | m/z (M+1) | Retention Time (min) | Purification |
|---|---|---|---|---|
| 137-1 | 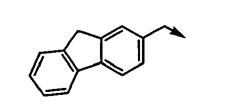 | 409.5 | 3.92 | B |
| 137-2 | 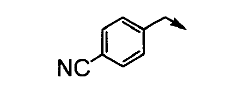 | 346.5 | 3.34 | A |
| 137-3 | 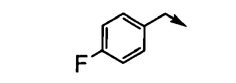 | 339.2 | 3.44 | A |
| 137-4 | 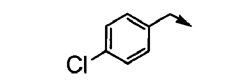 | 355.2 | 3.57 | A |
| 137-5 | 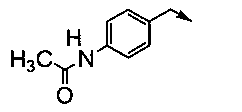 | 378.5 | 3.20 | A |
| 137-6 | 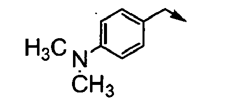 | 364.1 | 3.09 | A |
| 137-7 | 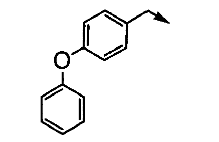 | 413.5 | 3.88 | A |
| 137-8 | 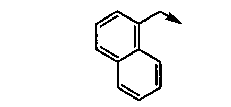 | 371.5 | 3.63 | A |
| 137-9 | 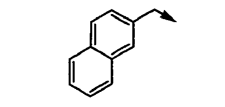 | 371.5 | 3.69 | A |
| 137-10 | 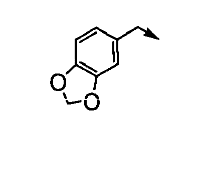 | 365.3 | 3.42 | A |

| Table 2 | | | | |
|---|---|---|---|---|
| 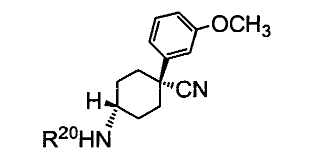 | | | | |

| No. | R²⁰ | m/z (M+1) | Retention Time (min) | Purification |
|---|---|---|---|---|
| 137-11 | 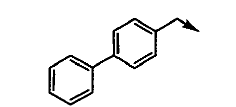 | 397.5 | 3.90 | B |
| 137-12 | 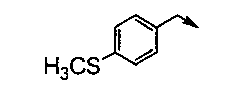 | 367.3 | 3.59 | A |
| 137-13 | 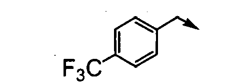 | 389.4 | 3.69 | A |
| 137-14 | 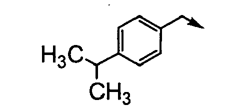 | 363.3 | 3.82 | A |
| 137-15 | 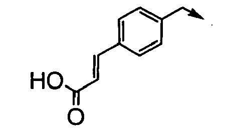 | 391.6 | 3.34 | A |
| 137-16 | 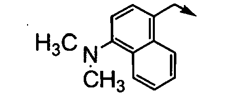 | 414.6 | 3.34 | A |
| 137-17 | 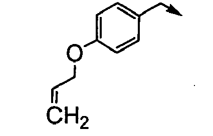 | 377.3 | 3.67 | A |
| 137-18 | 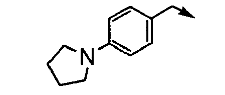 | 388.5 | 3.74 | A |
| 137-19 | 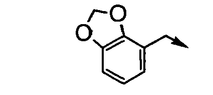 | 365.3 | 3.44 | A |
| 137-20 | 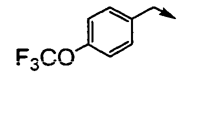 | 405.4 | 3.76 | A |

| Table 3 | | | | |
|---|---|---|---|---|
| 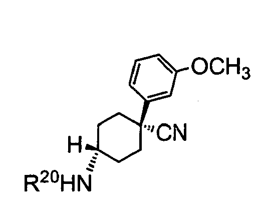 | | | | |

| No. | R²⁰ | m/z (M+1) | Retention Time (min) | Purification |
|---|---|---|---|---|
| 137-21 | 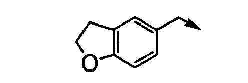 | 363.6 | 3.47 | B |
| 137-22 | 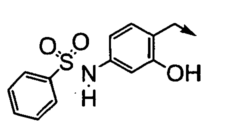 | 492.2 | 3.61 | B |
| 137-23 | 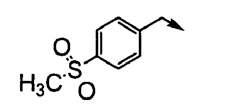 | 399.5 | 3.24 | A |
| 137-24 | 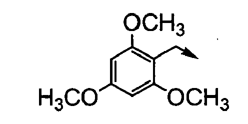 | 411.8 | 3.74 | B |
| 137-25 | 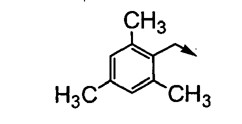 | 364.5 | 3.76 | B |
| 137-26 | 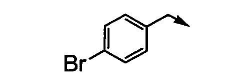 | 401.4 | 3.61 | A |
| 137-27 | 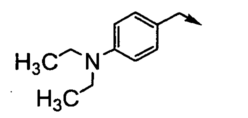 | 392.5 | 3.01 | A |
| 137-28 | 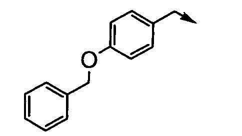 | 427.2 | 3.95 | B |
| 137-29 | 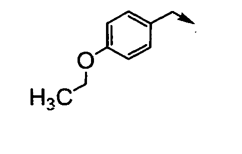 | 365.3 | 3.59 | A |

| Table 4 | | | | |
|---|---|---|---|---|
| 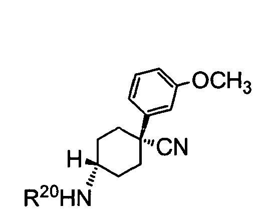 | | | | |

| No. | R²⁰ | m/z (M+1) | Retention Time (min) | Purification |
|---|---|---|---|---|
| 137-30 | 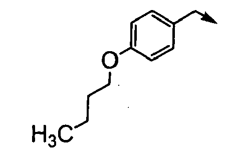 | 393.6 | 3.92 | A |
| 137-31 | 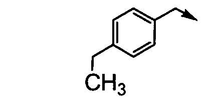 | 349.3 | 3.69 | A |
| 137-32 | 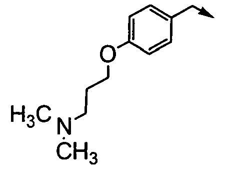 | 422.4 | 3.01 | A |
| 137-33 | 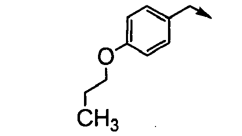 | 379.6 | 3.76 | A |
| 137-34 | 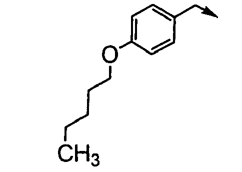 | 407.6 | 4.07 | A |
| 137-35 | 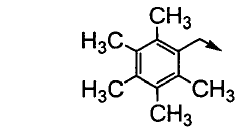 | 391.6 | 3.92 | B |
| 137-36 | 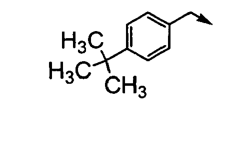 | 377.3 | 3.88 | A |

| Table 5 | | | | |
|---|---|---|---|---|
| 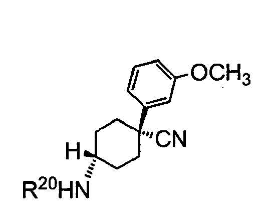 | | | | |

| No. | R²⁰ | m/z (M+1) | Retention Time (min) | Purification |
|---|---|---|---|---|
| 137-37 | 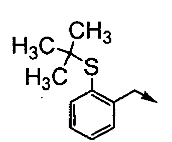 | 409.6 | 3.92 | A |
| 137-38 | 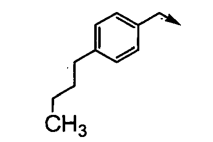 | 377.6 | 3.99 | A |
| 137-39 | 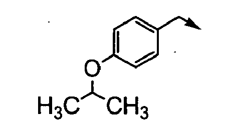 | 379.3 | 3.72 | A |
| 137-40 | 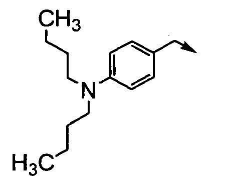 | 448.5 | 3.72 | A |
| 137-41 | 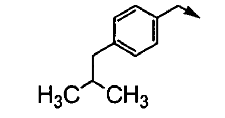 | 377.6 | 3.95 | A |
| 137-42 | 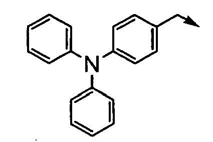 | 488.3 | 4.28 | A |
| 137-43 | 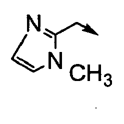 | 325.5 | 2.76 | A |

| Table 6 | | | | |
|---|---|---|---|---|
| 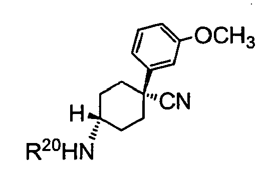 | | | | |

| No. | R²⁰ | m/z (M+1) | Retention Time (min) | Purification |
|---|---|---|---|---|
| 137-44 | 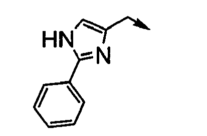 | 387.4 | 3.07 | A |
| 137-45 | 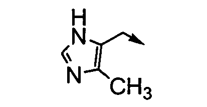 | 325.5 | 2.76 | A |
| 137-46 | 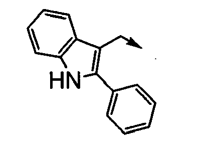 | 436.4 | 3.86 | A |
| 137-47 | 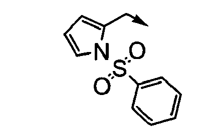 | 450.2 | 3.74 | A |
| 137-48 | 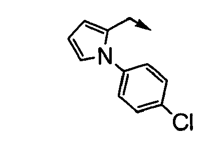 | 420.5 | 3.86 | A |
| 137-49 | 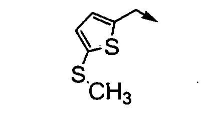 | 373.4 | 3.59 | A |
| 137-50 | 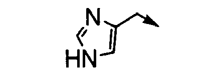 | 311.2 | 2.73 | A |
| 137-51 | 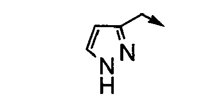 | 311.2 | 3.03 | A |
| 137-52 | 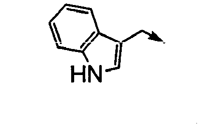 | 360.3 | 3.55 | A |

| Table 7 | | | | |
|---|---|---|---|---|
| 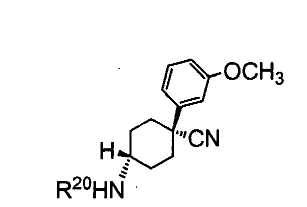 | | | | |

| No. | R²⁰ | m/z (M+1) | Retention Time (min) | Purification |
|---|---|---|---|---|
| 137-53 | 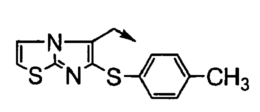 | 489.7 | 3.82 | A |
| 137-54 | 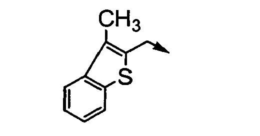 | 391.3 | 3.78 | A |
| 137-55 | 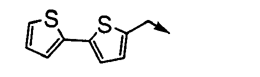 | 409.5 | 3.78 | B |
| 137-56 | 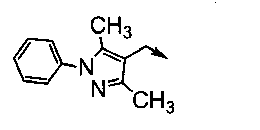 | 415.4 | 3.57 | A |
| 137-57 | 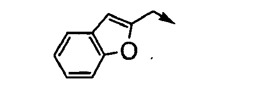 | 361.7 | 3.59 | A |
| 137-58 | 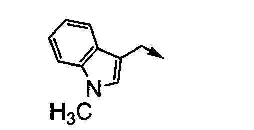 | 374.5 | 3.67 | A |
| 137-59 | 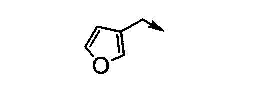 | 311.2 | 3.26 | A |
| 137-60 | 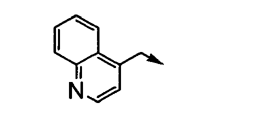 | 372.6 | 3.05 | A |
| 137-61 | 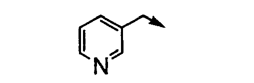 | 322.4 | 2.84 | A |
| 137-62 | 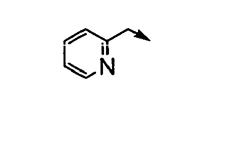 | 322.4 | 3.13 | A |

| Table 8 | | | | |
|---|---|---|---|---|
| 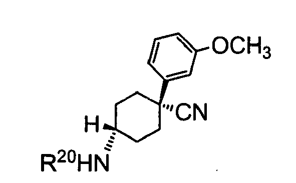 | | | | |

| No. | R²⁰ | m/z (M+1) | Retention Time(min) | Purification |
|---|---|---|---|---|
| 137-63 | 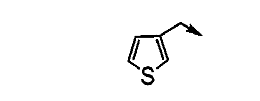 | 327.5 | 3.36 | B |
| 137-64 | 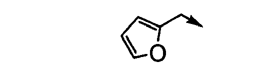 | 311.2 | 3.26 | A |
| 137-65 | 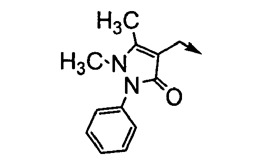 | 431.4 | 3.26 | A |
| 137-66 | 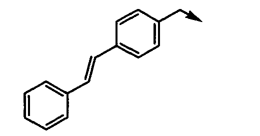 | 423.3 | 4.09 | B |
| 137-67 | 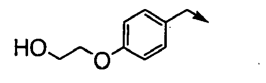 | 381.5 | 3.24 | A |
| 137-68 | 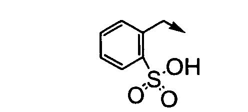 | 401.1 | 3.34 | A |
| 137-69 | 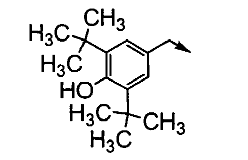 | 449.6 | 4.24 | A |
| 137-70 | 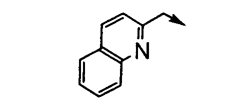 | 372.3 | 4.24 | A |
| 137-71 | 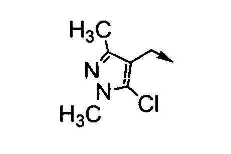 | 372.9 | 3.28 | A |

| Table 9 | | | | |
|---|---|---|---|---|
| 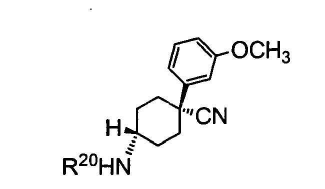 | | | | |

| No. | R²⁰ | m/z (M+1) | Retention Time (min) | Purification |
|---|---|---|---|---|
| 137-72 | 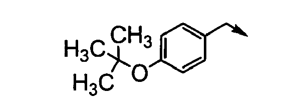 | 393.6 | 3.86 | A |
| 137-73 | 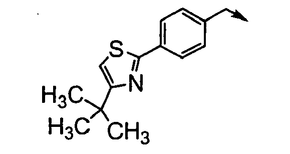 | 460.5 | 4.26 | A |
| 137-74 | 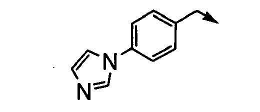 | 387.7 | 3.07 | A |
| 137-75 | 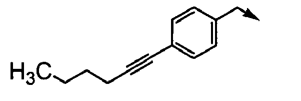 | 401.7 | 4.24 | A |
| 137-76 | 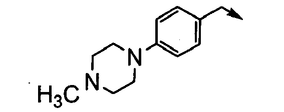 | 418.59 | 3.01 | A |
| 137-77 | 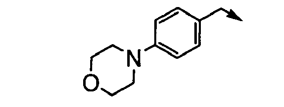 | 407.6 | 3.49 | A |
| 137-78 | 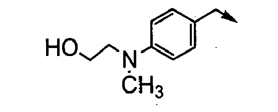 | 394.4 | 3.17 | A |
| 137-79 | 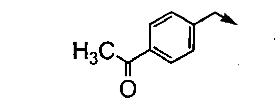 | 363.3 | 3.44 | A |
| 137-80 | 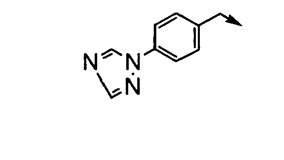 | 388.5 | 3.34 | A |

### Experiments

The action for enhancing LDL receptor expression of the compounds of the invention is evaluated in the following method.

### 1. Mesurement of LDL receptor expression

We investigated the effect of each test compound on the amount of LDL receptor in HepG2 cells, a human hepatoma cell line. Cells were seeded in each well of 6-well plates at the density of 7.5 x 10⁵ cells/well (day 1). On day 3, the culture media was changed to a media containing lipoprotein deficient serum and the test compound. On day 4, the cells were scraped and then collected by centrifugation operation. The collected cells were lysed in a buffer containing 0.1% TritonX-100, treated with centrifugation, and then the supernatant was given as a cell protein solution. Using the prepared cell protein solution, the LDL-R protein amount was determined with an immunoblotting procedure. The results are shown in Table 1.

HepG2 cells were treated with the compound of Examples 1-4, and then LDL receptor protein amount was determined by immunoblotting procedure. The compounds of Examples 1-4 at a concentration of 1 and 10 µM exhibited a superior activity enhancing the protein amount of LDL receptor compared with the control group.

### 2. Action of the compounds for LDL receptor activity in human hepatoma cell line

A preparation of 1,1'-dioctadecyl-3,3,3',3'-tetramethylindocarbocyanine perchlorate (DiI)-labeled LDL (DiI-LDL) was carried out as follows. That is, DiI (invitrogen, the U.S.) was mixed with human LDL (CHEMICON, the U.S.), and the mixture was kept for 8 hours at 37°C. Thereto a specific density solution (d=1.182) was added and mixed, and then treated with super centrifugation operation (32 k rpm, 14 hours ; using Beckman Ti55.2 rotator). After the centrifugation, the DiI labeled LDL fraction was taken up and prepared via a dialysis against saline.

The action on LDL receptor was determined with the amount of the DiI-LDL uptaken into HepG2 cells which was as an indicator. Namely, human hepatoma cell line HepG2 was bought from DAINIPPON PHARMACEUTICAL CO.,LTD (Osaka, Japan) and it was used for the experiment. The HepG2 cells were plated on a 96 well plate and incubated with Dulbecco's modified Eagle/F-12 medium (DMEM/F-12) containing 10% fetal bovine serum and antibiotics for 2-3 days at 37°C in a CO₂ incubator. After washing the cells, DMEM/F-12 media containing the test compound, 10% delipoprotein serum and antibiotics was added thereto and cells were incubated for 19 hours at 37°C. The DiI-LDL was added thereto, and cells were incubated for additional 5 hours at 37°C in a CO₂ incubator. For the measurement of the amount of nonspecific DiI-LDL uptake, another 30-50 times the nonlabeled LDL was added additively to each well. After washing cells with Dulbecco's phosphate buffer (SIGMA, the U.S.), Dulbecco's phosphate buffer was added to each well, and then the fluorescence value was measured with a fluorescence plate reader to determine the amount of the DiI-LDL uptaken into cells. After measuring the fluorescence, the Dulbecco's phosphate buffer was removed. Cells were dissolved in IN sodium hydroxide solution. Using a part of the solution, the protein amount was measured. The action of each compound on LDL receptor was estimated on the basis of 100% of the control group, using the value given by subtracting the nonspecifically uptaken amount from the caluculated fluorescence value/protein amount.

As shown in Table 10, the example compounds enhanced the amount of the DiI-LDL uptaken into the HepG2 cells and hence facilitated the activity of LDL receptor.

**Table 10**

| Example Compound | (Concentration) | Activity of LDL receptor(%) |
|---|---|---|
| 1-4 | (10 µM) | 134 |
| 6-3 | (10 µM) | 127 |
| 7-3 | (3 µM) | 125 |
| 12 | (10 µM) | 134 |
| 20 | (10 µM) | 123 |
| 43-2 | (10 µM) | 116 |
| 44 | (3 µM) | 135 |
| 49-f | (10 µM) | 135 |
| 55-1 | (1 µM) | 141 |
| 58-1 | (10 µM) | 152 |
| 82 | (10 µM) | 125 |
| 85 | (10 µM) | 149 |
| 89 | (10 µM) | 195 |
| 94 | (10 µM) | 136 |
| 105 | (10 µM) | 139 |

### INDUSTRIAL APPLICABILITY

The compound of the formula (1) of the invention or a prodrug thereof, or an acid addition salt thereof exhibits a potent action for enhancing LDL receptor expression in human hepatoma cell line HepG2 and hence directly or indirectly promotes protein expression of LDL receptor. Therefore, the compound or its derivative is useful for the prophylaxis or treatment of hyperlipidemia, or the prophylaxis or treatment of atherosclerosis per se or various diseases accompanied by atherosclerosis such as cerebral infarction, cerebral thrombosis, transient cerebral ischemia, angina pectoris, myocardial infarction, peripheral thrombus and occlusion.

## Claims

1. A medicament for enhancing low density lipoprotein receptor expression comprising as an active ingredient a compound of the formula (1): 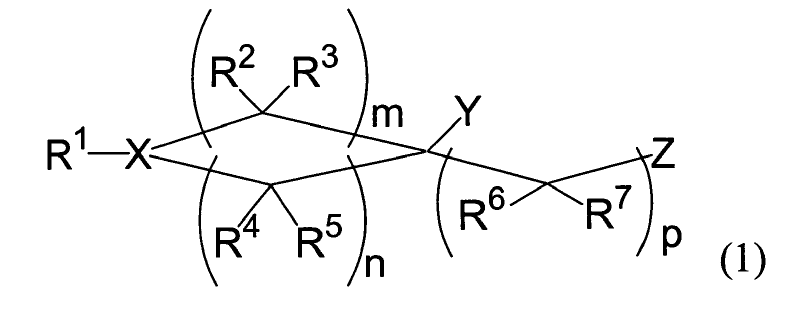 wherein
m, n, and p are independently an integer of 0 - 4, provided 3 ≤ m + n ≤ 8;
X is nitrogen atom or a group of the formula: C-R¹⁵;
R¹⁵ is hydrogen atom, a substituted or unsubstituted alkyl group, a substituted or unsubstituted aromatic group, or a group of the formula: -NR¹⁹R²⁰ wherein
R¹⁹ and R²⁰ are each independently hydrogen atom; a substituted or unsubstituted lower alkyl group; a substituted or unsubstituted cycloalkyl group; a saturated heterocyclic group comprising 3 - 8 carbon atoms as ring components which includes one -NR²¹- (R²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted lower alkoxycarbonyl group; a substituted or unsubstituted aromatic group; a substituted or unsubstituted aralkyl group; or a substituted or unsubstituted heteroarylalkyl group; or alternatively
R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group;
Y is a substituted or unsubstituted alkyl group; a substituted or unsubstituted alkenyl group; a substituted or unsubstituted alkynyl group; a substituted or unsubstituted cycloalkyl group; a substituted or unsubstituted aromatic group; or a group of the formula: - C(=O)R⁸ wherein R⁸ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
R¹ is hydrogen atom; a substituted or unsubstituted alkyl group; a substituted or unsubstituted alkenyl group; a substituted or unsubstituted alkynyl group; a substituted or unsubstituted cycloalkyl group; a saturated heterocyclic group comprising 3 - 8 carbon atoms as ring components which includes one -NR²³- (R²³ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted aromatic group; or a group of the formula: - C(=O)R¹⁴ wherein R¹⁴ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
R², R³, R⁴, R⁵, R⁶, and R⁷ are the same or different and are selected from the group consisted of hydrogen atom, hydroxyl group, a substituted or unsubstituted alkyl group, a substituted or unsubstituted alkoxy group, a substituted or unsubstituted alkoxycarbonyl group, a substituted or unsubstituted aralkyl group, a substituted or unsubstituted heteroarylalkyl group, a substituted or unsubstituted aralkyloxy group, and a substituted or unsubstituted heteroarylalkyloxy group; and when each of R², R³, R⁴, R⁵, R⁶, and/or R⁷ exists plurally, each thereof is independently selected from the aforementioned group; alternatively
one or plural combinations of R² and R³, R⁴ and R⁵, and R⁶ and R⁷ may combine to form oxo group; alternatively
R² and R⁴ may combine to form an alkylene group; alternatively
any two of the carbon atoms substituted with R² and R³, or R⁴ and R⁵ may combine to form double bond when the two carbons are located adjacently; and
Z is hydrogen atom, hydroxyl group, carboxy group, cyano group, phthalimide group, halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aromatic group, a substituted or unsubstituted lower alkoxycarbonyl group, a substituted or unsubstituted carbamoyl group, a substituted or unsubstituted benzyloxycarbonyl group, a substituted or unsubstituted aralkyloxy group, a substituted or unsubstituted heteroarylalkyloxy group, a substituted or unsubstituted aryloxy group, a substituted or unsubstituted heteroaryloxy group, a substituted or unsubstituted lower alkoxy group, a substituted or unsubstituted lower alkanoyloxy group, a substituted or unsubstituted lower alkylthio group, a substituted or unsubstituted lower alkylsulfinyl group, a substituted or unsubstituted lower alkylsulfonyl group, a substituted or unsubstituted benzenesulfonyloxy group, a substituted or unsubstituted lower alkoxycarbonylamino group, or a group of the formula: -NR⁹R¹⁰ wherein
R⁹ and R¹⁰ are each independently hydrogen atom, a substituted or unsubstituted lower alkyl group, a substituted or unsubstituted cycloalkyl group, a substituted or unsubstituted lower alkoxycarbonyl group, a substituted or unsubstituted aromatic group, a substituted or unsubstituted acyl group, a substituted or unsubstituted aralkyl group, or a substituted or unsubstituted heteroarylalkyl group; or alternatively
R⁹ and R¹⁰ may combine together with the nitrogen atom bound with R⁹ and R¹⁰ to form a saturated cyclic amino group comprising 3 - 8 carbon atoms as ring components, which may further include one -NR¹¹- (R¹¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group,
or a prodrug thereof, or a pharmaceutically acceptable salt thereof.

2. The medicament according to claim 1 for treating hyperlipidemia or arteriosclerosis.

3. A compound of the formula (1'): 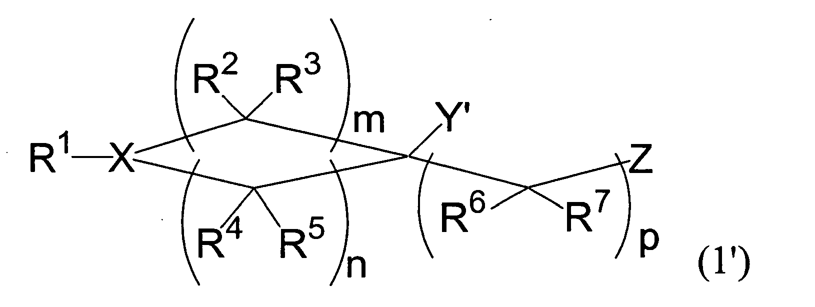 wherein
m, n, and p are independently an integer of 0 - 4, provided 3 ≤ m + n ≤ 8;
X is nitrogen atom or a group of the formula: C-R¹⁵;
R¹⁵ is hydrogen atom, a substituted or unsubstituted alkyl group, a substituted or unsubstituted aromatic group, or a group of the formula: -NR¹⁹R²⁰ wherein
R¹⁹ and R²⁰ are each independently hydrogen atom; a substituted or unsubstituted lower alkyl group; a substituted or unsubstituted cycloalkyl group; a saturated heterocyclic group comprising 3 - 8 carbon atoms as ring components which includes one -NR²¹- (R²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted lower alkoxycarbonyl group; a substituted or unsubstituted aromatic group, a substituted or unsubstituted aralkyl group; or a substituted or unsubstituted heteroarylalkyl group; or alternatively
R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group;
Y' is a substituted or unsubstituted cycloalkyl group; a substituted or unsubstituted aromatic group; or a group of the formula: -C(=O)R^{8a} wherein R^{8a} is a substituted or unsubstituted cycloalkyl group, or a substituted or unsubstituted aromatic group;
R¹ is hydrogen atom; a substituted or unsubstituted alkyl group; a substituted or unsubstituted alkenyl group; a substituted or unsubstituted alkynyl group, a substituted or unsubstituted cycloalkyl group; a saturated heterocyclic group comprising 3 - 8 carbon atoms as ring components which includes one -NR²³- (R²³ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; a substituted or unsubstituted aromatic group; or a group of the formula: - C(=O)R¹⁴ wherein R¹⁴ is a substituted or unsubstituted alkyl group, a substituted or unsubstituted alkenyl group, a substituted or unsubstituted alkynyl group, a substituted or unsubstituted cycloalkyl group, or a substituted or unsubstituted aromatic group;
R², R³, R⁴, R⁵, R⁶, and R⁷ are the same or different and are selected from the group consisted of hydrogen atom, hydroxyl group, a substituted or unsubstituted alkyl group, a substituted or unsubstituted alkoxy group, a substituted or unsubstituted alkoxycarbonyl group, a substituted or unsubstituted aralkyl group, a substituted or unsubstituted heteroarylalkyl group, a substituted or unsubstituted aralkyloxy group, or a substituted or unsubstituted heteroarylalkyloxy group; and when each of R², R³, R⁴, R⁵, R⁶, and/or R⁷ exists plurally, each thereof is independently selected from the aforementioned group; alternatively
one or plural combinations of R² and R³, R⁴ and R⁵, and R⁶ and R⁷ may combine to form oxo group; alternatively
R² and R⁴ may combine to form an alkylene group; alternatively
any two of the carbon atoms substituted with R² and R³ or R⁴ and R⁵ may combine to form double bond when the two carbons are located adjacently; and
Z is hydrogen atom, hydroxyl group, carboxy group, cyano group, phthalimide group, halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aromatic group, a substituted or unsubstituted lower alkoxycarbonyl group, a substituted or unsubstituted carbamoyl group, a substituted or unsubstituted benzyloxycarbonyl group, a substituted or unsubstituted aralkyloxy group, a substituted or unsubstituted heteroarylalkyloxy group, a substituted or unsubstituted aryloxy group, a substituted or unsubstituted heteroaryloxy group, a substituted or unsubstituted lower alkoxy group, a substituted or unsubstituted lower alkanoyloxy group, a substituted or unsubstituted lower alkylthio group, a substituted or unsubstituted lower alkylsulfinyl group, a substituted or unsubstituted lower alkylsulfonyl group, a substituted or unsubstituted benzenesulfonyloxy group, a substituted or unsubstituted lower alkoxycarbonylamino group, or a group of the formula: -NR⁹R¹⁰ wherein
R⁹ and R¹⁰ are each independently hydrogen atom, a substituted or unsubstituted lower alkyl group, a substituted or unsubstituted cycloalkyl group, a substituted or unsubstituted lower alkoxycarbonyl group, a substituted or unsubstituted aromatic group, a substituted or unsubstituted acyl group, a substituted or unsubstituted aralkyl group, or a substituted or unsubstituted heteroarylalkyl group; or alternatively
R⁹ and R¹⁰ may combine together with the nitrogen atom bound with R⁹ and R¹⁰ to form a saturated cyclic amino group comprising 3 - 8 carbon atoms as ring components, which may further include one -NR¹¹- (R¹¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom as a ring component and may optionally have one or more substituents on the carbon atoms of the saturated cyclic amino group; and
provided that Z is not cyano group when both Y'and R¹ are unsubstituted phenyl group,
or a prodrug thereof, or a pharmaceutically acceptable salt thereof.

4. The compound according to claim 3 wherein
X is nitrogen atom, and R² and R⁴ combine to form an alkylene; or alternatively
X is a group of the formula: C-R¹⁵,
or a prodrug thereof, or a pharmaceutically acceptable salt thereof.

5. The compound according to any one of claims 3 and 4 wherein Y' is a substituted or unsubstituted aromatic group,
or a prodrug thereof, or a pharmaceutically acceptable salt thereof.

6. The compound according to claim 5 wherein R¹ is a substituted or unsubstituted aromatic group,
or a prodrug thereof, or a pharmaceutically acceptable salt thereof.

7. The compound according to claim 6 wherein Y' is a substituted or unsubstituted phenyl group, or a substituted or unsubstituted pyridyl group,
or a prodrug thereof, or a pharmaceutically acceptable salt thereof.

8. The compound according to claim 7 wherein
R¹ is phenyl group, pyridyl group, pyrimidinyl group, benzoxazolyl group, or benzothiazolyl group, which may be optionally substituted with one or more substituents,
or a prodrug thereof, or a pharmaceutically acceptable salt thereof.

9. The compound according to claim 8 wherein
R¹ is a substituted phenyl group or a substituted pyridyl group, wherein the substituents on the phenyl group or pyridyl group are the same or different and are selected from one or more of hydroxyl group or a lower alkoxy group, or a prodrug thereof, or a pharmaceutically acceptable salt thereof.

10. The compound according to any one of claims 3 - 5
wherein
X is the formula: C-R¹⁵, and
R¹⁵ is a group of the formula: -NR¹⁹R²⁰,
or a prodrug thereof, or a pharmaceutically acceptable salt thereof.

11. The compound according to claim 10 wherein in the formula: -NR¹⁹R²⁰
R¹⁹ is hydrogen atom, and
R²⁰ is a substituted or unsubstituted aromatic group, a substituted or unsubstituted aralkyl group, or a substituted or unsubstituted heteroarylalkyl group, or alternatively
R¹⁹ and R²⁰ may combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) as a ring component and may optionally have one or more substituents on the carbon atoms of the saturated cyclic amino group,
or a prodrug thereof, or a pharmaceutically acceptable salt thereof.

12. The compound according to claim 10 wherein
R¹⁵ is a group of the formula: -NR¹⁹R²⁰,
R¹⁹ is hydrogen atom,
R²⁰ is a substituted or unsubstituted aromatic group, a substituted or unsubstituted aralkyl group, or a substituted or unsubstituted heteroarylalkyl group, and
the configuration between R¹⁵ and Y' is trans,
or a prodrug thereof, or a pharmaceutically acceptable salt thereof.

13. The compound according to claim 12 wherein R²⁰ is a substituted or unsubstituted aralkyl group, or a substituted or unsubstituted heteroarylalkyl group,
or a prodrug thereof, or a pharmaceutically acceptable salt thereof.

14. The compound according to claim 12 wherein R²⁰ is a substituted benzyl group wherein the substituent is sulfamoyl group,
or a prodrug thereof, or a pharmaceutically acceptable salt thereof.

15. The compound according to claim 10 wherein
R¹⁵ is a group of the formula: -NR¹⁹R²⁰;
R¹⁹ is hydrogen atom;
R²⁰ is a saturated heterocyclic group comprising 3 - 8 carbon atoms as ring components which includes one -NR²¹⁻(R²¹ is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) or one oxygen atom and may optionally have one or more substituents on the carbon atoms of the saturated heterocyclic group; and
the configuration between R¹⁵ and Y' is trans,
or a prodrug thereof, or a pharmaceutically acceptable salt thereof.

16. The compound according to claim 10 wherein
R¹⁵ is a group of the formula: -NR¹⁹R²⁰ wherein R¹⁹ and R²⁰ combine together with the nitrogen atom bound with R¹⁹ and R²⁰ to form a saturated cyclic amino group comprising 3 - 8 carbon atoms as ring components, which may further include one -NR²²- (R²² is hydrogen atom, a substituted or unsubstituted lower alkyl group, a substituted or unsubstituted aromatic group, a substituted or unsubstituted lower alkoxycarbonyl group, a substituted or unsubstituted aralkyl group, or a substituted or unsubstituted heteroarylalkyl group) as a ring component and may optionally have one or more substituents on the carbon atoms of the saturated cyclic amino group; and
the configuration between R¹⁵ and Y' is cis,
or a prodrug thereof, or a pharmaceutically acceptable salt thereof.

17. The compound according to any one of claims 9 - 16 wherein
every R², R³, R⁴, R⁵, R⁶, and R⁷ is hydrogen atom, or alternatively
one or plural combinations of R² and R³, R⁴ and R⁵, and R⁶ and R⁷ combine to form oxo group; and the others are all hydrogen atom,
or a prodrug thereof, or a pharmaceutically acceptable salt thereof.

18. The compound according to claim 17 wherein
every R², R³, R⁴, and R⁵ is hydrogen atom, and
R⁶ and R⁷ combine to form oxo group, or both R⁶ and R⁷ are hydrogen atom,
or a prodrug thereof, or a pharmaceutically acceptable salt thereof.

19. The compound according claim 18 wherein Z is hydroxyl group, cyano group, a lower alkoxy group or a group of the formula: -NR⁹R¹⁰,
or a prodrug thereof, or a pharmaceutically acceptable salt thereof.

20. The compound according to claim 19 wherein
Y' is a substituted phenyl group wherein the substituents on the phenyl group are the same or different and are selected from one or more of hydroxyl group or a lower alkoxy group,
or a prodrug thereof, or a pharmaceutically acceptable salt thereof.

21. The compound according to any one of claims 3 - 20
wherein Z is cyano group, or a prodrug thereof, or a pharmaceutically acceptable salt thereof.

22. The compound according to any one of claims 3 - 21
wherein
m is 2 or 3,
n is 2, and
every R², R³, R⁴ , R⁵, R⁶, and R⁷ is hydrogen atom,
or a prodrug thereof, or a pharmaceutically acceptable salt thereof.

23. The compound according to any one of claims 3 - 22
wherein p is 0, or a prodrug thereof, or a pharmaceutically acceptable salt thereof.

24. A pharmaceutical composition comprising as an active ingredient the compounds set forth in any one of claims 3 - 23, or a prodrug thereof, or a pharmaceutically acceptable salt thereof.

25. A medicament for enhancing low density lipoprotein receptor expression comprising as an active ingredient the compounds set forth in any one of claims 3 - 23, or a prodrug thereof, or a pharmaceutically acceptable salt thereof.

26. A hypolipidemic drug or antiarteriosclerotic drug comprising as an active ingredient the compound set forth in any one of claims 3 - 23, or a prodrug thereof, or a pharmaceutically acceptable salt thereof.

27. A method for treating hyperlipidemia or arteriosclerosis comprising administering to a pacient in need of the treatment a therapeutically effective dose of the compound set forth in any one of claims 3 - 23, or a prodrug thereof, or a pharmaceutically acceptable salt thereof.

28. Use of the compound set forth in any one of claims 3 - 23, or a prodrug thereof, or a pharmaceutically acceptable salt thereof, for the manufacture of a hypolipidemic drug or antiarteriosclerotic drug.
